# EUROPEAN PATENT SPECIFICATION

(11) **EP 1 953 542 B1**
(45) Date of publication and mention of the grant of the patent: **10.04.2013**
(21) Application number: 06822538.2
(22) Date of filing: 27.10.2006
(51) Int. Cl.: G01N 33/68

(54) **METHOD OF QUANTIFYING MEMBRANE PROTEIN BY USING MASS SPECTROMETER**
VERFAHREN ZUR QUANTIFIZIERUNG EINES MEMBRANPROTEINS UNTER VERWENDUNG EINES MASSENSPEKTROMETERS
PROCEDE DE QUANTIFICATION DE PROTEINE MEMBRANAIRE AU MOYEN D'UN SPECTROMETRE DE MASSE

(30) Priority: 08.11.2005 JP 2005324159
(43) Date of publication of application: 06.08.2008
(73) Proprietor: TOHOKU UNIVERSITY, Aoba-ku Sendai-shi, Miyagi 980-8577 (JP)
(72) Inventor: KAMIIE, Junichi, Sendai-shi, Miyagi 9808577 (JP); OTSUKI, Sumio, Sendai-shi, Miyagi 9808577 (JP); TERASAKI, Tetsuya, Sendai-shi, Miyagi 9808577 (JP)
(74) Representative: Harding, Charles Thomas
(86) International application number: PCT/JP2006/321577
(87) International publication number: WO 2007/055116

(56) References cited:
- WO-A-03/046148
- WO-A1-2004/002996
- JP-A- 2002 515 820
- JP-A- 2002 524 755
- JP-A- 2005 185 281
- BARNIDGE D R ET AL: "Absolute quantification of the G protein-coupled receptor rhodopsin by LC/MS/MS using proteolysis product peptides and synthetic peptide standards" ANALYTICAL CHEMISTRY, AMERICAN CHEMICAL SOCIETY. COLUMBUS, US, vol. 75, no. 3, 1 February 2003 (2003-02-01), pages 445-451, XP002360987 ISSN: 0003-2700
- JUNICHI KAMIIE ET AL: "Quantitative Atlas of Membrane Transporter Proteins: Development and Application of a Highly Sensitive Simultaneous LC/MS/MS Method Combined with Novel In-silico Peptide Selection Criteria" PHARMACEUTICAL RESEARCH, KLUWER ACADEMIC PUBLISHERS-PLENUM PUBLISHERS, NE, vol. 25, no. 6, 25 January 2008 (2008-01-25), pages 1469-1483, XP019613101 ISSN: 1573-904X
- BARNIDGE D.R. ET AL: 'Absolute quantification of the G protein-coupled receptor Rhodopsin by LC/MS/MS using proteolysis product peptides and synthetic peptide standards' ANALYTICAL CHEMISTRY vol. 75, no. 3, 01 February 2003, pages 445 - 451, XP003012639
- ROMIJN E.P. ET AL: 'Recent liquid chromatographic-(tandem) mass spectrometric applications in proteomics' JOURNAL OF CHROMATOGRAPHY A vol. 1000, no. 1/2, 06 June 2003, pages 589 - 608, XP003012640
- WHITELEGGE J: "Tandem mass spectrometry of integral membrane proteins for top-down proteomics", TRAC, TRENDS IN ANALYTICAL CHEMISTRY, ELSEVIER, AMSTERDAM, NL, vol. 24, no. 7, 1 July 2005 (2005-07-01), pages 576-582, XP004988690, ISSN: 0165-9936, DOI: 10.1016/J.TRAC.2005.04.010
- VENTER HENRIETTA ET AL: "Molecular dissection of membrane-transport proteins: Mass spectrometry and sequence determination of the galactose-H+ symport protein, GalP, of Escherichia coli and quantitative assay of the incorporation of (ring-2-13C)histidine and 15NH3", BIOCHEMICAL JOURNAL, THE BIOCHEMICAL SOCIETY, LONDON, GB, vol. 363, no. 2, 15 April 2002 (2002-04-15), pages 243-252, XP002288523, ISSN: 0264-6021, DOI: 10.1042/0264-6021:3630243
- HANS ZISCHKA ET AL: 'Improved mass spectrometric identification of gel-separated hydrophobic membrane proteins after sodium dodecyl sulfate removal by ion-pair extraction' PROTEOMICS vol. 4, no. 12, 01 December 2004, pages 3776 - 3782, XP055009576 ISSN: 1615-9853
- S. CLAVEROL: 'Characterization of protein variants and post-translational modifications: ESI-MSn analyses of intact proteins eluted from polyacrylamide gels' MOLECULAR & CELLULAR PROTEOMICS 25 June 2003, XP055009578 ISSN: 1535-9476

### SEQUENCE LISTING

<110> TOHOKU UNIVERSITY
<120> Method of quantifying membrane protein by using mass spectrometer.
<130> P033480EP
<140> 06822538.2
   <141> 2006-10-27
<150> JP2005-324159
   <151> 2005-11-08
<160> 1420
<170> PatentIn version 3.1
<210> 1
   <211> 10
   <212> PRT
   <213> Artificial Sequence
<220>
   <223> Fragment of ATP binding cassette,sub-family member G2
<400> 1
   Glu Asn Leu Gln Phe Ser Ala Ala Leu Arg
   1               5                   10
<210> 2
   <211> 12
   <212> PRT
   <213> Artificial Sequence
<220>
   <223> Fragment of ATP binding cassette, sub-family member G2
<400> 2
   Leu Ala Glu Ile Tyr Val Asn Ser Ser Phe Tyr Lys
   1               5                   10
<210> 3
   <211> 12
   <212> PRT
   <213> Artificial Sequence
<220>
   <223> Fragment of ATP binding cassette, sub-family member G2
<400> 3
   Leu Phe Asp Ser Leu Thr Leu Leu Ala Ser Gly Arg
   1               5                   10
<210> 4
   <211> 10
   <212> PRT
   <213> Artificial Sequence
<220>
   <223> Fragment of ATP binding cassette, sub-family member G2
<400> 4
   Ser Ser Leu Leu Asp Val Leu Ala Ala Arg
   1               5                   10
<210> 5
   <211> 9
   <212> PRT
   <213> Artificial Sequence
<220>
   <223> Fragment of ATP binding cassette, sub-family member G2
<400> 5
   Val Ile Gln Glu Leu Gly Leu Asp Lys
   1 5
<210> 6
   <211> 14
   <212> PRT
   <213> Artificial Sequence
<220>
   <223> Fragment of P-glycoprotein
<400> 6
   Ser Glu Ile Asp Ala Leu Glu Met Ser Ser Asn Asp Ser Arg
   1 5 10
<210> 7
   <211> 14
   <212> PRT
   <213> Artificial Sequence
<220>
   <223> Fragment of P-glycoprotein
<400> 7
   Glu Ala Leu Asp Glu Ser Ile Pro Pro Val Ser Phe Trp Arg
   1 5 10
<210> 8
   <211> 10
   <212> PRT
   <213> Artificial Sequence
<220>
   <223> Fragment of P-glycoprotein
<400> 8
   Ile Ala Thr Glu Ala Ile Glu Asn Phe Arg
   1 5 10
<210> 9
   <211> 16
   <212> PRT
   <213> Artificial Sequence
<220>
   <223> Fragment of P-glycoprotein
<400> 9
   Asn Ala Asp Val Ile Ala Gly Phe Asp Asp Gly Val Ile Val Glu Lys
   1 5 10 15
<210> 10
   <211> 17
   <212> PRT
   <213> Artificial Sequence
<220>
   <223> Fragment of P-glycoprotein
<400> 10
   Leu Tyr Asp Pro Thr Glu Gly Met Val Ser Val Asp Gly Gln Asp Ile
   1               5                   10                  15
   Arg
<210> 11
   <211> 25
   <212> PRT
   <213> Artificial Sequence
<220>
   <223> Fragment of P-glycoprotein
<400> 11
   Ile Leu Leu Leu Asp Glu Ala Thr Ser Ala Leu Asp Thr Glu Ser Glu
   1               5                   10                  15
   Ala Val Val Gln Val Ala Leu Asp Lys
               20                  25
<210> 12
   <211> 10
   <212> PRT
   <213> Artificial Sequence
<220>
   <223> Fragment of P-glycoprotein
<400> 12
   Thr Val Val Ser Leu Thr Gln Glu Gln Lys
   1               5                   10
<210> 13
   <211> 9
   <212> PRT
   <213> Artificial Sequence
<220>
   <223> Fragment of P-glycoprotein
<400> 13
   Ser Thr Val Val Gln Leu Leu Glu Arg
   1 5
<210> 14
   <211> 10
   <212> PRT
   <213> Artificial Sequence
<220>
   <223> Fragment of P-glycoprotein
<400> 14
   Glu Asn Val Thr Met Asp Glu Ile Glu Lys
   1 5 10
<210> 15
   <211> 9
   <212> PRT
   <213> Artificial Sequence
<220>
   <223> Fragment of P-glycoprotein
<400> 15
   Asn Thr Thr Gly Ala Leu Thr Thr Arg
   1 5
<210> 16
   <211> 8
   <212> PRT
   <213> Artificial Sequence
<220>
   <223> Fragment of P-glycoprotein
<400> 16
   Val Val Gln Glu Ala Leu Asp Lys
   1 5
<210> 17
   <211> 8
   <212> PRT
   <213> Artificial Sequence
<220>
   <223> Fragment of P-glycoprotein
<400> 17
   Phe Tyr Asp Pro Leu Ala Gly Lys
   1 5
<210> 18
   <211> 9
   <212> PRT
   <213> Artificial Sequence
<220>
   <223> Fragment of P-glycoprotein
<400> 18
   Ser Thr Thr Val Gln Leu Met Gln Arg
   1 5
<210> 19
   <211> 14
   <212> PRT
   <213> Artificial Sequence
<220>
   <223> Fragment of ATP-binding cassette, sub-family C member4 (ABCC4)
<400> 19
   Ser Gln His Leu Gly Glu Glu Leu Gln Gly Phe Trp Asp Lys
   1 5 10
<210> 20
   <211> 17
   <212> PRT
   <213> Artificial Sequence
<220>
   <223> Fragment of ATP-binding cassette, sub-family C member4 (ABCC4)
<400> 20
   Met Asp Thr Glu Leu Ala Glu Ser Gly Ser Asn Phe Ser Val Gly Gln
   1 5 10 15
   Arg
<210> 21
   <211> 21
   <212> PRT
   <213> Artificial Sequence
<220>
   <223> Fragment of ATP-binding cassette, sub-family C member4 (ABCC4)
<400> 21
   Asp Gly Ala Leu Glu Ser Gln Asp Thr Glu Asn Val Pro Val Thr Leu
   1 5 10 15
   Ser Glu Glu Asn Arg
   20
<210> 22
   <211> 13
   <212> PRT
   <213> Artificial Sequence
<220>
   <223> Fragment of ATP-binding cassette, sub-family C member4 (ABCC4)
<400> 22
   Ile Thr Ile Leu Val Thr His Gln Leu Gln Tyr Leu Lys
   1               5                   10
<210> 23
   <211> 16
   <212> PRT
   <213> Artificial Sequence
<220>
   <223> Fragment of ATP-binding cassette, sub-family C member4 (ABCC4)
<400> 23
   Ile Ala Tyr Val Ser Gln Gln Pro Trp Val Phe Ser Gly Thr Leu Arg
   1               5                   10                  15
<210> 24
   <211> 13
   <212> PRT
   <213> Artificial Sequence
<220>
   <223> Fragment of ATP-binding cassette, sub-family C member4 (ABCC4)
<400> 24
   Ile Gln Thr Phe Leu Leu Leu Asp Glu Ile Ser Gln Arg
   1               5                   10
<210> 25
   <211> 16
   <212> PRT
   <213> Artificial Sequence
<220>
   <223> Fragment of ATP-binding cassette, sub-family C member4 (ABCC4)
<400> 25
   Asp Leu Gln Leu Leu Glu Asp Gly Asp Leu Thr Val Ile Gly Asp Arg
   1               5                   10                  15
<210> 26
   <211> 13
   <212> PRT
   <213> Artificial Sequence
<220>
   <223> Fragment of ATP-binding cassette, sub-family C member4 (ABCC4)
<400> 26
   Met Val His Val Gln Asp Phe Thr Ala Phe Trp Asp Lys
   1               5                   10
<210> 27
   <211> 11
   <212> PRT
   <213> Artificial Sequence
<220>
   <223> Fragment of ATP-binding cassette, sub-family C member4 (ABCC4)
<400> 27
   Val Phe Phe Trp Trp Leu Asn Pro Leu Phe Lys
   1               5                   10
<210> 28
   <211> 17
   <212> PRT
   <213> Artificial Sequence
<220>
   <223> Fragment of ATP-binding cassette, sub-family C member4 (ABCC4)
<400> 28
   Asp Asn Glu Glu Ser Glu Gln Pro Pro Val Pro Gly Thr Pro Thr Leu
   1               5                   10                  15
   Arg
<210> 29
   <211> 8
   <212> PRT
   <213> Artificial Sequence
<220>
   <223> Fragment of ATP-binding cassette, sub-family C member4 (ABCC4)
<400> 29
   Ala Pro Val Leu Phe Phe Asp Arg
   1               5
<210> 30
   <211> 9
   <212> PRT
   <213> Artificial Sequence
<220>
   <223> Fragment of ATP-binding cassette, sub-family C member4 (ABCC4)
<400> 30
   Val Ala Met Glx His Met Ile Tyr Arg
   1 5
<210> 31
   <211> 23
   <212> PRT
   <213> Artificial Sequence
<220>
   <223> Fragment of ATP-binding cassette, sub-family C member4 (ABCC4)
<400> 31
   Ser Ser Leu Leu Ser Ala Val Leu Gly Glu Leu Ala Pro Ser His Gly
   1 5 10 15
   Leu Val Ser Val His Gly Arg
   20
<210> 32
   <211> 18
   <212> PRT
   <213> Artificial Sequence
<220>
   <223> Fragment of ATP-binding cassette, sub-family C member4 (ABCC4)
<400> 32
   Thr Phe Ser Glu Ser Ser Val Trp Ser Gln Gln Ser Ser Arg Pro Ser
   1 5 10 15
   Leu Lys
<210> 33
   <211> 9
   <212> PRT
   <213> Artificial Sequence
<220>
   <223> Fragment of ATP-binding cassette, sub-family C member4 (ABCC4)
<400> 33
   Val Ser Glu Ala Ile Val Ser Ile Arg
   1 5
<210> 34
   <211> 10
   <212> PRT
   <213> Artificial Sequence
<220>
   <223> Fragment of ATP-binding cassette, sub-family C member4 (ABCC4)
<400> 34
   Ser Gly Ile Asp Phe Gly Ser Leu Leu Lys
   1 5 10
<210> 35
   <211> 16
   <212> PRT
   <213> Artificial Sequence
<220>
   <223> Fragment of ATP-binding cassette, sub-family C member4 (ABCC4)
<400> 35
   Asp Leu Gln Leu Leu Glu Asp Gly Asp Leu Thr Val Ile Gly Asp Arg
   1 5 10 15
<210> 36
   <211> 16
   <212> PRT
   <213> Artificial Sequence
<220>
   <223> Fragment of ATP-binding cassette, sub-family C member4 (ABCC4)
<400> 36
   Met Ser Ile Ile Pro Gln Glu Pro Val Leu Phe Thr Gly Thr Met Arg
   1 5 10 15
<210> 37
   <211> 7
   <212> PRT
   <213> Artificial Sequence
<220>
   <223> ABCA1
<400> 37
   Ala Asp Val Ile Leu His Lys
   1 5
<210> 38
   <211> 6
   <212> PRT
   <213> Artificial Sequence
<220>
   <223> ABCA1
<400> 38
   Leu Ala Ala Ala Glu Arg
   1 5
<210> 39
   <211> 11
   <212> PRT
   <213> Artificial Sequence
<220>
   <223> ABCA1
<400> 39
   Ser Leu Asn Trp Tyr Glu Asp Asn Asn Tyr Lys
   1               5                   10
<210> 40
   <211> 11
   <212> PRT
   <213> Artificial Sequence
<220>
   <223> ABCA1
<400> 40
   Ile Leu Tyr Thr Pro Asp Thr Pro Ala Thr Arg
   1               5                   10
<210> 41
   <211> 9
   <212> PRT
   <213> Artificial Sequence
<220>
   <223> ABCA1
<400> 41
   Asp Gly Tyr Trp Asp Pro Gly Pro Arg
   1               5
<210> 42
   <211> 11
   <212> PRT
   <213> Artificial Sequence
<220>
   <223> ABCA1
<400> 42
   Ser Tyr Trp Phe Gly Glu Glu Ser Asp Glu Lys
   1               5                   10
<210> 43
   <211> 10
   <212> PRT
   <213> Artificial Sequence
<220>
   <223> ABCAL
<400> 43
   Leu Gly Val Ser Ile Gln Asn Leu Val Lys
   1 5 10
<210> 44
   <211> 8
   <212> PRT
   <213> Artificial Sequence
<220>
   <223> ABCA1
<400> 44
   Ile Ala Ile Ile Ser His Gly Lys
   1 5
<210> 45
   <211> 14
   <212> PRT
   <213> Artificial Sequence
<220>
   <223> ABCA1
<400> 45
   Glu Gly Ala Phe Val Glu Leu Phe His Glu Ile Asp Asp Arg
   1 5 10
<210> 46
   <211> 6
   <212> PRT
   <213> Artificial Sequence
<220>
   <223> ABCA1
<400> 46
   Asp Ser Ser Ala Asp Arg
   1 5
<210> 47
   <211> 7
   <212> PRT
   <213> Artificial Sequence
<220>
   <223> ABCA1
<400> 47
   Phe Leu Asn Ser Leu Gly Arg
   1 5
<210> 48
   <211> 12
   <212> PRT
   <213> Artificial Sequence
<220>
   <223> ABCA1
<400> 48
   Phe Val Ser Pro Leu Ser Trp Asp Leu Val Gly Arg
   1               5                   10
<210> 49
   <211> 14
   <212> PRT
   <213> Artificial Sequence
<220>
   <223> ABCA1
<400> 49
   Ile Leu Asp Gly Gly Gly Gln Asn Asp Ile Leu Glu Ile Lys
   1               5                   10
<210> 50
   <211> 7
   <212> PRT
   <213> Artificial Sequence
<220>
   <223> ABCA1
<400> 50
   Gly Asp Ala Phe Leu Asn Lys
   1               5
<210> 51
   <211> 10
   <212> PRT
   <213> Artificial Sequence
<220>
   <223> ABCA1
<400> 51
   Glu His Val Glu Phe Phe Ala Leu Leu Arg
   1               5                   10
<210> 52
   <211> 10
   <212> PRT
   <213> Artificial Sequence
<220>
   <223> ABCA1
<400> 52
   Phe Gly Asp Gly Tyr Thr Ile Val Val Arg
   1 5 10
<210> 53
   <211> 9
   <212> PRT
   <213> Artificial Sequence
<220>
   <223> ABCA1
<400> 53
   Asp Gln Ser Asp Asp Asp His Leu Lys
   1 5
<210> 54
   <211> 13
   <212> PRT
   <213> Artificial Sequence
<220>
   <223> ABCA2
<400> 54
   Ser Thr Val Ser Ser Phe Ser Leu Asp Ser Val Ala Arg
   1 5 10
<210> 55
   <211> 7
   <212> PRT
   <213> Artificial Sequence
<220>
   <223> ABCA2
<400> 55
   Asn Pro Gln Glu Leu Trp Arg
   1 5
<210> 56
   <211> 8
   <212> PRT
   <213> Artificial Sequence
<220>
   <223> ABCA2
<400> 56
   Leu Gly Gly Asn Pro Leu Phe Arg
   1 5
<210> 57
   <211> 9
   <212> PRT
   <213> Artificial Sequence
<220>
   <223> ABCA2
<400> 57
   Ile Leu Thr Val Pro Glu Ser Gln Lys
   1 5
<210> 58
   <211> 7
   <212> PRT
   <213> Artificial Sequence
<220>
   <223> ABCA2
<400> 58
   Gly Ala Leu Gln Gly Tyr Arg
   1 5
<210> 59
   <211> 7
   <212> PRT
   <213> Artificial Sequence
<220>
   <223> ABCA2
<400> 59
   Asn Gln Leu Asp Val Ala Lys
   1 5
<210> 60
   <211> 12
   <212> PRT
   <213> Artificial Sequence
<220>
   <223> ABCA2
<400> 60
   Ile Leu Tyr Ala Pro Ala Gly Ser Glu Val Asp Arg
   1 5 10
<210> 61
   <211> 7
   <212> PRT
   <213> Artificial Sequence
<220>
   <223> ABCA2
<400> 61
   Ser Phe Leu Glu Gln Gly Arg
   1 5
<210> 62
   <211> 6
   <212> PRT
   <213> Artificial Sequence
<220>
   <223> ABCA2
<400> 62
   Leu Gln Gln His Leu Arg
   1 5
<210> 63
   <211> 10
   <212> PRT
   <213> Artificial Sequence
<220>
   <223> ABCA2
<400> 63
   Trp Leu Gln Gln Tyr Val Ala Glu Leu Arg
   1 5 10
<210> 64
   <211> 7
   <212> PRT
   <213> Artificial Sequence
<220>
   <223> ABCA2
<400> 64
   Val Ser Val Asp Ile Phe Lys
   1 5
<210> 65
   <211> 8
   <212> PRT
   <213> Artificial Sequence
<220>
   <223> ABCA2
<400> 65
   Ser Tyr Trp Leu Gly Ser Gly Arg
   1 5
<210> 66
   <211> 12
   <212> PRT
   <213> Artificial Sequence
<220>
   <223> ABCA2
<400> 66
   Thr Glu Ala Trp Glu Trp Ser Trp Pro Trp Ala Arg
   1 5 10
<210> 67
   <211> 12
   <212> PRT
   <213> Artificial Sequence
<220>
   <223> ABCA2
<400> 67
   Leu Thr Val Glu Glu His Leu Trp Phe Tyr Ser Arg
   1 5 10
<210> 68
   <211> 7
   <212> PRT
   <213> Artificial Sequence
<220>
   <223> ABCA2
<400> 68
   Leu Phe Gln His Leu Glu Arg
   1 5
<210> 69
   <211> 11
   <212> PRT
   <213> Artificial Sequence
<220>
   <223> ABCA2
<400> 69
   Tyr Gly Ala Ile Thr Phe Gly Asn Val Leu Lys
   1 5 10
<210> 70
   <211> 9
   <212> PRT
   <213> Artificial Sequence
<220>
   <223> ABCA2
<400> 70
   Ser Ile Pro Ala Ser Phe Gly Thr Arg
   1 5
<210> 71
   <211> 7
   <212> PRT
   <213> Artificial Sequence
<220>
   <223> ABCA2
<400> 71
   Val Val Asn Ser Tyr Leu Lys
   1 5
<210> 72
   <211> 8
   <212> PRT
   <213> Artificial Sequence
<220>
   <223> ABCA2
<400> 72
   Glu His Leu Gln Leu Tyr Thr Arg
   1 5
<210> 73
   <211> 6
   <212> PRT
   <213> Artificial Sequence
<220>
   <223> ABCA2
<400> 73
   Val Gln Tyr Gln Leu Lys
   1 5
<210> 74
   <211> 12
   <212> PRT
   <213> Artificial Sequence
<220>
   <223> ABCA2
<400> 74
   Ser Glu His Ile Ser Leu Ala Gln Val Phe Ser Lys
   1 5 10
<210> 75
   <211> 7
   <212> PRT
   <213> Artificial Sequence
<220>
   <223> ABCA2
<400> 75
   Ser Ala Pro Thr Glu Leu Arg
   1 5
<210> 76
   <211> 8
   <212> PRT
   <213> Artificial Sequence
<220>
   <223> ABCA3
<400> 76
   Gln Leu Ala Leu Leu Leu Trp Lys
   1 5
<210> 77
   <211> 6
   <212> PRT
   <213> Artificial Sequence
<220>
   <223> ABCA3
<400> 77
   Gly Phe Pro Ser Glu Lys
   1 5
<210> 78
   <211> 7
   <212> PRT
   <213> Artificial Sequence
<220>
   <223> ABCA3
<400> 78
   Asp Phe Glu Asp Tyr Ile Arg
   1 5
<210> 79
   <211> 8
   <212> PRT
   <213> Artificial Sequence
<220>
   <223> ABCA3
<400> 79
   Glu Pro Leu Pro Leu Ala Val Lys
   1 5
<210> 80
   <211> 12
   <212> PRT
   <213> Artificial Sequence
<220>
   <223> ABCA3
<400> 80
   Glu Gly Phe Leu Ala Val Gln His Ala Val Asp Arg
   1 5 10
<210> 81
   <211> 8
   <212> PRT
   <213> Artificial Sequence
<220>
   <223> ABCA3
<400> 81
   Leu Thr Leu Gly Glu Tyr Gly Arg
   1 5
<210> 82
   <211> 11
   <212> PRT
   <213> Artificial Sequence
<220>
   <223> ABCA3
<400> 82
   Asp Ala Leu Gln Ala Glu Gly Gln Glu Pro Arg
   1 5 10
<210> 83
   <211> 13
   <212> PRT
   <213> Artificial Sequence
<220>
   <223> ABCA3
<400> 83
   Glu Val Leu Gly Asp Leu Glu Glu Phe Leu Ile Phe Arg
   1 5 10
<210> 84
   <211> 11
   <212> PRT
   <213> Artificial Sequence
<220>
   <223> ABCA3
<400> 84
   Ala Ser Val Glu Gly Gly Gly Phe Asn Glu Arg
   1 5 10
<210> 85
   <211> 7
   <212> PRT
   <213> Artificial Sequence
<220>
   <223> ABCA3
<400> 85
   Ser Ala Leu Gln Ala Ala Lys
   1 5
<210> 86
   <211> 6
   <212> PRT
   <213> Artificial Sequence
<220>
   <223> ABCA3
<400> 86
   Leu Glu Glu Leu Ser Lys
   1 5
<210> 87
   <211> 8
   <212> PRT
   <213> Artificial Sequence
<220>
   <223> ABCA3
<400> 87
   Val Pro Leu Leu Ala Val Asp Arg
   1 5
<210> 88
   <211> 10
   <212> PRT
   <213> Artificial Sequence
<220>
   <223> ABCA3
<400> 88
   Gly Leu Leu Leu Glu Pro His Ala Asn Lys
   1 5 10
<210> 89
   <211> 8
   <212> PRT
   <213> Artificial Sequence

<220>
   <223> ABCA3
<400> 89
   Phe Gly Ser Gly Tyr Ser Leu Arg
   1 5
<210> 90
   <211> 14
   <212> PRT
   <213> Artificial Sequence
<220>
   <223> ABCA3
<400> 90
   Val Gln Ser Glu Gly Gln Gln Glu Ala Leu Glu Glu Phe Lys
   1 5 10
<210> 91
   <211> 6
   <212> PRT
   <213> Artificial Sequence
<220>
   <223> ABC3
<400> 91
   Asp Leu Ser Trp Ala Lys
   1 5
<210> 92
   <211> 7
   <212> PRT
   <213> Artificial Sequence
<220>
   <223> ABCA3
<400> 92
   Val Phe Gly Ile Leu Glu Lys
   1 5
<210> 93
   <211> 11
   <212> PRT
   <213> Artificial Sequence
<220>
   <223> ABCA4
<400> 93
   Asp Glu Glu Thr Leu Thr Leu Phe Leu Ile Lys
   1 5 10
<210> 94
   <211> 9
   <212> PRT
   <213> Artificial Sequence
<220>
   <223> ABCA4
<400> 94
   Val Leu Pro Thr Leu Leu Asp Ser Arg
   1 5
<210> 95
   <211> 7
   <212> PRT
   <213> Artificial Sequence
<220>
   <223> ABCA4
<400> 95
   Ser Gln Gly Ile Asn Leu Arg
   1 5
<210> 96
   <211> 9
   <212> PRT
   <213> Artificial Sequence
<220>
   <223> ABCA4
<400> 96
   Asp Thr Leu Gly Asn Pro Thr Val Lys
   1 5
<210> 97
   <211> 6
   <212> PRT
   <213> Artificial Sequence
<220>
   <223> ABCA4
<400> 97
   Ser Ile Val Leu Glu Lys
   1 5
<210> 98
   <211> 10
   <212> PRT
   <213> Artificial Sequence
<220>
   <223> ABCA4
<400> 98
   Asp Ile Glu Thr Ser Leu Asp Ala Val Arg
   1 5 10
<210> 99
   <211> 8
   <212> PRT
   <213> Artificial Sequence
<220>
   <223> ABCA4
<400> 99
   Ile Ala Ile Ile Ala Gln Gly Arg
   1 5
<210> 100
   <211> 7
   <212> PRT
   <213> Artificial Sequence
<220>
   <223> ABCA4
<400> 100
   Ala Tyr Ala Ser Leu Phe Arg
   1 5
<210> 101
   <211> 7
   <212> PRT
   <213> Artificial Sequence
<220>
   <223> ABCA4
<400> 101
   Phe Trp Val Asn Glu Gln Arg
   1 5
<210> 102
   <211> 7
   <212> PRT
   <213> Artificial Sequence
<220>
   <223> ABCA4
<400> 102
   Glu Ile Pro Asp Phe Leu Lys
   1 5
<210> 103
   <211> 9
   <212> PRT
   <213> Artificial Sequence
<220>
   <223> ABCA4
<400> 103
   His Leu Glu Thr Glu Asp Asn Ile Lys
   1 5
<210> 104
   <211> 6
   <212> PRT
   <213> Artificial Sequence
<220>
   <223> ABCA4
<400> 104
   Val Trp Phe Asn Asn Lys
   1 5
<210> 105
   <211> 6
   <212> PRT
   <213> Artificial Sequence
<220>
   <223> ABCA4
<400> 105
   Ala Ala Gly Ala Ser Arg
   1 5
<210> 106
   <211> 7
   <212> PRT
   <213> Artificial Sequence
<220>
   <223> ABCA5
<400> 106
   Thr Asn Val Ser Leu Trp Lys
   1 5
<210> 107
   <211> 6
   <212> PRT
   <213> Artificial Sequence
<220>
   <223> ABCA5
<400> 107
   Glu Leu Glu Ser Thr Lys
   1 5
<210> 108
   <211> 13
   <212> PRT
   <213> Artificial Sequence
<220>
   <223> ABCA5
<400> 108
   Gly Ile Pro Ala Asn Asn Ile Ile Gln Glu Val Gln Lys
   1 5 10
<210> 109
   <211> 8
   <212> PRT
   <213> Artificial Sequence
<220>
   <223> ABCA5
<400> 109
   His Ile Val Trp Asn Leu Leu Lys
   1 5
<210> 110
   <211> 7
   <212> PRT
   <213> Artificial Sequence
<220>
   <223> ABCA5
<400> 110
   Asn Ala Val Val Pro Ile Lys
   1 5
<210> 111
   <211> 8
   <212> PRT
   <213> Artificial Sequence
<220>
   <223> ABCA5
<400> 111
   Ile Thr His Ala Leu Asp Leu Lys
   1 5
<210> 112
   <211> 7
   <212> PRT
   <213> Artificial Sequence
<220>
   <223> ABCA5
<400> 112
   Gly Tyr Phe Leu Glu Ile Lys
   1 5
<210> 113
   <211> 10
   <212> PRT
   <213> Artificial Sequence
<220>
   <223> ABCA5
<400> 113
   Asp Trp Ile Glu Asn Leu Glu Val Asp Arg
   1 5 10
<210> 114
   <211> 11
   <212> PRT
   <213> Artificial Sequence
<220>
   <223> ABCA5
<400> 114
   Glu Ile Gln Tyr Ile Phe Pro Asn Ala Ser Arg
   1 5 10
<210> 115
   <211> 11
   <212> PRT
   <213> Artificial Sequence
<220>
   <223> ABCA5
<400> 115
   Gln Glu Ser Phe Ser Ser Ile Leu Ala Tyr Lys
   1 5 10
<210> 116
   <211> 8
   <212> PRT
   <213> Artificial Sequence
<220>
   <223> ABCA6
<400> 116
   Thr Ala Leu Ala Pro Leu Leu Lys
   1 5
<210> 117
   <211> 10
   <212> PRT
   <213> Artificial Sequence
<220>
   <223> ABCA6
<400> 117
   Gly Thr Ser Val Ile Gly Ala Pro Asn Lys
   1 5 10
<210> 118
   <211> 7
   <212> PRT
   <213> Artificial Sequence
<220>
   <223> ABCA6
<400> 118
   Thr Leu Pro Phe Ile Thr Lys
   1 5
<210> 119
   <211> 8
   <212> PRT
   <213> Artificial Sequence
<220>
   <223> ABCA6
<400> 119
   Glu Asn Leu Ser Leu Phe Ala Lys
   1               5
<210> 120
   <211> 8
   <212> PRT
   <213> Artificial Sequence
<220>
   <223> ABCA6
<400> 120
   Asp Gln Val Trp Ser Leu Leu Arg
   1               5
<210> 121
   <211> 9
   <212> PRT
   <213> Artificial Sequence
<220>
   <223> ABCA6
<400> 121
   Leu Val Tyr Thr Leu Pro Leu Glu Arg
   1               5
<210> 122
   <211> 13
   <212> PRT
   <213> Artificial Sequence
<220>
   <223> ABCA6
<400> 122
   Thr Asn Thr Phe Pro Asp Leu Phe Ser Asp Leu Asp Lys
   1               5                   10
<210> 123
   <211> 14
   <212> PRT
   <213> Artificial Sequence
<220>
   <223> ABCA6
<400> 123
   His Gln Asn Ile Leu Leu Glu Val Asp Asp Phe Glu Asn Arg
   1 5 10
<210> 124
   <211> 6
   <212> PRT
   <213> Artificial Sequence
<220>
   <223> ABCA6
<400> 124
   Thr Phe Leu Glu Val Arg
   1 5
<210> 125
   <211> 6
   <212> PRT
   <213> Artificial Sequence
<220>
   <223> ABCA6
<400> 125
   Leu Val Ser Ala Phe Lys
   1 5
<210> 126
   <211> 11
   <212> PRT
   <213> Artificial Sequence
<220>
   <223> ABCA6
<400> 126
   Leu His Glu Gln Leu Asn Val Pro Val Gln Lys
   1 5 10
<210> 127
   <211> 7
   <212> PRT
   <213> Artificial Sequence
<220>
   <223> ABCA6
<400> 127
   Asp Tyr Ile Leu Glu Leu Lys
   1 5
<210> 128
   <211> 14
   <212> PRT
   <213> Artificial Sequence
<220>
   <223> ABCA6
<400> 128
   Glu Thr Ser Gln Val Thr Leu Val His Thr Glu Ile Leu Lys
   1 5 10
<210> 129
   <211> 10
   <212> PRT
   <213> Artificial Sequence
<220>
   <223> ABCA6
<400> 129
   Leu Phe Pro Gln Ala Ala Gly Gln Glu Arg
   1 5 10
<210> 130
   <211> 8
   <212> PRT
   <213> Artificial Sequence
<220>
   <223> ABCA6
<400> 130
   Tyr Ser Ser Leu Leu Thr Tyr Lys
   1 5
<210> 131
   <211> 15
   <212> PRT
   <213> Artificial Sequence
<220>
   <223> ABCA6
<400> 131
   Leu Pro Val Ala Asp Val Tyr Pro Leu Ser Gln Thr Phe His Lys
   1 5 10 15
<210> 132
   <211> 7
   <212> PRT
   <213> Artificial Sequence
<220>
   <223> ABCA6
<400> 132
   Val Phe Leu Glu Leu Ser Lys
   1 5
<210> 133
   <211> 8
   <212> PRT
   <213> Artificial Sequence
<220>
   <223> ABCA6
<400> 133
   Leu Leu Pro His Ser Asp Glu Pro
   1 5
<210> 134
   <211> 7
   <212> PRT
   <213> Artificial Sequence
<220>
   <223> ABCA7
<400> 134
   Gln Phe Gln Ser Pro Leu Arg
   1 5
<210> 135
   <211> 7
   <212> PRT
   <213> Artificial Sequence
<220>
   <223> ABCA8
<400> 135
   Leu Phe Pro Gln Ala Ala Arg
   1 5
<210> 136
   <211> 7
   <212> PRT
   <213> Artificial Sequence
<220>
   <223> ABCA9
<400> 136
   Gln His Ile Ser Asp Ala Lys
   1 5
<210> 137
   <211> 12
   <212> PRT
   <213> Artificial Sequence
<220>
   <223> ABCA10
<400> 137
   Asp Gly His Gly Asp Ser Pro Leu Phe Phe Leu Lys
   1 5 10
<210> 138
   <211> 10
   <212> PRT
   <213> Artificial Sequence
<220>
   <223> ABCA12
<400> 138
   Leu Leu Ala Ile Pro Ile Pro Asp Asn Arg
   1 5 10
<210> 139
   <211> 12
   <212> PRT
   <213> Artificial Sequence
<220>
   <223> ABCA13
<400> 139
   Phe His Pro Gln Asn Leu Pro Ala Asp Gly Phe Lys
   1 5 10
<210> 140
   <211> 14
   <212> PRT
   <213> Artificial Sequence
<220>
   <223> ABCB1
<400> 140
   Glu Ala Leu Asp Glu Ser Ile Pro Pro Val Ser Phe Trp Arg
   1 5 10
<210> 141
   <211> 10
   <212> PRT
   <213> Artificial Sequence
<220>
   <223> ABCB4
<400> 141
   Ile Ala Thr Glu Ala Ile Glu Asn Ile Arg
   1               5                   10
<210> 142
   <211> 9
   <212> PRT
   <213> Artificial Sequence
<220>
   <223> ABCB5
<400> 142
   Ser Ala Asp Leu Ile Val Thr Leu Lys
   1               5
<210> 143
   <211> 9
   <212> PRT
   <213> Artificial Sequence
<220>
   <223> ABCB11
<400> 143
   Ser Thr Ala Leu Gln Leu Ile Gln Arg
   1               5
<210> 144
   <211> 9
   <212> PRT
   <213> Artificial Sequence
<220>
   <223> ABCC1
<400> 144
   Thr Pro Ser Gly Asn Leu Val Asn Arg
   1               5
<210> 145
   <211> 6
   <212> PRT
   <213> Artificial Sequence
<220>
   <223> ABCC1
<400> 145
   Val Val Tyr Ser Ser Lys
   1 5
<210> 146
   <211> 6
   <212> PRT
   <213> Artificial Sequence
<220>
   <223> ABCC1
<400> 146
   Asp Pro Ala Gln Pro Lys
   1 5
<210> 147
   <211> 6
   <212> PRT
   <213> Artificial Sequence
<220>
   <223> ABCC1
<400> 147
   Phe Val Asn Asp Thr Lys
   1 5
<210> 148
   <211> 11
   <212> PRT
   <213> Artificial Sequence
<220>
   <223> ABCC1
<400> 148
   Asp Gly Gly Gly Thr Asn Ser Ile Thr Val Arg
   1 5 10
<210> 149
   <211> 9
   <212> PRT
   <213> Artificial Sequence
<220>
   <223> ABCC1
<400> 149
   Gly Val Asn Leu Ser Gly Gly Gln Lys
   1 5
<210> 150
   <211> 14
   <212> PRT
   <213> Artificial Sequence
<220>
   <223> ABCC1
<400> 150
   Gln Leu Ser Ser Ser Ser Ser Tyr Ser Gly Asp Ile Ser Arg
   1               5                   10
<210> 151
   <211> 7
   <212> PRT
   <213> Artificial Sequence
<220>
   <223> ABCC1
<400> 151
   Phe Tyr Val Ala Ser Ser Arg
   1               5
<210> 152
   <211> 8
   <212> PRT
   <213> Artificial Sequence
<220>
   <223> ABCC1
<400> 152
   Phe Ile His Gln Ser Asp Leu Lys
   1               5
<210> 153
   <211> 10
   <212> PRT
   <213> Artificial Sequence
<220>
   <223> ABCC1
<400> 153
   Ala Tyr Tyr Pro Ser Ile Val Ala Asn Arg
   1               5                   10
<210> 154
   <211> 9
   <212> PRT
   <213> Artificial Sequence
<220>
   <223> ABCC1
<400> 154
   Ser Ser Leu Thr Leu Gly Leu Phe Arg
   1 5
<210> 155
   <211> 7
   <212> PRT
   <213> Artificial Sequence
<220>
   <223> ABCC1
<400> 155
   Ile Gly Leu His Asp Leu Arg
   1 5
<210> 156
   <211> 16
   <212> PRT
   <213> Artificial Sequence
<220>
   <223> ABCC1
<400> 156
   Ile Thr Ile Ile Pro Gln Asp Pro Val Leu Phe Ser Gly Ser Leu Arg
   1 5 10 15
<210> 157
   <211> 9
   <212> PRT
   <213> Artificial Sequence
<220>
   <223> ABCC1
<400> 157
   Asp Phe Val Ser Ala Leu Pro Asp Lys
   1 5
<210> 158
   <211> 6
   <212> PRT
   <213> Artificial Sequence
<220>
   <223> ABCC1
<400> 158
   Val Ile Val Leu Asp Lys
   1 5
<210> 159
   <211> 8
   <212> PRT
   <213> Artificial Sequence
<220>
   <223> ABCC2
<400> 159
   Gln Leu Leu Asn Asn Ile Leu Arg
   1 5
<210> 160
   <211> 9
   <212> PRT
   <213> Artificial Sequence
<220>
   <223> ABCC2
<400> 160
   Ser Ser Gln Gln Asn Ser Gly Ala Arg
   1 5
<210> 161
   <211> 6
   <212> PRT
   <213> Artificial Sequence
<220>
   <223> ABCC2
<400> 161
   Leu Pro Gly Leu Asn Lys
   1 5
<210> 162
   <211> 9
   <212> PRT
   <213> Artificial Sequence
<220>
   <223> ABCC2
<400> 162
   Tyr Phe Ala Trp Glu Pro Ser Phe Arg
   1 5
<210> 163
   <211> 13
   <212> PRT
   <213> Artificial Sequence
<220>
   <223> ABCC2
<400> 163
   Tyr Leu Gly Gly Asp Asp Leu Asp Thr Ser Ala Ile Arg
   1 5 10
<210> 164
   <211> 9
   <212> PRT
   <213> Artificial Sequence
<220>
   <223> ABCC2
<400> 164
   Gly Ile Asn Leu Ser Gly Gly Gln Lys
   1 5
<210> 165
   <211> 9
   <212> PRT
   <213> Artificial Sequence
<220>
   <223> ABCC2
<400> 165
   Val Leu Gly Pro Asn Gly Leu Leu Lys
   1 5
<210> 166
   <211> 6
   <212> PRT
   <213> Artificial Sequence
<220>
   <223> ABCC2
<400> 166
   Asn Val Asn Ser Leu Lys
   1 5
<210> 167
   <211> 7
   <212> PRT
   <213> Artificial Sequence
<220>
   <223> ABCC2
<400> 167
   Glu Asp Glu Glu Leu Val Lys
   1 5
<210> 168
   <211> 6
   <212> PRT
   <213> Artificial Sequence
<220>
   <223> ABCC2
<400> 168
   Ile Asp Thr Asn Gln Lys
   1 5
<210> 169
   <211> 9
   <212> PRT
   <213> Artificial Sequence
<220>
   <223> ABCC2
<400> 169
   Ile Gln Phe Asn Asn Tyr Gln Val Arg
   1 5
<210> 170
   <211> 8
   <212> PRT
   <213> Artificial Sequence
<220>
   <223> ABCC2
<400> 170
   Pro Glu Leu Asp Leu Val Leu Arg
   1 5
<210> 171
   <211> 6
   <212> PRT
   <213> Artificial Sequence
<220>
   <223> ABCC2
<400> 171
   Ile Gly Val Val Gly Arg
   1 5
<210> 172
   <211> 11
   <212> PRT
   <213> Artificial Sequence
<220>
   <223> ABCC2
<400> 172
   Glu Ala Gly Ile Glu Asn Val Asn Ser Thr Lys
   1 5 10
<210> 173
   <211> 10
   <212> PRT
   <213> Artificial Sequence
<220>
   <223> ABCC3
<400> 173
   Ala Glu Gly Glu Ile Ser Asp Pro Phe Arg
   1 5 10
<210> 174
   <211> 10
   <212> PRT
   <213> Artificial Sequence
<220>
   <223> ABCC3
<400> 174
   Gly Tyr Ile Ile Leu Ser His Leu Ser Lys
   1 5 10
<210> 175
   <211> 8
   <212> PRT
   <213> Artificial Sequence
<220>
   <223> ABCC3
<400> 175
   Leu Phe Phe Trp Trp Phe Thr Lys
   1 5
<210> 176
   <211> 6
   <212> PRT
   <213> Artificial Sequence
<220>
   <223> ABCC3
<400> 176
   Gln Val Glu Gly Ile Arg
   1 5
<210> 177
   <211> 8
   <212> PRT
   <213> Artificial Sequence
<220>
   <223> ABCC3
<400> 177
   Gln Gly Glu Leu Gln Leu Leu Arg
   1 5
<210> 178
   <211> 7
   <212> PRT
   <213> Artificial Sequence
<220>
   <223> ABCC3
<400> 178
   Val Gln Val Thr Glu Ala Lys
   1 5
<210> 179
   <211> 10
   <212> PRT
   <213> Artificial Sequence
<220>
   <223> ABCC3
<400> 179
   Ala Asp Gly Ala Leu Thr Gln Glu Glu Lys
   1 5 10
<210> 180
   <211> 10
   <212> PRT
   <213> Artificial Sequence
<220>
   <223> ABCC3
<400> 180
   Val Leu His Gln Ala Leu Leu His Asn Lys
   1 5 10

<210> 181
   <211> 13
   <212> PRT
   <213> Artificial Sequence
<220>
   <223> ABCC3
<400> 181
   Ser Pro Gln Ser Phe Phe Asp Thr Thr Pro Ser Gly Arg
   1 5 10
<210> 182
   <211> 9
   <212> PRT
   <213> Artificial Sequence
<220>
   <223> ABCC3
<400> 182
   Asp Phe Glu Ile Ile Ser Asp Thr Lys
   1 5
<210> 183
   <211> 6
   <212> PRT
   <213> Artificial Sequence
<220>
   <223> ABCC3
<400> 183
   Val Asp Ala Asn Gln Arg
   1 5
<210> 184
   <211> 8
   <212> PRT
   <213> Artificial Sequence
<220>
   <223> ABCC3
<400> 184
   Pro Pro Glu Gly Trp Pro Pro Arg
   1 5
<210> 185
   <211> 6
   <212> PRT
   <213> Artificial Sequence
<220>
   <223> ABCC3
<400> 185
   Gly Glu Val Glu Phe Arg
   1 5
<210> 186
   <211> 11
   <212> PRT
   <213> Artificial Sequence
<220>
   <223> ABCC3
<400> 186
   Asp Leu Ser Leu His Val His Gly Gly Glu Lys
   1 5 10
<210> 187
   <211> 11
   <212> PRT
   <213> Artificial Sequence
<220>
   <223> ABCC5
<400> 187
   Ser Leu Ser Glu Ala Ser Val Ala Val Asp Arg
   1 5 10
<210> 188
   <211> 9
   <212> PRT
   <213> Artificial Sequence
<220>
   <223> ABCC6
<400> 188
   Ala Pro Glu Thr Glu Pro Phe Leu Arg
   1 5
<210> 189
   <211> 12
   <212> PRT
   <213> Artificial Sequence
<220>
   <223> ABCC6
<400> 189
   Ala Thr Phe Trp Trp Val Ser Gly Leu Val Trp Arg
   1 5 10
<210> 190
   <211> 7
   <212> PRT
   <213> Artificial Sequence
<220>
   <223> ABCC6
<400> 190
   Asp Leu Trp Ser Leu Gly Arg
   1 5
<210> 191
   <211> 10
   <212> PRT
   <213> Artificial Sequence
<220>
   <223> ABCC6
<400> 191
   Glu Asn Ser Ser Glu Glu Leu Val Ser Arg
   1 5 10
<210> 192
   <211> 7
   <212> PRT
   <213> Artificial Sequence
<220>
   <223> ABCC6
<400> 192
   Leu Thr Ser Ser Ile Leu Arg
   1 5
<210> 193
   <211> 11
   <212> PRT
   <213> Artificial Sequence
<220>
   <223> ABCC6
<400> 193
   Phe His Gly Trp Glu Gly Ala Phe Leu Asp Arg
   1 5 10
<210> 194
   <211> 8
   <212> PRT
   <213> Artificial Sequence
<220>
   <223> ABCC6
<400> 194
   Gly Gln Glu Leu Gly Ala Leu Arg
   1 5
<210> 195
   <211> 12
   <212> PRT
   <213> Artificial Sequence
<220>
   <223> ABCC6
<400> 195
   Gly Glu Gly Glu Thr Glu Pro Gly Thr Ser Thr Lys
   1               5                   10
<210> 196
   <211> 7
   <212> PRT
   <213> Artificial Sequence
<220>
   <223> ABCC6
<400> 196
   Ala Gly Trp Pro Ala Gly Lys
   1               5
<210> 197
   <211> 7
   <212> PRT
   <213> Artificial Sequence
<220>
   <223> ABCC6
<400> 197
   Asp Ser Ile Gln Tyr Gly Arg
   1               5
<210> 198
   <211> 7
   <212> PRT
   <213> Artificial Sequence
<220>
   <223> ABCC6
<400> 198
   Leu Leu Trp Asp Val Val Arg
       1               5
<210> 199
   <211> 9
   <212> PRT
   <213> Artificial Sequence
<220>
   <223> ABCC6
<400> 199
   Thr Pro Ile Gly His Leu Leu Asn Arg
   1               5
<210> 200
   <211> 12
   <212> PRT
   <213> Artificial Sequence
<220>
   <223> ABCC6
<400> 200
   Thr Gln Ala Pro Phe Val Ala Gln Asn Asn Ala Arg
   1               5                   10
<210> 201
   <211> 6
   <212> PRT
   <213> Artificial Sequence
<220>
   <223> ABCC6
<400> 201
   Val Asp Glu Ser Gln Arg
   1               5
<210> 202
   <211> 14
   <212> PRT
   <213> Artificial Sequence
<220>
   <223> ABCC6
<400> 202
   Asn Trp Thr Asp Leu Glu Asn Ser Ile Val Ser Val Glu Arg
   1               5                   10
<210> 203
   <211> 9
   <212> PRT
   <213> Artificial Sequence
<220>
   <223> ABCC6
<400> 203
   Gly Glu Asp Leu Ser Val Gly Gln Lys
   1               5
<210> 204
   <211> 8
   <212> PRT
   <213> Artificial Sequence
<220>
   <223> ABCC6
<400> 204
   Leu Ala Gln Glu Ser Gly Leu Val
   1               5
<210> 205
   <211> 13
   <212> PRT
   <213> Artificial Sequence
<220>
   <223> ABCC7
<400> 205
   Gly Leu Pro Leu Val His Thr Leu Ile Thr Val Ser Lys
   1               5                   10
<210> 206
   <211> 7
   <212> PRT
   <213> Artificial Sequence
<220>
   <223> ABCC7
<400> 206
   Ile Gln Asp Phe Leu Gln Lys
   1               5
<210> 207
   <211> 6
   <212> PRT
   <213> Artificial Sequence
<220>
   <223> ABCC7
<400> 207
   Tyr Ile Thr Val His Lys
   1               5
<210> 208
   <211> 7
   <212> PRT
   <213> Artificial Sequence
<220>
   <223> ABCC7
<400> 208
   Ala Gly Gly Ile Leu Asn Arg
   1 5
<210> 209
   <211> 7
   <212> PRT
   <213> Artificial Sequence
<220>
   <223> ABCC7
<400> 209
   Gln Leu Glu Ser Glu Gly Arg
   1 5
<210> 210
   <211> 6
   <212> PRT
   <213> Artificial Sequence
<220>
   <223> ABCC7
<400> 210
   Asn Gly Gln Leu Ser Lys
   1 5
<210> 211
   <211> 8
   <212> PRT
   <213> Artificial Sequence
<220>
   <223> ABCC7
<400> 211
   Val Ala Asp Glu Val Gly Leu Arg
   1 5
<210> 212
   <211> 9
   <212> PRT
   <213> Artificial Sequence
<220>
   <223> ABCC7
<400> 212
   Ser Val Ile Glu Gln Phe Pro Gly Lys
   1 5
<210> 213
   <211> 7
   <212> PRT
   <213> Artificial Sequence
<220>
   <223> ABCC7
<400> 213
   Gln Tyr Asp Ser Ile Gln Lys
   1 5
<210> 214
   <211> 8
   <212> PRT
   <213> Artificial Sequence
<220>
   <223> ABCC7
<400> 214
   Gln Ala Ile Ser Pro Ser Asp Arg
   1 5
<210> 215
   <211> 8
   <212> PRT
   <213> Artificial Sequence
<220>
   <223> ABCC8
<400> 215
   Thr Val Val Thr Ile Ala His Arg
   1 5
<210> 216
   <211> 7
   <212> PRT
   <213> Artificial Sequence
<220>
   <223> ABCC8
<400> 216
   Ala Leu Thr Asn Tyr Gln Arg
   1 5
<210> 217
   <211> 6
   <212> PRT
   <213> Artificial Sequence
<220>
   <223> ABCC8
<400> 217
   Gly Ala Ile Gln Thr Lys
   1 5
<210> 218
   <211> 9
   <212> PRT
   <213> Artificial Sequence
<220>
   <223> ABCC8
<400> 218
   Leu Tyr Ala Trp Glu Asn Ile Phe Arg
   1 5
<210> 219
   <211> 11
   <212> PRT
   <213> Artificial Sequence
<220>
   <223> ABCC8
<400> 219
   Leu Ser Glu Phe Leu Ser Ser Ala Glu Ile Arg
   1 5 10
<210> 220
   <211> 7
   <212> PRT
   <213> Artificial Sequence
<220>
   <223> ABCC8
<400> 220
   Tyr Gln Ala Val Pro Leu Arg
   1 5
<210> 221
   <211> 6
   <212> PRT
   <213> Artificial Sequence
<220>
   <223> ABCC8
<400> 221
   Asp Gly Thr Ile Gln Arg
   1 5
<210> 222
   <211> 6
   <212> PRT
   <213> Artificial Sequence
<220>
   <223> ABCC8
<400> 222
   Ala Glu Ile Pro Trp Arg
   1 5
<210> 223
   <211> 13
   <212> PRT
   <213> Artificial Sequence
<220>
   <223> ABCC8
<400> 223
   Trp Thr Asp Ser Ala Leu Thr Leu Thr Pro Ala Ala Arg
   1 5 10
<210> 224
   <211> 13
   <212> PRT
   <213> Artificial Sequence
<220>
   <223> ABCC8
<400> 224
   Phe Phe Glu Thr Thr Pro Leu Gly Ser Ile Leu Asn Arg
   1 5 10
<210> 225
   <211> 9
   <212> PRT
   <213> Artificial Sequence
<220>
   <223> ABCC9
<400> 225
   Asn Leu His His Asn Leu Leu Asn Lys
   1 5
<210> 226
   <211> 9
   <212> PRT
   <213> Artificial Sequence
<220>
   <223> ABCC9
<400> 226
   Ser Thr Leu Asp Tyr Ser Thr Glu Arg
   1 5
<210> 227
   <211> 7
   <212> PRT
   <213> Artificial Sequence
<220>
   <223> ABCC9
<400> 227
   Ala Ile Ile Ser Val Gln Lys
   1 5
<210> 228
   <211> 9
   <212> PRT
   <213> Artificial Sequence
<220>
   <223> ABCC9
<400> 228
   Asp Val Glu Leu Tyr Glu His Trp Lys
   1 5
<210> 229
   <211> 7
   <212> PRT
   <213> Artificial Sequence
<220>
   <223> ABCC9
<400> 229
   Ile Ile Leu Gly Pro Ile Arg
   1 5
<210> 230
   <211> 13
   <212> PRT
   <213> Artificial Sequence
<220>
   <223> ABCC9
<400> 230
   Phe Phe Asp Thr Thr Pro Leu Gly Leu Ile Leu Asn Arg
   1 5 10
<210> 231
   <211> 9
   <212> PRT
   <213> Artificial Sequence
<220>
   <223> ABCC9
<400> 231
   Ser Ser Leu Ser Leu Ala Phe Phe Arg
   1 5
<210> 232
   <211> 10
   <212> PRT
   <213> Artificial Sequence
<220>
   <223> ABCC9
<400> 232
   Ile Val Ile Asp Gly Ile Asp Ile Ser Lys
   1 5 10
<210> 233
   <211> 9
   <212> PRT
   <213> Artificial Sequence
<220>
   <223> ABCC10
<400> 233
   Val Phe Thr Ala Leu Ala Leu Val Arg
   1 5
<210> 234
   <211> 12
   <212> PRT
   <213> Artificial Sequence
<220>
   <223> ABCC10
<400> 234
   Phe Ser Tyr Ala Trp Leu Ala Pro Leu Leu Ala Arg
   1 5 10
<210> 235
   <211> 8
   <212> PRT
   <213> Artificial Sequence
<220>
   <223> ABCC10
<400> 235
   Leu Gln Pro Thr Tyr Leu Ala Arg
   1 5
<210> 236
   <211> 11
   <212> PRT
   <213> Artificial Sequence
<220>
   <223> ABCC10
<400> 236
   Val Phe Gln Ala His Trp Gln Glu Gly Ala Arg
   1 5 10
<210> 237
   <211> 6
   <212> PRT
   <213> Artificial Sequence
<220>
   <223> ABCC10
<400> 237
   Val Thr Leu Gln Ala Arg
   1 5
<210> 238
   <211> 10
   <212> PRT
   <213> Artificial Sequence
<220>
   <223> ABCC10
<400> 238
   Leu Val Thr Glu Leu Leu Ser Gly Ile Arg
   1 5 10
<210> 239
   <211> 6
   <212> PRT
   <213> Artificial Sequence
<220>
   <223> ABCC10
<400> 239
   Gly His Val Ala Val Arg
   1 5
<210> 240
   <211> 8
   <212> PRT
   <213> Artificial Sequence
<220>
   <223> ABCC10
<400> 240
   Thr Phe Asp Ala Gln Leu Tyr Lys
   1 5
<210> 241
   <211> 9
   <212> PRT
   <213> Artificial Sequence
<220>
   <223> ABCC10
<400> 241
   Gly Val Thr Leu Ser Gly Gly Gln Arg
   1 5
<210> 242
   <211> 6
   <212> PRT
   <213> Artificial Sequence
<220>
   <223> ABCC10
<400> 242
   Ala Val Tyr Gln Glu Lys
   1 5
<210> 243
   <211> 12
   <212> PRT
   <213> Artificial Sequence
<220>
   <223> ABCC10
<400> 243
   Glu Gly Leu Glu Glu Glu Gln Ser Thr Ser Gly Arg
   1 5 10
<210> 244
   <211> 15
   <212> PRT
   <213> Artificial Sequence
<220>
   <223> ABCC10
<400> 244
   Asn Ala Ala Asp Trp Trp Leu Ser His Trp Ile Ser Gln Leu Lys
   1               5                   10                  15
<210> 245
   <211> 10
   <212> PRT
   <213> Artificial Sequence
<220>
   <223> ABCC10
<400> 245
   Ala Ala Pro Asn Gly Ser Ser Asp Ile Arg
   1               5                   10
<210> 246
   <211> 6
   <212> PRT
   <213> Artificial Sequence
<220>
   <223> ABCC10
<400> 246
   Leu Gly Ile Val Gly Arg
   1               5
<210> 247
   <211> 9
   <212> PRT
   <213> Artificial Sequence
<220>
   <223> ABCC10
<400> 247
   Ser Ser Leu Leu Leu Val Leu Phe Arg
   1               5
<210> 248
   <211> 8
   <212> PRT
   <213> Artificial Sequence
<220>
   <223> ABCC10
<400> 248
   Leu Leu Glu Pro Ser Ser Gly Arg
   1 5
<210> 249
   <211> 10
   <212> PRT
   <213> Artificial Sequence
<220>
   <223> ABCC10
<400> 249
   Glu Asn Leu Asp Pro Gln Gly Leu His Lys
   1 5 10
<210> 250
   <211> 7
   <212> PRT
   <213> Artificial Sequence
<220>
   <223> ABCC10
<400> 250
   Ala Leu Trp Gln Ala Leu Lys
   1 5
<210> 251
   <211> 7
   <212> PRT
   <213> Artificial Sequence
<220>
   <223> ABCC10
<400> 251
   Ser Leu Ser Leu Gly Gln Arg
   1 5
<210> 252
   <211> 7
   <212> PRT
   <213> Artificial Sequence
<220>
   <223> ABCC10
<400> 252
   Ala Leu Leu Thr Asp Ala Lys
   1 5
<210> 253
   <211> 9
   <212> PRT
   <213> Artificial Sequence
<220>
   <223> ABCC10
<400> 253
   Leu Asn Thr Ile Leu Asn Ser Asp Arg
   1 5
<210> 254
   <211> 8
   <212> PRT
   <213> Artificial Sequence
<220>
   <223> ABCC10
<400> 254
   Val Leu Val Leu Gln Ala Gly Arg
   1 5
<210> 255
   <211> 11
   <212> PRT
   <213> Artificial Sequence
<220>
   <223> ABCC10
<400> 255
   Val Val Glu Leu Asp Ser Pro Ala Thr Leu Arg
   1 5 10
<210> 256
   <211> 8
   <212> PRT
   <213> Artificial Sequence
<220>
   <223> ABCC11
<400> 256
   Phe Asn Leu Asp Pro Phe Asp Arg
   1 5
<210> 257
   <211> 14
   <212> PRT
   <213> Artificial Sequence
<220>
   <223> ABCC11
<400> 257
   Thr Tyr Thr Leu Gln Asp Gly Pro Trp Ser Gln Gln Glu Arg
   1 5 10
<210> 258
   <211> 7
   <212> PRT
   <213> Artificial Sequence
<220>
   <223> ABCC11
<400> 258
   Asn Pro Glu Ala Pro Gly Arg
   1 5
<210> 259
   <211> 8
   <212> PRT
   <213> Artificial Sequence
<220>
   <223> ABCC11
<400> 259
   Ala Ala Val Pro Pro Trp Gly Lys
   1 5
<210> 260
   <211> 13
   <212> PRT
   <213> Artificial Sequence
<220>
   <223> ABCC11
<400> 260
   Ser Leu Ser Phe Ser Ser Ser Trp Ile Ile Asn Gln Arg
   1 5 10
<210> 261
   <211> 10
   <212> PRT
   <213> Artificial Sequence
<220>
   <223> ABCC11
<400> 261
   Ala Ala Val Ser Ser Phe Ala Phe Glu Lys
   1 5 10
<210> 262
   <211> 12
   <212> PRT
   <213> Artificial Sequence
<220>
   <223> ABCC11
<400> 262
   Ala Gln His His Thr Ser Glu Val Ser Asp Gln Arg
   1 5 10
<210> 263
   <211> 7
   <212> PRT
   <213> Artificial Sequence
<220>
   <223> ABCC11
<400> 263
   Ile Asn Leu Val Val Ser Lys
   1 5
<210> 264
   <211> 14
   <212> PRT
   <213> Artificial Sequence
<220>
   <223> ABCC11
<400> 264
   Ile Gly Leu Glu Thr Glu Ala Gln Phe Thr Ala Val Glu Arg
   1 5 10
<210> 265
   <211> 15
   <212> PRT
   <213> Artificial Sequence
<220>
   <223> ABCC11
<400> 265
   Asp Asn Thr Pro Thr Val Leu His Gly Ile Asn Leu Thr Ile Arg
   1 5 10 15
<210> 266
   <211> 10
   <212> PRT
   <213> Artificial Sequence
<220>
   <223> ABCC11
<400> 266
   Gly His Glu Val Val Gly Ile Val Gly Arg
   1 5 10
<210> 267
   <211> 12
   <212> PRT
   <213> Artificial Sequence
<220>
   <223> ABCC11
<400> 267
   His Thr Asp Gln Gln Ile Trp Asp Ala Leu Glu Arg
   1 5 10
<210> 268
   <211> 11
   <212> PRT
   <213> Artificial Sequence
<220>
   <223> ABCC12
<400> 268
   Phe Ser Ile Ala Ile Leu Pro Phe Ser Ile Lys
   1 5 10
<210> 269
   <211> 8
   <212> PRT
   <213> Artificial Sequence
<220>
   <223> ABCC12
<400> 269
   Glu Asn Ile Leu Phe Gly Glu Lys
   1 5
<210> 270
   <211> 7
   <212> PRT
   <213> Artificial Sequence
<220>
   <223> ABCC13
<400> 270
   Glu Asp Leu Phe Glu Leu Lys
   1 5
<210> 271
   <211> 10
   <212> PRT
   <213> Artificial Sequence
<220>
   <223> ABCG1
<400> 271
   Gly Ala Val Leu Ile Asn Gly Leu Pro Arg
   1 5 10
<210> 272
   <211> 7
   <212> PRT
   <213> Artificial Sequence
<220>
   <223> ABCG1
<400> 272
   Gly Leu Ala Gln Gly Gly Arg
   1 5
<210> 273
   <211> 10
   <212> PRT
   <213> Artificial Sequence
<220>
   <223> ABCG1
<400> 273
   Glu His Leu Asn Tyr Trp Tyr Ser Leu Lys
   1 5 10
<210> 274
   <211> 15
   <212> PRT
   <213> Artificial Sequence
<220>
   <223> ABCG1
<400> 274
   Tyr Gly Phe Glu Gly Val Ile Leu Ser Ile Tyr Gly Leu Asp Arg
   1 5 10 15
<210> 275
   <211> 8
   <212> PRT
   <213> Artificial Sequence
<220>
   <223> ABCG1
<400> 275
   Glu Leu Asp Val Glu Asn Ala Lys
   1 5
<210> 276
   <211> 11
   <212> PRT
   <213> Artificial Sequence
<220>
   <223> ABCG4
<400> 276
   Gly Val Val Thr Asn Leu Ile Pro Tyr Leu Lys
   1 5 10
<210> 277
   <211> 10
   <212> PRT
   <213> Artificial Sequence
<220>
   <223> ABCG4
<400> 277
   Glu His Leu Asn Tyr Trp Tyr Ser Leu Lys
   1 5 10
<210> 278
   <211> 6
   <212> PRT
   <213> Artificial Sequence
<220>
   <223> ABCG4
<400> 278
   Ala Tyr Tyr Leu Ala Lys
   1 5
<210> 279
   <211> 8
   <212> PRT
   <213> Artificial Sequence
<220>
   <223> ABCG5
<400> 279
   Asp Ser Pro Gly Val Phe Ser Lys
   1 5
<210> 280
   <211> 13
   <212> PRT
   <213> Artificial Sequence
<220>
   <223> ABCG5
<400> 280
   Ala Gly Thr Phe Leu Gly Glu Val Tyr Val Asn Gly Arg
   1 5 10
<210> 281
   <211> 10
   <212> PRT
   <213> Artificial Sequence
<220>
   <223> ABCG5
<400> 281
   Ile Val Val Leu Thr Ile His Gln Pro Arg
   1 5 10
<210> 282
   <211> 14
   <212> PRT
   <213> Artificial Sequence
<220>
   <223> ABCG5
<400> 282
   Ala Val Ser Asp Gln Glu Ser Gln Asp Gly Leu Tyr Gln Lys
   1 5 10
<210> 283
   <211> 9
   <212> PRT
   <213> Artificial Sequence
<220>
   <223> ABCG5
<400> 283
   Ile Ile Ser Tyr Phe Thr Phe Gln Lys
   1 5
<210> 284
   <211> 10
   <212> PRT
   <213> Artificial Sequence
<220>
   <223> ABCG8
<400> 284
   Ala Ser Leu Leu Asp Val Ile Thr Gly Arg
   1 5 10
<210> 285
   <211> 8
   <212> PRT
   <213> Artificial Sequence
<220>
   <223> SLC10A1
<400> 285
   Gly Ile Tyr Asp Gly Asp Leu Lys
   1 5
<210> 286
   <211> 7
   <212> PRT
   <213> Artificial Sequence
<220>
   <223> SLC15A1
<400> 286
   Thr Leu Pro Val Phe Pro Lys
   1 5
<210> 287
   <211> 9
   <212> PRT
   <213> Artificial Sequence
<220>
   <223> SLC15A2
<400> 287
   Ser Gln Asp Phe His Phe His Leu Lys
   1 5
<210> 288
   <211> 7
   <212> PRT
   <213> Artificial Sequence
<220>
   <223> SLC16A1
<400> 288
   Ser Ile Thr Val Phe Phe Lys
   1               5
<210> 289
   <211> 7
   <212> PRT
   <213> Artificial Sequence
<220>
   <223> SLC16A7
<400> 289
   Ala Val Thr Val Phe Phe Lys
   1               5
<210> 290
   <211> 14
   <212> PRT
   <213> Artificial Sequence
<220>
   <223> SLC21A2
<400> 290
   Val Asn Thr Ala Ala Val Asn Leu Val Pro Gly Asp Pro Arg
   1               5                   10
<210> 291
   <211> 11
   <212> PRT
   <213> Artificial Sequence
<220>
   <223> SLC21A3
<400> 291
   Glu Gly Leu Glu Thr Asn Ala Asp Ile Ile Lys
   1               5                   10
<210> 292
   <211> 8
   <212> PRT
   <213> Artificial Sequence
<220>
   <223> SLC21A6
<400> 292
   Leu Asn Thr Val Gly Ile Ala Lys
   1               5
<210> 293
   <211> 9
   <212> PRT
   <213> Artificial Sequence
<220>
   <223> SLC21A8
<400> 293
   Ile Tyr Asn Ser Val Phe Phe Gly Arg
   1 5
<210> 294
   <211> 7
   <212> PRT
   <213> Artificial Sequence
<220>
   <223> SLC21A9
<400> 294
   Val Leu Leu Gln Thr Leu Arg
   1 5
<210> 295
   <211> 11
   <212> PRT
   <213> Artificial Sequence
<220>
   <223> SLC21A11
<400> 295
   Ser Gly Glu Leu Gln Gly Asp Glu Ala Gln Arg
   1 5 10
<210> 296
   <211> 10
   <212> PRT
   <213> Artificial Sequence
<220>
   <223> SLC21A12
<400> 296
   Tyr Glu Val Glu Leu Asp Ala Gly Val Arg
   1 5 10
<210> 297
   <211> 8
   <212> PRT
   <213> Artificial Sequence
<220>
   <223> SLC21A14
<400> 297
   Leu Tyr Asp Ser Asn Val Phe Arg
   1 5
<210> 298
   <211> 11
   <212> PRT
   <213> Artificial Sequence
<220>
   <223> SLC21A15
<400> 298
   Asn Pro Val His Leu Asp Gln Asn Asp Pro Arg
   1 5 10
<210> 299
   <211> 12
   <212> PRT
   <213> Artificial Sequence
<220>
   <223> SLC21A19
<400> 299
   Ile Phe Gly Thr Ile Pro Gly Pro Ser Ile Phe Lys
   1 5 10
<210> 300
   <211> 12
   <212> PRT
   <213> Artificial Sequence
<220>
   <223> SLC21A20
<400> 300
   His Leu Pro Gly Thr Ala Glu Ile Gln Ala Gly Lys
   1 5 10
<210> 301
   <211> 10
   <212> PRT
   <213> Artificial Sequence
<220>
   <223> SLC22A1
<400> 301
   Leu Ser Pro Ser Phe Ala Asp Leu Phe Arg
   1 5 10
<210> 302
   <211> 8
   <212> PRT
   <213> Artificial Sequence
<220>
   <223> SLC22A2
<400> 302
   Ser Leu Pro Ala Ser Leu Gln Arg
   1 5
<210> 303
   <211> 8
   <212> PRT
   <213> Artificial Sequence
<220>
   <223> SLC22A3
<400> 303
   Phe Leu Gln Gly Val Phe Gly Lys
   1 5
<210> 304
   <211> 8
   <212> PRT
   <213> Artificial Sequence
<220>
   <223> SLC22A4
<400> 304
   Ala Phe Ile Leu Asp Leu Phe Arg
   1 5
<210> 305
   <211> 30
   <212> PRT
   <213> Artificial Sequence
<220>
   <223> SLC22A5
<400> 305
   Leu Ile Phe Phe Leu Leu Ser Ala Ser Ile Ile Pro Asn Gly Phe Thr
   1 5 10 15
   Gly Leu Ser Ser Val Phe Leu Ile Ala Thr Pro Glu His Arg
   20 25 30
<210> 306
   <211> 8
   <212> PRT
   <213> Artificial Sequence
<220>
   <223> SLC22A6
<400> 306
   Thr Ser Leu Ala Val Leu Gly Lys
   1 5
<210> 307
   <211> 9
   <212> PRT
   <213> Artificial Sequence
<220>
   <223> SLC22A7
<400> 307
   Asn Val Ala Leu Leu Ala Leu Pro Arg
   1 5
<210> 308
   <211> 8
   <212> PRT
   <213> Artificial Sequence
<220>
   <223> SLC22A8
<400> 308
   Tyr Thr Ala Ser Asp Leu Phe Arg
   1 5
<210> 309
   <211> 9
   <212> PRT
   <213> Artificial Sequence
<220>
   <223> SLC22A9
<400> 309
   Asp Thr Leu Thr Leu Glu Ile Leu Lys
   1 5
<210> 310
   <211> 9
   <212> PRT
   <213> Artificial Sequence
<220>
   <223> SLC22A10
<400> 310
   Asn Leu Pro Leu Pro Asp Thr Ile Lys
   1               5
<210> 311
   <211> 8
   <212> PRT
   <213> Artificial Sequence
<220>
   <223> SLC22A11
<400> 311
   Val Val Phe Ala Val Leu Gly Lys
   1               5
<210> 312
   <211> 10
   <212> PRT
   <213> Artificial Sequence
<220>
   <223> SLC22A12
<400> 312
   Gly Gly Ala Ile Leu Gly Pro Leu Val Arg
   1               5                   10
<210> 313
   <211> 12
   <212> PRT
   <213> Artificial Sequence
<220>
   <223> SLC22A13
<400> 313
   Thr Gly Pro Ser Gly Asn Ala Leu Asp Leu Phe Arg
   1               5                   10
<210> 314
   <211> 15
   <212> PRT
   <213> Artificial Sequence
<220>
   <223> SLC22A14
<400> 314
   Asp Gln Pro Leu Ser Glu Ser Leu Asn His Ser Ser Gln Ile Arg
   1 5 10 15
<210> 315
   <211> 13
   <212> PRT
   <213> Artificial Sequence
<220>
   <223> SLC22A15
<400> 315
   Val Gly Gly Ile Ile Ala Pro Phe Ile Pro Ser Leu Lys
   1 5 10
<210> 316
   <211> 17
   <212> PRT
   <213> Artificial Sequence
<220>
   <223> SLC22A16
<400> 316
   Asp Tyr Val Thr Val Gln Leu Gln Asn Gly Glu Ile Trp Glu Leu Ser
   1 5 10 15
   Arg
<210> 317
   <211> 16
   <212> PRT
   <213> Artificial Sequence

<220>
   <223> SLC22A17
<400> 317
   Asn Leu Leu Ile Leu Gly Phe Thr Asn Phe Ile Ala His Ala Ile Arg
   1 5 10 15
<210> 318
   <211> 12
   <212> PRT
   <213> Artificial Sequence
<220>
   <223> SLC22A18
<400> 318
   Thr Leu Gly Pro Thr Val Gly Gly Leu Leu Tyr Arg
   1 5 10
<210> 319
   <211> 9
   <212> PRT
   <213> Artificial Sequence
<220>
   <223> SLC29A1
<400> 319
   Trp Leu Pro Ser Leu Val Leu Ala Arg
   1               5
<210> 320
   <211> 11
   <212> PRT
   <213> Artificial Sequence
<220>
   <223> SLC29A2
<400> 320
   Val Ala Leu Thr Leu Asp Leu Asp Leu Glu Lys
   1               5                   10
<210> 321
   <211> 13
   <212> PRT
   <213> Artificial Sequence
<220>
   <223> SLC28A1
<400> 321
   Ala Gly Ser Ser Phe Val Phe Gly Glu Ala Leu Val Lys
   1               5                   10
<210> 322
   <211> 13
   <212> PRT
   <213> Artificial Sequence
<220>
   <223> SLC28A3
<400> 322
   Thr Asp Pro Gly Phe Ile Ala Phe Asp Trp Leu Gly Arg
   1               5                   10
<210> 323
   <211> 11
   <212> PRT
   <213> Artificial Sequence
<220>
   <223> SLC19A1
<400> 323
   Thr Ile Ile Thr Phe Ile Val Ser Asp Val Arg
   1 5 10
<210> 324
   <211> 12
   <212> PRT
   <213> Artificial Sequence
<220>
   <223> SLC1A1
<400> 324
   Ser Asp Thr Ile Ser Phe Thr Gln Thr Ser Gln Phe
   1 5 10
<210> 325
   <211> 11
   <212> PRT
   <213> Artificial Sequence
<220>
   <223> SLC1A2
<400> 325
   Ser Glu Leu Asp Thr Ile Asp Ser Gln His Arg
   1 5 10
<210> 326
   <211> 12
   <212> PRT
   <213> Artificial Sequence
<220>
   <223> SLC1A3
<400> 326
   Val Thr Ala Ala Asp Ala Phe Leu Asp Leu Ile Arg
   1 5 10
<210> 327
   <211> 11
   <212> PRT
   <213> Artificial Sequence
<220>
   <223> SLC1A4
<400> 327
   Asp Ile Ile Val Leu Val Thr Ser Leu Gly Lys
   1 5 10
<210> 328
   <211> 13
   <212> PRT
   <213> Artificial Sequence
<220>
   <223> SLC1A5
<400> 328
   Asn Ile Phe Pro Ser Asn Leu Val Ser Ala Ala Phe Arg
   1 5 10
<210> 329
   <211> 6
   <212> PRT
   <213> Artificial Sequence
<220>
   <223> SLC1A6
<400> 329
   Val Gly Trp Leu Gln Arg
   1 5
<210> 330
   <211> 6
   <212> PRT
   <213> Artificial Sequence .
<220>
   <223> SLC1A7
<400> 330
   Thr Thr Pro Val Val Lys
   1 5
<210> 331
   <211> 10
   <212> PRT
   <213> Artificial Sequence
<220>
   <223> SLC2A1
<400> 331
   Thr Phe Asp Glu Ile Ala Ser Gly Phe Arg
   1 5 10
<210> 332
   <211> 6
   <212> PRT
   <213> Artificial Sequence
<220>
   <223> SLC6A12
<400> 332
   Gly Gln Trp Thr Asn Lys
   1 5
<210> 333
   <211> 9
   <212> PRT
   <213> Artificial Sequence
<220>
   <223> SLC7A5
<400> 333
   Val Gln Asp Ala Phe Ala Ala Ala Lys
   1 5
<210> 334
   <211> 15
   <212> PRT
   <213> Artificial Sequence
<220>
   <223> SLC17A1
<400> 334
   Glu Tyr Ile Thr Ser Ser Leu Val Gln Gln Val Ser Ser Ser Arg
   1 5 10 15
<210> 335
   <211> 16
   <212> PRT
   <213> Artificial Sequence
<220>
   <223> SLC17A2
<400> 335
   Glu His Ile Leu Ser Ser Leu Ala Gln Gln Pro Ser Ser Pro Gly Arg
   1 5 10 15
<210> 336
   <211> 8
   <212> PRT
   <213> Artificial Sequence
<220>
   <223> SLC17A3
<400> 336
   Glu Tyr Ile Ile Ser Ser Leu Lys
   1 5
<210> 337
   <211> 8
   <212> PRT
   <213> Artificial Sequence
<220>
   <223> SLC17A4
<400> 337
   Ala Asp Val Gln Asp Trp Ala Lys
   1 5
<210> 338
   <211> 9
   <212> PRT
   <213> Artificial Sequence
<220>
   <223> SLC17A5
<400> 338
   Thr Pro Leu Leu Pro Gly Ala Pro Arg
   1 5
<210> 339
   <211> 6
   <212> PRT
   <213> Artificial Sequence
<220>
   <223> SLC17A6
<400> 339
   Ile Leu Ala Pro Gly Lys
   1 5
<210> 340
   <211> 8
   <212> PRT
   <213> Artificial Sequence
<220>
   <223> SLC17A7
<400> 340
   Gly Gly His Val Val Val Gln Lys
   1 5
<210> 341
   <211> 18
   <212> PRT
   <213> Artificial Sequence
<220>
   <223> SLC17A8
<400> 341
   Thr Tyr Ile Glu Thr Ser Ile Gly Glu Gly Ala Asn Val Val Ser Leu
   1               5                   10                  15
   Ser Lys
<210> 342
   <211> 7
   <212> PRT
   <213> Artificial Sequence
<220>
   <223> SLC29A4
<400> 342
   Leu Leu Leu Pro Asp Glu Arg
   1               5
<210> 343
   <211> 14
   <212> PRT
   <213> Artificial Sequence
<220>
   <223> SLC44A1
<400> 343
   Leu Pro Val Pro Ala Ser Ala Pro Ile Pro Phe Phe His Arg
   1               5                   10
<210> 344
   <211> 8
   <212> PRT
   <213> Artificial Sequence
<220>
   <223> SLC44A2
<400> 344
   Tyr Leu Thr Tyr Leu Asn Ala Arg
   1               5
<210> 345
   <211> 7
   <212> PRT
   <213> Artificial Sequence
<220>
   <223> SLC44A3
<400> 345
   Ser Phe Pro Leu Phe Asn Arg
   1 5
<210> 346
   <211> 8
   <212> PRT
   <213> Artificial Sequence
<220>
   <223> SLC44A4
<400> 346
   Gly Val Gln Asn Pro Val Ala Arg
   1 5
<210> 347
   <211> 10
   <212> PRT
   <213> Artificial Sequence
<220>
   <223> SLC44A5
<400> 347
   Thr Ile Leu Phe Tyr Phe Asn Leu Leu Arg
   1 5 10
<210> 348
   <211> 11
   <212> PRT
   <213> Artificial Sequence
<220>
   <223> MATE1
<400> 348
   Asp His Val Gly Tyr Ile Phe Thr Thr Asp Arg
   1 5 10
<210> 349
   <211> 8
   <212> PRT
   <213> Artificial Sequence
<220>
   <223> MATE2
<400> 349
   Ala Ser Phe His Leu Phe Gln Lys
   1 5
<210> 350
   <211> 6
   <212> PRT
   <213> Artificial Sequence
<220>
   <223> SLC2A2
<400> 350
   Leu Asp Glu Glu Val Lys
   1 5
<210> 351
   <211> 8
   <212> PRT
   <213> Artificial Sequence
<220>
   <223> SLC2A2
<400> 351
   Glu Glu Ala Ser Ser Glu Gln Lys
   1 5
<210> 352
   <211> 7
   <212> PRT
   <213> Artificial Sequence
<220>
   <223> SLC2A8
<400> 352
   Ala Ala Pro Pro Ala Pro Arg
   1 5
<210> 353
   <211> 12
   <212> PRT
   <213> Artificial Sequence
<220>
   <223> SLC2A8
<400> 353
   Thr Leu Glu Gln Ile Thr Ala His Phe Glu Gly Arg
   1 5 10
<210> 354
   <211> 8
   <212> PRT
   <213> Artificial Sequence
<220>
   <223> SLC2A9
<400> 354
   Ala Phe Gln Thr Phe Leu Gly Lys
   1 5
<210> 355
   <211> 9
   <212> PRT
   <213> Artificial Sequence
<220>
   <223> SLC2A9
<400> 355
   Leu Val Ser Val Leu Glu Leu Leu Arg
   1 5
<210> 356
   <211> 9
   <212> PRT
   <213> Artificial Sequence
<220>
   <223> SLC2A9
<400> 356
   Ile Asp Ser Ala Val Thr Asp Gly Lys
   1 5
<210> 357
   <211> 12
   <212> PRT
   <213> Artificial Sequence
<220>
   <223> SLC2A10
<400> 357
   Asp Leu Ile Pro Leu Gln Gly Gly Glu Ala Pro Lys
   1 5 10
<210> 358
   <211> 6
   <212> PRT
   <213> Artificial Sequence
<220>
   <223> SLC2A10
<400> 358
   Thr Asn Glu Asp Gln Arg
   1 5
<210> 359
   <211> 9
   <212> PRT
   <213> Artificial Sequence
<220>
   <223> SLC2A10
<400> 359
   Ser Gly Asp Pro Ser Ala Pro Pro Arg
   1 5
<210> 360
   <211> 15
   <212> PRT
   <213> Artificial Sequence
<220>
   <223> SLC2A10
<400> 360
   Leu Ala Leu Ser Ser Ala Leu Pro Gly Pro Pro Leu Pro Ala Arg
   1 5 10 15
<210> 361
   <211> 8
   <212> PRT
   <213> Artificial Sequence
<220>
   <223> SLC4A5
<400> 361
   Gly His Leu Ser Gly Leu Gln Lys
   1 5
<210> 362
   <211> 7
   <212> PRT
   <213> Artificial Sequence
<220>
   <223> SLC4A5
<400> 362
   Val Glu Glu Gly Gly Glu Arg
   1 5

<210> 363
   <211> 7
   <212> PRT
   <213> Artificial Sequence
<220>
   <223> SLC4A5
<400> 363
   Ser Leu Ala Asp Ile Gly Lys
   1               5
<210> 364
   <211> 7
   <212> PRT
   <213> Artificial Sequence
<220>
   <223> SLC4A5
<400> 364
   Ser Val Ser Thr Thr Asn Arg
   1               5
<210> 365
   <211> 16
   <212> PRT
   <213> Artificial Sequence
<220>
   <223> SLC4A5
<400> 365
   Ser Pro Gly Ala Gly Pro Ser Leu His His Ser Thr Glu Asp Leu Arg
   1               5                   10                  15
<210> 366
   <211> 7
   <212> PRT
   <213> Artificial Sequence
<220>
   <223> SLC4A5
<400> 366
   Gln Ser Ala Asn Tyr Gly Arg
   1               5
<210> 367
   <211> 24
   <212> PRT
   <213> Artificial Sequence
<220>
   <223> SLC4A5
<400> 367
   Asp Ser Glu Ala Ser Asn Val Leu Val Gly Glu Val Asp Phe Leu Asp
   1               5                   10                  15
   Gln Pro Phe Ile Ala Phe Val Arg
               20
<210> 368
   <211> 11
   <212> PRT
   <213> Artificial Sequence
<220>
   <223> SLC4A5
<400> 368
   Phe Leu Phe Ile Leu Leu Gly Pro Ser Gly Arg
   1               5                   10
<210> 369
   <211> 27
   <212> PRT
   <213> Artificial Sequence
<220>
   <223> SLC4A5
<400> 369
   Glu Asp Leu Ile Ala Gly Ile Asp Glu Phe Leu Asp Glu Val Ile Val
   1               5                   10                  15
   Leu Pro Pro Gly Glu Trp Asp Pro Asn Ile Arg
               20                  25
<210> 370
   <211> 7
   <212> PRT
   <213> Artificial Sequence
<220>
   <223> SLC4A5
<400> 370
   Leu Leu Phe Asp Phe Ser Lys
   1               5
<210> 371
   <211> 9
   <212> PRT
   <213> Artificial Sequence
<220>
   <223> SLC4A5
<400> 371
   Gly Trp Phe Val Ala Pro Phe Gly Lys
   1               5
<210> 372
   <211> 21
   <212> PRT
   <213> Artificial Sequence
<220>
   <223> SLC4A5
<400> 372
   Val Thr Gly Ile Ile Val Phe Ile Leu Thr Gly Ile Ser Val Phe Leu
   1               5                   10                  15
   Ala Pro Ile Leu Lys
               20
<210> 373
   <211> 9
   <212> PRT
   <213> Artificial Sequence
<220>
   <223> SLC4A5
<400> 373
   His Gln Pro Asp His Ala Phe Leu Arg
   1               5
<210> 374
   <211> 11
   <212> PRT
   <213> Artificial Sequence
<220>
   <223> SLC4A7
<400> 374
   Phe Leu Phe Leu Leu Leu Gly Pro Ala Gly Lys
   1               5                   10
<210> 375
   <211> 9
   <212> PRT
   <213> Artificial Sequence
<220>
   <223> SLC4A7
<400> 375
   Ala Pro Gln Tyr His Glu Ile Gly Arg
   1 5
<210> 376
   <211> 12
   <212> PRT
   <213> Artificial Sequence
<220>
   <223> SLC4A7
<400> 376
   Glu Ala Ala His His Ala Gly Pro Glu Leu Gln Arg
   1 5 10
<210> 377
   <211> 10
   <212> PRT
   <213> Artificial Sequence
<220>
   <223> SLC4A7
<400> 377
   Asp Tyr Gln Leu Ser Tyr Leu Ser Leu Arg
   1 5 10
<210> 378
   <211> 8
   <212> PRT
   <213> Artificial Sequence
<220>
   <223> SLC4A7
<400> 378
   Phe Glu Pro Thr His Pro Glu Arg
   1 5
<210> 379
   <211> 9
   <212> PRT
   <213> Artificial Sequence
<220>
   <223> SLC4A7
<400> 379
   His Gln Pro Asp Leu Ile Tyr Leu Arg
   1 5
<210> 380
   <211> 12
   <212> PRT
   <213> Artificial Sequence
<220>
   <223> SLC5A9
<400> 380
   Thr Glu Thr Ala Pro His Ile Ala Leu Asp Ser Arg
   1 5 10
<210> 381
   <211> 11
   <212> PRT
   <213> Artificial Sequence
<220>
   <223> SLC5A9
<400> 381
   Leu Thr Ser Ile Glu Glu Glu Pro Leu Trp Arg
   1 5 10
<210> 382
   <211> 8
   <212> PRT
   <213> Artificial Sequence
<220>
   <223> SLC5A11
<400> 382
   Glu Asp Ala Phe His Ile Phe Arg
   1 5
<210> 383
   <211> 7
   <212> PRT
   <213> Artificial Sequence
<220>
   <223> SLC5A11
<400> 383
   His Asp Pro Val Val Gln Lys
   1 5
<210> 384
   <211> 11
   <212> PRT
   <213> Artificial Sequence
<220>
   <223> SLC5A11
<400> 384
   Leu Val Leu Glu Leu Leu Pro Pro Gly Leu Arg
   1 5 10
<210> 385
   <211> 15
   <212> PRT
   <213> Artificial Sequence
<220>
   <223> SLC5A11
<400> 385
   Ala Glu Ala Ile Ile Val Ser Leu Glu Glu Asn Pro Leu Val Lys
   1 5 10 15
<210> 386
   <211> 9
   <212> PRT
   <213> Artificial Sequence
<220>
   <223> SLC6A9
<400> 386
   Thr Asp Gly Asp Thr Leu Leu Gln Arg
   1 5
<210> 387
   <211> 13
   <212> PRT
   <213> Artificial Sequence
<220>
   <223> SLC6A9
<400> 387
   Ala Gln Ile Pro Ile Val Gly Ser Asn Gly Ser Ser Arg
   1 5 10
<210> 388
   <211> 12
   <212> PRT
   <213> Artificial Sequence
<220>
   <223> SLC6A13
<400> 388
   Ile Ser Asp Gly Ile Gln His Leu Gly Ala Leu Arg
   1 5 10
<210> 389
   <211> 7
   <212> PRT
   <213> Artificial Sequence
<220>
   <223> SLC7A2
<400> 389
   Ala Ala Leu Thr Phe Ala Arg
   1 5
<210> 390
   <211> 12
   <212> PRT
   <213> Artificial Sequence
<220>
   <223> SLC7A2
<400> 390
   Ala Gly Ser Gly Thr Phe Asp Glu Leu Leu Ser Lys
   1 5 10
<210> 391
   <211> 7
   <212> PRT
   <213> Artificial Sequence
<220>
   <223> SLC7A2
<400> 391
   Gln Ile Gly Gln Phe Leu Arg
   1 5
<210> 392
   <211> 7
   <212> PRT
   <213> Artificial Sequence
<220>
   <223> SLC7A2
<400> 392
   Glu Ser Ala Trp Val Asn Lys
   1 5
<210> 393
   <211> 7
   <212> PRT
   <213> Artificial Sequence
<220>
   <223> SLC7A2
<400> 393
   Ile Ser Glu Glu Phe Leu Lys
   1 5
<210> 394
   <211> 8
   <212> PRT
   <213> Artificial Sequence
<220>
   <223> SLC7A2
<400> 394
   Asp Gly Leu Gly Ser Ser Pro Arg
   1 5
<210> 395
   <211> 8
   <212> PRT
   <213> Artificial Sequence
<220>
   <223> SLC7A2
<400> 395
   His Ser Leu Glu Gly His Leu Arg
   1 5
<210> 396
   <211> 11
   <212> PRT
   <213> Artificial Sequence
<220>
   <223> SLC7A2
<400> 396
   Ser Ala Ile Gln Ala Asn Asp His His Pro Arg
   1 5 10
<210> 397
   <211> 11
   <212> PRT
   <213> Artificial Sequence
<220>
   <223> SLC7A2
<400> 397
   Asn Leu Ser Ser Pro Phe Ile Phe His Glu Lys
   1 5 10
<210> 398
   <211> 9
   <212> PRT
   <213> Artificial Sequence
<220>
   <223> SLC7A6
<400> 398
   Val Gln Asp Thr Phe Thr Tyr Ala Lys
   1 5
<210> 399
   <211> 7
   <212> PRT
   <213> Artificial Sequence
<220>
   <223> SLC7A6
<400> 399
   Leu Phe Phe Val Gly Ser Arg
   1 5
<210> 400
   <211> 8
   <212> PRT
   <213> Artificial Sequence
<220>
   <223> SLC7A6
<400> 400
   Asn Val Leu Ala Ala Ile Thr Arg
   1 5
<210> 401
   <211> 8
   <212> PRT
   <213> Artificial Sequence
<220>
   <223> SLC7A9
<400> 401
   Ser Ile Gln Ser Gln Glu Pro Lys
   1 5
<210> 402
   <211> 22
   <212> PRT
   <213> Artificial Sequence
<220>
   <223> SLC10A6
<400> 402
   Ile Gly Ala Val Val Gly Gly Val Leu Leu Leu Val Val Ala Val Ala
   1 5 10 15
   Gly Val Val Leu Ala Lys
   20
<210> 403
   <211> 37
   <212> PRT
   <213> Artificial Sequence
<220>
   <223> SLC10A6
<400> 403
   Gly Ser Trp Asn Ser Asp Ile Thr Leu Leu Thr Ile Ser Phe Ile Phe
   1 5 10 15
   Pro Leu Ile Gly His Val Thr Gly Phe Leu Leu Ala Leu Phe Thr His
   20 25 30
   Gln Ser Trp Gln Arg
   35
<210> 404
   <211> 6
   <212> PRT
   <213> Artificial Sequence
<220>
   <223> SLC13A3
<400> 404
   Ser Leu Phe Gly Gln Lys
   1 5
<210> 405
   <211> 6
   <212> PRT
   <213> Artificial Sequence
<220>
   <223> SLC13A3
<400> 405
   Thr Asn Ala Glu Asp Arg
   1 5
<210> 406
   <211> 10
   <212> PRT
   <213> Artificial Sequence
<220>
   <223> SLC13A3
<400> 406
   Glu Glu Tyr Gln Asn Leu Gly Pro Ile Lys
   1 5 10
<210> 407
   <211> 6
   <212> PRT
   <213> Artificial Sequence
<220>
   <223> SLC13A3
<400> 407
   Trp Trp Phe Asp Phe Lys
   1 5
<210> 408
   <211> 8
   <212> PRT
   <213> Artificial Sequence
<220>
   <223> SLC13A5
<400> 408
   Thr Leu Leu Trp Val Gly Ala Lys
   1 5
<210> 409
   <211> 15
   <212> PRT
   <213> Artificial Sequence
<220>
   <223> SLC16A2
<400> 409
   Gly Gly Gly Gly Leu Asp Val Gly Gly Gly Gly Glu Gly Ser Arg
   1 5 10 15
<210> 410
   <211> 13
   <212> PRT
   <213> Artificial Sequence
<220>
   <223> SLC16A2
<400> 410
   Val His Glu Pro Glu Pro Thr Pro Thr Val Glu Thr Arg
   1 5 10
<210> 411
   <211> 10
   <212> PRT
   <213> Artificial Sequence
<220>
   <223> SLC16A2
<400> 411
   Tyr Val Glu Glu Glu Phe Ser Glu Ile Lys
   1 5 10
<210> 412
   <211> 8
   <212> PRT
   <213> Artificial Sequence
<220>
   <223> SLC16A4
<400> 412
   Ser Glu Asn Asn Ser Gly Ile Lys
   1 5
<210> 413
   <211> 7
   <212> PRT
   <213> Artificial Sequence
<220>
   <223> SLC16A4
<400> 413
   Ser Asp Glu Glu Ser Asp Lys
   1 5
<210> 414
   <211> 9
   <212> PRT
   <213> Artificial Sequence
<220>
   <223> SLC16A4
<400> 414
   Gln Leu Phe Asp Ile Ser Leu Phe Arg
   1 5
<210> 415
   <211> 10
   <212> PRT
   <213> Artificial Sequence
<220>
   <223> SLC10A2
<400> 415
   Ala Asn Gly Gly Phe Gln Pro Asp Glu Lys
   1 5 10
<210> 416
   <211> 12
   <212> PRT
   <213> Artificial Sequence
<220>
   <223> SLC10A3
<400> 416
   Val Thr Ser Leu Asp Thr Glu Val Leu Thr Ile Lys
   1 5 10
<210> 417
   <211> 14
   <212> PRT
   <213> Artificial Sequence
<220>
   <223> SLC10A3
<400> 417
   Leu Leu Ser Ile His Glu Thr Leu His Val Pro Ile Ser Lys
   1 5 10
<210> 418
   <211> 7
   <212> PRT
   <213> Artificial Sequence
<220>
   <223> SLC10A4
<400> 418
   Val Ala Asp Tyr Ile Val Lys
   1 5
<210> 419
   <211> 8
   <212> PRT
   <213> Artificial Sequence
<220>
   <223> SLC10A5
<400> 419
   Thr Glu Ile Leu Phe Phe Thr Lys
   1 5
<210> 420
   <211> 14
   <212> PRT
   <213> Artificial Sequence
<220>
   <223> SLC10A5
<400> 420
   Ile Leu Gly Leu Ser Gly Thr Phe His Ile Pro Val Ser Lys
   1 5 10
<210> 421
   <211> 19
   <212> PRT
   <213> Artificial Sequence
<220>
   <223> SLC10A5
<400> 421
   Ile Val Ser Thr Leu Leu Phe Ile Leu Val Pro Val Ser Ile Gly Ile
   1 5 10 15
   Val Ile Lys
<210> 422
   <211> 7
   <212> PRT
   <213> Artificial Sequence
<220>
   <223> SLC11A1
<400> 422
   Leu Gly Val Val Thr Gly Lys
   1 5
<210> 423
   <211> 17
   <212> PRT
   <213> Artificial Sequence
<220>
   <223> SLC11A2
<400> 423
   Leu Leu Trp Ile Leu Leu Leu Ala Thr Leu Val Gly Leu Leu Leu Gln
   1 5 10 15
   Arg
<210> 424
   <211> 9
   <212> PRT
   <213> Artificial Sequence
<220>
   <223> SLC12A1
<400> 424
   Asn Thr Gly Ser Ile Ser Gly Pro Lys
   1 5
<210> 425
   <211> 10
   <212> PRT
   <213> Artificial Sequence
<220>
   <223> SLC12A1
<400> 425
   Ser Leu Leu Gln Ala Ser Gly Leu Gly Arg
   1 5 10
<210> 426
   <211> 11
   <212> PRT
   <213> Artificial Sequence
<220>
   <223> SLC12A1
<400> 426
   Asn Val Ala Val Thr Pro Ser Ser Ala Asp Arg
   1 5 10
<210> 427
   <211> 7
   <212> PRT
   <213> Artificial Sequence
<220>
   <223> SLC12A1
<400> 427
   Ala Leu Gln Phe Phe Ala Lys
   1 5
<210> 428
   <211> 12
   <212> PRT
   <213> Artificial Sequence
<220>
   <223> SLC12A2
<400> 428
   Gly Pro Ile Val Pro Leu Asn Val Ala Asp Gln Lys
   1 5 10
<210> 429
   <211> 8
   <212> PRT
   <213> Artificial Sequence
<220>
   <223> SLC12A2
<400> 429
   Asp Leu Pro Pro Ile Leu Leu Val
   1 5
<210> 430
   <211> 6
   <212> PRT
   <213> Artificial Sequence
<220>
   <223> SLC12A2
<400> 430
   Asp Gly Gly Gly Val Arg
   1 5
<210> 431
   <211> 10
   <212> PRT
   <213> Artificial Sequence
<220>
   <223> SLC12A2
<400> 431
   Gly Gly Gly Ala Tyr Tyr Leu Ile Ser Arg
   1 5 10
<210> 432
   <211> 12
   <212> PRT
   <213> Artificial Sequence
<220>
   <223> SLC12A2
<400> 432
   Ala Phe Tyr Ala Pro Val His Ala Asp Asp Leu Arg
   1 5 10
<210> 433
   <211> 10
   <212> PRT
   <213> Artificial Sequence
<220>
   <223> SLC12A3
<400> 433
   Ser Gly Gly Thr Tyr Phe Leu Ile Ser Arg
   1 5 10
<210> 434
   <211> 6
   <212> PRT
   <213> Artificial Sequence
<220>
   <223> SLC12A3
<400> 434
   Gly Phe Phe Ser Tyr Arg
   1 5
<210> 435
   <211> 8
   <212> PRT
   <213> Artificial Sequence
<220>
   <223> SLC12A3
<400> 435
   Asp Pro Ala Ile Ala Ile Pro Lys
   1 5
<210> 436
   <211> 12
   <212> PRT
   <213> Artificial Sequence
<220>
   <223> SLC12A4
<400> 436
   His Gly Leu Pro Ser Ala Asp Ala Pro Ser Leu Lys
   1 5 10
<210> 437
   <211> 7
   <212> PRT
   <213> Artificial Sequence
<220>
   <223> SLC12A4
<400> 437
   Leu Leu Gln Ala Ile Ala Lys
   1 5
<210> 438
   <211> 8
   <212> PRT
   <213> Artificial Sequence
<220>
   <223> SLC12A4
<400> 438
   Asp Asn Ile Ile Pro Phe Leu Arg
   1 5
<210> 439
   <211> 6
   <212> PRT
   <213> Artificial Sequence
<220>
   <223> SLC12A4
<400> 439
   Val Phe Gly His Gly Lys
   1 5
<210> 440
   <211> 8
   <212> PRT
   <213> Artificial Sequence
<220>
   <223> SLC12A4
<400> 440
   Gly Leu Ser Leu Ser Ala Ala Arg
   1 5
<210> 441
   <211> 9
   <212> PRT
   <213> Artificial Sequence
<220>
   <223> SLC12A4
<400> 441
   Leu Leu Thr Phe Ala Ser Gln Leu Lys
   1 5
<210> 442
   <211> 7
   <212> PRT
   <213> Artificial Sequence
<220>
   <223> SLC12A5
<400> 442
   Leu Leu Gln Ala Ile Ser Arg
   1 5
<210> 443
   <211> 14
   <212> PRT
   <213> Artificial Sequence
<220>
   <223> SLC12A5
<400> 443
   Asp Gly Ile Val Pro Phe Leu Gln Val Phe Gly His Gly Lys
   1 5 10
<210> 444
   <211> 8
   <212> PRT
   <213> Artificial Sequence
<220>
   <223> SLC12A5
<400> 444
   Gly Leu Ser Leu Ser Ala Ala Arg
   1 5
<210> 445
   <211> 20
   <212> PRT
   <213> Artificial Sequence
<220>
   <223> SLC12A5
<400> 445
   Val Asp Gln Asp Gln Asn Val Val His Pro Gln Leu Leu Ser Leu Thr
   1 5 10 15
   Ser Gln Leu Lys
   20
<210> 446
   <211> 7
   <212> PRT
   <213> Artificial Sequence
<220>
   <223> SLC12A6
<400> 446
   Val Ser Ser Leu Leu Asn Arg
   1 5
<210> 447
   <211> 11
   <212> PRT
   <213> Artificial Sequence
<220>
   <223> SLC12A6
<400> 447
   Ala Ala Ile Phe His Ser Asp Asp Ala Leu Lys
   1 5 10
<210> 448
   <211> 8
   <212> PRT
   <213> Artificial Sequence
<220>
   <223> SLC12A6
<400> 448
   Asp Asn Ile Ile Pro Phe Leu Arg
   1 5
<210> 449
   <211> 6
   <212> PRT
   <213> Artificial Sequence
<220>
   <223> SLC12A6
<400> 449
   Val Phe Gly His Ser Lys
   1 5
<210> 450
   <211> 8
   <212> PRT
   <213> Artificial Sequence
<220>
   <223> SLC12A6
<400> 450
   Gly Leu Ser Leu Ser Ala Ala Arg
   1 5
<210> 451
   <211> 9
   <212> PRT
   <213> Artificial Sequence
<220>
   <223> SLC12A6
<400> 451
   Leu Leu Thr Phe Ala Ser Gln Leu Lys
   1 5
<210> 452
   <211> 6
   <212> PRT
   <213> Artificial Sequence
<220>
   <223> SLC12A6
<400> 452
   Gln Ser Glu Asp Ala Arg
   1 5
<210> 453
   <211> 7
   <212> PRT
   <213> Artificial Sequence
<220>
   <223> SLC12A6
<400> 453
   Thr Phe Ile Gly Thr Val Arg
   1 5
<210> 454
   <211> 13
   <212> PRT
   <213> Artificial Sequence
<220>
   <223> SLC12A6
<400> 454
   Val Thr Thr Ala Ala His Leu Ala Leu Leu Val Ala Lys
   1               5                   10
<210> 455
   <211> 7
   <212> PRT
   <213> Artificial Sequence
<220>
   <223> SLC12A7
<400> 455
   Leu Leu Gln Ala Ile Ala Arg
   1               5
<210> 456
   <211> 14
   <212> PRT
   <213> Artificial Sequence
<220>
   <223> SLC12A7
<400> 456
   Asp Gly Ile Val Pro Phe Leu Gln Val Phe Gly His Gly Lys
   1               5                   10
<210> 457
   <211> 8
   <212> PRT
   <213> Artificial Sequence
<220>
   <223> SLC12A7
<400> 457
   Gly Leu Ser Leu Asn Ala Ala Arg
   1               5
<210> 458
   <211> 9
   <212> PRT
   <213> Artificial Sequence
<220>
   <223> SLC12A7
<400> 458
   Leu Leu Ser Phe Thr Ser Gln Leu Lys
   1 5
<210> 459
   <211> 7
   <212> PRT
   <213> Artificial Sequence
<220>
   <223> SLC12A7
<400> 459
   Asn Phe Val Asp Thr Val Arg
   1 5
<210> 460
   <211> 13
   <212> PRT
   <213> Artificial Sequence
<220>
   <223> SLC12A7
<400> 460
   Asp Thr Thr Ala Ala His Gln Ala Leu Leu Val Ala Lys
   1 5 10
<210> 461
   <211> 11
   <212> PRT
   <213> Artificial Sequence
<220>
   <223> SLC12A7
<400> 461
   Asn Thr Ala Ser His Thr Ala Ala Ala Ala Arg
   1 5 10
<210> 462
   <211> 9
   <212> PRT
   <213> Artificial Sequence
<220>
   <223> SLC12A7
<400> 462
   Thr Gln Ala Pro Pro Thr Pro Asp Lys
   1 5
<210> 463
   <211> 9
   <212> PRT
   <213> Artificial Sequence
<220>
   <223> SLC12A7
<400> 463
   Arg Asp Thr Ser Leu Ser Gly Phe Lys
   1               5
<210> 464
   <211> 8
   <212> PRT
   <213> Artificial Sequence
<220>
   <223> SLC12A7
<400> 464
   Leu Asn Gly Val Val Leu Asn Lys
   1               5
<210> 465
   <211> 17
   <212> PRT
   <213> Artificial Sequence
<220>
   <223> SLC12A8
<400> 465
   Ala Ser Pro Gly Leu His Leu Gly Ser Ala Ser Asn Phe Ser Phe Phe
   1               5                   10                  15
   Arg
<210> 466
   <211> 9
   <212> PRT
   <213> Artificial Sequence
<220>
   <223> SLC12A8
<400> 466
   Tyr His His Ser Ser Leu Val Asn Arg
   1               5
<210> 467
   <211> 7
   <212> PRT
   <213> Artificial Sequence
<220>
   <223> SLC12A9
<400> 467
   Ile Leu His Ala Leu Ala Arg
   1 5
<210> 468
   <211> 12
   <212> PRT
   <213> Artificial Sequence
<220>
   <223> SLC12A9
<400> 468
   Asp Asp Leu Phe Gly Val Ile Leu Ala Pro Ala Lys
   1 5 10
<210> 469
   <211> 12
   <212> PRT
   <213> Artificial Sequence
<220>
   <223> SLC12A9
<400> 469
   Ala Phe Val Asp Leu Thr Leu Ser Pro Ser Val Arg
   1 5 10
<210> 470
   <211> 8
   <212> PRT
   <213> Artificial Sequence
<220>
   <223> SLC12A9
<400> 470
   Gln Gly Ala Gln His Leu Leu Arg
   1 5
<210> 471
   <211> 6
   <212> PRT
   <213> Artificial Sequence
<220>
   <223> SLC12A9
<400> 471
   Ala Pro Gly Ser Pro Arg
   1 5
<210> 472
   <211> 16
   <212> PRT
   <213> Artificial Sequence
<220>
   <223> SLC12A9
<400> 472
   Ala Leu Asn Pro Gln Asp Tyr Val Ala Thr Val Ala Asp Ala Leu Lys
   1 5 10 15
<210> 473
   <211> 6
   <212> PRT
   <213> Artificial Sequence
<220>
   <223> SLC12A9
<400> 473
   Asn Val Val Leu Ala Arg
   1 5
<210> 474
   <211> 7
   <212> PRT
   <213> Artificial Sequence
<220>
   <223> SLC12A9
<400> 474
   Ala Leu Leu Ser Gln Leu Arg
   1 5
<210> 475
   <211> 7
   <212> PRT
   <213> Artificial Sequence
<220>
   <223> SLC12A9
<400> 475
   Ser Ala Asn Ala Leu Val Arg
   1 5
<210> 476
   <211> 10
   <212> PRT
   <213> Artificial Sequence
<220>
   <223> SLC12A9
<400> 476
   Gly Thr Gly Gly Gly Pro Gly Gly Pro Lys
   1 5 10
<210> 477
   <211> 5
   <212> PRT
   <213> Artificial Sequence
<220>
   <223> SLC13A1
<400> 477
<210> 478
   <211> 6
   <212> PRT
   <213> Artificial Sequence
<220>
   <223> SLC13A2
<400> 478
<210> 479
   <211> 5
   <212> PRT
   <213> Artificial Sequence
<220>
   <223> SLC13A2
<400> 479
   Thr Val Asn Gln Lys
   1 5
<210> 480
   <211> 10
   <212> PRT
   <213> Artificial Sequence
<220>
   <223> SLC13A4
<400> 480
   Ser Asn Glu Val Ala Ala Glu Tyr Phe Lys
   1 5 10
<210> 481
   <211> 15
   <212> PRT
   <213> Artificial Sequence
<220>
   <223> SLC13A4
<400> 481
   Asn Ser Gln Pro Ser Leu Glu Leu Ile Phe Val Asn Glu Asp Arg
   1 5 10 15
<210> 482
   <211> 6
   <212> PRT
   <213> Artificial Sequence
<220>
   <223> SLC19A1
<400> 482
   Asn Ser Asn Ile Tyr Lys
   1 5
<210> 483
   <211> 7
   <212> PRT
   <213> Artificial Sequence
<220>
   <223> SLC14A2
<400> 483
   Tyr Gln Ala Tyr Asp Val Ser
   1 5
<210> 484
   <211> 6
   <212> PRT
   <213> Artificial Sequence
<220>
   <223> SLC15A3
<400> 484
   Gln Pro Leu Leu Pro Arg
   1 5
<210> 485
   <211> 25
   <212> PRT
   <213> Artificial Sequence
<220>
   <223> SLC15A3
<400> 485
   Ala Val Ala Leu Ser Leu Leu Leu Tyr Leu Ala Ala Ser Gly Leu Leu
   1 5 10 15
   Pro Ala Thr Ala Phe Pro Asp Gly Arg
   20 25
<210> 486
   <211> 8
   <212> PRT
   <213> Artificial Sequence
<220>
   <223> SLC15A3
<400> 486
   Leu Ile Asp Pro Leu Leu Leu Arg
   1 5
<210> 487
   <211> 8
   <212> PRT
   <213> Artificial Sequence
<220>
   <223> SLC15A3
<400> 487
   Leu Leu Pro Ser Ala Leu Gln Lys
   1 5
<210> 488
   <211> 10
   <212> PRT
   <213> Artificial Sequence
<220>
   <223> SLC15A3
<400> 488
   Ala Ser Gln Gly Pro Ala Ser His Ser Arg
   1 5 10
<210> 489
   <211> 12
   <212> PRT
   <213> Artificial Sequence
<220>
   <223> SLC15A4
<400> 489
   Ala Asn Ile Thr Pro Phe Gly Ala Asp Gln Val Lys
   1 5 10
<210> 490
   <211> 6
   <212> PRT
   <213> Artificial Sequence
<220>
   <223> SLC15A4
<400> 490
   Thr Leu Asn Ser His Lys
   1 5
<210> 491
   <211> 13
   <212> PRT
   <213> Artificial Sequence
<220>
   <223> SLC16A10
<400> 491
   Val Leu Ile Asp Ser Val Gly Leu Phe Tyr Thr Leu Arg
   1 5 10
<210> 492
   <211> 15
   <212> PRT
   <213> Artificial Sequence
<220>
   <223> SLC16A11
<400> 492
   Ser Leu Gly Leu Ala Phe Pro Asp Leu Ala Glu His Phe Asp Arg
   1 5 10 15
<210> 493
   <211> 29
   <212> PRT
   <213> Artificial Sequence
<220>
   <223> SLC16A11
<400> 493
   Ser Ala Gln Asp Thr Ala Trp Ile Ser Ala Leu Ala Leu Ala Val Gln
   1 5 10 15
   Gln Ala Ala Ser Pro Val Gly Ser Ala Leu Ser Thr Arg
   20 25
<210> 494
   <211> 15
   <212> PRT
   <213> Artificial Sequence
<220>
   <223> SLC16A12
<400> 494
   His Gly Glu Pro Val Ala Thr Ala Val Pro Gly Tyr Ser Leu Thr
   1               5                   10                  15
<210> 495
   <211> 14
   <212> PRT
   <213> Artificial Sequence
<220>
   <223> SLC16A13
<400> 495
   Ala Gln Leu Thr Ser Leu Leu His His Gly Pro Phe Leu Arg
   1               5                   10
<210> 496
   <211> 7
   <212> PRT
   <213> Artificial Sequence

<220>
   <223> SLC16A14
<400> 496
   Ser Thr Gly Gln Gln Gly Arg
   1               5
<210> 497
   <211> 7
   <212> PRT
   <213> Artificial Sequence
<220>
   <223> SLC16A3
<400> 497
   Ala Val Ser Val Phe Phe Lys
   1               5
<210> 498
   <211> 8
   <212> PRT
   <213> Artificial Sequence
<220>
   <223> SLC16A3
<400> 498
   Asp Leu Gly Val Pro Asp Thr Lys
   1 5
<210> 499
   <211> 6
   <212> PRT
   <213> Artificial Sequence
<220>
   <223> SLC16A5
<400> 499
   Ala Val Leu Gln Ala Lys
   1 5
<210> 500
   <211> 9
   <212> PRT
   <213> Artificial Sequence
<220>
   <223> SLC16A6
<400> 500
   Ile Gly Ala Gly Phe Val Leu Asn Arg
   1 5
<210> 501
   <211> 7
   <212> PRT
   <213> Artificial Sequence
<220>
   <223> SLC16A8
<400> 501
   Ala Val Ser Val Phe Phe Arg
   1 5
<210> 502
   <211> 9
   <212> PRT
   <213> Artificial Sequence
<220>
   <223> SLC16A8
<400> 502
   Ala Phe Ala Val Tyr Ala Val Thr Lys
   1               5
<210> 503
   <211> 6
   <212> PRT
   <213> Artificial Sequence
<220>
   <223> SLC16A8
<400> 503
   Leu Val Asp Val Leu Lys
   1               5
<210> 504
   <211> 7
   <212> PRT
   <213> Artificial Sequence
<220>
   <223> SLC16A9
<400> 504
   Gln Asp Ser Leu Leu His Lys
   1               5
<210> 505
   <211> 8
   <212> PRT
   <213> Artificial Sequence
<220>
   <223> SLC16A9
<400> 505
   Asn Pro Thr Val Thr His Thr Lys
   1               5
<210> 506
   <211> 10
   <212> PRT
   <213> Artificial Sequence
<220>
   <223> SLC16A9
<400> 506
   Leu Leu Leu Gly Ile Leu Ala Asp Phe Lys
   1               5                   10
<210> 507
   <211> 7
   <212> PRT
   <213> Artificial Sequence
<220>
   <223> SLC18A1
<400> 507
   Val Ser Pro Glu Ser Ala Lys
   1 5
<210> 508
   <211> 16
   <212> PRT
   <213> Artificial Sequence
<220>
   <223> SLC18A1
<400> 508
   Glu Phe Pro Leu Gly Glu Asp Ser Asp Glu Glu Pro Asp His Glu Glu
   1 5 10 15
<210> 509
   <211> 7
   <212> PRT
   <213> Artificial Sequence
<220>
   <223> SLC18A2
<400> 509
   Val Gln Pro Glu Ser Gln Lys
   1 5
<210> 510
   <211> 9
   <212> PRT
   <213> Artificial Sequence
<220>
   <223> SLC18A2
<400> 510
   Gly Thr Pro Leu Thr Thr Leu Leu Lys
   1 5
<210> 511
   <211> 9
   <212> PRT
   <213> Artificial Sequence
<220>
   <223> SLC18A3
<400> 511
   Tyr Pro Thr Glu Ser Glu Asp Val Lys
   1 5
<210> 512
   <211> 7
   <212> PRT
   <213> Artificial Sequence
<220>
   <223> SLC18A3
<400> 512
   Tyr Pro Glu Glu Pro Glu Arg
   1 5
<210> 513
   <211> 11
   <212> PRT
   <213> Artificial Sequence
<220>
   <223> SLC18A3
<400> 513
   Ala Asn Leu Pro Val Gly Thr Pro Ile His Arg
   1 5 10
<210> 514
   <211> 7
   <212> PRT
   <213> Artificial Sequence
<220>
   <223> SLC18A3
<400> 514
   Asn Val Gly Leu Leu Thr Arg
   1 5
<210> 515
   <211> 16
   <212> PRT
   <213> Artificial Sequence
<220>
   <223> SLC18A3
<400> 515
   Asp Val Leu Leu Asp Glu Pro Pro Gln Gly Leu Tyr Asp Ala Val Arg
   1 5 10 15
<210> 516
   <211> 5
   <212> PRT
   <213> Artificial Sequence
<220>
   <223> SLC19A2
<400> 516
   Val Asn Gly Ile Lys
   1               5
<210> 517
   <211> 25
   <212> PRT
   <213> Artificial Sequence
<220>
   <223> SLC19A2
<400> 517
   Val Gln Asn Gly Gly Ile Val Thr Asp Thr Pro Ala Ser Asn His Leu
   1               5                   10                  15
   Pro Gly Trp Glu Asp Ile Glu Ser Lys
               20                  25
<210> 518
   <211> 34
   <212> PRT
   <213> Artificial Sequence
<220>
   <223> SLC19A3
<400> 518
   Asn Leu Thr Ser Ala Glu Ile Thr Asn Glu Ile Phe Pro Val Trp Thr
   1               5                   10                  15
   Tyr Ser Tyr Leu Val Leu Leu Leu Pro Val Phe Val Leu Thr Asp Tyr
               20                  25                  30
   Val Arg
<210> 519
   <211> 32
   <212> PRT
   <213> Artificial Sequence
<220>
   <223> SLC19A3
<400> 519
   Ala Pro Ser Gln Asp Ser Ser Ile Tyr Asn Gly Ala Val Glu Ala Ile
   1 5 10 15
   Ala Thr Phe Gly Gly Ala Val Ala Ala Phe Ala Val Gly Tyr Val Lys
   20 25 30
<210> 520
   <211> 6
   <212> PRT
   <213> Artificial Sequence
<220>
   <223> SLC20A1
<400> 520
   Gly Gln Glu Gly Val Lys
   1 5
<210> 521
   <211> 6
   <212> PRT
   <213> Artificial Sequence
<220>
   <223> SLC20A1
<400> 521
   Trp Ser Glu Leu Ile Lys
   1 5
<210> 522
   <211> 6
   <212> PRT
   <213> Artificial Sequence
<220>
   <223> SLC20A1
<400> 522
   Ala Phe Ile Leu His Lys
   1 5
<210> 523
   <211> 9
   <212> PRT
   <213> Artificial Sequence
<220>
   <223> SLC20A1
<400> 523
   Ala Asp Pro Val Pro Asn Gly Leu Arg
   1 5
<210> 524
   <211> 9
   <212> PRT
   <213> Artificial Sequence
<220>
   <223> SLC20A1
<400> 524
   Leu Ser Val Gly Asp Ile Glu Asn Lys
   1 5
<210> 525
   <211> 20
   <212> PRT
   <213> Artificial Sequence
<220>
   <223> SLC20A1
<400> 525
   His Pro Val Ser Glu Val Gly Pro Ala Thr Val Pro Leu Gln Ala Val
   1 5 10 15
   Val Glu Glu Arg
   20
<210> 526
   <211> 5
   <212> PRT
   <213> Artificial Sequence
<220>
   <223> SLC20A1
<400> 526
   Thr Val Ser Phe Lys
   1 5
<210> 527
   <211> 10
   <212> PRT
   <213> Artificial Sequence
<220>
   <223> SLC20A1
<400> 527
   Leu Gly Asp Leu Glu Glu Ala Pro Glu Arg
   1 5 10
<210> 528
   <211> 7
   <212> PRT
   <213> Artificial Sequence
<220>
   <223> SLC20A1
<400> 528
   Leu Pro Ser Val Asp Leu Lys
   1 5
<210> 529
   <211> 6
   <212> PRT
   <213> Artificial Sequence
<220>
   <223> SLC20A1
<400> 529
   Asp Ser Gly Leu Tyr Lys
   1 5
<210> 530
   <211> 5
   <212> PRT
   <213> Artificial Sequence
<220>
   <223> SLC20A1
<400> 530
   Glu Leu Leu His Lys
   1 5
<210> 531
   <211> 5
   <212> PRT
   <213> Artificial Sequence
<220>
   <223> SLC20A1
<400> 531
   Leu His Leu Ala Lys
   1 5
<210> 532
   <211> 11
   <212> PRT
   <213> Artificial Sequence
<220>
   <223> SLC20A1
<400> 532
   Val Gly Ser Val Val Ser Val Gly Trp Leu Arg
   1 5 10
<210> 533
   <211> 6
   <212> PRT
   <213> Artificial Sequence
<220>
   <223> SLC20A2
<400> 533
   Glu Gly Ala Leu Ser Arg
   1 5
<210> 534
   <211> 8
   <212> PRT
   <213> Artificial Sequence
<220>
   <223> SLC20A2
<400> 534
   Val Ser Asp Glu Ser Leu Ser Lys
   1 5
<210> 535
   <211> 10
   <212> PRT
   <213> Artificial Sequence
<220>
   <223> SLC20A2
<400> 535
   Val Gln Glu Ala Glu Ser Pro Val Phe Lys
   1 5 10
<210> 536
   <211> 6
   <212> PRT
   <213> Artificial Sequence
<220>
   <223> SLC20A2
<400> 536
   Glu Leu Pro Gly Ala Lys
   1 5
<210> 537
   <211> 30
   <212> PRT
   <213> Artificial Sequence
<220>
   <223> SLC20A2
<400> 537
   Ala Asn Asp Asp Ser Thr Ile Pro Leu Thr Gly Ala Ala Gly Glu Thr
   1 5 10 15
   Leu Gly Thr Ser Glu Gly Thr Ser Ala Gly Ser His Pro Arg
   20 25 30
<210> 538
   <211> 5
   <212> PRT
   <213> Artificial Sequence
<220>
   <223> SLC20A2
<400> 538
   Ala Ala Tyr Gly Arg
   1 5
<210> 539
   <211> 15
   <212> PRT
   <213> Artificial Sequence
<220>
   <223> SLC20A2
<400> 539
   Ser Pro Ile Ser Asn Gly Thr Phe Gly Phe Asp Gly His Thr Arg
   1 5 10 15
<210> 540
   <211> 11
   <212> PRT
   <213> Artificial Sequence
<220>
   <223> SLC20A2
<400> 540
   Ser Asp Gly His Val Tyr His Thr Val His Lys
   1 5 10
<210> 541
   <211> 6
   <212> PRT
   <213> Artificial Sequence
<220>
   <223> SLC20A2
<400> 541
   Asp Ser Gly Leu Tyr Lys
   1               5
<210> 542
   <211> 5
   <212> PRT
   <213> Artificial Sequence
<220>
   <223> SLC20A2
<400> 542
   Asp Leu Leu His Lys
   1               5
<210> 543
   <211> 5
   <212> PRT
   <213> Artificial Sequence
<220>
   <223> SLC20A2
<400> 543
   Ile His Ile Asp Arg
   1               5
<210> 544
   <211> 12
   <212> PRT
   <213> Artificial Sequence
<220>
   <223> SLC20A2
<400> 544
   Leu Ala Ser Glu Leu Ala Asp Pro Asp Gln Pro Arg
   1               5                   10
<210> 545
   <211> 11
   <212> PRT
   <213> Artificial Sequence
<220>
   <223> SLC20A2
<400> 545
   Val Gly Ser Val Val Ala Val Gly Trp Ile Arg
   1 5 10
<210> 546
   <211> 5
   <212> PRT
   <213> Artificial Sequence
<220>
   <223> SLC20A2
<400> 546
   Ala Val Asp Trp Arg
   1 5
<210> 547
   <211> 8
   <212> PRT
   <213> Artificial Sequence
<220>
   <223> SLC23A1
<400> 547
   Ala Gln Glu Asp Leu Glu Gly Arg
   1 5
<210> 548
   <211> 11
   <212> PRT
   <213> Artificial Sequence
<220>
   <223> SLC23A1
<400> 548
   Asp Pro Ser Thr Pro Leu Pro Thr Glu Pro Lys
   1 5 10
<210> 549
   <211> 6
   <212> PRT
   <213> Artificial Sequence
<220>
   <223> SLC23A1
<400> 549
   Gly Leu Ile Gln Trp Lys
   1 5
<210> 550
   <211> 7
   <212> PRT
   <213> Artificial Sequence
<220>
   <223> SLC23A1
<400> 550
   Gly Phe Ser Ser Ser Ser Lys
   1 5
<210> 551
   <211> 6
   <212> PRT
   <213> Artificial Sequence
<220>
   <223> SLC23A2
<400> 551
   Tyr Glu Asp Glu Ala Lys
   1 5
<210> 552
   <211> 7
   <212> PRT
   <213> Artificial Sequence
<220>
   <223> SLC23A2
<400> 552
   Phe Pro Leu Pro Ile Tyr Lys
   1 5
<210> 553
   <211> 5
   <212> PRT
   <213> Artificial Sequence
<220>
   <223> SLC23A2
<400> 553
   Leu Gln Leu Phe Lys
   1 5
<210> 554
   <211> 6
   <212> PRT
   <213> Artificial Sequence
<220>
   <223> SLC23A2
<400> 554
   Tyr Gly Phe Tyr Ala Arg
   1               5
<210> 555
   <211> 11
   <212> PRT
   <213> Artificial Sequence
<220>
   <223> SLC23A2
<400> 555
   Gln Gly Val Leu Leu Val Ala Pro Trp Phe Lys
   1               5                   10
<210> 556
   <211> 9
   <212> PRT
   <213> Artificial Sequence
<220>
   <223> SLC23A3
<400> 556
   Ser Pro Leu Asn Pro Ser Gln Leu Arg
   1               5
<210> 557
   <211> 22
   <212> PRT
   <213> Artificial Sequence
<220>
   <223> SLC23A3
<400> 557
   Leu Pro Leu Val Gln Ala Pro Ser Leu Glu Phe Leu Ile Pro Ala Leu
   1               5                   10                  15
   Val Leu Thr Ser Gln Lys
               20
<210> 558
   <211> 14
   <212> PRT
   <213> Artificial Sequence
<220>
   <223> SLC23A3
<400> 558
   His Gln Leu Thr Leu Leu Ser Leu Ser Ser Gly Ser Phe Arg
   1 5 10
<210> 559
   <211> 16
   <212> PRT
   <213> Artificial Sequence
<220>
   <223> SLC23A3
<400> 559
   Gly Leu Gly Gln Gly Leu Pro Ser Pro Phe Thr Ala Gln Glu Ala Arg
   1 5 10 15
<210> 560
   <211> 5
   <212> PRT
   <213> Artificial Sequence
<220>
   <223> SLC23A3
<400> 560
   Ala Gln Val Tyr Arg
   1 5
<210> 561
   <211> 16
   <212> PRT
   <213> Artificial Sequence
<220>
   <223> SLC24A1
<400> 561
   Gly Leu Ser Ser Leu Trp Ala Ala Val Ser Ser His Gln Pro Ile Lys
   1 5 10 15
<210> 562
   <211> 11
   <212> PRT
   <213> Artificial Sequence
<220>
   <223> SLC24A1
<400> 562
   Thr Leu Thr Tyr Tyr Thr Ser Thr Ser Ser Arg
   1 5 10
<210> 563
   <211> 8
   <212> PRT
   <213> Artificial Sequence
<220>
   <223> SLC24A1
<400> 563
   Ser Tyr Ser Pro Thr Gln Val Arg
   1 5
<210> 564
   <211> 6
   <212> PRT
   <213> Artificial Sequence
<220>
   <223> SLC24A1
<400> 564
   Tyr Thr Pro Ser Pro Arg
   1 5
<210> 565
   <211> 9
   <212> PRT
   <213> Artificial Sequence
<220>
   <223> SLC24A1
<400> 565
   Asp Ser Asp Ile Thr Ala Thr Tyr Lys
   1 5
<210> 566
   <211> 9
   <212> PRT
   <213> Artificial Sequence
<220>
   <223> SLC24A1
<400> 566
   Ala Phe Thr Ala Ala Trp Ser Leu Arg
   1 5
<210> 567
   <211> 9
   <212> PRT
   <213> Artificial Sequence
<220>
   <223> SLC24A1
<400> 567
   Thr Ser Val Ser Ala Ile Lys Thr Ala
   1 5
<210> 568
   <211> 6
   <212> PRT
   <213> Artificial Sequence
<220>
   <223> SLC24A1
<400> 568
   Pro Ala Ile Val Trp Arg
   1 5
<210> 569
   <211> 12
   <212> PRT
   <213> Artificial Sequence
<220>
   <223> SLC24A1
<400> 569
   Glu Ile Leu Asn Leu Thr Trp Trp Pro Leu Phe Arg
   1 5 10
<210> 570
   <211> 5
   <212> PRT
   <213> Artificial Sequence
<220>
   <223> SLC24A1
<400> 570
   Glu Gln Leu Ser Arg
   1 5
<210> 571
   <211> 10
   <212> PRT
   <213> Artificial Sequence
<220>
   <223> SLC24A1
<400> 571
   Glu Glu Glu Ser Leu Asn Gln Gly Ala Arg
   1 5 10
<210> 572
   <211> 15
   <212> PRT
   <213> Artificial Sequence
<220>
   <223> SLC24A1
<400> 572
   Leu Pro Ala Val Thr Val Thr Pro Ala Pro Val Pro Asp Ile Lys
   1 5 10 15
<210> 573
   <211> 20
   <212> PRT
   <213> Artificial Sequence
<220>
   <223> SLC24A1
<400> 573
   Gly Asn Glu Gly Glu Thr Glu Ser Gln Glu Leu Ser Ala Glu Asn His
   1 5 10 15
   Gly Glu Ala Lys
   20
<210> 574
   <211> 14
   <212> PRT
   <213> Artificial Sequence
<220>
   <223> SLC24A1
<400> 574
   Gly Asn Glu Glu Pro Leu Ser Leu Asp Trp Pro Glu Thr Arg
   1 5 10
<210> 575
   <211> 8
   <212> PRT
   <213> Artificial Sequence
<220>
   <223> SLC24A2
<400> 575
   Val Ala Gln Gly Tyr His Gln Arg
   1 5
<210> 576
   <211> 8
   <212> PRT
   <213> Artificial Sequence
<220>
   <223> SLC24A2
<400> 576
   Thr Leu Leu Asp Leu Asn Asp Lys
   1 5
<210> 577
   <211> 12
   <212> PRT
   <213> Artificial Sequence
<220>
   <223> SLC24A2
<400> 577
   Ile Leu Asp Tyr Thr Pro Gln Pro Pro Leu Ser Lys
   1 5 10
<210> 578
   <211> 12
   <212> PRT
   <213> Artificial Sequence
<220>
   <223> SLC24A2
<400> 578
   Glu Ile Leu Asn Leu Thr Trp Trp Pro Leu Phe Arg
   1 5 10
<210> 579
   <211> 6
   <212> PRT
   <213> Artificial Sequence
<220>
   <223> SLC24A2
<400> 579
   Ala Ser Ile Leu His Lys
   1 5
<210> 580
   <211> 10
   <212> PRT
   <213> Artificial Sequence
<220>
   <223> SLC24A2
<400> 580
   Gln Asn Gly Ala Ala Asn His Val Glu Lys
   1 5 10
<210> 581
   <211> 5
   <212> ?RT
   <213> Artificial Sequence
<220>
   <223> SLC24A3
<400> 581
   Leu Ile Asn Ser Arg
   1 5
<210> 582
   <211> 12
   <212> PRT
   <213> Artificial Sequence
<220>
   <223> SLC24A3
<400> 582
   Ala Tyr Thr Asn Gly Glu Ser Glu Val Ala Ile Lys
   1 5 10
<210> 583
   <211> 15
   <212> PRT
   <213> Artificial Sequence
<220>
   <223> SLC24A3
<400> 583
   His Thr Val Glu Asn Gly Thr Gly Pro Ser Ser Ala Pro Asp Arg
   1 5 10 15
<210> 584
   <211> 5
   <212> PRT
   <213> Artificial Sequence
<220>
   <223> SLC24A3
<400> 584
   Leu Glu Thr Val Lys
   1 5
<210> 585
   <211> 9
   <212> PRT
   <213> Artificial Sequence
<22C>
   <223> SLC24A4
<400> 585
   Phe Thr Phe Pro Glu Ala Gly Leu Arg
   1 5
<210> 586
   <211> 6
   <212> PRT
   <213> Artificial Sequence
<220>
   <223> SLC24A4
<400> 586
   Ile Ile Ile Asn Glu Arg
   1 5
<210> 587
   <211> 15
   <212> PRT
   <213> Artificial Sequence
<220>
   <223> SLC24A5
<400> 587
   Thr Ala Phe Ile Asn Gly Ser Ala Pro Ala Glu Val Asn Ser Arg
   1 5 10 15
<210> 588
   <211> 6
   <212> PRT
   <213> Artificial Sequence
<220>
   <223> SLC24A6
<400> 588
   Asp Asp Gln Asn Trp Lys
   1 5
<210> 589
   <211> 5
   <212> PRT
   <213> Artificial Sequence
<220>
   <223> SLC24A6
<400> 589
   Gln Gly Tyr Pro Arg
   1 5
<210> 590
   <211> 6
   <212> PRT
   <213> Artificial Sequence
<220>
   <223> SLC24A6
<400> 590
   Ser His Thr Glu Val Lys
   1 5
<210> 591
   <211> 11
   <212> PRT
   <213> Artificial Sequence
<220>
   <223> SLC25A1
<400> 591
   Ala Leu Ala Ala Ala Ala Pro Ala Ser Gly Lys
   1 5 10
<210> 592
   <211> 8
   <212> PRT
   <213> Artificial Sequence
<220>
   <223> SLC25A1
<400> 592
   Thr Gln Leu Gln Leu Asp Glu Arg
   1 5
<210> 593
   <211> 9
   <212> PRT
   <213> Artificial Sequence
<220>
   <223> SLC25A1
<400> 593
   Ser His Gly Val Leu Gly Leu Tyr Arg
   1 5
<210> 594
   <211> 5
   <212> PRT
   <213> Artificial Sequence
<220>
   <223> SLC25A1
<400> 594
   Leu Asp Ser Thr Arg
   1 5
<210> 595
   <211> 11
   <212> PRT
   <213> Artificial Sequence
<220>
   <223> SLC25A1
<400> 595
   Phe Ile His Asp Gln Thr Ser Pro Asn Pro Lys
   1 5 10
<210> 596
   <211> 7
   <212> PRT
   <213> Artificial Sequence
<220>
   <223> SLC25A1
<400> 596
   Gly Phe Phe His Gly Val Arg
   1 5
<210> 597
   <211> 5
   <212> PRT
   <213> Artificial Sequence
<220>
   <223> SLC25A1
<400> 597
   Glu Gln Gly Leu Lys
   1 5
<210> 598
   <211> 10
   <212> PRT
   <213> Artificial Sequence
<220>
   <223> SLC25A10
<400> 598
   Val His Leu Gln Thr Gln Gln Glu Val Lys
   1 5 10
<210> 599
   <211> 8
   <212> PRT
   <213> Artificial Sequence
<220>
   <223> SLC25A10
<400> 599
   Phe Ala Ile Tyr Glu Thr Val Arg
   1 5
<210> 600
   <211> 11
   <212> PRT
   <213> Artificial Sequence
<220>
   <223> SLC25A10
<400> 600
   Gly Ser Gln Gly Pro Leu Pro Phe His Glu Lys
   1 5 10
<210> 601
   <211> 8
   <212> PRT
   <213> Artificial Sequence
<220>
   <223> SLC25A10
<400> 601
   Leu Gly Pro Leu Ala Phe Tyr Lys
   1 5
<210> 602
   <211> 11
   <212> PRT
   <213> Artificial Sequence
<220>
   <223> SLC25A11
<400> 602
   Thr Ser Phe His Ala Leu Thr Ser Ile Leu Lys
   1 5 10
<210> 603
   <211> 5
   <212> PRT
   <213> Artificial Sequence
<220>
   <223> SLC25A11
<400> 603
   Ala Glu Gly Leu Arg
   1 5
<210> 604
   <211> 8
   <212> PRT
   <213> Artificial Sequence
<220>
   <223> SLC25A11
<400> 604
   Asn Val Phe Asn Ala Leu Ile Arg
   1 5
<210> 605
   <211> 9
   <212> PRT
   <213> Artificial Sequence
<220>
   <223> SLC25A11
<400> 605
   Glu Glu Gly Val Leu Thr Leu Trp Arg
   1 5
<210> 606
   <211> 8
   <212> PRT
   <213> Artificial Sequence
<220>
   <223> SLC25A11
<400> 606
   Asn Gly Leu Asp Val Leu Phe Lys
   1 5
<210> 607
   <211> 9
   <212> PRT
   <213> Artificial Sequence
<220>
   <223> SLC25A11
<400> 607
   Tyr Glu Gly Phe Phe Ser Leu Trp Lys
   1 5
<210> 608
   <211> 15
   <212> PRT
   <213> Artificial Sequence
<220>
   <223> SLC25A12
<400> 608
   Asn Ile Phe Leu Gln Tyr Ala Ser Thr Glu Val Asp Gly Glu Arg
   1 5 10 15
<210> 609
   <211> 13
   <212> PRT
   <213> Artificial Sequence
<220>
   <223> SLC25A12
<400> 609
   Tyr Leu Gly Leu Tyr Asn Asp Pro Asn Ser Asn Pro Lys
   1 5 10
<210> 610
   <211> 13
   <212> PRT
   <213> Artificial Sequence
<220>
   <223> SLC25A12
<400> 610
   Ile Val Gln Leu Leu Ala Gly Val Ala Asp Gln Thr Lys
   1 5 10
<210> 611
   <211> 12
   <212> PRT
   <213> Artificial Sequence
<220>
   <223> SLC25A12
<400> 611
   Ser Gly Asn Gly Glu Val Thr Phe Glu Asn Val Lys
   1 5 10
<210> 612
   <211> 6
   <212> PRT
   <213> Artificial Sequence
<220>
   <223> SLC25A12
<400> 612
   Gln Ala Phe Ala Leu Lys
   1 5
<210> 613
   <211> 9
   <212> PRT
   <213> Artificial Sequence
<220>
   <223> SLC25A12
<400> 613
   Glu Glu Phe Ala Gln Ser Ala Ile Arg
   1 5
<210> 614
   <211> 24
   <212> PRT
   <213> Artificial Sequence
<220>
   <223> SLC25A12
<400> 614
   Tyr Gly Gln Val Thr Pro Leu Glu Ile Asp Ile Leu Tyr Gln Leu Ala
   1 5 10 15
   Asp Leu Tyr Asn Ala Ser Gly Arg
   20
<210> 615
   <211> 8
   <212> PRT
   <213> Artificial Sequence
<220>
   <223> SLC25A12
<400> 615
   Leu Thr Leu Ala Asp Ile Glu Arg
   1 5
<210> 616
   <211> 18
   <212> PRT
   <213> Artificial Sequence
<220>
   <223> SLC25A12
<400> 616
   Ile Ala Pro Leu Ala Glu Gly Ala Leu Pro Tyr Asn Leu Ala Glu Leu
   1 5 10 15
   Gln Arg
<210> 617
   <211> 9
   <212> PRT
   <213> Artificial Sequence
<220>
   <223> SLC25A12
<400> 617
   Tyr Glu Gly Phe Phe Gly Leu Tyr Arg
   1 5
<210> 618
   <211> 12
   <212> PRT
   <213> Artificial Sequence
<220>
   <223> SLC25A12
<400> 618
   Leu Gln Val Ala Gly Glu Ile Thr Thr Gly Pro Arg
   1 5 10
<210> 619
   <211> 8
   <212> PRT
   <213> Artificial Sequence
<220>
   <223> SLC25A12
<400> 619
   Val Ser Ala Leu Asn Val Leu Arg
   1 5
<210> 620
   <211> 9
   <212> PRT
   <213> Artificial Sequence
<220>
   <223> SLC25A12
<400> 620
   Asp Leu Gly Ile Phe Gly Leu Tyr Lys
   1 5
<210> 621
   <211> 6
   <212> PRT
   <213> Artificial Sequence
<220>
   <223> SLC25A12
<400> 621
   Leu Gln Val Ala Ala Arg
   1 5
<210> 622
   <211> 9
   <212> PRT
   <213> Artificial Sequence
<220>
   <223> SLC25A12
<400> 622
   Glu Glu Gly Pro Ser Ala Phe Trp Lys
   1 5
<210> 623
   <211> 16
   <212> PRT
   <213> Artificial Sequence
<220>
   <223> SLC25A12
<400> 623
   Ile Ala Asp Leu Pro Pro Ala Asn Pro Asp His Ile Gly Gly Tyr Arg
   1 5 10 15
<210> 624
   <211> 12
   <212> PRT
   <213> Artificial Sequence
<220>
   <223> SLC25A12
<400> 624
   Leu Ala Thr Ala Thr Phe Ala Gly Ile Glu Asn Lys
   1 5 10
<210> 625
   <211> 7
   <212> PRT
   <213> Artificial Sequence
<220>
   <223> SLC25A12
<400> 625
   Phe Gly Leu Tyr Leu Pro Lys
   1 5
<210> 626
   <211> 10
   <212> PRT
   <213> Artificial Sequence
<220>
   <223> SLC25A12
<400> 626
   Ser Pro Ser Val Ala Val Val Gln Pro Lys
   1 5 10
<210> 627
   <211> 7
   <212> PRT
   <213> Artificial Sequence
<220>
   <223> SLC25A12
<400> 627
   Ala Ala Val Ala Ala Thr Gln
   1 5
<210> 628
   <211> 5
   <212> PRT
   <213> Artificial Sequence
<220>
   <223> SLC25A13
<400> 628
   Thr Ile Phe Leu Lys
   1 5
<210> 629
   <211> 6
   <212> PRT
   <213> Artificial Sequence
<220>
   <223> SLC25A13
<400> 629
   Tyr Ala Ser Ile Glu Lys
   1 5
<210> 630
   <211> 13
   <212> PRT
   <213> Artificial Sequence
<220>
   <223> SLC25A13
<400> 630
   Tyr Leu Asn Ile Phe Gly Glu Ser Gln Pro Asn Pro Lys
   1 5 10

<210> 631
   <211> 13
   <212> PRT
   <213> Artificial Sequence
<220>
   <223> SLC25A13
<400> 631
   Thr Val Glu Leu Leu Ser Gly Val Val Asp Gln Thr Lys
   1 5 10
<210> 632
   <211> 9
   <212> PRT
   <213> Artificial Sequence
<220>
   <223> SLC25A13
<400> 632
   Gly Glu Val Thr Phe Glu Asp Val Lys
   1 5
<210> 633
   <211> 27
   <212> PRT
   <213> Artificial Sequence
<220>
   <223> SLC25A13
<400> 633
   Gln Val Phe Gly Gln Thr Thr Ile His Gln His Ile Pro Phe Asn Trp
   1 5 10 15
   Asp Ser Glu Phe Val Gln Leu His Phe Gly Lys
   20 25
<210> 634
   <211> 6
   <212> PRT
   <213> Artificial Sequence
<220>
   <223> SLC25A13
<400> 634
   Gln Ala Phe Val Gln Arg
<210> 635
   <211> 7
   <212> PRT
   <213> Artificial Sequence
<220>
   <223> SLC25A13
<400> 635
   Val Thr Ala Ile Asp Phe Arg
   1 5
<210> 636
   <211> 9
   <212> PRT
   <213> Artificial Sequence
<220>
   <223> SLC25A13
<400> 636
   Glu Glu Phe Val Leu Ala Ala Gln Lys
   1 5
<210> 637
   <211> 18
   <212> PRT
   <213> Artificial Sequence
<220>
   <223> SLC25A13
<400> 637
   Ile Ala Pro Leu Glu Glu Gly Thr Leu Pro Phe Asn Leu Ala Glu Ala
   1 5 10 15
   Gln Arg
<210> 638
   <211> 9
   <212> PRT
   <213> Artificial Sequence
<220>
   <223> SLC25A13
<400> 638
   Tyr Glu Gly Phe Phe Gly Leu Tyr Arg
   1 5
<210> 639
   <211> 12
   <212> PRT
   <213> Artificial Sequence
<220>
   <223> SLC25A13
<400> 639
   Leu Gln Val Ala Gly Glu Ile Thr Thr Gly Pro Arg
   1 5 10
<210> 640
   <211> 8
   <212> PRT
   <213> Artificial Sequence
<220>
   <223> SLC25A13
<400> 640
   Val Ser Ala Leu Ser Val Val Arg
   1 5
<210> 641
   <211> 9
   <212> PRT
   <213> Artificial Sequence
<220>
   <223> SLC25A13
<400> 641
   Asp Leu Gly Phe Phe Gly Ile Tyr Lys
   1               5
<210> 642
   <211> 6
   <212> PRT
   <213> Artificial Sequence
<220>
   <223> SLC25A13
<400> 642
   Leu Gln Val Ala Ala Arg
   1               5
<210> 643
   <211> 15
   <212> PRT
   <213> Artificial Sequence
<220>
   <223> SLC25A13
<400> 643
   Ile Asn Leu Pro Ala Pro Asn Pro Asp His Val Gly Gly Tyr Lys
   1               5                   10                  15
<210> 644
   <211> 12
   <212> PRT
   <213> Artificial Sequence
<220>
   <223> SLC25A13
<400> 644
   Leu Ala Val Ala Thr Phe Ala Gly Ile Glu Asn Lys
   1               5                   10
<210> 645
   <211> 6
   <212> PRT
   <213> Artificial Sequence
<220>
   <223> SLC25A13
<400> 645
   Ala Ile Gly Gly Gly Pro
   1 5
<210> 646
   <211> 11
   <212> PRT
   <213> Artificial Sequence
<220>
   <223> SLC25A14
<400> 646
   Leu Gln Val Gln Gly Gln Ser Ile Asp Ala Arg
   1 5 10
<210> 647
   <211> 17
   <212> PRT
   <213> Artificial Sequence
<220>
   <223> SLC25A14
<400> 647
   Glu Glu Gly Val Leu Ala Leu Tyr Ser Gly Ile Ala Pro Ala Leu Leu
   1 5 10 15
   Arg
<210> 648
   <211> 8
   <212> PRT
   <213> Artificial Sequence
<220>
   <223> SLC25A14
<400> 648
   Gln Ala Ser Tyr Gly Thr Ile Lys
   1 5
<210> 649
   <211> 10
   <212> PRT
   <213> Artificial Sequence
<220>
   <223> SLC25A14
<400> 649
   Ala Ile Val Gly His Val Asp Leu Tyr Lys
   1 5 10
<210> 650
   <211> 8
   <212> PRT
   <213> Artificial Sequence
<220>
   <223> SLC25A14
<400> 650
   Gly Thr Val Asp Gly Ile Leu Lys
   1 5
<210> 651
   <211> 8
   <212> PRT
   <213> Artificial Sequence
<220>
   <223> SLC25A15
<400> 651
   Thr Tyr Ser Gln Val Gly Phe Arg
   1 5
<210> 652
   <211> 10
   <212> PRT
   <213> Artificial Sequence
<220>
   <223> SLC25A15
<400> 652
   Ser Gln Asn Thr Val Trp Ser Val Ile Lys
   1 5 10
<210> 653
   <211> 7
   <212> PRT
   <213> Artificial Sequence
<220>
   <223> SLC25A15
<400> 653
   Ser Phe Phe Ala Ser Gly Arg
   1 5
<210> 654
   <211> 8
   <212> PRT
   <213> Artificial Sequence
<220>
   <223> SLC25A16
<400> 654
   Thr Thr Val Ala Pro Leu Asp Arg
   1 5
<210> 655
   <211> 11
   <212> PRT
   <213> Artificial Sequence
<220>
   <223> SLC25A16
<400> 655
   Val Leu Leu Gln Ala His Asn His His Tyr Lys
   1 5 10
<210> 656
   <211> 9
   <212> PRT
   <213> Artificial Sequence
<220>
   <223> SLC25A16
<400> 656
   His Leu Gly Val Phe Ser Ala Leu Arg
   1 5
<210> 657
   <211> 5
   <212> PRT
   <213> Artificial Sequence
<220>
   <223> SLC25A16
<400> 657
   Ala Val Pro Gln Lys
   1 5
<210> 658
   <211> 8
   <212> PRT
   <213> Artificial Sequence
<220>
   <223> SLC25A16
<400> 658
   Glu Gly Phe Leu Gly Leu Tyr Lys
   1 5
<210> 659
   <211> 6
   <212> PRT
   <213> Artificial Sequence
<220>
   <223> SLC25A16
<400> 659
   Thr Leu Ile Thr Thr Lys
   1 5
<210> 660
   <211> 9
   <212> PRT
   <213> Artificial Sequence
<220>
   <223> SLC25A16
<400> 660
   Leu Gly Ile Ser Gly His Val His Arg
   1 5
<210> 661
   <211> 6
   <212> PRT
   <213> Artificial Sequence
<220>
   <223> SLC25A16
<400> 661
   Leu Ala Phe Gln Val Lys
   1 5
<210> 662
   <211> 9
   <212> PRT
   <213> Artificial Sequence
<220>
   <223> SLC25A16
<400> 662
   Tyr Thr Gly Ile Ile His Ala Phe Lys
   1 5
<210> 663
   <211> 5
   <212> PRT
   <213> Artificial Sequence
<220>
   <223> SLC25A16
<400> 663
   Thr Ile Tyr Ala Lys
   1 5
<210> 664
   <211> 9
   <212> PRT
   <213> Artificial Sequence
<220>
   <223> SLC25A16
<400> 664
   Glu Gly Gly Phe Phe Gly Phe Tyr Arg
   1 5
<210> 665
   <211> 8
   <212> PRT
   <213> Artificial Sequence
<220>
   <223> SLC25A16
<400> 665
   Gly Leu Ser Leu Asn Tyr Ile Arg
   1 5
<210> 666
   <211> 6
   <212> PRT
   <213> Artificial Sequence
<220>
   <223> SLC25A16
<400> 666
   Gln Phe Phe His Leu Asn
   1 5
<210> 667
   <211> 9
   <212> PRT
   <213> Artificial Sequence
<220>
   <223> SLC25A17
<400> 667
   Glu Glu Gly Leu Leu Ala Pro Tyr Arg
   1 5
<210> 668
   <211> 5
   <212> PRT
   <213> Artificial Sequence
<220>
   <223> SLC25A17
<400> 668
   Ala Leu Trp Val Lys
   1 5
<210> 669
   <211> 5
   <212> PRT
   <213> Artificial Sequence
<220>
   <223> SLC25A17
<400> 669
   Leu Gln Gly Ala Lys
   1 5
<210> 670
   <211> 10
   <212> PRT
   <213> Artificial Sequence
<220>
   <223> SLC25A17
<400> 670
   Asn Glu Asp Ile Val Pro Thr Asn Tyr Lys
   1 5 10
<210> 671
   <211> 11
   <212> PRT
   <213> Artificial Sequence
<220>
   <223> SLC25A17
<400> 671
   Gly Ile Ile Asp Ala Phe His Gln Ile Ile Arg
   1 5 10
<210> 672
   <211> 6
   <212> PRT
   <213> Artificial Sequence
<220>
   <223> SLC25A17
<400> 672
   Leu Asn Pro Glu Asn Arg
   1 5
<210> 673
   <211> 6
   <212> PRT
   <213> Artificial Sequence
<220>
   <223> SLC25A17
<400> 673
   Thr Leu Gly Ser Leu Arg
   1 5
<210> 674
   <211> 9
   <212> PRT
   <213> Artificial Sequence
<220>
   <223> SLC25A17
<400> 674
   Asn Ile Leu Tyr Leu Leu His Gln Arg
   1 5
<210> 675
   <211> 5
   <212> PRT
   <213> Artificial Sequence
<220>
   <223> SLC25A17
<400> 675
   Gly Leu Glu Ala Lys
   1 5
<210> 676
   <211> 7
   <212> PRT
   <213> Artificial Sequence
<220>
   <223> SLC25A18
<400> 676
   Leu Gln Asn Gln His Gly Lys
   1 5
<210> 677
   <211> 13
   <212> PRT
   <213> Artificial Sequence
<220>
   <223> SLC25A18
<400> 677
   Gly Ala Ala Val Asn Leu Thr Leu Val Thr Pro Glu Lys
   1 5 10
<210> 678
   <211> 8
   <212> PRT
   <213> Artificial Sequence
<220>
   <223> SLC25A18
<400> 678
   Ile Gln Leu Gln Asp Ala Gly Arg
   1 5
<210> 679
   <211> 16
   <212> PRT
   <213> Artificial Sequence
<220>
   <223> SLC25A18
<400> 679
   Leu Ala Val His His Gln Gly Ser Ala Ser Ala Pro Ser Thr Ser Arg
   1 5 10 15
<210> 680
   <211> 9
   <212> PRT
   <213> Artificial Sequence
<220>
   <223> SLC25A18
<400> 680
   Thr Gln Gly Leu Ala Gly Leu Tyr Arg
   1 5
<210> 681
   <211> 9
   <212> PRT
   <213> Artificial Sequence
<220>
   <223> SLC25A19
<400> 681
   Tyr His Gly Ile Leu Gln Ala Ser Arg
   1 5
<210> 682
   <211> 13
   <212> PRT
   <213> Artificial Sequence
<220>
   <223> SLC25A19
<400> 682
   Gln Ile Leu Gln Glu Glu Gly Pro Thr Ala Phe Trp Lys
   1 5 10
<210> 683
   <211> 7
   <212> PRT
   <213> Artificial Sequence
<220>
   <223> SLC25A19
<400> 683
   Gly Ser Val Tyr Asp Ala Arg
   1 5
<210> 684
   <211> 6
   <212> PRT
   <213> Artificial Sequence
<220>
   <223> SLC25A19
<400> 684
   Val Tyr Asn Thr Leu Arg
   1 5
<210> 685
   <211> 9
   <212> PRT
   <213> Artificial Sequence
<220>
   <223> SLC25A19
<400> 685
   Ser Glu Gly Pro Gln Val Phe Tyr Lys
   1               5
<210> 686
   <211> 5
   <212> PRT
   <213> Artificial Sequence
<220>
   <223> SLC25A19
<400> 686
   Gln Val Leu Gln Lys
   1               5
<210> 687
   <211> 8
   <212> PRT
   <213> Artificial Sequence
<220>
   <223> SLC25A19
<400> 687
   Glu Gly Ala Leu Gly Phe Phe Lys
   1               5
<210> 688
   <211> 8
   <212> PRT
   <213> Artificial Sequence
<220>
   <223> SLC25A2
<400> 688
   Thr Tyr Ala Gln Val Gly Leu Arg
   1               5
<210> 689
   <211> 10
   <212> PRT
   <213> Artificial Sequence
<220>
   <223> SLC25A2
<400> 689
   Ser His Asn Thr Ile Trp Ser Val Val Lys
   1               5                   10
<210> 690
   <211> 7
   <212> PRT
   <213> Artificial Sequence
<220>
   <223> SLC25A2
<400> 690
   Ser Phe Phe Ala Ser Gly Arg
   1 5
<210> 691
   <211> 12
   <212> PRT
   <213> Artificial Sequence
<220>
   <223> SLC25A2
<400> 691
   Asn Glu Gly Ile Val Ala Leu Tyr Ser Gly Leu Lys
   1 5 10
<210> 692
   <211> 5
   <212> PRT
   <213> Artificial Sequence
<220>
   <223> SLC25A2
<400> 692
   Gln Leu Glu Ala Tyr
   1 5
<210> 693
   <211> 8
   <212> PRT
   <213> Artificial Sequence
<220>
   <223> SLC25A20
<400> 693
   Glu Gly Ile Thr Gly Leu Tyr Arg
   1 5
<210> 694
   <211> 6
   <212> PRT
   <213> Artificial Sequence
<220>
   <223> SLC25A20
<400> 694
   Tyr Pro Asn Gly Phe Arg
   1 5
<210> 695
   <211> 9
   <212> PRT
   <213> Artificial Sequence
<220>
   <223> SLC25A20
<400> 695
   Asp Glu Gly Val Thr Ser Leu Tyr Lys
   1 5
<210> 696
   <211> 9
   <212> PRT
   <213> Artificial Sequence
<220>
   <223> SLC25A20
<400> 696
   Phe Leu Asn Trp Ala Thr Pro Asn Leu
   1 5
<210> 697
   <211> 5
   <212> PRT
   <213> Artificial Sequence
<220>
   <223> SLC25A21
<400> 697
   Phe Gln Ile Gln Arg
   1 5
<210> 698
   <211> 7
   <212> PRT
   <213> Artificial Sequence
<220>
   <223> SLC25A21
<400> 698
   Ser Leu Val Asp Ser Phe Arg
   1 5
<210> 699
   <211> 7
   <212> PRT
   <213> Artificial Sequence
<220>
   <223> SLC25A21
<400> 699
   Val Gly Leu Gln Ala Asn Arg
   1 5
<210> 700
   <211> 14
   <212> PRT
   <213> Artificial Sequence
<220>
   <223> SLC25A21
<400> 700
   Asn Thr Phe Ala Glu Gln Pro Ser Thr Val Gly Tyr Ala Arg
   1 5 10
<210> 701
   <211> 8
   <212> PRT
   <213> Artificial Sequence
<220>
   <223> SLC25A21
<400> 701
   Gly Leu Thr Ala Thr Leu Gly Arg
   1 5
<210> 702
   <211> 12
   <212> PRT
   <213> Artificial Sequence
<220>
   <223> SLC25A21
<400> 702
   Ile Gln Gly Pro Gln Pro Val Pro Gly Glu Ile Lys
   1 5 10
<210> 703
   <211> 5
   <212> PRT
   <213> Artificial Sequence
<220>
   <223> SLC25A21
<400> 703
   Gly Leu Leu Pro Lys
   1 5
<210> 704
   <211> 7
   <212> PRT
   <213> Artificial Sequence
<220>
   <223> SLC25A22
<400> 704
   Gln Ile Ser Leu Pro Ala Lys
   1 5
<210> 705
   <211> 9
   <212> PRT
   <213> Artificial Sequence
<220>
   <223> SLC25A22
<400> 705
   Leu Gln Asn Gln Gln Asn Gly Gln Arg
   1 5
<210> 706
   <211> 13
   <212> PRT
   <213> Artificial Sequence
<220>
   <223> SLC25A22
<400> 706
   Gly Ala Ala Val Asn Leu Thr Leu Val Thr Pro Glu Lys
   1 5 10
<210> 707
   <211> 8
   <212> PRT
   <213> Artificial Sequence
<220>
   <223> SLC25A22
<400> 707
   Leu Ala Ala Asn Asp Phe Phe Arg
   1 5
<210> 708
   <211> 5
   <212> PRT
   <213> Artificial Sequence
<220>
   <223> SLC25A22
<400> 708
   His Gln Leu Ser Lys
   1 5
<210> 709
   <211> 5
   <212> PRT
   <213> Artificial Sequence
<220>
   <223> SLC25A22
<400> 709
   Leu Thr Leu Leu Lys
   1 5
<210> 710
   <211> 8
   <212> PRT
   <213> Artificial Sequence
<220>
   <223> SLC25A22
<400> 710
   Ile Gln Leu Gln Asp Ala Gly Arg
   1 5
<210> 711
   <211> 7
   <212> PRT
   <213> Artificial Sequence
<220>
   <223> SLC25A22
<400> 711
   Gly Ile Ala Gly Leu Tyr Lys
   1 5
<210> 712
   <211> 6
   <212> PRT
   <213> Artificial Sequence
<220>
   <223> SLC25A22
<400> 712
   Leu Gln Ser Leu Gln Arg
   1               5
<210> 713
   <211> 9
   <212> PRT
   <213> Artificial Sequence
<220>
   <223> SLC25A22
<400> 713
   His Glu Gly Pro Ser Ala Phe Leu Lys
   1               5
<210> 714
   <211> 7
   <212> PRT
   <213> Artificial Sequence
<220>
   <223> SLC25A23
<400> 714
   Val Asp Val His Glu Leu Arg
   1               5
<210> 715
   <211> 5
   <212> PRT
   <213> Artificial Sequence
<220>
   <223> SLC25A23
<400> 715
   Gln Gly Leu Ala Arg
   1               5
<210> 716
   <211> 5
   <212> PRT
   <213> Artificial Sequence
<220>
   <223> SLC25A23
<400> 716
   Tyr Leu Gln Glu Arg
   1 5
<210> 717
   <211> 16
   <212> PRT
   <213> Artificial Sequence
<220>
   <223> SLC25A23
<400> 717
   Asn Gln Asp Gly His Ile Asp Val Ser Glu Ile Gln Gln Ser Phe Arg
   1 5 10 15
<210> 718
   <211> 13
   <212> PRT
   <213> Artificial Sequence
<220>
   <223> SLC25A23
<400> 718
   Ala Leu Gly Ile Ser Ile Ser Leu Glu Gln Ala Glu Lys
   1 5 10
<210> 719
   <211> 19
   <212> PRT
   <213> Artificial Sequence
<220>
   <223> SLC25A23
<400> 719
   Asp His Phe Leu Leu His Ser Leu Glu Asn Val Glu Asp Val Leu Tyr
   1 5 10 15
   Phe Trp Lys
<210> 720
   <211> 13
   <212> PRT
   <213> Artificial Sequence
<220>
   <223> SLC25A23
<400> 720
   Gln Leu Val Ala Gly Ala Val Ala Gly Ala Val Ser Arg
   1 5 10
<210> 721
   <211> 8
   <212> PRT
   <213> Artificial Sequence
<220>
   <223> SLC25A23
<400> 721
   Thr Gly Thr Ala Pro Leu Asp Arg
   1 5
<210> 722
   <211> 8
   <212> PRT
   <213> Artificial Sequence
<220>
   <223> SLC25A23
<400> 722
   Leu Asn Ile Leu Gly Gly Leu Arg
   1 5
<210> 723
   <211> 8
   <212> PRT
   <213> Artificial Sequence
<220>
   <223> SLC25A23
<400> 723
   Gly Asn Gly Ile Asn Val Leu Lys
   1 5
<210> 724
   <211> 8
   <212> PRT
   <213> Artificial Sequence
<220>
   <223> SLC25A23
<400> 724
   Ile Ala Pro Glu Ser Ala Ile Lys
   1 5
<210> 725
   <211> 24
   <212> PRT
   <213> Artificial Sequence
<220>
   <223> SLC25A23
<400> 725
   Gly Tyr Leu Pro Asn Val Leu Gly Ile Ile Pro Tyr Ala Gly Ile Asp
   1               5                   10                  15
   Leu Ala Val Tyr Glu Thr Leu Lys
               20
<210> 726
   <211> 8
   <212> PRT
   <213> Artificial Sequence
<220>
   <223> SLC25A23
<400> 726
   Gln Ala Leu Gly Val Thr Ser Arg
   1               5
<210> 727
   <211> 9
   <212> PRT
   <213> Artificial Sequence
<220>
   <223> SLC25A24
<400> 727
   Ile Phe Thr Thr Gly Asp Val Asn Lys
   1               5
<210> 728
   <211> 16
   <212> PRT
   <213> Artificial Sequence
<220>
   <223> SLC25A24
<400> 728
   Asp Tyr Phe Leu Phe Asn Pro Val Thr Asp Ile Glu Glu Ile Ile Arg
   1               5                   10                  15
<210> 729
   <211> 13
   <212> PRT
   <213> Artificial Sequence
<220>
   <223> SLC25A24
<400> 729
   Gln Leu Leu Ala Gly Gly Ile Ala Gly Ala Val Ser Arg
   1 5 10
<210> 730
   <211> 8
   <212> PRT
   <213> Artificial Sequence
<220>
   <223> SLC25A24
<400> 730
   Thr Ser Thr Ala Pro Leu Asp Arg
   1 5
<210> 731
   <211> 5
   <212> PRT
   <213> Artificial Sequence
<220>
   <223> SLC25A24
<400> 731
   Glu Gly Gly Ile Arg
   1 5
<210> 732
   <211> 8
   <212> PRT
   <213> Artificial Sequence
<220>
   <223> SLC25A24
<400> 732
   Gly Asn Gly Thr Asn Val Ile Lys
   1 5
<210> 733
   <211> 8
   <212> PRT
   <213> Artificial Sequence
<220>
   <223> SLC25A24
<400> 733
   Ile Ala Pro Glu Thr Ala Val Lys
   1 5
<210> 734
   <211> 6
   <212> PRT
   <213> Artificial Sequence
<220>
   <223> SLC25A24
<400> 734
   Ile Gly Thr Phe Glu Arg
   1 5
<210> 735
   <211> 5
   <212> PRT
   <213> Artificial Sequence
<220>
   <223> SLC25A24
<400> 735
   Leu Ala Val Gly Lys
   1 5
<210> 736
   <211> 9
   <212> PRT
   <213> Artificial Sequence
<220>
   <223> SLC25A24
<400> 736
   His Glu Gly Leu Gly Ala Phe Tyr Lys
   1 5
<210> 737
   <211> 24
   <212> PRT
   <213> Artificial Sequence
<220>
   <223> SLC25A24
<400> 737
   Gly Tyr Val Pro Asn Leu Leu Gly Ile Ile Pro Tyr Ala Gly Ile Asp
   1 5 10 15
   Leu Ala Val Tyr Glu Leu Leu Lys
   20
<210> 738
   <211> 9
   <212> PRT
   <213> Artificial Sequence
<220>
   <223> SLC25A24
<400> 738
   Ser Tyr Trp Leu Asp Asn Phe Ala Lys
   1 5
<210> 739
   <211> 8
   <212> PRT
   <213> Artificial Sequence
<220>
   <223> SLC25A24
<400> 739
   Glu Gly Ile Pro Gly Leu Tyr Arg
   1 5
<210> 740
   <211> 8
   <212> PRT
   <213> Artificial Sequence
<220>
   <223> SLC25A24
<400> 740
   Gln Thr Leu Gly Val Thr Gln Lys
   1 5
<210> 741
   <211> 7
   <212> PRT
   <213> Artificial Sequence
<220>
   <223> SLC25A25
<400> 741
   Ile Val Gln Ala Gly Asp Lys
   1 5
<210> 742
   <211> 5
   <212> PRT
   <213> Artificial Sequence
<220>
   <223> SLC25A25
<400> 742
   Asp Leu Gly Val Lys
   1 5
<210> 743
   <211> 8
   <212> PRT
   <213> Artificial Sequence
<220>
   <223> SLC25A25
<400> 743
   Ile Ser Glu Gln Gln Ala Glu Lys
   1 5
<210> 744
   <211> 19
   <212> PRT
   <213> Artificial Sequence
<220>
   <223> SLC25A25
<400> 744
   Asp Tyr His Leu Leu His Pro Val Glu Asn Ile Pro Glu Ile Ile Leu
   1 5 10 15
   Tyr Trp Lys
<210> 745
   <211> 22
   <212> PRT
   <213> Artificial Sequence
<220>
   <223> SLC25A25
<400> 745
   His Ser Thr Ile Phe Asp Val Gly Glu Asn Leu Thr Val Pro Asp Glu
   1 5 10 15
   Phe Thr Val Glu Glu Arg
   20
<210> 746
   <211> 13
   <212> PRT
   <213> Artificial Sequence
<220>
   <223> SLC25A25
<400> 746
   His Leu Val Ala Gly Gly Gly Ala Gly Ala Val Ser Arg
   1               5                   10
<210> 747
   <211> 8
   <212> PRT
   <213> Artificial Sequence
<220>
   <223> SLC25A25
<400> 747
   Gly Asn Gly Ile Asn Val Leu Lys
   1               5
<210> 748
   <211> 8
   <212> PRT
   <213> Artificial Sequence
<220>
   <223> SLC25A25
<400> 748
   Ile Ala Pro Glu Ser Ala Ile Lys
   1               5
<210> 749
   <211> 8
   <212> PRT
   <213> Artificial Sequence
<220>
   <223> SLC25A25
<400> 749
   Glu Gly Val Ala Ala Phe Tyr Lys
   1               5
<210> 750
   <211> 9
   <212> PRT
   <213> Artificial Sequence
<220>
   <223> SLC25A25
<400> 750
   Thr Glu Gly Ala Phe Gly Leu Tyr Arg
   1 5
<210> 751
   <211> 16
   <212> PRT
   <213> Artificial Sequence
<220>
   <223> SLC25A25
<400> 751
   Val Ile Pro Ala Val Ser Ile Ser Tyr Val Val Tyr Glu Asn Leu Lys
   1 5 10 15
<210> 752
   <211> 8
   <212> PRT
   <213> Artificial Sequence
<220>
   <223> SLC25A25
<400> 752
   Ile Thr Leu Gly Val Gln Ser Arg
   1 5
<210> 753
   <211> 7
   <212> PRT
   <213> Artificial Sequence
<220>
   <223> SLC25A26
<400> 753
   Val Pro Ser Glu Val Val Lys
   1 5
<210> 754
   <211> 8
   <212> PRT
   <213> Artificial Sequence
<220>
   <223> SLC25A26
<400> 754
   Ala Gln Val Ser Ala Ser Thr Arg
   1 5
<210> 755
   <211> 19
   <212> PRT
   <213> Artificial Sequence
<220>
   <223> SLC25A26
<400> 755
   Thr Phe Gln Ile Phe Ser Asn Ile Leu Tyr Glu Glu Gly Ile Gln Gly
   1 5 10 15
   Leu Tyr Arg
<210> 756
   <211> 5
   <212> PRT
   <213> Artificial Sequence
<220>
   <223> SLC25A26
<400> 756
   Ser Thr Val Leu Arg
   1 5
<210> 757
   <211> 7
   <212> PRT
   <213> Artificial Sequence
<220>
   <223> SLC25A27
<400> 757
   Leu Leu Pro Leu Thr Gln Arg
   1 5
<210> 758
   <211> 6
   <212> PRT
   <213> Artificial Sequence
<220>
   <223> SLC25A27
<400> 758
   Leu Gly Asp Gly Ala Arg
   1 5
<210> 759
   <211> 6
   <212> PRT
   <213> Artificial Sequence
<220>
   <223> SLC25A27
<400> 759
   Glu Ser Ala Pro Tyr Arg
   1 5
<210> 760
   <211> 13
   <212> PRT
   <213> Artificial Sequence
<220>
   <223> SLC25A27
<400> 760
   Thr Ala Leu Gly Ile Ile Glu Glu Glu Gly Phe Leu Lys
   1 5 10
<210> 761
   <211> 6
   <212> PRT
   <213> Artificial Sequence
<220>
   <223> SLC25A27
<400> 761
   Glu Val Val Phe Gly Lys
   1 5
<210> 762
   <211> 8
   <212> PRT
   <213> Artificial Sequence
<220>
   <223> SLC25A27
<400> 762
   Gly Val His His Ala Phe Ala Lys
   1 5
<210> 763
   <211> 8
   <212> PRT
   <213> Artificial Sequence
<220>
   <223> SLC25A27
<400> 763
   Ile Leu Ala Glu Gly Gly Ile Arg
   1 5
<210> 764
   <211> 5
   <212> PRT
   <213> Artificial Sequence
<220>
   <223> SLC25A27
<400> 764
   Gly Leu Leu Tyr Lys
   1 5
<210> 765
   <211> 13
   <212> PRT
   <213> Artificial Sequence

<220>
   <223> SLC25A28
<400> 765
   Ala Val Trp Gln Asn Glu Gly Ala Gly Ala Phe Tyr Arg
   1 5 10
<210> 766
   <211> 13
   <212> PRT
   <213> Artificial Sequence
<220>
   <223> SLC25A28
<400> 766
   Thr Val Tyr Gln Val Gly Gly Val Thr Ala Tyr Phe Arg
   1 5 10
<210> 767
   <211> 9
   <212> PRT
   <213> Artificial Sequence
<220>
   <223> SLC25A29
<400> 767
   Gln Glu Ser Val Leu Gly Leu Tyr Lys
   1 5
<210> 768
   <211> 10
   <212> PRT
   <213> Artificial Sequence
<220>
   <223> SLC25A29
<400> 768
   Leu Gln Leu Gln Asp Ala Gly Pro Ala Arg
   1 5 10
<210> 769
   <211> 17
   <212> PRT
   <213> Artificial Sequence
<220>
   <223> SLC25A29
<400> 769
   Glu Thr Pro Ser Phe Gly Val Tyr Phe Leu Thr Tyr Asp Ala Leu Thr
   1 5 10 15
   Arg
<210> 770
   <211> 7
   <212> PRT
   <213> Artificial Sequence
<220>
   <223> SLC25A29
<400> 770
   Leu Gln Ala Asp Gly Leu Arg
   1 5
<210> 771
   <211> 5
   <212> PRT
   <213> Artificial Sequence
<220>
   <223> SLC25A29
<400> 771
   Ala Glu Gly Trp Arg
   1 5
<210> 772
   <211> 26
   <212> PRT
   <213> Artificial Sequence
<220>
   <223> SLC25A29
<400> 772
   Gly Glu Glu Ala Gly Pro Glu Gly Glu Ala Val Pro Ala Ala Pro Ala
   1               5                   10                  15
   Gly Pro Ala Leu Ala Gln Pro Ser Ser Leu
               20                  25
<210> 773
   <211> 20
   <212> PRT
   <213> Artificial Sequence
<220>
   <223> SLC25A3
<400> 773
   Ala Asn Pro Phe Asn Thr Pro His Leu Gln Leu Val His Asp Gly Leu
   1               5                   10                  15
   Gly Asp Leu Arg
               20
<210> 774
   <211> 11
   <212> PRT
   <213> Artificial Sequence
<220>
   <223> SLC25A3
<400> 774
   Gly Ile Phe Asn Gly Phe Ser Val Thr Leu Lys
   1               5                   10
<210> 775
   <211> 12
   <212> PRT
   <213> Artificial Sequence
<220>
   <223> SLC25A3
<400> 775
   Ile Gln Thr Gln Pro Gly Tyr Ala Asn Thr Leu Arg
   1               5                   10
<210> 776
   <211> 5
   <212> PRT
   <213> Artificial Sequence
<220>
   <223> SLC25A3
<400> 776
   Glu Glu Gly Leu Lys
   1 5
<210> 777
   <211> 7
   <212> PRT
   <213> Artificial Sequence
<220>
   <223> SLC25A3
<400> 777
   Thr Val Glu Ala Leu Tyr Lys
   1 5
<210> 778
   <211> 11
   <212> PRT
   <213> Artificial Sequence
<220>
   <223> SLC25A30
<400> 778
   Leu Gln Ile Gln Gly Gln Thr Asn Asp Ala Lys
   1 5 10
<210> 779
   <211> 5
   <212> PRT
   <213> Artificial Sequence
<220>
   <223> SLC25A30
<400> 779
   Glu Glu Gly Leu Lys
   1 5
<210> 780
   <211> 8
   <212> PRT
   <213> Artificial Sequence
<220>
   <223> SLC25A30
<400> 780
   Gln Ala Ser Tyr Gly Thr Ile Lys
   1 5
<210> 781
   <211> 8
   <212> PRT
   <213> Artificial Sequence
<220>
   <223> SLC25A30
<400> 781
   Gly Val Ser Leu Thr Ala Gln Arg
   1 5
<210> 782
   <211> 9
   <212> PRT
   <213> Artificial Sequence
<220>
   <223> SLC25A30
<400> 782
   Asn Glu Gly Phe Phe Ala Leu Tyr Lys
   1 5
<210> 783
   <211> 8
   <212> PRT
   <213> Artificial Sequence
<220>
   <223> SLC25A30
<400> 783
   Gly Phe Trp Pro Asn Trp Leu Arg
   1 5
<210> 784
   <211> 13
   <212> PRT
   <213> Artificial Sequence
<220>
   <223> SLC25A31
<400> 784
   Asp Leu Leu Ala Gly Gly Val Ala Ala Ala Val Ser Lys
   1 5 10
<210> 785
   <211> 8
   <212> PRT
   <213> Artificial Sequence
<220>
   <223> SLC25A31
<400> 785
   Thr Ala Val Ala Pro Ile Glu Arg
   1 5
<210> 786
   <211> 10
   <212> PRT
   <213> Artificial Sequence
<220>
   <223> SLC25A31
<400> 786
   Leu Leu Leu Gln Val Gln Ala Ser Ser Lys
   1 5 10
<210> 787
   <211> 7
   <212> PRT
   <213> Artificial Sequence
<220>
   <223> SLC25A31
<400> 787
   Gln Ile Ser Pro Glu Ala Arg
   1 5
<210> 788
   <211> 9
   <212> PRT
   <213> Artificial Sequence
<220>
   <223> SLC25A31
<400> 788
   Glu Gln Gly Phe Phe Ser Phe Trp Arg
   1 5
<210> 789
   <211> 8
   <212> PRT
   <213> Artificial Sequence
<220>
   <223> SLC25A31
<400> 789
   Gly Asn Leu Ala Asn Val Ile Arg
   1 5
<210> 790
   <211> 12
   <212> PRT
   <213> Artificial Sequence
<220>
   <223> SLC25A31
<400> 790
   Tyr Phe Pro Thr Gln Ala Leu Asn Phe Ala Phe Lys
   1 5 10
<210> 791
   <211> 7
   <212> PRT
   <213> Artificial Sequence
<220>
   <223> SLC25A31
<400> 791
   Leu Gly Val Asp Ile Gly Lys
   1 5
<210> 792
   <211> 22
   <212> PRT
   <213> Artificial Sequence
<220>
   <223> SLC25A31
<400> 792
   Ser Asp Gly Ile Ala Gly Leu Tyr Gln Gly Phe Gly Val Ser Val Gln
   1 5 10 15
   Gly Ile Ile Val Tyr Arg
   20
<210> 793
   <211> 11
   <212> PRT
   <213> Artificial Sequence
<220>
   <223> SLC25A31
<400> 793
   Ala Ser Tyr Phe Gly Ala Tyr Asp Thr Val Lys
   1 5 10
<210> 794
   <211> 12
   <212> PRT
   <213> Artificial Sequence
<220>
   <223> SLC25A31
<400> 794
   Ile Tyr Gln His Glu Gly Ile Ser Ser Phe Phe Arg
   1 5 10
<210> 795
   <211> 8
   <212> PRT
   <213> Artificial Sequence
<220>
   <223> SLC25A31
<400> 795
   Gly Ala Phe Ser Asn Val Leu Arg
   1 5
<210> 796
   <211> 13
   <212> PRT
   <213> Artificial Sequence
<220>
   <223> SLC25A31
<400> 796
   Gly Thr Gly Gly Ala Leu Val Leu Val Leu Tyr Asp Lys
   1 5 10
<210> 797
   <211> 10
   <212> PRT
   <213> Artificial Sequence
<220>
   <223> SLC25A31
<400> 797
   Glu Phe Phe His Ile Asp Ile Gly Gly Arg
   1 5 10
<210> 798
   <211> 25
   <212> PRT
   <213> Artificial Sequence
<220>
   <223> SLC25A32
<400> 798
   Tyr Glu Asn Leu Ile Ala Gly Val Ser Gly Gly Val Leu Ser Asn Leu
   1 5 10 15
   Ala Leu His Pro Leu Asp Leu Val Lys
   20 25
<210> 799
   <211> 5
   <212> PRT
   <213> Artificial Sequence
<220>
   <223> SLC25A32
<400> 799
   Leu Asp Gly Leu Arg
   1 5
<210> 800
   <211> 5
   <212> PRT
   <213> Artificial Sequence
<220>
   <223> SLC25A32
<400> 800
   Tyr Glu Gly Val Arg
   1 5
<210> 801
   <211> 7
   <212> PRT
   <213> Artificial Sequence
<220>
   <223> SLC25A32
<400> 801
   Tyr Asn Gln His Ile Asn Arg
   1 5
<210> 802
   <211> 10
   <212> PRT
   <213> Artificial Sequence
<220>
   <223> SLC25A34
<400> 802
   Leu Gln Leu Gln Gly Glu Leu Gln Ala Arg
   1 5 10
<210> 803
   <211> 9
   <212> PRT
   <213> Artificial Sequence
<220>
   <223> SLC25A34
<400> 803
   Ala Asp Gly Leu Trp Gly Leu Gln Lys
   1 5
<210> 804
   <211> 17
   <212> PRT
   <213> Artificial Sequence
<220>
   <223> SLC25A34
<400> 804
   Gln Gln Gly Leu Leu Gly Leu Trp Gln Gly Val Gly Gly Ala Val Pro
   1 5 10 15
   Arg
<210> 805
   <211> 5
   <212> PRT
   <213> Artificial Sequence
<220>
   <223> SLC25A34
<400> 805
   Ala Trp Val Gln Lys
   1 5
<210> 806
   <211> 11
   <212> PRT
   <213> Artificial Sequence
<220>
   <223> SLC25A34
<400> 806
   Leu Tyr Asn Gln Pro Val Asp Thr Ala Gly Arg
   1 5 10
<210> 807
   <211> 9
   <212> PRT
   <213> Artificial Sequence
<220>
   <223> SLC25A34
<400> 807
   Gln Glu Gly Pro Leu Ala Leu Tyr Lys
   1 5
<210> 808
   <211> 8
   <212> PRT
   <213> Artificial Sequence
<220>
   <223> SLC25A34
<400> 808
   Gly Leu Gly Pro Ala Tyr Leu Arg
   1 5
<210> 809
   <211> 11
   <212> PRT
   <213> Artificial Sequence
<220>
   <223> SLC25A35
<400> 809
   Asn Val Phe His Ala Phe Ile Thr Ile Gly Lys
   1 5 10
<210> 810
   <211> 9
   <212> PRT
   <213> Artificial Sequence
<220>
   <223> SLC25A35
<400> 810
   Val Asp Gly Leu Ala Ala Leu Gln Lys
   1 5
<210> 811
   <211> 24
   <212> PRT
   <213> Artificial Sequence
<220>
   <223> SLC25A35
<400> 811
   Leu Gly Thr Tyr Gly Leu Ala Glu Ala Gly Gly Tyr Leu His Thr Ala
   1               5                   10                  15
   Glu Gly Thr His Ser Pro Ala Arg
               20
<210> 812
   <211> 18
   <212> PRT
   <213> Artificial Sequence
<220>
   <223> SLC25A35
<400> 812
   Thr His Leu Gln Ala Gln Ala Ala Ser Glu Ile Ala Val Gly His Gln
   1               5                   10                  15
   Tyr Lys
<210> 813
   <211> 8
   <212> PRT
   <213> Artificial Sequence
<220>
   <223> SLC25A35
<400> 813
   His Gly Leu Val Gly Leu Trp Arg
   1               5
<210> 814
   <211> 8
   <212> PRT
   <213> Artificial Sequence
<220>
   <223> SLC25A35
<400> 814
   Gly Ala Leu Gly Gly Leu Pro Arg
   1               5
<210> 815
   <211> 15
   <212> PRT
   <213> Artificial Sequence
<220>
   <223> SLC25A35
<400> 815
   Asp Leu Leu Ser Gln Trp Glu Ile Phe Pro Pro Gln Ser Trp Lys
   1 5 10 15
<210> 816
   <211> 11
   <212> PRT
   <213> Artificial Sequence
<220>
   <223> SLC25A35
<400> 816
   Leu Tyr Asn Gln Pro Thr Asp Ala Gln Gly Lys
   1 5 10
<210> 817
   <211> 5
   <212> PRT
   <213> Artificial Sequence
<220>
   <223> SLC25A36
<400> 817
   Val Ile Leu Glu Lys
   1 5
<210> 818
   <211> 13
   <212> PRT
   <213> Artificial Sequence
<220>
   <223> SLC25A36
<400> 818
   Gly Leu Gly Pro Asn Leu Val Gly Val Ala Pro Ser Arg
   1 5 10
<210> 819
   <211> 6
   <212> PRT
   <213> Artificial Sequence
<220>
   <223> SLC25A36
<400> 819
   Leu Gln Leu Asp Ala Arg
   1 5
<210> 820
   <211> 5
   <212> PRT
   <213> Artificial Sequence
<220>
   <223> SLC25A36
<400> 820
   Leu Leu Glu Tyr Lys
   1 5
<210> 821
   <211> 20
   <212> PRT
   <213> Artificial Sequence
<220>
   <223> SLC25A36
<400> 821
   Ser Phe Phe Gln Thr Leu Ser Leu Leu Val Gln Glu Glu Gly Tyr Gly
   1 5 10 15
   Ser Leu Tyr Arg
   20
<210> 822
   <211> 8
   <212> PRT
   <213> Artificial Sequence
<220>
   <223> SLC25A36
<400> 822
   Gly Leu Thr Thr His Leu Val Arg
   1 5
<210> 823
   <211> 11
   <212> PRT
   <213> Artificial Sequence
<220>
   <223> SLC25A37
<400> 823
   Ser Gly Ser Val Gly Ser Gln Ala Val Ala Arg
   1 5 10
<210> 824
   <211> 11
   <212> PRT
   <213> Artificial Sequence
<220>
   <223> SLC25A37
<400> 824
   Ala Gln Tyr Thr Ser Ile Tyr Gly Ala Leu Lys
   1 5 10
<210> 825
   <211> 9
   <212> PRT
   <213> Artificial Sequence
<220>
   <223> SLC25A37
<400> 825
   Thr Glu Gly Leu Gly Ala Phe Tyr Arg
   1 5
<210> 826
   <211> 13
   <212> PRT
   <213> Artificial Sequence
<220>
   <223> SLC25A37
<400> 826
   Thr Val Tyr Gln Leu Asn Gly Leu Pro Ala Thr Ser Lys
   1 5 10
<210> 827
   <211> 6
   <212> PRT
   <213> Artificial Sequence
<220>
   <223> SLC25A4
<400> 827
   Gln Ile Ser Ala Glu Lys
   1 5
<210> 828
   <211> 9
   <212> PRT
   <213> Artificial Sequence
<220>
   <223> SLC25A4
<400> 828
   Glu Gln Gly Phe Leu Ser Phe Trp Arg
   1 5
<210> 829
   <211> 5
   <212> PRT
   <213> Artificial Sequence
<220>
   <223> SLC25A4
<400> 829
   Ser Asp Gly Leu Arg
   1 5
<210> 830
   <211> 6
   <212> PRT
   <213> Artificial Sequence
<220>
   <223> SLC25A5
<400> 830
   Gln Ile Thr Ala Asp Lys
   1 5
<210> 831
   <211> 5
   <212> PRT
   <213> Artificial Sequence
<220>
   <223> SLC25A5
<400> 831
   Ser Asp Gly Ile Lys
   1 5
<210> 832
   <211> 6
   <212> PRT
   <213> Artificial Sequence
<220>
   <223> SLC25A6
<400> 832
   Gln Ile Ala Ala Asp Lys
   1 5
<210> 833
   <211> 9
   <212> PRT
   <213> Artificial Sequence
<220>
   <223> SLC25A6
<400> 833
   Glu Gln Gly Val Leu Ser Phe Trp Arg
   1 5
<210> 834
   <211> 5
   <212> PRT
   <213> Artificial Sequence
<220>
   <223> SLC25A6
<400> 834
   Ser Asp Gly Ile Arg
   1 5
<210> 835
   <211> 9
   <212> PRT
   <213> Artificial Sequence
<220>
   <223> SLC25A7
<400> 835
   Glu Thr Ala Pro Ser Leu Gly Ser Lys
   1 5
<210> 836
   <211> 9
   <212> PRT
   <213> Artificial Sequence
<220>
   <223> SLC25A7
<400> 836
   Tyr Thr Gly Thr Tyr Asn Ala Tyr Arg
   1 5
<210> 837
   <211> 13
   <212> PRT
   <213> Artificial Sequence
<220>
   <223> SLC25A7
<400> 837
   Ile Ile Ala Thr Thr Glu Gly Leu Thr Gly Leu Trp Lys
   1               5                   10
<210> 838
   <211> 5
   <212> PRT
   <213> Artificial Sequence
<220>
   <223> SLC25A7
<400> 838
   Glu Ala Phe Val Lys
   1               5
<210> 839
   <211> 10
   <212> PRT
   <213> Artificial Sequence
<220>
   <223> SLC25A7
<400> 839
   Phe Ile Asn Ser Pro Pro Gly Gln Tyr Lys
   1               5                   10
<210> 840
   <211> 12
   <212> PRT
   <213> Artificial Sequence
<220>
   <223> SLC25A8
<400> 840
   Leu Gln Ile Gln Gly Glu Ser Gln Gly Pro Val Arg
   1               5                   10
<210> 841
   <211> 5
   <212> PRT
   <213> Artificial Sequence
<220>
   <223> SLC25A8
<400> 841
   Gln Phe Tyr Thr Lys
   1 5
<210> 842
   <211> 10
   <212> PRT
   <213> Artificial Sequence
<220>
   <223> SLC25A8
<400> 842
   Gly Ser Glu His Ala Ser Ile Gly Ser Arg
   1 5 10
<210> 843
   <211> 6
   <212> PRT
   <213> Artificial Sequence
<220>
   <223> SLC25A8
<400> 843
   Phe Gln Ala Gln Ala Arg
   1 5
<210> 844
   <211> 5
   <212> PRT
   <213> Artificial Sequence
<220>
   <223> SLC25A8
<400> 844
   Glu Glu Gly Phe Arg
   1 5
<210> 845
   <211> 5
   <212> PRT
   <213> Artificial Sequence
<220>
   <223> SLC25A8
<400> 845
   Asp Ala Leu Leu Lys
   1 5
<210> 846
   <211> 14
   <212> PRT
   <213> Artificial Sequence
<220>
   <223> SLC25A9
<400> 846
   Leu Gln Ile Gln Gly Glu Asn Gln Ala Val Gln Thr Ala Arg
   1 5 10
<210> 847
   <211> 5
   <212> PRT
   <213> Artificial Sequence
<220>
   <223> SLC25A9
<400> 847
   Leu Val Gln Tyr Arg
   1 5
<210> 848
   <211> 6
   <212> PRT
   <213> Artificial Sequence
<220>
   <223> SLC25A9
<400> 848
   Gln Val Tyr Thr Pro Lys
   1 5
<210> 849
   <211> 11
   <212> PRT
   <213> Artificial Sequence
<220>
   <223> SLC26A1
<400> 849
   Ala Leu Val Gln Asp Leu Leu Pro Ala Thr Arg
   1 5 10
<210> 850
   <211> 6
   <212> PRT
   <213> Artificial Sequence
<220>
   <223> SLC26A1
<400> 850
   His Leu Leu Gly Val Arg
   1 5
<210> 851
   <211> 5
   <212> PRT
   <213> Artificial Sequence
<220>
   <223> SLC26A1
<400> 851
   Glu Leu Ser Asp Arg
   1 5
<210> 852
   <211> 7
   <212> PRT
   <213> Artificial Sequence
<220>
   <223> SLC26A1
<400> 852
   Ser His Gly Tyr Ser Val Arg
   1 5
<210> 853
   <211> 6
   <212> PRT
   <213> Artificial Sequence
<220>
   <223> SLC26A1
<400> 853
   Thr Ala Leu Leu Ala Arg
   1 5
<210> 854
   <211> 23
   <212> PRT
   <213> Artificial Sequence
<220>
   <223> SLC26A1
<400> B54
<210> 855
   <211> 10
   <212> PRT
   <213> Artificial Sequence
<220>
   <223> SLC26A1
<400> 855
   Phe Gly Gly Pro Leu Tyr Tyr Ala Asn Lys
   1 5 10
<210> 856
   <211> 5
   <212> PRT
   <213> Artificial Sequence
<220>
   <223> SLC26A1
<400> 856
   Asp Ile Leu Ser Arg
   1 5
<210> 857
   <211> 29
   <212> PRT
   <213> Artificial Sequence
<220>
   <223> SLC26A1
<400> 857
   Gly Gly Phe Leu Gly Glu Gly Pro Gly Asp Thr Ala Glu Glu Glu Gln
   1 5 10 15
   Leu Phe Leu Ser Val His Asp Ala Val Gln Thr Ala Arg
   20 25
<210> 858
   <211> 9
   <212> PRT
   <213> Artificial Sequence
<220>
   <223> SLC26A1
<400> 858
   Glu Leu Glu Ala Thr Asp Ala His Leu
   1 5
<210> 859
   <211> 7
   <212> PRT
   <213> Artificial Sequence
<220>
   <223> SLC26A10
<400> 859
   Ser Leu Gly Ser Ala Phe Lys
   1 5
<210> 860
   <211> 15
   <212> PRT
   <213> Artificial Sequence
<220>
   <223> SLC26A10
<400> 860
   Leu Val Pro Glu Pro Leu Val Gly Asn Leu Ser Gly Ile Glu Lys
   1 5 10 15
<210> 861
   <211> 7
   <212> PRT
   <213> Artificial Sequence
<220> .
   <223> SLC26A10
<400> 861
   Glu Gln Leu Asp Ala Gln Arg
   1 5
<210> 862
   <211> 25
   <212> PRT
   <213> Artificial Sequence
<220>
   <223> SLC26A10
<400> 862
   Ala Leu Thr Ser Gly Ala Ala Leu His Val Leu Leu Ser Gln Leu Pro
   1               5                   10                  15
   Ser Leu Leu Gly Leu Ser Leu Pro Arg
               20                  25
<210> 863
   <211> 11
   <212> PRT
   <213> Artificial Sequence
<220>
   <223> SLC26A10
<400> 863
   Thr Leu Ala Ser Leu Leu Thr Ala Leu Pro Arg
   1               5                   10
<210> 864
   <211> 20
   <212> PRT
   <213> Artificial Sequence
<220>
   <223> SLC26A10
<400> 864
   Ser Ser Pro Ala Glu Leu Thr Ile Ser Ala Leu Ser Leu Ala Leu Leu
   1               5                   10                  15
   Val Pro Val Lys
               20
<210> 865
   <211> 5
   <212> PRT
   <213> Artificial Sequence
<220>
   <223> SLC26A10
<400> 865
   Glu Leu Asn Val Arg
   1               5
<210> 866
   <211> 26
   <212> PRT
   <213> Artificial Sequence
<220>
   <223> SLC26A10
<400> 866
   Tyr Gln Val Gln Ile Val Gly Leu Leu Pro Gly Gly Phe Pro Gln Pro
   1 5 10 15
   Leu Leu Pro Asn Leu Ala Glu Leu Pro Arg
   20 25
<210> 867
   <211> 26
   <212> PRT
   <213> Artificial Sequence
<220>
   <223> SLC26A10
<400> 867
   Ile Leu Ala Asp Ser Leu Pro Ile Ala Leu Val Ser Phe Ala Val Ser
   1 5 10 15
   Ala Ser Leu Ala Ser Ile His Ala Asp Lys
   20 25
<210> 868
   <211> 18
   <212> PRT
   <213> Artificial Sequence
<220>
   <223> SLC26A10
<400> 868
   Val Val Val Leu Asp Phe Ser Gly Val Thr Phe Ala Asp Ala Ala Gly
   1 5 10 15
   Ala Arg
<210> 869
   <211> 6
   <212> PRT
   <213> Artificial Sequence
<220>
   <223> SLC26A10
<400> 869
   Glu Val Val Gln Val Arg
   1 5
<210> 870
   <211> 6
   <212> PRT
   <213> Artificial Sequence
<220>
   <223> SLC26A10
<400> 870
   Val Gly Leu Leu Asp Arg
   1               5
<210> 871
   <211> 23
   <212> PRT
   <213> Artificial Sequence
<220>
   <223> SLC26A10
<400> 871
   Val Thr Pro Asp Gln Leu Phe Val Ser Val Gln Asp Ala Ala Ala Tyr
   1               5                   10                  15
   Ala Leu Gly Ser Leu Leu Arg
               20
<210> 872
   <211> 14
   <212> PRT
   <213> Artificial Sequence
<220>
   <223> SLC26A11
<400> 872
   Leu Gly Phe Leu Leu Asp Phe Ile Ser Tyr Pro Val Ile Lys
   1               5                   10
<210> 873
   <211> 16
   <212> PRT
   <213> Artificial Sequence
<220>
   <223> SLC26A11
<400> 873
   Gly Phe Thr Ser Ala Ala Ala Val Thr Ile Gly Phe Gly Gln Ile Lys
   1               5                   10                  15
<210> 874
   <211> 5
   <212> PRT
   <213> Artificial Sequence
<220>
   <223> SLC26A11
<400> 874
   Ile Ala Glu Thr Arg
   1 5
<210> 875
   <211> 10
   <212> PRT
   <213> Artificial Sequence
<220>
   <223> SLC26A11
<400> 875
   Gly Leu Val Trp Ala Ala Thr Thr Ala Arg
   1 5 10
<210> 876
   <211> 6
   <212> PRT
   <213> Artificial Sequence
<220>
   <223> SLC26A11
<400> 876
   Glu Glu Ile Leu Ser Arg
   1 5
<210> 877
   <211> 8
   <212> PRT
   <213> Artificial Sequence
<220>
   <223> SLC26A11
<400> 877
   Ala Leu Glu Val Ser Pro Pro Arg
   1 5
<210> 878
   <211> 16
   <212> PRT
   <213> Artificial Sequence
<220>
   <223> SLC26A11
<400> 878
   Gln Gly Val Ala Leu Ala Phe Val Gly Leu Gln Val Pro Val Leu Arg
   1 5 10 15
<210> 879
   <211> 8
   <212> ?RT
   **<213>** Artificial Sequence
<220>
   <223> SLC26A11
<400> 879
   Val Leu Leu Ser Ala Asp Leu Lys
   1 5
<210> 880
   <211> 8
   <212> PRT
   <213> Artificial Sequence
<220>
   <223> SLC26A11
<400> 880
   Glu Asp Ser Ile Leu Asp Gln Lys
   1 5
<210> 881
   <211> 5
   <212> PRT
   <213> Artificial Sequence
<220>
   <223> SLC26A11
<400> 881
   Val Ala Leu Leu Lys
   1 5
<210> 882
   <211> 8
   <212> PRT
   <213> Artificial Sequence
<220>
   <223> SLC26A2
<400> 882
   Glu Gln His Asn Val Ser Pro Arg
   1 5
<210> 883
   <211> 19
   <212> PRT
   <213> Artificial Sequence
<220>
   <223> SLC26A2
<400> 883
   Asp Ser Ala Glu Gly Asn Asp Ser Tyr Pro Ser Gly Ile His Leu Glu
   1 5 10 15
   Leu Gln Arg
<210> 884
   <211> 7
   <212> PRT
   <213> Artificial Sequence
<220>
   <223> SLC26A2
<400> 884
   Glu Ser Ser Thr Asp Phe Lys
   1 5
<210> 885
   <211> 5
   <212> PRT
   <213> Artificial Sequence
<220>
   <223> SLC26A2
<400> 885
   Ile Leu Ile Glu Arg
   1 5
<210> 886
   <211> 6
   <212> PRT
   <213> Artificial Sequence
<220>
   <223> SLC26A2
<400> 886
   Ser Asp Thr Asn Phe Lys
   1 5
<210> 887
   <211> 7
   <212> PRT
   <213> Artificial Sequence
<220>
   <223> SLC26A2
<400> 887
   Glu Leu Asn Glu His Phe Lys
   1 5
<210> 888
   <211> 12
   <212> PRT
   <213> Artificial Sequence
<220>
   <223> 5LC26A2
<400> 888
   Ser Val Leu Gly Val Ile Thr Ile Val Asn Leu Arg
   1 5 10
<210> 889
   <211> 20
   <212> PRT
   <213> Artificial Sequence
<220>
   <223> SLC26A2
<400> 889
   Ser Ser Leu Leu Gly Leu Val Glu Glu Ser Glu Val Phe Glu Ser Val
   1 5 10 15
   Ser Ala Tyr Lys
   20
<210> 890
   <211> 10
   <212> PRT
   <213> Artificial Sequence
<220>
   <223> SLC26A2
<400> 890
   Phe Val Ala Pro Leu Tyr Tyr Ile Asn Lys
   1 5 10
<210> 891
   <211> 5
   <212> PRT
   <213> Artificial Sequence
<220>
   <223> SLC26A2
<400> 891
   Ser Ala Leu Tyr Lys
   1 5
<210> 892
   <211> 9
   <212> PRT
   <213> Artificial Sequence
<220>
   <223> SLC26A2
<400> 892
   Gln Thr Val Asn Pro Ile Leu Ile Lys
   1 5
<210> 893
   <211> 7
   <212> PRT
   <213> Artificial Sequence
<220>
   <223> SLC26A3
<400> 893
   Thr Phe Leu Asp His Leu Lys
   1 5
<210> 894
   <211> 5
   <212> PRT
   <213> Artificial Sequence
<220>
   <223> SLC26A3
<400> 894
   Ala Val Pro Asp Arg
   1 5
<210> 895
   <211> 21
   <212> PRT
   <213> Artificial Sequence
<220>
   <223> SLC26A3
<400> 895
   Asn Ala Thr Thr Leu Gly Leu Pro Asn Asn Ser Asn Asn Ser Ser Leu
   1 5 10 15
   Leu Asp Asp Glu Arg
   20
<210> 896
   <211> 18
   <212> PRT
   <213> Artificial Sequence
<220>
   <223> SLC26A3
<400> 896
   Phe Ile Phe Gln Leu Thr Val Pro Ser His Thr Asp Pro Val Ser Ile
   1 5 10 15
   Phe Lys

<210> 897
   <211> 11
   <212> PRT
   <213> Artificial Sequence
<220>
   <223> SLC26A3
<400> 897
   Val Leu Tyr Ser Val Phe Ser Gln Ile Glu Lys
   1 5 10
<210> 898
   <211> 10
   <212> PRT
   <213> Artificial Sequence
<220>
   <223> SLC26A3
<400> 898
   Ser Ala Val Gln Glu Ser Thr Gly Gly Lys
   1 5 10
<210> 899
   <211> 5
   <212> PRT
   <213> Artificial Sequence
<220>
   <223> SLC26A3
<400> 899
   Thr Gln Phe Pro Lys
   1 5
<210> 900
   <211> 5
   <212> PRT
   <213> Artificial Sequence
<220>
   <223> SLC26A3
<400> 900
   Thr Asn Ile Tyr Lys
   1 5
<210> 901
   <211> 9
   <212> PRT
   <213> Artificial Sequence
<220>
   <223> SLC26A3
<400> 901
   Gln Gly Leu Leu Gln Val Thr Pro Lys
   1 5
<210> 902
   <211> 8
   <212> PRT
   <213> Artificial Sequence
<220>
   <223> SLC26A3
<400> 902
   Ser Ile Leu Gln Glu Phe Ile Arg
   1 5
<210> 903
   <211> 15
   <212> PRT
   <213> Artificial Sequence
<220>
   <223> SLC26A3
<400> 903
   Val Asp Val Tyr Ile Val Gly Thr Asp Asp Asp Phe Ile Glu Lys
   1 5 10 15
<210> 904
   <211> 7
   <212> PRT
   <213> Artificial Sequence
<220>
   <223> SLC26A3
<400> 904
   Phe Asn Pro Ser Gln Glu Lys
   1               5
<210> 905
   <211> 12
   <212> PRT
   <213> Artificial Sequence
<220>
   <223> SLC26A3
<400> 905
   Ile Asp Phe Thr Ile Asn Thr Asn Gly Gly Leu Arg
   1               5                   10
<210> 906
   <211> 9
   <212> PRT
   <213> Artificial Sequence
<220>
   <223> SLC26A3
<400> 906
   Val Tyr Glu Val Pro Val Glu Thr Lys
   1               5
<210> 907
   <211> 5
   <212> PRT
   <213> Artificial Sequence
<220>
   <223> SLC26A4
<400> 907
   Glu Ser Leu Ala Lys
   1               5
<210> 908
   <211> 11
   <212> PRT
   <213> Artificial Sequence
<220>
   <223> SLC26A4
<400> 908
   Thr Leu Val Pro Ile Leu Glu Trp Leu Pro Lys
   1 5 10
<210> 909
   <211> 5
   <212> PRT
   <213> Artificial Sequence
<220>
   <223> SLC26A4
<400> 909
   Glu Leu Asn Asp Arg
   1 5
<210> 910
   <211> 8
   <212> PRT
   <213> Artificial Sequence
<220>
   <223> SLC26A4
<400> 910
   Asn Tyr Asn Ala Gly Ile Val Lys
   1 5
<210> 911
   <211> 9
   <212> PRT
   <213> Artificial Sequence
<220>
   <223> SLC26A4
<400> 911
   Asn Ile Glu Glu Pro Gln Gly Val Lys
   1 5
<210> 912
   <211> 9
   <212> PRT
   <213> Artificial Sequence
<220>
   <223> SLC26A4
<400> 912
   Ser Thr Val Gly Phe Asp Ala Ile Arg
   1 5
<210> 913
   <211> 15
   <212> PRT
   <213> Artificial Sequence
<220>
   <223> SLC26A4
<400> 913
   Glu Ile Glu Ile Gln Val Asp Trp Asn Ser Glu Leu Pro Val Lys
   1 5 10 15
<210> 914
   <211> 5
   <212> PRT
   <213> Artificial Sequence
<220>
   <223> SLC26A4
<400> 914
   Val Asn Val Pro Lys
   1 5
<210> 915
   <211> 8
   <212> PRT
   <213> Artificial Sequence
<220>
   <223> SLC26A5
<400> 915
   Val Pro Asp Ser Ile Ala Asp Lys
   1 5
<210> 916
   <211> 6
   <212> PRT
   <213> Artificial Sequence
<220>
   <223> SLC26A5
<400> 916
   Trp Leu Pro Ala Tyr Lys
   1 5
<210> 917
   <211> 5
   <212> PRT
   <213> Artificial Sequence
<220>
   <223> SLC26A5
<400> 917
   Glu Phe Asn Glu Arg
   1               5
<210> 918
   <211> 6
   <212> PRT
   <213> Artificial Sequence
<220>
   <223> SLC26A6
<400> 918
   Trp Gly Ser Ala Pro Arg
   1               5
<210> 919
   <211> 5
   <212> PRT
   <213> Artificial Sequence
<220>
   <223> SLC26A6
<400> 919
   Thr His Gln Trp Arg
   1               5
<210> 920
   <211> 17
   <212> PRT
   <213> Artificial Sequence
<220>
   <223> SLC26A6
<400> 920
   Ala Tyr Ala Leu Leu Leu Gln His Leu Pro Val Leu Val Trp Leu Pro
   1               5                   10                  15
   Arg
<210> 921
   <211> 16
   <212> PRT
   <213> Artificial Sequence
<220>
   <223> SLC26A6
<400> 921
   Gly Tyr Thr Thr Ala Ala Ala Val Gln Val Phe Val Ser Gln Leu Lys
   1               5                   10                  15
<210> 922
   <211> 5
   <212> PRT
   <213> Artificial Sequence
<220>
   <223> SLC26A6
<400> 922
   Leu Pro Gln Ser Lys
   1               5
<210> 923
   <211> 26
   <212> PRT
   <213> Artificial Sequence
<220>
   <223> SLC26A6
<400> 923
   Phe Glu Val Asp Val Val Gly Asn Ile Pro Ala Gly Leu Val Pro Pro
   1               5                   10                  15
   Val Ala Pro Asn Thr Gln Leu Phe Ser Lys
               20                  25
<210> 924
   <211> 9
   <212> PRT
   <213> Artificial Sequence
<220>
   <223> SLC26A6
<400> 924
   Asp Val Ala Glu Tyr Ser Glu Ala Lys
   1               5
<210> 925
   <211> 18
   <212> PRT
   <213> Artificial Sequence
<220>
   <223> SLCA26A6
<400> 925
   Ser Ser Ala Thr Val Tyr Phe Ala Asn Ala Glu Phe Tyr Ser Asp Ala
   1 5 10 15
   Leu Lys
<210> 926
   <211> 8
   <212> PRT
   <213> Artificial Sequence
<220>
   <223> SLC26A6
<400> 926
   Ala Pro Asp Gly Ser Thr Leu Lys
   1 5
<210> 927
   <211> 7
   <212> PRT
   <213> Artificial Sequence
<220>
   <223> SLC26A6
<400> 927
   Asn Ile Phe His Asp Phe Arg
   1 5
<210> 928
   <211> 18
   <212> PRT
   <213> Artificial Sequence
<220>
   <223> SLC26A7
<400> 928
   Leu Glu Ala Leu Leu Leu Ser Leu Leu Ser Ile Val Val Leu Val Leu
   1 5 10 15
   Val Lys
<210> 929
   <211> 10
   <212> PRT
   <213> Artificial Sequence
<220>
   <223> SLC26A7
<400> 929
   Thr Ala Gly Leu Tyr Ser Thr Gly Ala Lys
   1 5 10
<210> 930
   <211> 14
   <212> PRT
   <213> Artificial Sequence
<220>
   <223> SLC26A8
<400> 930
   Ile Leu Leu Leu Gly Gln Ile Pro Asn Thr Asn Ile Tyr Arg
   1 5 10
<210> 931
   <211> 9
   <212> PRT
   <213> Artificial Sequence
<220>
   <223> SLC26A8
<400> 931
   Glu Ile Ile Thr Ile Pro Gly Val Lys
   1 5
<210> 932
   <211> 6
   <212> PRT
   <213> Artificial Sequence
<220>
   <223> SLC26A8
<400> 932
   Ile Leu Tyr Thr Glu Arg
   1 5
<210> 933
   <211> 5
   <212> PRT
   <213> Artificial Sequence
<220>
   <223> SLC26A9
<400> 933
   Tyr Val Val Asp Arg
   1 5
<210> 934
   <211> 7
   <212> PRT
   <213> Artificial Sequence
<220>
   <223> SLC26A9
<400> 934
   Thr Tyr Pro Val Gly Glu Lys
   1 5
<210> 935
   <211> 15
   <212> PRT
   <213> Artificial Sequence
<220>
   <223> SLC26A9
<400> 935
   Ala Val Val Phe Gly Leu Leu Pro Val Leu Ser Trp Leu Pro Lys
   1 5 10 15
<210> 936
   <211> 24
   <212> PRT
   <213> Artificial Sequence
<220>
   <223> SLC26A9
<400> 936
   Asn Leu Pro His Thr Asn Ile Ala Ser Leu Ile Phe Ala Leu Ile Ser
   1 5 10 15
   Gly Ala Phe Leu Val Leu Val Lys
   20
<210> 937
   <211> 5
   <212> PRT
   <213> Artificial Sequence
<220>
   <223> SLC26A9
<400> 937
   Glu Leu Asn Ala Arg
   1 5
<210> 938
   <211> 14
   <212> PRT
   <213> Artificial Sequence
<220>
   <223> SLC26A9
<400> 938
   Gly Phe Pro Thr Pro Val Ser Pro Val Val Ser Gln Trp Lys
   1 5 10
<210> 939
   <211> 5
   <212> PRT
   <213> Artificial Sequence
<220>
   <223> SLC26A9
<400> 939
   Thr Leu Ala Asn Lys
   1 5
<210> 940
   <211> 12
   <212> PRT
   <213> Artificial Sequence
<220>
   <223> SLC26A9
<400> 940
   Ser Val Leu Gly Ala Leu Ile Ala Val Asn Leu Lys
   1 5 10
<210> 941
   <211> 8
   <212> PRT
   <213> Artificial Sequence
<220>
   <223> SLC26A9
<400> 941
   Ala Gln Asp Ile Gln Gly Ile Lys
   1 5
<210> 942
   <211> 5
   <212> PRT
   <213> Artificial Sequence
<220>
   <223> SLC26A9
<400> 942
   Val Leu Leu Ala Lys
   1 5
<210> 943
   <211> 7
   <212> PRT
   <213> Artificial Sequence
<220>
   <223> SLC26A9
<400> 943
   Leu Ser Ser Thr Tyr Gly Lys
   1 5
<210> 944
   <211> 18
   <212> PRT
   <213> Artificial Sequence
<220>
   <223> SLC26A9
<400> 944
   His Val Phe Pro Ser Ile His Asp Ala Val Leu Phe Ala Gln Ala Asn
   1 5 10 15
   Ala Arg
<210> 945
   <211> 27
   <212> PRT
   <213> Artificial Sequence
<220>
   <223> SLC26A9
<400> 945
   Asp Val Thr Pro Gly His Asn Phe Gln Gly Ala Pro Gly Asp Ala Glu
   1 5 10 15
   Leu Ser Leu Tyr Asp Ser Glu Glu Asp Ile Arg
   20 25
<210> 946
   <211> 8
   <212> PRT
   <213> Artificial Sequence
<220>
   <223> SLC27A1
<400> 946
   Ile Phe Gln Ala Val Val Gln Arg
   1 5
<210> 947
   <211> 12
   <212> PRT
   <213> Artificial Sequence
<220>
   <223> SLC27A1
<400> 947
   Leu Leu Pro Gln Val Asp Thr Thr Gly Thr Phe Lys
   1 5 10
<210> 948
   <211> 15
   <212> PRT
   <213> Artificial Sequence
<220>
   <223> SLC27A1
<400> 948
   Gly Glu Asn Val Ser Thr Thr Glu Val Glu Gly Val Leu Ser Arg
   1 5 10 15
<210> 949
   <211> 13
   <212> PRT
   <213> Artificial Sequence
<220>
   <223> SLC27A2
<400> 949
   Thr Ser Asn Thr Asp Gly Ile Asp Ser Phe Leu Asp Lys
   1 5 10
<210> 950
   <211> 9
   <212> PRT
   <213> Artificial Sequence
<220>
   <223> SLC27A2
<400> 950
   Ala Leu His Asp His Leu Gly Leu Arg
   1 5
<210> 951
   <211> 14
   <212> PRT
   <213> Artificial Sequence
<220>
   <223> SLC27A2
<400> 951
   Val Asp Glu Val Ser Thr Glu Pro Ile Pro Glu Ser Trp Arg
   1 5 10
<210> 952
   <211> 12
   <212> PRT
   <213> Artificial Sequence
<220>
   <223> SLC27A2
<400> 952
   Ile Gln Asp Thr Ile Glu Ile Thr Gly Thr Phe Lys
   1 5 10
<210> 953
   <211> 8
   <212> PRT
   <213> Artificial Sequence
<220>
   <223> SLC27A2
<400> 953
   Leu Ala Leu Gly Asn Gly Leu Arg
   1 5
<210> 954
   <211> 14
   <212> PRT
   <213> Artificial Sequence
<220>
   <223> SLC27A3
<400> 954
   Leu Ala Val Gly Ser Gly Leu Arg Pro Asp Thr Trp Glu Arg
   1 5 10
<210> 955
   <211> 12
   <212> PRT
   <213> Artificial Sequence
<220>
   <223> SLC27A3
<400> 955
   Leu Gln Glu Ser Leu Ala Thr Thr Glu Thr Phe Lys
   1 5 10
<210> 956
   <211> 9
   <212> PRT
   <213> Artificial Sequence
<220>
   <223> SLC27A3
<400> 956
   His Ile Phe Pro Phe Ser Leu Ile Arg
   1 5
<210> 957
   <211> 8
   <212> PRT
   <213> Artificial Sequence
<220>
   <223> SLC27A3
<400> 957
   Leu Ala Glu Leu Ala Gln Gln Arg
   1 5
<210> 958
   <211> 15
   <212> PRT
   <213> Artificial Sequence
<220>
   <223> SLC27A4
<400> 958
   Thr Ala Leu Ile Phe Glu Gly Thr Asp Thr His Trp Thr Phe Arg
   1 5 10 15
<210> 959
   <211> 8
   <212> PRT
   <213> Artificial Sequence
<220>
   <223> SLC27A4
<400> 959
   Ala Ala Ile Val Val His Ser Arg
   1 5
<210> 960
   <211> 15
   <212> PRT
   <213> Artificial Sequence
<220>
   <223> SLC27A4
<400> 960
   Gly Glu Asn Val Ser Thr Thr Glu Val Glu Gly Thr Leu Ser Arg
   1 5 10 15
<210> 961
   <211> 10
   <212> PRT
   <213> Artificial Sequence
<220>
   <223> SLC27A4
<400> 961
   Gln Ser Ile Trp Thr Asn Phe Ser Ser Arg
   1 5 10
<210> 962
   <211> 7
   <212> PRT
   <213> Artificial Sequence
<220>
   <223> SLC27A4
<400> 962
   Leu Leu Pro Glu Leu His Lys
   1 5
<210> 963
   <211> 12
   <212> PRT
   <213> Artificial Sequence
<220>
   <223> SLC27A5
<400> 963
   Gln Pro Pro Asp Thr Phe Val Asp Ala Phe Glu Arg
   1 5 10
<210> 964
   <211> 10
   <212> PRT
   <213> Artificial Sequence
<220>
   <223> SLC27A5
<400> 964
   Ala Asp Val Trp Glu Thr Phe Gln Gln Arg
   1 5 10
<210> 965
   <211> 11
   <212> PRT
   <213> Artificial Sequence
<220>
   <223> SLC27A5
<400> 965
   Trp Leu Pro Ala Asp Val Ile Phe Leu Ala Lys
   1 5 10
<210> 966
   <211> 11
   <212> PRT
   <213> Artificial Sequence
<220>
   <223> SLC27A6
<400> 966
   Ala Ala Val Ile Ser Gln Leu Gln Val Leu Arg
   1 5 10
<210> 967
   <211> 8
   <212> PRT
   <213> Artificial Sequence
<220>
   <223> SLC27A6
<400> 967
   Leu Ala Ile Gly Asn Gly Ile Arg
   1 5
<210> 968
   <211> 12
   <212> PRT
   <213> Artificial Sequence
<220>
   <223> SLC27A6
<400> 968
   His Gln Leu Val Glu Asp Gly Phe Asn Pro Leu Lys
   1 5 10
<210> 969
   <211> 9
   <212> PRT
   <213> Artificial Sequence
<220>
   <223> SLC27A6
<400> 969
   Leu Leu Ser Thr Phe Asp Leu Ile Lys
   1 5
<210> 970
   <211> 6
   <212> PRT
   <213> Artificial Sequence
<220>
   <223> SLC28A2
<400> 970
   Gln Trp Ile Ser Val Arg
   1 5
<210> 971
   <211> 6
   <212> PRT
   <213> Artificial Sequence
<220>
   <223> SLC28A2
<400> 971
   Ser Glu Glu Gly Val Lys
   1 5
<210> 972
   <211> 16
   <212> PRT
   <213> Artificial Sequence
<220>
   <223> SLC28A2
<400> 972
   Phe Phe Ile Asn Glu Phe Val Ala Tyr Gln Gln Leu Ser Gln Tyr Lys
   1 5 10 15
<210> 973
   <211> 9
   <212> PRT
   <213> Artificial Sequence
<220>
   <223> SLC29A3
<400> 973
   Ala Asp Gln Glu Ala Leu Leu Glu Lys
   1               5
<210> 974
   <211> 9
   <212> PRT
   <213> Artificial Sequence
<220>
   <223> SLC29A3
<400> 974
   Gly Ser Gly Ser Leu Trp Thr Thr Lys
   1               5
<210> 975
   <211> 14
   <212> PRT
   <213> Artificial Sequence
<220>
   <223> SLC29A3
<400> 975
   Gln Leu Thr Ala Trp Ile Gln Val Pro Gly Pro Asn Ser Lys
   1               5                   10
<210> 976
   <211> 9
   <212> PRT
   <213> Artificial Sequence
<220>
   <223> SLC29A3
<400> 976
   Ala Leu Pro Gly Phe Val Leu Leu Arg
   1               5
<210> 977
   <211> 6
   <212> PRT
   <213> Artificial Sequence
<220>
   <223> SLC2A11
<400> 977
   Thr Gln Ile Gln Gly Arg
   1               5
<210> 978
   <211> 7
   <212> PRT
   <213> Artificial Sequence
<220>
   <223> SLC2A11
<400> 978
   Ala Gly Val Pro Glu Ala Lys
   1 5
<210> 979
   <211> 7
   <212> PRT
   <213> Artificial Sequence
<220>
   <223> SLC2A11
<400> 979
   Thr Phe Gln Glu Ile Ser Lys
   1 5
<210> 980
   <211> 7
   <212> PRT
   <213> Artificial Sequence
<220>
   <223> SLC2A11
<400> 980
   Ala Gln Gly Pro Thr Trp Arg
   1 5
<210> 981
   <211> 7
   <212> PRT
   <213> Artificial Sequence
<220>
   <223> SLC2A12
<400> 981
   Gly Ile Ser Ser His Ser Arg
   1 5
<210> 982
   <211> 17
   <212> PRT
   <213> Artificial Sequence
<220>
   <223> SLC2A12
<400> 982
   Val Ile Ser Thr Ile Pro Ala Thr Leu Leu Val Asp His Val Gly Ser
   1 5 10 15
   Lys
<210> 983
   <211> 5
   <212> PRT
   <213> Artificial Sequence
<220>
   <223> SLC2A13
<400> 983
   Gly Gln Thr Gln Lys
   1 5
<210> 984
   <211> 5
   <212> PRT
   <213> Artificial Sequence
<220>
   <223> SLC2A13
<400> 984
   Trp Leu Ile Gln Lys
   1 5
<210> 985
   <211> 7
   <212> PRT
   <213> Artificial Sequence
<220>
   <223> SLC2A13
<400> 985
   Glu Thr Leu Leu Ala Gly Arg
   1 5
<210> 986
   <211> 6
   <212> PRT
   <213> Artificial Sequence
<220>
   <223> SLC2A13
<400> 986
   Tyr Ile Glu Tyr Ile Arg
   1 5
<210> 987
   <211> 11
   <212> PRT
   <213> Artificial Sequence
<220>
   <223> SLC2A13
<400> 987
   Glu Val Gly Ser Ala Gly Pro Val Ile Cys Arg
   1 5 10
<210> 988
   <211> 15
   <212> PRT
   <213> Artificial Sequence
<220>
   <223> SLC2A13
<400> 988
   Gly Ser Asn Tyr His Leu Ser Asp Asn Asp Ala Ser Asp Val Glu
   1 5 10 15
<210> 989
   <211> 6
   <212> PRT
   <213> Artificial Sequence
<220>
   <223> SLC2A14
<400> 989
   Phe Leu Leu Ile Asn Arg
   1 5
<210> 990
   <211> 7
   <212> PRT
   <213> Artificial Sequence
<220>
   <223> SLC2A14
<400> 990
   Thr Phe Glu Asp Ile Thr Arg
   1 5
<210> 991
   <211> 11
   <212> PRT
   <213> Artificial Sequence
<220>
   <223> SLC2A14
<400> 991
   Ala Phe Glu Gly Gln Ala His Gly Ala Asp Arg
   1 5 10
<210> 992
   <211> 10
   <212> PRT
   <213> Artificial Sequence
<220>
   <223> SLC2A3
<400> 992
   Ala Phe Glu Gly Gln Ala His Ser Gly Lys
   1 5 10
<210> 993
   <211> 11
   <212> PRT
   <213> Artificial Sequence
<220>
   <223> SLC2A3
<400> 993
   Glu Glu Asp Gln Ala Thr Glu Ile Leu Gln Arg
   1 5 10
<210> 994
   <211> 7
   <212> PRT
   <213> Artificial Sequence
<220>
   <223> SLC2A3
<400> 994
   Thr Phe Glu Asp Ile Ala Arg
   1 5
<210> 995
   <211> 6
   <212> PRT
   <213> Artificial Sequence
<220>
   <223> SLC2A3
<400> 995
   Glu Thr Pro Gly Asn Ala
   1 5
<210> 996
   <211> 15
   <212> PRT
   <213> Artificial Sequence
<220>
   <223> SLC2A4
<400> 996
   Leu Thr Gly Trp Ala Asp Val Ser Gly Val Leu Ala Glu Leu Lys
   1 5 10 15
<210> 997
   <211> 11
   <212> PRT
   <213> Artificial Sequence
<220>
   <223> SLC2A9
<400> 997
   Thr Phe Asp Gln Ile Ser Ala Ala Phe His Arg
   1 5 10
<210> 998
   <211> 7
   <212> PRT
   <213> Artificial Sequence
<220>
   <223> SLC2A5
<400> 998
   Gly Trp Asp Ser Val Asp Arg
   1 5
<210> 999
   <211> 11
   <212> PRT
   <213> Artificial Sequence
<220>
   <223> SLC2A5
<400> 999
   Thr Phe Ile Glu Ile Asn Gln Ile Phe Thr Lys
   1 5 10
<210> 1000
   <211> 7
   <212> PRT
   <213> Artificial Sequence
<220>
   <223> SLC2A6
<400> 1000
   Val Gly Thr Leu Gln Asn Lys
   1 5
<210> 1001
   <211> 10
   <212> PRT
   <213> Artificial Sequence
<220>
   <223> SLC2A6
<400> 1001
   Ser Leu Asp Pro Asp Leu His Leu Thr Lys
   1 5 10
<210> 1002
   <211> 6
   <212> PRT
   <213> Artificial Sequence
<220>
   <223> SLC2A7
<400> 1002
   Tyr Ser Leu Ile Gln Lys
   1 5
<210> 1003
   <211> 10
   <212> PRT
   <213> Artificial Sequence
<220>
   <223> SLC30A1
<400> 1003
   Leu Asp Pro Ala Asp Pro Glu Asn Pro Arg
   1 5 10
<210> 1004
   <211> 7
   <212> PRT
   <213> Artificial Sequence
<220>
   <223> SLC30A1
<400> 1004
   Asn Thr Phe Gly Trp Ile Arg
   1 5
<210> 1005
   <211> 8
   <212> PRT
   <213> Artificial Sequence
<220>
   <223> SLC30A1
<400> 1005
   Ile Ile Ala Thr Val His Ile Lys
   1 5
<210> 1006
   <211> 16
   <212> PRT
   <213> Artificial Sequence
<220>
   <223> SLC30A10
<400> 1006
   Gly Ala Thr Val Phe Ala Asn Val Ala Glu Leu Ile His Asn Thr Arg
   1 5 10 15
<210> 1007
   <211> 6
   <212> PRT
   <213> Artificial Sequence
<220>
   <223> SLC30A10
<400> 1007
   Leu Ala Arg Pro Glu Arg
   1 5
<210> 1008
   <211> 6
   <212> PRT
   <213> Artificial Sequence
<220>
   <223> SLC30A11
<400> 1008
   Thr Gln Ile Gln Gly Arg
   1 5
<210> 1009
   <211> 20
   <212> PRT
   <213> Artificial Sequence
<220>
   <223> SLC30A11
<400> 1009
   Ser Leu Leu Val Asn Asn Ile Phe Val Val Ser Ala Ala Ile Leu Phe
   1 5 10 15
   Gly Phe Ser Arg
   20
<210> 1010
   <211> 7
   <212> PRT
   <213> Artificial Sequence
<220>
   <223> SLC30A11
<400> 1010
   Ala Gln Gly Pro Thr Trp Arg
   1 5
<210> 1011
   <211> 11
   <212> PRT
   <213> Artificial Sequence
<220>
   <223> SLC30A2
<400> 1011
   Gln His Leu Leu Asp Ala Arg Pro Ala Ile Arg
   1 5 10
<210> 1012
   <211> 8
   <212> PRT
   <213> Artificial Sequence
<220>
   <223> SLC30A2
<400> 1012
   Gly Val Asp Phe Thr Ala Val Arg
   1 5
<210> 1013
   <211> 8
   <212> PRT
   <213> Artificial Sequence
<220>
   <223> SLC30A3
<400> 1013
   Gly Gly Ala Gly Gly Ser Leu Arg
   1 5
<210> 1014
   <211> 10
   <212> PRT
   <213> Artificial Sequence
<220>
   <223> SLC30A3
<400> 1014
   Asp Thr Leu Leu Ser Val Pro Gly Val Arg
   1 5 10
<210> 1015
   <211> 8
   <212> PRT
   <213> Artificial Sequence
<220>
   <223> SLC30A3
<400> 1015
   Asn Val Gly Phe Glu Pro Val Arg
   1 5
<210> 1016
   <211> 12
   <212> PRT
   <213> Artificial Sequence
<220>
   <223> SLC30A3
<400> 1016
   Asp Pro Leu Pro Pro Pro Gly Leu Thr Pro Glu Arg
   1 5 10
<210> 1017
   <211> 7
   <212> PRT
   <213> Artificial Sequence
<220>
   <223> SLC30A4
<400> 1017
   Phe Thr Phe Gly Phe His Arg
   1 5
<210> 1018
   <211> 10
   <212> PRT
   <213> Artificial Sequence
<220>
   <223> SLC30A4
<400> 1018
   Asn His Gly Gln Asp Ser Leu Ala Val Arg
   1 5 10
<210> 1019
   <211> 16
   <212> PRT
   <213> Artificial Sequence
<220>
   <223> SLC30A4
<400> 1019
   Ser Thr Ala Ile Val His Ile Gln Leu Ile Pro Gly Ser Ser Ser Lys
   1 5 10 15
<210> 1020
   <211> 14
   <212> PRT
   <213> Artificial Sequence
<220>
   <223> SLC30A4
<400> 1020
   His Ser His Ser His Ser Leu Pro Ser Asn Ser Pro Thr Arg
   1 5 10
<210> 1021
   <211> 7
   <212> PRT
   <213> Artificial Sequence
<220>
   <223> SLC30A4
<400> 1021
   Trp Glu Glu Val Gln Ser Lys
   1 5
<210> 1022
   <211> 8
   <212> PRT
   <213> Artificial Sequence
<220>
   <223> SLC30A5
<400> 1022
   His Gln Ile Ile Gly Ser Leu Lys
   1 5
<210> 1023
   <211> 8
   <212> PRT
   <213> Artificial Sequence
<220>
   <223> SLC30A5
<400> 1023
   Val Gly Phe His Thr Ala Ser Arg
   1 5
<210> 1024
   <211> 6
   <212> PRT
   <213> Artificial Sequence
<220>
   <223> SLC30A6
<400> 1024
   Leu Leu Gln Glu Phe Arg
   1 5
<210> 1025
   <211> 11
   <212> PRT
   <213> Artificial Sequence
<220>
   <223> SLC30A6
<400> 1025
   Phe Leu Glu Gln Pro Glu Ile His Thr Gly Arg
   1 5 10
<210> 1026
   <211> 16
   <212> PRT
   <213> Artificial Sequence
<220>
   <223> SLC30A6
<400> 1026
   Val Leu Leu Gln Thr Thr Pro Pro His Val Ile Gly Gln Leu Asp Lys
   1 5 10 15
<210> 1027
   <211> 10
   <212> PRT
   <213> Artificial Sequence
<220>
   <223> SLC30A7
<400> 1027
   Leu Ile Val Ala Pro Asp Ala Asp Ala Arg
   1 5 10
<210> 1028
   <211> 11
   <212> PRT
   <213> Artificial Sequence
<220>
   <223> SLC30A7
<400> 1028
   Ala Leu Ala Pro Pro Asp Val His His Glu Arg
   1 5 10
<210> 1029
   <211> 16
   <212> PRT
   <213> Artificial Sequence
<220>
   <223> SLC30A7
<400> 1029
   Trp Ile Leu Ser Gln Thr His Asn Ile Phe Thr Gln Ala Gly Val Arg
   1 5 10 15
<210> 1030
   <211> 7
   <212> PRT
   <213> Artificial Sequence
<220>
   <223> SLC30A8
<400> 1030
   Leu Thr Phe Gly Trp His Arg
   1 5

<210> 1031
   <211> 9
   <212> PRT
   <213> Artificial Sequence
<220>
   <223> SLC30A8
<400> 1031
   Gly Ala Asn Glu Tyr Ala Tyr Ala Lys
   1 5
<210> 1032
   <211> 9
   <212> PRT
   <213> Artificial Sequence
<220>
   <223> SLC30A8
<400> 1032
   Glu Val Gln Ala Asn Ala Ser Val Arg
   1 5
<210> 1033
   <211> 8
   <212> PRT
   <213> Artificial Sequence
<220>
   <223> SLC30A8
<400> 1033
   Ser Leu Asn Tyr Ser Gly Val Lys
   1 5
<210> 1034
   <211> 7
   <212> PRT
   <213> Artificial Sequence
<220>
   <223> SLC30A8
<400> 1034
   Thr Tyr Leu Val Asn Asp Lys
   1 5
<210> 1035
   <211> 15
   <212> PRT
   <213> Artificial Sequence
<220>
   <223> SLC30A9
<400> 1035
   His Gly Glu Asn Ile Ile Asp Thr Leu Gly Ala Glu Val Asp Arg
   1               5                   10                  15
<210> 1036
   <211> 12
   <212> PRT
   <213> Artificial Sequence
<220>
   <223> SLC30A9
<400> 1036
   Leu Thr Glu Leu Leu Glu Asn Asp Pro Ser Val Arg
   1               5                   10
<210> 1037
   <211> 8
   <212> PRT
   <213> Artificial Sequence
<220>
   <223> SLC30A9
<400> 1037
   Ala Glu Val Asp Phe Asp Gly Arg
   1               5
<210> 1038
   <211> 8
   <212> PRT
   <213> Artificial Sequence
<220>
   <223> SLC30A9
<400> 1038
   Ala Thr Asp Leu Gly Leu Gly Lys
   1               5
<210> 1039
   <211> 9
   <212> PRT
   <213> Artificial Sequence
<220>
   <223> SLC30A9
<400> 1039
   Ser Ile Gln Pro Glu Gln Val Gln Arg
   1 5
<210> 1040
   <211> 15
   <212> PRT
   <213> Artificial Sequence
<220>
   <223> SLC30A9
<400> 1040
   Val Pro Ser Phe Glu Thr Ala Glu Gly Ile Gly Ala Glu Leu Lys
   1 5 10 15
<210> 1041
   <211> 5
   <212> PRT
   <213> Artificial Sequence
<220>
   <223> SLC31A1
<400> 1041
   Glu Ser Leu Leu Arg
   1 5
<210> 1042
   <211> 6
   <212> PRT
   <213> Artificial Sequence
<220>
   <223> SLC31A1
<400> 1042
   Ser Gln Val Ser Ile Arg
   1 5
<210> 1043
   <211> 4
   <212> PRT
   <213> Artificial Sequence
<220>
   <223> SLC31A2
<400> 1043
   Thr His His Arg
   1
<210> 1044
   <211> 11
   <212> PRT
   <213> Artificial Sequence
<220>
   <223> SLC32AI
<400> 1044
   Leu Ser Asn Val Ala Thr Ser Val Ser Asn Lys
   1 5 10
<210> 1045
   <211> 8
   <212> PRT
   <213> Artificial Sequence
<220>
   <223> SLC32A1
<400> 1045
   Ser Leu Phe Gln Glu Gly Ser Arg
   1 5
<210> 1046
   <211> 7
   <212> PRT
   <213> Artificial Sequence
<220>
   <223> SLC32A1
<400> 1046
   Ser Trp Gly Leu Thr Leu Arg
   1 5
<210> 1047
   <211> 13
   <212> PRT
   <213> Artificial Sequence
<220>
   <223> SLC33A1
<400> 1047
   Ile Gly Phe Ser Ala Ala Asp Ala Val Thr Gly Leu Lys
   1 5 10
<210> 1048
   <211> 8
   <212> PRT
   <213> Artificial Sequence
<220>
   <223> SLC33A1
<400> 1048
   Leu Leu Gly Asn Thr Asp Asp Arg
   1 5
<210> 1049
   <211> 8
   <212> PRT
   <213> Artificial Sequence
<220>
   <223> SLC34A1
<400> 1049
   Ile Ile Thr Glu Pro Phe Thr Lys
   1 5
<210> 1050
   <211> 12
   <212> PRT
   <213> Artificial Sequence
<220>
   <223> SLC34A1
<400> 1050
   Leu Gly Ser Pro Ala Val Ser Pro Leu Pro Val Arg
   1 5 10
<210> 1051
   <211> 10
   <212> PRT
   <213> Artificial Sequence
<220>
   <223> SLC34A1
<400> 1051
   Trp Leu Gln Thr Trp Asp Phe Leu Pro Arg
   1 5 10
<210> 1052
   <211> 14
   <212> PRT
   <213> Artificial Sequence
<220>
   <223> SLC34A1
<400> 1052
   Val Phe Leu Glu Glu Leu Pro Pro Ala Thr Pro Ser Pro Arg
   1 5 10
<210> 1053
   <211> 12
   <212> PRT
   <213> Artificial Sequence
<220>
   <223> SLC34A1
<400> 1053
   Leu Ala Leu Glu Glu Glu Gln Lys Pro Glu Ser Arg
   1 5 10
<210> 1054
   <211> 8
   <212> PRT
   <213> Artificial Sequence
<220>
   <223> SLC34A2
<400> 1054
   Val Ile Thr Asp Pro Phe Thr Lys
   1 5
<210> 1055
   <211> 10
   <212> PRT
   <213> Artificial Sequence
<220>
   <223> SLC34A2
<400> 1055
   Thr Asn Asp Asn Gly Thr Pro Val Ala Lys
   1 5 10
<210> 1056
   <211> 12
   <212> PRT
   <213> Artificial Sequence
<220>
   <223> SLC34A2
<400> 1056
   Phe Gln Asn Gly Glu Asp Ala Pro Asp Ile Leu Lys
   1 5 10
<210> 1057
   <211> 8
   <212> PRT
   <213> Artificial Sequence
<220>
   <223> SLC34A3
<400> 1057
   Asn Ser Thr Ala Pro Ala Asp Arg
   1 5
<210> 1058
   <211> 14
   <212> PRT
   <213> Artificial Sequence
<220>
   <223> SLC34A3
<400> 1058
   Leu Ser Glu Leu Ala Leu Gly Ala Ala Ser Leu Thr Pro Arg
   1 5 10
<210> 1059
   <211> 7
   <212> PRT
   <213> Artificial Sequence
<220>
   <223> SLC34A3
<400> 1059
   Asp Thr Ser Gln Pro Trp Lys
   1 5
<210> 1060
   <211> 8
   <212> PRT
   <213> Artificial Sequence
<220>
   <223> SLC34A3
<400> 1060
   His Phe Gly Val Val Thr Ala Arg
   1 5
<210> 1061
   <211> 8
   <212> PRT
   <213> Artificial Sequence
<220>
   <223> SLC34A3
<400> 1061
   Arg Pro Ala Trp Leu Pro Val Arg
   1 5
<210> 1062
   <211> 9
   <212> PRT
   <213> Artificial Sequence
<220>
   <223> SLC35A1
<400> 1062
   Ser Ser Asp Thr Ser Leu Trp Val Arg
   1 5
<210> 1063
   <211> 9
   <212> PRT
   <213> Artificial Sequence
<220>
   <223> SLC35A2
<400> 1063
   Gly Ser Ser Gly Ser Val Trp Leu Arg
   1 5
<210> 1064
   <211> 11
   <212> PRT
   <213> Artificial Sequence
<220>
   <223> SLC35A2
<400> 1064
   Gly Asp Leu Ile Thr Glu Pro Phe Leu Pro Lys
   1 5 10
<210> 1065
   <211> 17
   <212> PRT
   <213> Artificial Sequence
<220>
   <223> SLC35A2
<400> 1065
   Gly Phe Ala Thr Ser Leu Ser Ile Val Leu Ser Thr Val Ala Ser Ile
   1 5 10 15
   Arg
<210> 1066
   <211> 6
   <212> PRT
   <213> Artificial Sequence
<220>
   <223> SLC35A3
<400> 1066
   Gln Ser Val Trp Ile Arg
   1 5
<210> 1067
   <211> 9
   <212> PRT
   <213> Artificial Sequence
<220>
   <223> SLC35A3
<400> 1067
   Asn Gly Phe Phe Gln Gly Tyr Asn Arg
   1 5
<210> 1068
   <211> 16
   <212> PRT
   <213> Artificial Sequence
<220>
   <223> SLC35A4
<400> 1068
   Val Pro Phe Arg Pro Ser Ser Ala Val Leu Leu Thr Glu Leu Thr Lys
   1 5 10 15
<210> 1069
   <211> 7
   <212> PRT
   <213> Artificial Sequence
<220>
   <223> SLC35A4
<400> 1069
   His Gly Ser Ser Ile Thr Arg
   1 5
<210> 1070
   <211> 9
   <212> PRT
   <213> Artificial Sequence
<220>
   <223> SLC35A5
<400> 1070
   Thr Leu Gln His Asn Leu Ala Gly Arg
   1 5
<210> 1071
   <211> 9
   <212> PRT
   <213> Artificial Sequence
<220>
   <223> SLC35A5
<400> 1071
   Ser Asp Glu Ser Asp Glu Asp Thr Phe
   1 5
<210> 1072
   <211> 10
   <212> PRT
   <213> Artificial Sequence
<220>
   <223> SLC35A5
<400> 1072
   Ser Ser Gly Asp Gly Glu Glu Leu Glu Arg
   1 5 10
<210> 1073
   <211> 6
   <212> PRT
   <213> Artificial Sequence
<220>
   <223> SLC35B1
<400> 1073
   Tyr Gly Glu Gly Ala Lys
   1 5
<210> 1074
   <211> 10
   <212> PRT
   <213> Artificial Sequence
<220>
   <223> SLC35B2
<400> 1074
   Ala Val Pro Val Glu Ser Pro Val Gln Lys
   1 5 10
<210> 1075
   <211> 10
   <212> PRT
   <213> Artificial Sequence
<220>
   <223> SLC35B2
<400> 1075
   Ala Ser Asp Glu Val Pro Leu Ala Pro Arg
   1 5 10
<210> 1076
   <211> 12
   <212> PRT
   <213> Artificial Sequence
<220>
   <223> SLC35B2
<400> 1076
   Ser Tyr Gly Ala Thr Ala Thr Ser Pro Gly Glu Arg
   1 5 10
<210> 1077
   <211> 11
   <212> PRT
   <213> Artificial Sequence
<220>
   <223> SLC35B2
<400> 1077
   Phe Val Ser Phe Pro Thr Gln Val Leu Ala Lys
   1 5 10
<210> 1078
   <211> 6
   <212> PRT
   <213> Artificial Sequence
<220>
   <223> SLC35B3
<400> 1078
   Thr Leu Ala Gln Thr Val
   1 5
<210> 1079
   <211> 9
   <212> PRT
   <213> Artificial Sequence
<220>
   <223> SLC35B3
<400> 1079
   Tyr Ile Ser Ile Thr Val Pro Ser Lys
   1 5
<210> 1080
   <211> 10
   <212> PRT
   <213> Artificial Sequence
<220>
   <223> SLC35B3
<400> 1080
   Leu Pro Ser Leu Tyr Asp Leu Ile Asn Lys
   1 5 10
<210> 1081
   <211> 5
   <212> PRT
   <213> Artificial Sequence
<220>
   <223> SLC35B4
<400> 1081
   Ser Glu Pro Gln Lys
   1 5
<210> 1082
   <211> 4
   <212> PRT
   <213> Artificial Sequence
<220>
   <223> SLC35B4
<400> 1082
   Gln Phe Gly Lys
   1
<210> 1083
   <211> 9
   <212> PRT
   <213> Artificial Sequence
<220>
   <223> SLC35C1
<400> 1083
   Tyr Leu Leu Asp Ser Pro Ser Leu Arg
   1 5
<210> 1084
   <211> 14
   <212> PRT
   <213> Artificial Sequence
<220>
   <223> SLC35C1
<400> 1084
   Phe Thr Ser Pro Leu Thr His Asn Val Ser Gly Thr Ala Lys
   1               5                   10
<210> 1085
   <211> 10
   <212> PRT
   <213> Artificial Sequence
<220>
   <223> SLC35C2
<400> 1085
   Trp Ala Leu Asp Val Ala Phe Leu Trp Lys
   1               5                   10
<210> 1086
   <211> 13
   <212> PRT
   <213> Artificial Sequence
<220>
   <223> SLC35C2
<400> 1086
   Gly Leu Gly Ser Ser Pro Asp Leu Glu Leu Leu Leu Arg
   1               5                   10
<210> 1087
   <211> 9
   <212> PRT
   <213> Artificial Sequence
<220>
   <223> SLC35C2
<400> 1087
   Phe Gln Asp Thr Gly Leu Leu Leu Arg
   1               5
<210> 1088
   <211> 7
   <212> PRT
   <213> Artificial Sequence
<220>
   <223> SLC35D1
<400> 1088
   Gln Ser Glu Ala Asn Asn Lys
   1 5
<210> 1089
   <211> 7
   <212> PRT
   <213> Artificial Sequence
<220>
   <223> SLC35D1
<400> 1089
   Ser Val Leu Thr Asn Tyr Arg
   1 5
<210> 1090
   <211> 21
   <212> PRT
   <213> Artificial Sequence
<220>
   <223> SLC35D2
<400> 1090
   Leu Phe Pro Leu Pro Leu Leu Tyr Val Gly Asn His Ile Ser Gly Leu
   1 5 10 15
   Ser Ser Thr Ser Lys
   20
<210> 1091
   <211> 4
   <212> PRT
   <213> Artificial Sequence
<220>
   <223> SLC35D2
<400> 1091
   Glu Leu Gly Lys
   1
<210> 1092
   <211> 4
   <212> PRT
   <213> Artificial Sequence
<220>
   <223> SLC35D2
<400> 1092
   Leu Pro Ser Arg
   1
<210> 1093
   <211> 15
   <212> PRT
   <213> Artificial Sequence
<220>
   <223> SLC35D3
<400> 1093
   Leu Gly Leu Ile Ala Val Pro Pro Phe Gly Leu Ser Leu Ala Arg
   1 5 10 15
<210> 1094
   <211> 15
   <212> PRT
   <213> Artificial Sequence
<220>
   <223> SLC35D3
<400> 1094
   Ser Glu Gly Gly Glu Ala Ala Gly Gly Pro Ala Gln Glu Ser Arg
   1 5 10 15
<210> 1095
   <211> 21
   <212> PRT
   <213> Artificial Sequence
<220>
   <223> SLC35D3
<400> 1095
   Ser Phe Ala Gly Val Ala Val Leu Ser Thr Leu Gln Ser Ser Leu Thr
   1 5 10 15
   Leu Trp Ser Leu Arg
   20
<210> 1096
   <211> 13
   <212> PRT
   <213> Artificial Sequence
<220>
   <223> SLC35E1
<400> 1096
   His Leu Leu Pro Val Thr Thr Ala Asp Leu Ser Ser Lys
   1 5 10
<210> 1097
   <211> 12
   <212> PRT
   <213> Artificial Sequence
<220>
   <223> SLC35E1
<400> 1097
   Ile Met Val Ile Thr Val Ser Leu Ile Met Leu Arg
   1 5 10
<210> 1098
   <211> 12
   <212> PRT
   <213> Artificial Sequence
<220>
   <223> SLC35E1
<400> 1098
   Asn Asn Ile Leu Thr Asp His Phe Gln Tyr Ser Arg
   1 5 10
<210> 1099
   <211> 11
   <212> PRT
   <213> Artificial Sequence
<220>
   <223> SLC35E2
<400> 1099
   Asn Val Ala Val Ser Phe Ala Glu Thr Val Lys
   1 5 10
<210> 1100
   <211> 12
   <212> PRT
   <213> Artificial Sequence
<220>
   <223> SLC35E2
<400> 1100
   Phe Ala Thr Val Val Leu Gly Leu Val Ser Leu Lys
   1 5 10
<210> 1101
   <211> 11
   <212> PRT
   <213> Artificial Sequence
<220>
   <223> SLC35E2
<400> 1101
   Val Phe Phe Thr Asp Val Pro Val Ile Gly Arg
   1 5 10
<210> 1102
   <211> 13
   <212> PRT
   <213> Artificial Sequence
<220>
   <223> SLC35E2
<400> 1102
   Ile Ser Pro Val Thr Phe Ser Val Ala Ser Thr Val Lys
   1 5 10
<210> 1103
   <211> 12
   <212> PRT
   <213> Artificial Sequence
<220>
   <223> SLC35E2
<400> 1103
   Ser Pro Leu Ser Trp Gly Ser Leu Phe Gly His Arg
   1 5 10
<210> 1104
   <211> 15
   <212> PRT
   <213> Artificial Sequence
<220>
   <223> SLC35E2
<400> 1104
   Ala Pro Asp Asp Thr Val Glu Pro Leu Leu Pro Gln Asp Pro Arg
   1 5 10 15
<210> 1105
   <211> 15
   <212> PRT
   <213> Artificial Sequence
<220>
   <223> SLC35E2
<400> 1105
   His Ala Leu Ser Ile Trp Leu Ser Val Ile Val Phe Gly Asn Lys
   1 5 10 15
<210> 1106
   <211> 16
   <212> PRT
   <213> Artificial Sequence
<220>
   <223> SLC35E2
<400> 1106
   Thr Pro Ala Leu Glu Glu Leu Val Pro Gly Ser Glu Glu Lys Pro Lys
   1 5 10 15
<210> 1107
   <211> 16
   <212> PRT
   <213> Artificial Sequence
<220>
   <223> SLC35E2
<400> 1107
   Gln His Gln Gln Glu Ala Leu Gln Ser Leu Ala Ala Ala Thr Gly Arg
   1 5 10 15
<210> 1108
   <211> 7
   <212> PRT
   <213> Artificial Sequence
<220>
   <223> SLC35E3
<400> 1108
   Leu Asp Ile Phe Ala Pro Lys
   1 5
<210> 1109
   <211> 8
   <212> PRT
   <213> Artificial Sequence
<220>
   <223> SLC35E3
<400> 1109
   Leu Ser Glu Gln Glu Gly Ser Arg
   1 5
<210> 1110
   <211> 9
   <212> PRT
   <213> Artificial Sequence
<220>
   <223> SLC35E3
<400> 1110
   Gly Asp His Gly Ile Val Val Asp Arg
   1 5
<210> 1111
   <211> 12
   <212> PRT
   <213> Artificial Sequence
<220>
   <223> SLC35E4
<400> 1111
   Ser Val Gln Gln Ser Ala Leu Leu Gln Glu Glu Arg
   1 5 10
<210> 1112
   <211> 6
   <212> PRT
   <213> Artificial Sequence
<220>
   <223> SLC35E4
<400> 1112
   Leu Leu Phe Gly Ser Arg
   1 5
<210> 1113
   <211> 8
   <212> PRT
   <213> Artificial Sequence
<220>
   <223> SLC35F1
<400> 1113
   His Gln Gly Ala Gly Glu Asn Lys
   1 5
<210> 1114
   <211> 13
   <212> PRT
   <213> Artificial Sequence
<220>
   <223> SLC35F2
<400> 1114
   Leu Glu Glu Asn Leu Gln Glu Thr His Ser Ala Val Leu
   1 5 10
<210> 1115
   <211> 8
   <212> PRT
   <213> Artificial Sequence
<220>
   <223> SLC35F2
<400> 1115
   Leu Phe Thr Trp Asn Ile Leu Lys
   1 5
<210> 1116
   <211> 10
   <212> PRT
   <213> Artificial Sequence
<220>
   <223> SLC35F3
<400> 1116
   Phe Phe Gly Asp Asn Gly Leu Thr Leu Lys
   1 5 10
<210> 1117
   <211> 7
   <212> PRT
   <213> Artificial Sequence
<220>
   <223> SLC35F3
<400> 1117
   Leu Leu Leu Gly Ser Ala Lys
   1 5
<210> 1118
   <211> 11
   <212> PRT
   <213> Artificial Sequence
<220>
   <223> SLC35F5
<400> 1118
   Gln Leu Pro Ser Ser His Ala Leu Glu Ala Lys
   1 5 10
<210> 1119
   <211> 8
   <212> PRT
   <213> Artificial Sequence
<220>
   <223> SLC35F5
<400> 1119
   Leu Thr Ala Thr Gln Val Ala Lys
   1 5
<210> 1120
   <211> 14
   <212> PRT
   <213> Artificial Sequence
<220>
   <223> SLC36A1
<400> 1120
   Ile Pro Asp Pro Ser His Leu Pro Leu Val Ala Pro Trp Lys
   1 5 10
<210> 1121
   <211> 8
   <212> PRT
   <213> Artificial Sequence
<220>
   <223> SLC36A2
<400> 1121
   Leu Pro Leu Val Ala Ser Trp Lys
   1 5
<210> 1122
   <211> 13
   <212> PRT
   <213> Artificial Sequence
<220>
   <223> SLC36A2
<400> 1122
   Gly Ile Thr Val Phe Gln Ala Leu Ile His Leu Val Lys
   1 5 10
<210> 1123
   <211> 10
   <212> PRT
   <213> Artificial Sequence
<220>
   <223> SLC36A2
<400> 1123
   Trp Ala Leu Pro Leu Asp Leu Ser Ile Arg
   1 5 10
<210> 1124
   <211> 8
   <212> PRT
   <213> Artificial Sequence
<220>
   <223> SLC36A3
<400> 1124
   Trp Asn Leu Ala Leu Ser Pro Arg
   1 5
<210> 1125
   <211> 7
   <212> PRT
   <213> Artificial Sequence
<220>
   <223> SLC36A3
<400> 1125
   Ala His Ala Val Trp Gly Arg
   1 5
<210> 1126
   <211> 6
   <212> PRT
   <213> Artificial Sequence
<220>
   <223> SLC36A4
<400> 1126
   Gln Ala Ala Trp Gly Arg
   1 5
<210> 1127
   <211> 10
   <212> PRT
   <213> Artificial Sequence
<220>
   <223> SLC36A4
<400> 1127
   Gln Val His Glu Gly Phe Leu Glu Ser Lys
   1 5 10
<210> 1128
   <211> 15
   <212> PRT
   <213> Artificial Sequence
<220>
   <223> SLC36A4
<400> 1128
   Gly Asn Ile Gly Thr Gly Leu Leu Gly Leu Pro Leu Ala Ile Lys
   1 5 10 15
<210> 1129
   <211> 18
   <212> PRT
   <213> Artificial Sequence
<220>
   <223> SLC36A4
<400> 1129
   Gly Ser Ile Thr Leu Asn Leu Pro Gln Asp Val Trp Leu Tyr Gln Ser
   1 5 10 15
   Val Lys
<210> 1130
   <211> 7
   <212> PRT
   <213> Artificial Sequence
<220>
   <223> SLC37A1
<400> 1130
   Phe Ile Ile Ser Phe Ser Arg
   1 5
<210> 1131
   <211> 6
   <212> PRT
   <213> Artificial Sequence
<220>
   <223> SLC37A1
<400> 1131
   Leu Pro Ala Gly Ile Arg
   1 5
<210> 1132
   <211> 5
   <212> PRT
   <213> Artificial Sequence
<220>
   <223> SLC37A1
<400> 1132
   Gly Glu Leu His Lys
   1 5
<210> 1133
   <211> 11
   <212> PRT
   <213> Artificial Sequence
<220>
   <223> SLC37A2
<400> 1133
   Ser Ser Leu Ala Pro Gly Val Trp Phe Phe Arg
   1 5 10
<210> 1134
   <211> 9
   <212> PRT
   <213> Artificial Sequence
<220>
   <223> SLC37A2
<400> 1134
   Glu Ser Gly Leu Glu Thr Val Ala Lys
   1 5
<210> 1135
   <211> 6
   <212> PRT
   <213> Artificial Sequence
<220>
   <223> SLC37A3
<400> 1135
   Gln Glu Leu Ile Gln Arg
   1 5
<210> 1136
   <211> 6
   <212> PRT
   <213> Artificial Sequence
<220>
   <223> SLC37A3
<400> 1136
   Glu Ala Glu Ala Asp Lys
   1               5
<210> 1137
   <211> 5
   <212> PRT
   <213> Artificial Sequence
<220>
   <223> SLC37A3
<400> 1137
   Ser Pro Asn Asp Lys
   1               5
<210> 1138
   <211> 10
   <212> PRT
   <213> Artificial Sequence
<220>
   <223> SLC37A4
<400> 1138
   Met Ala Ala Gln Gly Tyr Gly Tyr Tyr Arg
   1               5                   10
<210> 1139
   <211> 15
   <212> PRT
   <213> Artificial Sequence
<220>
   <223> SLC37A4
<400> 1139
   Thr Cys Cys Thr Asp Trp Gly Gln Phe Phe Leu Ile Gln Glu Lys
   1               5                   10                  15
<210> 1140
   <211> 9
   <212> PRT
   <213> Artificial Sequence
<220>
   <223> SLC38A1
<400> 1140
   Asp Asp Ile Leu Ile Leu Thr Val Arg
   1 5
<210> 1141
   <211> 8
   <212> PRT
   <213> Artificial Sequence
<220>
   <223> SLC38A1
<400> 1141
   Ile Thr Asp Gln Asp Gly Asp Lys
   1 5
<210> 1142
   <211> 7
   <212> PRT
   <213> Artificial Sequence
<220>
   <223> SLC38A2
<400> 1142
   Ala Phe Gly Leu Val Gly Lys
   1 5
<210> 1143
   <211> 16
   <212> PRT
   <213> Artificial Sequence
<220>
   <223> SLC38A2
<400> 1143
   Thr Ala Asn Glu Gly Gly Ser Leu Leu Tyr Glu Gln Leu Gly Tyr Lys
   1 5 10 15
<210> 1144
   <211> 16
   <212> PRT
   <213> Artificial Sequence
<220>
   <223> SLC38A2
<400> 1144
   Tyr Glu Leu Pro Leu Val Ile Gln Ala Leu Thr Asn Ile Glu Asp Lys
   1 5 10 15
<210> 1145
   <211> 7
   <212> PRT
   <213> Artificial Sequence
<220>
   <223> SLC38A3
<400> 1145
   Ala Phe Gly Thr Pro Gly Lys
   1 5
<210> 1146
   <211> 16
   <212> PRT
   <213> Artificial Sequence
<220>
   <223> SLC38A3
<400> 1146
   Ser Glu Leu Pro Leu Val Ile Gln Thr Phe Leu Asn Leu Glu Glu Lys
   1 5 10 15
<210> 1147
   <211> 8
   <212> PRT
   <213> Artificial Sequence
<220>
   <223> SLC38A3
<400> 1147
   Ala Tyr Glu Gln Leu Gly Tyr Arg
   1 5
<210> 1148
   <211> 7
   <212> PRT
   <213> Artificial Sequence
<220>
   <223> SLC38A4
<400> 1148
   Tyr Phe Val Phe Asn Ser Arg
   1 5
<210> 1149
   <211> 10
   <212> PRT
   <213> Artificial Sequence
<220>
   <223> SLC38A4
<400> 1149
   Thr Ser Val Ile Thr Leu Leu Gly Pro Lys
   1 5 10
<210> 1150
   <211> 7
   <212> PRT
   <213> Artificial Sequence
<220>
   <223> SLC3BA5
<400> 1150
   Ala Phe Gly Pro Ala Gly Lys
   1 5
<210> 1151
   <211> 8
   <212> PRT
   <213> Artificial Sequence
<220>
   <223> SLC38A5
<400> 1151
   Ala Tyr Glu Gln Leu Gly Gln Arg
   1 5
<210> 1152
   <211> 6
   <212> PRT
   <213> Artificial Sequence
<220>
   <223> SLC38A5
<400> 1152
   Ala Phe Ser Trp Pro Arg
   1 5
<210> 1153
   <211> 14
   <212> PRT
   <213> Artificial Sequence
<220>
   <223> SLC38A5
<400> 1153
   Ile Val Pro Ser Glu Val Glu Pro Phe Leu Ser Trp Pro Lys
   1 5 10
<210> 1154
   <211> 7
   <212> PRT
   <213> Artificial Sequence
<220>
   <223> SLC38A5
<400> 1154
   Glu Gly Phe Leu Pro Ser Arg
   1 5
<210> 1155
   <211> 6
   <212> PRT
   <213> Artificial Sequence
<220>
   <223> SLC38A6
<400> 1155
   Leu Phe His Phe Ser Lys
   1 5
<210> 1156
   <211> 17
   <212> PRT
   <213> Artificial Sequence
<220>
   <223> SLC38A6
<400> 1156
   Thr Glu Leu Pro Ala Ala Ile Ala Glu Phe Leu Thr Gly Asp Tyr Ser
   1 5 10 15
   Arg
<210> 1157
   <211> 7
   <212> PRT
   <213> Artificial Sequence
<220>
   <223> SLC38A6
<400> 1157
   Val Glu Ser Glu Leu Leu Lys
   1 5
<210> 1158
   <211> 7
   <212> PRT
   <213> Artificial Sequence
<220>
   <223> SLC39A1
<400> 1158
   Leu Leu Gln Ser His Leu Arg
   1 5
<210> 1159
   <211> 12
   <212> PRT
   <213> Artificial Sequence
<220>
   <223> SLC39A1
<400> 1159
   Glu Gln Ser Gly Pro Ser Pro Leu Glu Glu Thr Arg
   1 5 10
<210> 1160
   <211> 7
   <212> PRT
   <213> Artificial Sequence
<220>
   <223> SLC39A10
<400> 1160
   Asn Gly His Asp Pro Gly Arg
   1 5
<210> 1161
   <211> 6
   <212> PRT
   <213> Artificial Sequence
<220>
   <223> SLC39A10
<400> 1161
   Val His Lys Pro Asp Arg
   1 5
<210> 1162
   <211> 7
   <212> PRT
   <213> Artificial Sequence
<220>
   <223> SLC39A10
<400> 1162
   Ile Val Phe Asp Ile Gln Phe
   1 5
<210> 1163
   <211> 10
   <212> PRT
   <213> Artificial Sequence
<220>
   <223> SLC39A11
<400> 1163
   Thr Ala Ser Ala Thr Phe Glu Ser Ala Arg
   1 5 10
<210> 1164
   <211> 8
   <212> PRT
   <213> Artificial Sequence
<220>
   <223> SLC39A11
<400> 1164
   Gly Ala Gly Phe Ser Thr Trp Arg
   1 5
<210> 1165
   <211> 10
   <212> PRT
   <213> Artificial Sequence
<220>
   <223> SLC39A12
<400> 1165
   Phe Tyr Leu His Ser Leu Leu Ser Leu Arg
   1 5 10
<210> 1166
   <211> 7
   <212> PRT
   <213> Artificial Sequence
<220>
   <223> SLC39A12
<400> 1166
   Gly Leu Ser Leu Ile Ser Lys
   1 5

<210> 1167
   <211> 9
   <212> PRT
   <213> Artificial Sequence
<220>
   <223> SLC39A13
<400> 1167
   Ala Gly Gly Ser Gln Pro Ala Leu Arg
   1 5
<210> 1168
   <211> 5
   <212> PRT
   <213> Artificial Sequence
<220>
   <223> SLC39A13
<400> 1168
   Ala Gly Phe Asp Arg
   1 5
<210> 1169
   <211> 4
   <212> PRT
   <213> Artificial Sequence
<220>
   <223> SLC39A13
<400> 1169
   Leu Asp Asn Lys
   1
<210> 1170
   <211> 12
   <212> PRT
   <213> Artificial Sequence
<220>
   <223> SLC39A14
<400> 1170
   Ala Leu Leu Asn His Leu Asp Val Gly Val Gly Arg
   1 5 10
<210> 1171
   <211> 13
   <212> PRT
   <213> Artificial Sequence
<220>
   <223> SLC39A14
<400> 1171
   Tyr Gly Glu Gly Asp Ser Leu Thr Leu Gln Gln Leu Lys
   1 5 10
<210> 1172
   <211> 9
   <212> PRT
   <213> Artificial Sequence
<220>
   <223> SLC39A2
<400> 1172
   Leu Val His Leu Gly Thr Ser Ser Arg
   1 5
<210> 1173
   <211> 4
   <212> PRT
   <213> Artificial Sequence
<220>
   <223> SLC39A2
<400> 1173
   Gly Pro Leu Arg
   1
<210> 1174
   <211> 8
   <212> PRT
   <213> Artificial Sequence
<220>
   <223> SLC39A2
<400> 1174
   Trp Phe Gln Ile Asp Ala Ala Arg
   1 5
<210> 1175
   <211> 5
   <212> PRT
   <213> Artificial Sequence
<220>
   <223> SLC39A2
<400> 1175
<21C> 1176
   <211> 5
   <212> PRT
   <213> Artificial Sequence
<220>
   <223> SLC39A3
<400> 1176
<210> 1177
   <211> 4
   <212> PRT
   <213> Artificial Sequence
<220>
   <223> SLC39A3
<400> 1177
   Asp Ala Ala Lys
   1
<210> 1178
   <211> 7
   <212> PRT
   <213> Artificial Sequence
<220>
   <223> SLC39A3
<400> 1178
   Ile Ile Glu Thr Asp Phe Glu
   1 5
<210> 1179
   <211> 5
   <212> PRT
   <213> Artificial Sequence
<220>
   <223> SLC39A3
<400> 1179
   Leu Leu Val Ala Lys
   1 5
<210> 1180
   <211> 7
   <212> PRT
   <213> Artificial Sequence
<220>
   <223> SLC39A4
<400> 1180
   Ala Ala Gly Gln Thr Pro Lys
   1 5
<210> 1181
   <211> 5
   <212> PRT
   <213> Artificial Sequence
<220>
   <223> SLC39A4
<400> 1181
   Leu Gly Val Gly Arg
   1 5
<210> 1182
   <211> 19
   <212> PRT
   <213> Artificial Sequence
<220>
   <223> SLC39A4
<400> 1182
   Ala Gly Leu Trp Ala Ser His Ala Asp His Leu Leu Ala Leu Leu Glu
   1 5 10 15
   Ser Pro Lys
<210> 1183
   <211> 12
   <212> PRT
   <213> Artificial Sequence
<220>
   <223> SLC39A4
<400> 1183
   Ala Leu Thr Pro Gly Leu Ser Trp Leu Leu Gln Arg
   1 5 10
<210> 1184
   <211> 10
   <212> PRT
   <213> Artificial Sequence
<220>
   <223> SLC39A5
<400> 1184
   Leu Gly Gln His Gly Pro Leu Thr Gly Arg
   1 5 10
<210> 1185
   <211> 12
   <212> PRT
   <213> Artificial Sequence
<220>
   <223> SLC39A5
<400> 1185
   Gly Pro Ala Pro Ser Gly Leu Asp Leu Leu His Arg
   1 5 10
<210> 1186
   <211> 9
   <212> PRT
   <213> Artificial Sequence
<220>
   <223> SLC39A5
<400> 1186
   Leu Leu His Ser Leu Gly Leu Gly Arg
   1 5
<210> 1187
   <211> 6
   <212> PRT
   <213> Artificial Sequence
<220>
   <223> SLC39A6
<400> 1187
   Val Asp Thr Asp Asp Arg
   1 5
<210> 1188
   <211> 11
   <212> PRT
   <213> Artificial Sequence
<220>
   <223> SLC39A6
<400> 1188
   Tyr Glu Ser Gln Leu Ser Thr Asn Glu Glu Lys
   1 5 10
<210> 1189
   <211> 6
   <212> PRT
   <213> Artificial Sequence
<220>
   <223> SLC39A6
<400> 1189
   Thr Glu Gly Tyr Leu Arg
   1 5
<210> 1190
   <211> 7
   <212> PRT
   <213> Artificial Sequence
<220>
   <223> SLC39A7
<400> 1190
   Gly Ser His Gly His Gly Arg
   1 5
<210> 1191
   <211> 17
   <212> PRT
   <213> Artificial Sequence
<220>
   <223> SLC39A7
<400> 1191
   Gly Gly His Gly His Ser His Gly His Gly His Ala His Ser His Thr
   1 5 10 15
   Arg
<210> 1192
   <211> 22
   <212> PRT
   <213> Artificial Sequence
<220>
   <223> SLC39A7
<400> 1192
   Gly His Gly His Asp His Glu His Ser His Gly Gly Tyr Gly Glu Ser
   1 5 10 15
   Gly Ala Pro Gly Ile Lys
   20
<210> 1193
   <211> 5
   <212> PRT
   <213> Artificial Sequence
<220>
   <223> SLC39A8
<400> 1193
   Thr His Gln Pro Lys
   1 5
<210> 1194
   <211> 7
   <212> PRT
   <213> Artificial Sequence
<220>
   <223> SLC39A8
<400> 1194
   Val Asp Ser Tyr Val Glu Lys
   1 5
<210> 1195
   <211> 16
   <212> PRT
   <213> Artificial Sequence
<220>
   <223> SLC39A8
<400> 1195
   Ala Val Ala Val Phe Gly Gly Phe Tyr Leu Leu Phe Phe Phe Glu Arg
   1 5 10 15
<210> 1196
   <211> 4
   <212> PRT
   <213> Artificial Sequence
<220>
   <223> SLC39A9
<400> 1196
   Gly Leu Ser Arg
   1
<210> 1197
   <211> 4
   <212> PRT
   <213> Artificial Sequence
<220>
   <223> SLC39A9
<400> 1197
   Ala Ala Glu Lys
   1
<210> 1198
   <211> 15
   <212> PRT
   <213> Artificial Sequence
<220>
   <223> SLC39A9
<400> 1198
   His His Gln Ala Ser Glu Thr His Asn Val Ile Ala Ser Asp Lys
   1 5 10 15
<210> 1199
   <211> 7
   <212> PRT
   <213> Artificial Sequence
<220>
   <223> SLC3A1
<400> 1199
   Asp Gly Asn Gly Asp Leu Lys
   1 5
<210> 1200
   <211> 8
   <212> PRT
   <213> Artificial Sequence
<220>
   <223> SLC3A1
<400> 1200
   Leu Ser Thr Asn Ser Ala Asp Lys
   1 5
<210> 1201
   <211> 10
   <212> PRT
   <213> Artificial Sequence
<220>
   <223> SLC3A1
<400> 1201
   Val Asp Thr Ser Gly Ile Phe Leu Asp Lys
   1 5 10
<210> 1202
   <211> 11
   <212> PRT
   <213> Artificial Sequence
<220>
   <223> SLC3A1
<400> 1202
   Gly Val Asp Gly Phe Ser Leu Asp Ala Val Lys
   1 5 10
<210> 1203
   <211> 10
   <212> PRT
   <213> Artificial Sequence
<220>
   <223> SLC3A1
<400> 1203
   Leu Asp Tyr Ile Thr Ala Leu Asn Ile Lys
   1 5 10
<210> 1204
   <211> 12
   <212> PRT
   <213> Artificial Sequence
<220>
   <223> SLC3A2
<400> 1204
   Leu Leu Thr Ser Phe Leu Pro Ala Gln Leu Leu Arg
   1 5 10
<210> 1205
   <211> 10
   <212> PRT
   <213> Artificial Sequence
<220>
   <223> SLC3A2
<400> 1205
   Val Ile Leu Asp Leu Thr Pro Asn Tyr Arg
   1 5 10
<210> 1206
   <211> 5
   <212> PRT
   <213> Artificial Sequence
<220>
   <223> SLC3A2
<400> 1206
   Leu Ser Asp Gln Arg
   1 5
<210> 1207
   <211> 6
   <212> PRT
   <213> Artificial Sequence
<220>
   <223> SLC3A2
<400> 1207
   Phe Thr Gly Leu Ser Lys
   1 5
<210> 1208
   <211> 11
   <212> PRT
   <213> Artificial Sequence
<220>
   <223> SLC3A2
<400> 1208
   Ala Asp Leu Leu Leu Ser Thr Gln Pro Gly Arg
   1 5 10
<210> 1209
   <211> 7
   <212> PRT
   <213> Artificial Sequence
<220>
   <223> SLC40A1
<400> 1209
   Thr Pro Ala Leu Ala Val Lys
   1 5
<210> 1210
   <211> 6
   <212> PRT
   <213> Artificial Sequence
<220>
   <223> SLC40A1
<400> 1210
   Ala Gly Asp His Asn Arg
   1 5
<210> 1211
   <211> 11
   <212> PRT
   <213> Artificial Sequence
<220>
   <223> SLC40A1
<400> 1211
   Phe Ile Gln Gly Glu Ser Ile Thr Pro Thr Lys
   1 5 10
<210> 1212
   <211> 9
   <212> PRT
   <213> Artificial Sequence
<220>
   <223> SLC41A1
<400> 1212
   Gly Pro Ala Pro Pro Ser Pro Leu Lys
   1 5
<210> 1213
   <211> 15
   <212> PRT
   <213> Artificial Sequence
<220>
   <223> SLC41A1
<400> 1213
   Val Thr Glu Val Phe Ile Leu Val Pro Ala Leu Leu Gly Leu Lys
   1 5 10 15
<210> 1214
   <211> 6
   <212> PRT
   <213> Artificial Sequence
<220>
   <223> SLC41A1
<400> 1214
   Asp Thr Asp Val Gly Asp
   1 5
<210> 1215
   <211> 9
   <212> PRT
   <213> Artificial Sequence
<220>
   <223> SLC41A2
<400> 1215
   Leu Ser Thr Ala Val Asn Ile Gly Lys
   1 5
<210> 1216
   <211> 8
   <212> PRT
   <213> Artificial Sequence
<220>
   <223> SLC41A2
<400> 1216
   Leu Asn Thr Ile Gln Ser Asp Lys
   1 5
<210> 1217
   <211> 10
   <212> PRT
   <213> Artificial Sequence
<220>
   <223> SLC41A2
<400> 1217
   Thr Phe Phe Gly Pro Gly Val Asn Asn Lys
   1 5 10
<210> 1218
   <211> 13
   <212> PRT
   <213> Artificial Sequence
<220>
   <223> SLC41A3
<400> 1218
   Asp Leu Leu Thr Leu Val Pro Pro Leu Val Gly Leu Lys
   1 5 10
<210> 1219
   <211> 7
   <212> PRT
   <213> Artificial Sequence
<220>
   <223> SLC41A3
<400> 1219
   Gln Ser Pro Pro Ile Val Lys
   1 5
<210> 1220
   <211> 9
   <212> PRT
   <213> Artificial Sequence
<220>
   <223> SLC42A1
<400> 1220
   Phe Leu Thr Pro Leu Phe Thr Thr Lys
   1 5
<210> 1221
   <211> 6
   <212> PRT
   <213> Artificial Sequence
<220>
   <223> SLC42A1
<400> 1221
   Phe Asn Ile Gly Ile Lys
   1 5
<210> 1222
   <211> 9
   <212> PRT
   <213> Artificial Sequence
<220>
   <223> SLC42A2
<400> 1222
   Val Leu Tyr Arg Pro Gln Leu Glu Lys
   1 5
<210> 1223
   <211> 9
   <212> PRT
   <213> Artificial Sequence
<220>
   <223> SLC42A2
<400> 1223
   Phe Phe Thr Pro Ile Leu Glu Ser Lys
   1 5
<210> 1224
   <211> 13
   <212> PRT
   <213> Artificial Sequence
<220>
   <223> SLC42A3
<400> 1224
   Tyr Asp Phe Glu Ala Asp Ala His Trp Trp Ser Glu Arg
   1 5 10
<210> 1225
   <211> 6
   <212> PRT
   <213> Artificial Sequence
<220>
   <223> SLC42A3
<400> 1225
   Gly Asp Ser Gln His Arg
   1 5
<210> 1226
   <211> 4
   <212> PRT
   <213> Artificial Sequence
<220>
   <223> SLC42A3
<400> 1226
   Thr Gln Gly Lys
   1
<210> 1227
   <211> 19
   <212> PRT
   <213> Artificial Sequence
<220>
   <223> SLC42A3
<400> 1227
<210> 1228
   <211> 9
   <212> PRT
   <213> Artificial Sequence
<220>
   <223> SLC43A1
<400> 1228
   Leu Ser Gly Leu Ala Leu Asp His Lys
   1 5
<210> 1229
   <211> 9
   <212> PRT
   <213> Artificial Sequence
<220>
   <223> SLC43A1
<400> 1229
   Gly Thr Ser Glu Asn Leu Pro Glu Arg
   1 5
<210> 1230
   <211> 6
   <212> PRT
   <213> Artificial Sequence
<220>
   <223> SLC43A1
<400> 1230
   Asp Gly Val Ala Thr Lys
   1 5
<210> 1231
   <211> 8
   <212> PRT
   <213> Artificial Sequence
<220>
   <223> SLC43A2
<400> 1231
   Phe Leu Val Ser Gly Asp Gln Lys
   1 5
<210> 1232
   <211> 10
   <212> PRT
   <213> Artificial Sequence
<220>
   <223> SLC43A2
<400> 1232
   Glu Gln Val Ala Leu Gln Glu Gly His Lys
   1 5 10
<210> 1233
   <211> 9
   <212> PRT
   <213> Artificial Sequence
<220>
   <223> SLC43A2
<400> 1233
   Phe Ser Trp Leu Gly Phe Asp His Lys
   1 5
<210> 1234
   <211> 9
   <212> PRT
   <213> Artificial Sequence
<220>
   <223> SLC43A3
<400> 1234
   Glu Glu Thr Pro Gly Ala Gly Gln Lys
   1 5
<210> 1235
   <211> 8
   <212> PRT
   <213> Artificial Sequence
<220>
   <223> SLC43A3
<400> 1235
   Glu Thr Ala Glu His Glu Asn Arg
   1 5
<210> 1236
   <211> 7
   <212> PRT
   <213> Artificial Sequence
<220>
   <223> SLC43A3
<400> 1236
   Glu Ser Pro Ser Ala Ile Ala
   1 5
<210> 1237
   <211> 12
   <212> PRT
   <213> Artificial Sequence
<220>
   <223> SLC45A1
<400> 1237
   Ser Gln Val Thr Gly Tyr Ser Gly Ser Val Thr Arg
   1 5 10
<210> 1238
   <211> 19
   <212> PRT
   <213> Artificial Sequence
<220>
   <223> SLC45A1
<400> 1238
   His Leu Ser His Arg Asp Ile Gly Ile Ala Leu Ala Asp Val Thr Gly
   1 5 10 15
   Asn His Lys
<210> 1239
   <211> 10
   <212> PRT
   <213> Artificial Sequence
<220>
   <223> SLC45A1
<400> 1239
   Val Gly Ser Leu Asp Thr Ser Lys Pro Arg
   1 5 10
<210> 1240
   <211> 7
   <212> PRT
   <213> Artificial Sequence
<220>
   <223> SLC45A1
<400> 1240
   Ala Pro Asp Gly Phe Tyr Arg
   1 5
<210> 1241
   <211> 11
   <212> PRT
   <213> Artificial Sequence
<220>
   <223> SLC45A2
<400> 1241
   Leu Ile Met His Ser Met Ala Met Phe Gly Arg
   1 5 10
<210> 1242
   <211> 10
   <212> PRT
   <213> Artificial Sequence
<220>
   <223> SLC45A2
<400> 1242
   Met Gly Ser Asn Ser Gly Gln Ala Gly Arg
   1 5 10
<210> 1243
   <211> 4
   <212> PRT
   <213> Artificial Sequence
<220>
   <223> SLC45A2
<400> 1243
   His Ile Tyr Lys
   1
<210> 1244
   <211> 8
   <212> PRT
   <213> Artificial Sequence
<220>
   <223> SLC45A3
<400> 1244
   Asn Leu Gly Ala Leu Leu Pro Arg
   1 5
<210> 1245
   <211> 10
   <212> PRT
   <213> Artificial Sequence
<220>
   <223> SLC45A3
<400> 1245
   Val Val Val Gly Glu Pro Thr Glu Ala Arg
   1 5 10
<210> 1246
   <211> 6
   <212> PRT
   <213> Artificial Sequence
<220>
   <223> SLC45A3
<400> 1246
   Gln Val Phe Leu Pro Lys
   1 5
<210> 1247
   <211> 5
   <212> PRT
   <213> Artificial Sequence
<220>
   <223> SLC45A3
<400> 1247
   Val Val Pro Gly Arg
   1 5
<210> 1248
   <211> 5
   <212> PRT
   <213> Artificial Sequence
<220>
   <223> SLC45A3
<400> 1248
   Ser Asp Leu Ala Lys
   1 5
<210> 1249
   <211> 8
   <212> PRT
   <213> Artificial Sequence
<220>
   <223> SLC45A4
<400> 1249
   Ala Ser His His Pro Pro Ala Arg
   1 5
<210> 1250
   <211> 9
   <212> PRT
   <213> Artificial Sequence
<220>
   <223> SLC45A4
<400> 1250
   Gly Leu Arg Pro Ser Pro Gly Asp Arg
   1 5
<210> 1251
   <211> 6
   <212> PRT
   <213> Artificial Sequence
<220>
   <223> SLC45A4
<400> 1251
   Asn Tyr Leu Val Pro Leu
   1 5
<210> 1252
   <211> 14
   <212> PRT
   <213> Artificial Sequence
<220>
   <223> SLC4A1
<400> 1252
   Ile Pro Pro Asp Ser Glu Ala Thr Leu Val Leu Val Gly Arg
   1 5 10
<210> 1253
   <211> 13
   <212> PRT
   <213> Artificial Sequence
<220>
   <223> SLC4A1
<400> 1253
   Ala Asp Phe Leu Glu Gln Pro Val Leu Gly Phe Val Arg
   1 5 10
<210> 1254
   <211> 8
   <212> PRT
   <213> Artificial Sequence
<220>
   <223> SLC4A1
<400> 1254
   Leu Ser Val Pro Asp Gly Phe Lys
   1               5
<210> 1255
   <211> 10
   <212> PRT
   <213> Artificial Sequence
<220>
   <223> SLC4A1
<400> 1255
   Gly Trp Val Ile His Pro Leu Gly Leu Arg
   1               5                   10
<210> 1256
   <211> 14
   <212> PRT
   <213> Artificial Sequence
<220>
   <223> SLC4A1
<400> 1256
   Ala Ser Thr Pro Gly Ala Ala Ala Gln Ile Gln Glu Val Lys
   1               5                   10
<210> 1257
   <211> 9
   <212> PRT
   <213> Artificial Sequence
<220>
   <223> SLC4A1
<400> 1257
   Tyr His Pro Asp Val Pro Tyr Val Lys
   1               5
<210> 1258
   <211> 8
   <212> PRT
   <213> Artificial Sequence
<220>
   <223> SLC4A1
<400> 1258
   Val Leu Leu Pro Leu Ile Phe Arg
   1               5
<210> 1259
   <211> 10
   <212> PRT
   <213> Artificial Sequence
<220>
   <223> SLC4A10
<400> 1259
   Asn Leu Leu Ile Thr Ala Asp Asn Ser Lys
   1               5                   10
<210> 1260
   <211> 10
   <212> PRT
   <213> Artificial Sequence
<220>
   <223> SLC4A10
<400> 1260
   Ile Phe Gly Gly Leu Ile Leu Asp Ile Lys
   1               5                   10
<210> 1261
   <211> 11
   <212> PRT
   <213> Artificial Sequence
<220>
   <223> SLC4A10
<400> 1261
   Phe Leu Phe Ile Leu Leu Gly Pro Leu Gly Lys
   1               5                   10
<210> 1262
   <211> 7
   <212> PRT
   <213> Artificial Sequence
<220>
   <223> SLC4A10
<400> 1262
   Thr Val Val Ala Phe Val Arg
   1               5
<210> 1263
   <211> 7
   <212> PRT
   <213> Artificial Sequence
<220>
   <223> SLC4A10
<400> 1263
   Gly Leu Gly Gly Gln Gln Lys
   1 5
<210> 1264
   <211> 7
   <212> PRT
   <213> Artificial Sequence
<220>
   <223> SLC4A10
<400> 1264
   Ser Phe Ala Asp Ile Gly Lys
   1 5
<210> 1265
   <211> 13
   <212> PRT
   <213> Artificial Sequence
<220>
   <223> SLC4A10
<400> 1265
   Thr Leu Phe Ile Gly Val His Val Pro Leu Gly Gly Arg
   1 5 10
<210> 1266
   <211> 12
   <212> PRT
   <213> Artificial Sequence
<220>
   <223> SLC4A11
<400> 1266
   Glu Gln Thr Ala Tyr Pro Pro Thr His Tyr Ile Arg
   1 5 10
<210> 1267
   <211> 14
   <212> PRT
   <213> Artificial Sequence
<220>
   <223> SLC4A11
<400> 1267
   Val Glu Asn Gly His Ile Tyr Asp Thr Ile Val Asn Val Lys
   1 5 10
<210> 1268
   <211> 13
   <212> PRT
   <213> Artificial Sequence
<220>
   <223> SLC4A11
<400> 1268
   Tyr Tyr Tyr Gly His Tyr Leu Asp Asp Tyr His Thr Lys
   1 5 10
<210> 1269
   <211> 15
   <212> PRT
   <213> Artificial Sequence
<220>
   <223> SLC4A11
<400> 1269
   Phe Pro Leu Tyr Pro Leu Asp Phe Thr Asp Gly Ile Ile Gly Lys
   1 5 10 15
<210> 1270
   <211> 7
   <212> PRT
   <213> Artificial Sequence
<220>
   <223> SLC4A11
<400> 1270
   Asp Phe Val Pro Phe Gly Lys
   1 5
<210> 1271
   <211> 7
   <212> PRT
   <213> Artificial Sequence
<220>
   <223> SLC4A11
<400> 1271
   Glu Ala Leu Val His Gln Arg
   1 5
<210> 1272
   <211> 10
   <212> PRT
   <213> Artificial Sequence
<220>
   <223> SLC4A11
<400> 1272
   Phe Val Ile Leu Val Leu Ala Pro Pro Lys
   1 5 10
<210> 1273
   <211> 19
   <212> PRT
   <213> Artificial Sequence
<220>
   <223> SLC4A11
<400> 1273
   Val His Leu Leu Ser Asp Thr Ile Gln Gly Val Thr Ala Thr Val Thr
   1 5 10 15
   Gly Val Arg
<210> 1274
   <211> 5
   <212> PRT
   <213> Artificial Sequence
<220>
   <223> SLC4A2
<400> 1274
   Asn Gln Glu Leu Arg
   1 5
<210> 1275
   <211> 5
   <212> PRT
   <213> Artificial Sequence
<220>
   <223> SLC4A2
<400> 1275
   Ala Leu Leu Leu Lys
   1 5
<210> 1276
   <211> 14
   <212> PRT
   <213> Artificial Sequence
<220>
   <223> SLC4A2
<400> 1276
   Ile Pro Pro Asp Ser Glu Ala Thr Leu Val Leu Val Gly Arg
   1 5 10
<210> 1277
   <211> 13
   <212> PRT
   <213> Artificial Sequence
<220>
   <223> SLC4A2
<400> 1277
   Ala Asp Phe Leu Glu Gln Pro Val Leu Gly Phe Val Arg
   1 5 10
<210> 1278
   <211> 8
   <212> PRT
   <213> Artificial Sequence
<220>
   <223> SLC4A2
<400> 1278
   Leu Ser Val Pro Asp Gly Phe Lys
   1 5
<210> 1279
   <211> 10
   <212> PRT
   <213> Artificial Sequence
<220>
   <223> SLC4A2
<400> 1279
   Gly Trp Val Ile His Pro Leu Gly Leu Arg
   1 5 10
<210> 1280
   <211> 14
   <212> PRT
   <213> Artificial Sequence
<220>
   <223> SLC4A2
<400> 1280
   Ala Ser Thr Pro Gly Ala Ala Ala Gln Ile Gln Glu Val Lys
   1 5 10
<210> 1281
   <211> 9
   <212> PRT
   <213> Artificial Sequence
<220>
   <223> SLC4A2
<400> 1281
   Tyr His Pro Asp Val Pro Tyr Val Lys
   1 5
<210> 1282
   <211> 8
   <212> PRT
   <213> Artificial Sequence
<220>
   <223> SLC4A2
<400> 1282
   Val Leu Leu Pro Leu Ile Phe Arg
   1 5
<210> 1283
   <211> 14
   <212> PRT
   <213> Artificial Sequence
<220>
   <223> SLC4A3
<400> 1283
   Leu Thr Val Pro Thr Gly Leu Ser Val Thr Ser Pro Asp Lys
   1 5 10
<210> 1284
   <211> 9
   <212> PRT
   <213> Artificial Sequence
<220>
   <223> SLC4A3
<400> 1284
   Tyr Pro His Tyr Pro Ser Asp Leu Arg
   1   5
<210> 1285
   <211> 10
   <212> PRT
   <213> Artificial Sequence
<220>
   <223> SLC4A3
<400> 1285
   Thr Gly Ser Val Phe Gly Gly Leu Val Arg
   1 5 10
<210> 1286
   <211> 8
   <212> PRT
   <213> Artificial Sequence
<220>
   <223> SLC4A3
<400> 1286
   Gly Gly Tyr Thr Ala Pro Gly Lys
   1 5
<210> 1287
   <211> 7
   <212> PRT
   <213> Artificial Sequence
<220>
   <223> SLC4A3
<400> 1287
   Ser Val Ala Ala Phe Gln Arg
   1 5
<210> 1288
   <211> 7
   <212> PRT
   <213> Artificial Sequence
<220>
   <223> SLC4A3
<400> 1288
   Asp Ser Gly Phe Phe Pro Arg
   1 5
<210> 1289
   <211> 8
   <212> PRT
   <213> Artificial Sequence
<220>
   <223> SLC4A3
<400> 1289
   His Ser His Pro Asn Asp Asp Lys
   1 5
<210> 1290
   <211> 11
   <212> PRT
   <213> Artificial Sequence
<220>
   <223> SLC4A3
<400> 1290
   Gly Ser Pro Ser Gly Leu Ala Pro Ile Leu Arg
   1 5 10
<210> 1291
   <211> 14
   <212> PRT
   <213> Artificial Sequence
<220>
   <223> SLC4A3
<400> 1291
   Asp Gly Asp Gly Thr Thr Asp Leu Ala Leu Ser Ser Pro Arg
   1 5 10
<210> 1292
   <211> 7
   <212> PRT
   <213> Artificial Sequence
<220>
   <223> SLC4A3
<400> 1292
   Leu Pro Pro Thr Ser Ala Arg
   1 5
<210> 1293
   <211> 6
   <212> PRT
   <213> Artificial Sequence
<220>
   <223> SLC4A3
<400> 1293
   Leu Pro Pro Pro His Lys
   1 5
<210> 1294
   <211> 9
   <212> PRT
   <213> Artificial Sequence
<220>
   <223> SLC4A4
<400> 1294
   His Gln Pro Asp Phe Ile Tyr Leu Arg
   1 5
<210> 1295
   <211> 12
   <212> PRT
   <213> Artificial Sequence
<220>
   <223> SLC4A4
<400> 1295
   Leu Ala Asp Tyr Tyr Pro Ile Asn Ser Asn Phe Lys
   1 5 10
<210> 1296
   <211> 7
   <212> PRT
   <213> Artificial Sequence
<220>
   <223> SLC4A4
<400> 1296
   Leu Leu Phe Asn Phe Ser Lys
   1 5
<210> 1297
   <211> 7
   <212> PRT
   <213> Artificial Sequence
<220>
   <223> SLC4A4
<400> 1297
   Ser Leu Pro Ser Ser Asp Lys
   1 5
<210> 1298
   <211> 9
   <212> PRT
   <213> Artificial Sequence
<220>
   <223> SLC4A4
<400> 1298
   Phe Leu Phe Ile Leu Leu Gly Pro Lys
   1 5
<210> 1299
   <211> 7
   <212> PRT
   <213> Artificial Sequence
<220>
   <223> SLC4A4
<400> 1299
   Ser Leu Ala Asp Ile Gly Lys
   1 5
<210> 1300
   <211> 4
   <212> PRT
   <213> Artificial Sequence
<220>
   <223> SLC4A4
<400> 1300
   Ser Asn Leu Arg
   1
<210> 1301
   <211> 7
   <212> PRT
   <213> Artificial Sequence
<220>
   <223> SLC4A8
<400> 1301
   Leu Leu Ser Ser Pro Gly Lys
   1 5
<210> 1302
   <211> 5
   <212> PRT
   <213> Artificial Sequence

<220>
   <223> SLC4A8
<400> 1302
   Tyr Phe Pro Thr Arg
   1 5
<210> 1303
   <211> 10
   <212> PRT
   <213> Artificial Sequence
<220>
   <223> SLC4A8
<400> 1303
   Leu Phe Gly Gly Leu Val Leu Asp Ile Lys
   1 5 10
<210> 1304
   <211> 11
   <212> PRT
   <213> Artificial Sequence
<220>
   <223> SLC4A8
<400> 1304
   Phe Leu Phe Ile Leu Leu Gly Pro Val Gly Lys
   1 5 10
<210> 1305
   <211> 8
   <212> PRT
   <213> Artificial Sequence
<220>
   <223> SLC4A8
<400> 1305
   Asn Asn Leu Ile Pro Ile Val Arg
   1 5
<210> 1306
   <211> 5
   <212> PRT
   <213> Artificial Sequence
<220>
   <223> SLC4A8
<400> 1306
   Thr His Gly Gln Lys
   1 5
<210> 1307
   <211> 7
   <212> PRT
   <213> Artificial Sequence
<220>
   <223> SLC4A8
<400> 1307
   Thr Leu Tyr Val Gly Val Arg
   1 5
<210> 1308
   <211> 9
   <212> PRT
   <213> Artificial Sequence
<220>
   <223> SLC4A9
<400> 1308
   His Gln Pro Asp Leu Leu Leu Leu Arg
   1 5
<210> 1309
   <211> 7
   <212> PRT
   <213> Artificial Sequence
<220>
   <223> SLC4A9
<400> 1309
   Phe Phe Pro Ser Val Val Arg
   1 5
<210> 1310
   <211> 10
   <212> PRT
   <213> Artificial Sequence
<220>
   <223> SLC4A9
<400> 1310
   Asp Tyr Ser Leu Asp Tyr Leu Pro Phe Arg
   1 5 10
<210> 1311
   <211> 10
   <212> PRT
   <213> Artificial Sequence
<220>
   <223> SLC4A9
<400> 1311
   Leu Phe Gly Gly Leu Ile Gln Asp Val Arg
   1 5 10
<210> 1312
   <211> 11
   <212> PRT
   <213> Artificial Sequence
<220>
   <223> SLC5A1
<400> 1312
   Gly Thr Val Gly Gly Phe Phe Leu Ala Gly Arg
   1 5 10
<210> 1313
   <211> 12
   <212> PRT
   <213> Artificial Sequence
<220>
   <223> SLC5A12
<400> 1313
   Leu His Ile Phe Asp Phe Asp Val Asp Pro Leu Arg
   1 5 10
<210> 1314
   <211> 11
   <212> PRT
   <213> Artificial Sequence
<220>
   <223> SLC5A2
<400> 1314
   Gly Thr Val Gly Gly Tyr Phe Leu Ala Gly Arg
   1 5 10
<210> 1315
   <211> 15
   <212> PRT
   <213> Artificial Sequence
<220>
   <223> SLC5A2
<400> 1315
   Leu Tyr Leu Ser Val Leu Ser Leu Phe Leu Tyr Ile Phe Thr Lys
   1 5 10 15
<210> 1316
   <211> 6
   <212> PRT
   <213> Artificial Sequence
<220>
   <223> SLC5A2
<400> 1316
   Ser Leu Thr His Ile Lys
   1 5
<210> 1317
   <211> 6
   <212> PRT
   <213> Artificial Sequence
<220>
   <223> SLC5A2
<400> 1317
   Leu Val Phe Ser Leu Arg
   1 5
<210> 1318
   <211> 6
   <212> PRT
   <213> Artificial Sequence
<220>
   <223> SLC5A3
<400> 1318
   Asn Ile Ala His Ala Lys
   1 5
<210> 1319
   <211> 8
   <212> PRT
   <213> Artificial Sequence
<220>
   <223> SLC5A3
<400> 1319
   Leu Ile Leu Ala Phe Ala Tyr Arg
   1 5
<210> 1320
   <211> 11
   <212> PRT
   <213> Artificial Sequence
<220>
   <223> SLC5A4
<400> 1320
   Gly Thr Ile Gly Gly Phe Phe Leu Ala Gly Arg
   1 5 10
<210> 1321
   <211> 8
   <212> PRT
   <213> Artificial Sequence
<220>
   <223> SLC5A4
<400> 1321
   Ala Asp Ser Phe His Ile Phe Arg
   1               5
<210> 1322
   <211> 6
   <212> PRT
   <213> Artificial Sequence
<220>
   <223> SLC5A5
<400> 1322
   Leu Val Ile Ile Ser Lys
   1               5
<210> 1323
   <211> 8
   <212> PRT
   <213> Artificial Sequence
<220>
   <223> SLC5A5
<400> 1323
   Val Leu Pro Ser Ser Ala Ala Arg
   1               5
<210> 1324
   <211> 7
   <212> PRT
   <213> Artificial Sequence
<220>
   <223> SLC5A5
<400> 1324
   Leu Phe Phe Leu Gly Gln Lys
   1               5
<210> 1325
   <211> 7
   <212> PRT
   <213> Artificial Sequence
<220>
   <223> SLC6A1
<400> 1325
   Ala Ala Asp Leu Pro Asp Arg
   1 5
<210> 1326
   <211> 8
   <212> PRT
   <213> Artificial Sequence
<220>
   <223> SLC6A1
<400> 1326
   Gly Val Thr Leu Pro Gly Ala Lys
   1 5
<210> 1327
   <211> 8
   <212> PRT
   <213> Artificial Sequence
<220>
   <223> SLC6A11
<400> 1327
   Ala Leu Pro Leu Gly Asn Gly Lys
   1 5
<210> 1328
   <211> 9
   <212> PRT
   <213> Artificial Sequence
<220>
   <223> SLC6A11
<400> 1328
   Phe Tyr Leu Tyr Pro Asp Leu Ser Arg
   1 5
<210> 1329
   <211> 9
   <212> PRT
   <213> Artificial Sequence
<220>
   <223> SLC6A11
<400> 1329
   Leu Thr Thr Pro Ser Thr Asp Leu Lys
   1 5
<210> 1330
   <211> 9
   <212> PRT
   <213> Artificial Sequence
<220>
   <223> SLC6A14
<400> 1330
   Gly Ala Thr Leu Glu Gly Ala Ser Lys
   1 5
<210> 1331
   <211> 7
   <212> PRT
   <213> Artificial Sequence
<220>
   <223> SLC6A15
<400> 1331
   Gly Ile Gln Ser Ser Gly Lys
   1 5
<210> 1332
   <211> 12
   <212> PRT
   <213> Artificial Sequence
<220>
   <223> SLC6A15
<400> 1332
   Ala Phe Leu Leu Asn Gly Ser Ile Asp Gly Ile Arg
   1 5 10
<210> 1333
   <211> 10
   <212> PRT
   <213> Artificial Sequence
<220>
   <223> SLC6A16
<400> 1333
   Phe Gly Leu Gln Gln Leu Val Val Ala Lys
   1 5 10
<210> 1334
   <211> 12
   <212> PRT
   <213> Artificial Sequence
<220>
   <223> SLC6A17
<400> 1334
   Ile Leu Gly Tyr Leu Asn Thr Asn Val Leu Ser Arg
   1 5 10
<210> 1335
   <211> 17
   <212> PRT
   <213> Artificial Sequence
<220>
   <223> SLC6A17
<400> 1335
   His Phe His Leu Leu Ser Asp Gly Ser Asn Thr Leu Ser Val Ser Tyr
   1 5 10 15
   Lys
<210> 1336
   <211> 7
   <212> PRT
   <213> Artificial Sequence
<220>
   <223> SLC6A18
<400> 1336
   Val Ala Gln Leu Pro Leu Lys
   1 5
<210> 1337
   <211> 11
   <212> PRT
   <213> Artificial Sequence
<220>
   <223> SLC6A19
<400> 1337
   Leu Val Leu Pro Asn Pro Gly Leu Asp Ala Arg
   1 5 10
<210> 1338
   <211> 7
   <212> PRT
   <213> Artificial Sequence
<220>
   <223> SLC6A2
<400> 1338
   Asp Gly Asp Ala Gln Pro Arg
   1 5
<210> 1339
   <211> 10
   <212> PRT
   <213> Artificial Sequence
<220>
   <223> SLC6A20
<400> 1339
   Gly Tyr Leu Ala Ser Ala Tyr Pro Ser Lys
   1 5 10
<210> 1340
   <211> 8
   <212> PRT
   <213> Artificial Sequence
<220>
   <223> SLC6A20
<400> 1340
   Ile Ile Ser Ser His Leu Pro Lys
   1 5
<210> 1341
   <211> 7
   <212> PRT
   <213> Artificial Sequence
<220>
   <223> SLC6A20
<400> 1341
   Gly Asp Ala Asp Pro Val Ala
   1 5
<210> 1342
   <211> 12
   <212> PRT
   <213> Artificial Sequence
<220>
   <223> SLC6A3
<400> 1342
   Gly Val Thr Leu Pro Gly Ala Ile Asp Gly Ile Arg
   1 5 10
<210> 1343
   <211> 9
   <212> PRT
   <213> Artificial Sequence
<220>
   <223> SLC6A3
<400> 1343
   Ala Tyr Leu Ser Val Asp Phe Tyr Arg
   1 5
<210> 1344
   <211> 10
   <212> PRT
   <213> Artificial Sequence
<220>
   <223> SLC6A3
<400> 1344
   His Ser Val Pro Ile Gly Asp Val Ala Lys
   1 5 10
<210> 1345
   <211> 8
   <212> PRT
   <213> Artificial Sequence
<220>
   <223> SLC6A4
<400> 1345
   Val Val Pro Thr Pro Gly Asp Lys
   1 5
<210> 1346
   <211> 7
   <212> PRT
   <213> Artificial Sequence
<220>
   <223> SLC6A4
<400> 1346
   His Val Leu Gln Ile His Arg
   1 5
<210> 1347
   <211> 9
   <212> PRT
   <213> Artificial Sequence
<220>
   <223> SLC6A4
<400> 1347
   Leu Ile Ile Thr Pro Gly Thr Phe Lys
   1 5
<210> 1348
   <211> 15
   <212> PRT
   <213> Artificial Sequence
<220>
   <223> SLC6A5
<400> 1348
   Ser Ala Ser Thr Gly Ala Gln Thr Phe Gln Ser Ala Asp Ala Arg
   1 5 10 15
<210> 1349
   <211> 9
   <212> PRT
   <213> Artificial Sequence
<220>
   <223> SLC6A5
<400> 1349
   Ala Gln Ala Ala Ser Ala Ala Leu Arg
   1 5
<210> 1350
   <211> 16
   <212> PRT
   <213> Artificial Sequence
<220>
   <223> SLC6A6
<400> 1350
   Asn Val Leu Ser Leu Ser Pro Gly Ile Asp His Pro Gly Ser Leu Lys
   1 5 10 15
<210> 1351
   <211> 12
   <212> PRT
   <213> Artificial Sequence
<220>
   <223> SLC6A6
<400> 1351
   Gly Leu Thr Leu Pro Gly Ala Gly Ala Gly Ile Lys
   1 5 10
<210> 1352
   <211> 9
   <212> PRT
   <213> Artificial Sequence
<220>
   <223> SLC6A6
<400> 1352
   Phe Tyr Leu Tyr Pro Asp Ile Thr Arg
   1 5
<210> 1353
   <211> 8
   <212> PRT
   <213> Artificial Sequence
<220>
   <223> SLC6A6
<400> 1353
   Tyr Val Pro Leu Thr Tyr Asn Lys
   1 5
<210> 1354
   <211> 6
   <212> PRT
   <213> Artificial Sequence
<220>
   <223> SLC6A6
<400> 1354
   Tyr Leu Leu Thr Pro Arg
   1 5
<210> 1355
   <211> 7
   <212> PRT
   <213> Artificial Sequence
<220>
   <223> SLC6A7
<400> 1355
   Leu Gln Gly Ala His Leu Arg
   1 5
<210> 1356
   <211> 6
   <212> PRT
   <213> Artificial Sequence
<220>
   <223> SLC6A7
<400> 1356
   Ile Ser Pro Leu Phe Lys
   1 5
<210> 1357
   <211> 17
   <212> PRT
   <213> Artificial Sequence
<220>
   <223> SLC6A7
<400> 1357
   Gly Ile Gln Phe Tyr Leu Thr Pro Gln Phe His His Leu Leu Ser Ser
   1 5 10 15
   Lys
<210> 1358
   <211> 14
   <212> PRT
   <213> Artificial Sequence
<220>
   <223> SLC6A8
<400> 1358
   Gly Pro Leu Ile Ala Pro Gly Pro Asp Gly Ala Pro Ala Lys
   1 5 10
<210> 1359
   <211> 13
   <212> PRT
   <213> Artificial Sequence
<220>
   <223> SLC6A8
<400> 1359
   Gly Asp Gly Pro Val Gly Leu Gly Thr Pro Gly Gly Arg
   1 5 10
<210> 1360
   <211> 6
   <212> PRT
   <213> Artificial Sequence
<220>
   <223> SLC6A8
<400> 1360
   Leu Ala Val Pro Pro Arg
   1 5
<210> 1361
   <211> 8
   <212> PRT
   <213> Artificial Sequence
<220>
   <223> SLC7A1
<400> 1361
   Phe Leu Ala Asn Val Asn Asp Arg
   1 5
<210> 1362
   <211> 9
   <212> PRT
   <213> Artificial Sequence
<220>
   <223> SLC7A10
<400> 1362
   Ile Gln Asp Met Phe Thr Gly Gly Lys
   1 5
<210> 1363
   <211> 11
   <212> PRT
   <213> Artificial Sequence
<220>
   <223> SLC7A11
<400> 1363
   Gly Gly Tyr Leu Gln Gly Asn Val Asn Gly Arg
   1 5 10
<210> 1364
   <211> 7
   <212> PRT
   <213> Artificial Sequence
<220>
   <223> SLC7A11
<400> 1364
   Leu Pro Ser Leu Gly Asn Lys
   1 5
<210> 1365
   <211> 7
   <212> PRT
   <213> Artificial Sequence
<220>
   <223> SLC7A11
<400> 1365
   Gly Gln Thr Gln Asn Phe Lys
   1               5
<210> 1366
   <211> 6
   <212> PRT
   <213> Artificial Sequence
<220>
   <223> SLC7A11
<400> 1366
   Asp Ala Phe Ser Gly Arg
   1               5
<210> 1367
   <211> 6
   <212> PRT
   <213> Artificial Sequence
<220>
   <223> SLC7A11
<400> 1367
   Asp Ser Ser Ile Thr Arg
   1               5
<210> 1368
   <211> 7
   <212> PRT
   <213> Artificial Sequence
<220>
   <223> SLC7A11
<400> 1368
   Leu Phe Tyr Val Ala Ser Arg
   1               5
<210> 1369
   <211> 17
   <212> PRT
   <213> Artificial Sequence
<220>
   <223> SLC7A13
<400> 1369
   Val Ser Ile Leu Ser Phe Ile Ser Leu Thr Gly Val Val Phe Leu Ile
   1 5 10 15
   Arg
<210> 1370
   <211> 23
   <212> PRT
   <213> Artificial Sequence
<220>
   <223> SLC7A13
<400> 1370
   Val Phe Leu Ser Phe Pro Leu Ala Thr Ile Val Ile Asp Val Gly Leu
   1 5 10 15
   Val Val Ile Pro Leu Val Lys
   20
<210> 1371
   <211> 8
   <212> PRT
   <213> Artificial Sequence
<220>
   <223> SLC7A14
<400> 1371
   Leu Ser Thr Ile Thr Trp Ile Arg
   1 5
<210> 1372
   <211> 6
   <212> PRT
   <213> Artificial Sequence
<220>
   <223> SLC7A3
<400> 1372
   Ser Asn Gln Pro Ser Arg
   1 5
<210> 1373
   <211> 14
   <212> PRT
   <213> Artificial Sequence
<220>
   <223> SLC7A3
<400> 1373
   Thr Val Asp Leu Asp Pro Gly Thr Leu Tyr Val His Ser Val
   1 5 10
<210> 1374
   <211> 10
   <212> PRT
   <213> Artificial Sequence
<220>
   <223> SLC7A4
<400> 1374
   Gly Leu Pro Thr Ile Ala Ser Leu Ala Arg
   1 5 10
<210> 1375
   <211> 17
   <212> PRT
   <213> Artificial Sequence
<220>
   <223> SLC7A4
<400> 1375
   Ser Ser Pro Pro Ser Ser Pro Gly Pro Ala Ser Pro Gly Pro Leu Thr
   1 5 10 15
   Lys
<210> 1376
   <211> 7
   <212> PRT
   <213> Artificial Sequence
<220>
   <223> SLC7A7
<400> 1376
   Ile Val Gly Ser Ala Thr Arg
   1 5
<210> 1377
   <211> 9
   <212> PRT
   <213> Artificial Sequence
<220>
   <223> SLC7A8
<400> 1377
   Ser Gly Gly Asp Tyr Ser Tyr Val Lys
   1 5
<210> 1378
   <211> 7
   <212> PRT
   <213> Artificial Sequence
<220>
   <223> SLC7A8
<400> 1378
   Leu Phe Phe Ala Gly Ala Arg
   1 5
<210> 1379
   <211> 8
   <212> PRT
   <213> Artificial Sequence
<220>
   <223> SLC7A8
<400> 1379
   Asp Val Ala Gly Gln Pro Gln Pro
   1 5
<210> 1380
   <211> 8
   <212> PRT
   <213> Artificial Sequence
<220>
   <223> SLC8A1
<400> 1380
   Gly Asn Val Ile Val Pro Tyr Lys
   1 5
<210> 1381
   <211> 22
   <212> PRT
   <213> Artificial Sequence
<220>
   <223> SLC8A1
<400> 1381
   Asp Ser Val Thr Ala Val Val Phe Val Ala Leu Gly Thr Ser Val Pro
   1 5 10 15
   Asp Thr Phe Ala Ser Lys
   20
<210> 1382
   <211> 6
   <212> PRT
   <213> Artificial Sequence
<220>
   <223> SLC8A1
<400> 1382
   Gln Pro Leu Thr Ser Lys
   1 5
<210> 1383
   <211> 7
   <212> PRT
   <213> Artificial Sequence
<220>
   <223> SLC8A2
<400> 1383
   Thr Val Asp Gly Thr Ala Arg
   1 5
<210> 1384
   <211> 21
   <212> PRT
   <213> Artificial Sequence
<220>
   <223> SLC8A2
<400> 1384
   Asp Ser Val Asn Ala Val Val Phe Val Ala Leu Gly Thr Ser Ile Pro
   1 5 10 15
   Asp Thr Phe Ala Ser
   20
<210> 1385
   <211> 8
   <212> PRT
   <213> Artificial Sequence
<220>
   <223> SLC8A3
<400> 1385
   Gly Thr Val Ile Val Pro Phe Arg
   1 5
<210> 1386
   <211> 24
   <212> PRT
   <213> Artificial Sequence
<220>
   <223> SLC8A3
<400> 1386
   Leu Gln Pro Leu Thr Ser Ala Phe Leu His Phe Gly Leu Val Thr Phe
   1 5 10 15
   Val Leu Phe Leu Asn Gly Leu Arg
   20
<210> 1387
   <211> 11
   <212> PRT
   <213> Artificial Sequence
<220>
   <223> SLC9A1
<400> 1387
   Ile Asn Asn Tyr Leu Thr Val Pro Ala His Lys
   1 5 10
<210> 1388
   <211> 14
   <212> PRT
   <213> Artificial Sequence
<220>
   <223> SLC9A1
<400> 1388
   Ser His Gly Leu Gln Leu Ser Pro Thr Ala Ser Thr Ile Arg
   1 5 10
<210> 1389
   <211> 6
   <212> PRT
   <213> Artificial Sequence
<220>
   <223> SLC9A1
<400> 1389
   Phe Thr Ser His Ile Arg
   1               5
<210> 1390
   <211> 6
   <212> PRT
   <213> Artificial Sequence
<220>
   <223> SLC9A1
<400> 1390
   Ser His Thr Thr Ile Lys
   1               5
<210> 1391
   <211> 11
   <212> PRT
   <213> Artificial Sequence
<220>
   <223> SLC9A1
<400> 1391
   Asp Gln Phe Ile Ile Ala Tyr Gly Gly Leu Arg
   1               5                   10
<210> 1392
   <211> 7
   <212> PRT
   <213> Artificial Sequence
<220>
   <223> SLC9A1
<400> 1392
   Ile Leu Pro Ala Leu Ser Lys
   1               5
<210> 1393
   <211> 6
   <212> PRT
   <213> Artificial Sequence
<220>
   <223> SLC9A1
<400> 1393
   Val Leu Gly Leu Ser Arg
   1               5
<210> 1394
   <211> 11
   <212> PRT
   <213> Artificial Sequence
<220>
   <223> SLC9A2
<400> 1394
   Asp Gln Phe Ile Ile Ala Tyr Gly Gly Leu Arg
   1               5                   10
<210> 1395
   <211> 6
   <212> PRT
   <213> Artificial Sequence
<220>
   <223> SLC9A2
<400> 1395
   Phe Thr His Asn Ile Arg
   1               5
<210> 1396
   <211> 6
   <212> PRT
   <213> Artificial Sequence
<220>
   <223> SLC9A2
<400> 1396
   Ser Tyr Thr Thr Ile Lys
   1               5
<210> 1397
   <211> 13
   <212> PRT
   <213> Artificial Sequence
<220>
   <223> SLC9A2
<400> 1397
   Ala Leu Gly Val Phe Val Leu Thr Gln Val Ile Asn Arg
   1               5                   10
<210> 1398
   <211> 7
   <212> PRT
   <213> Artificial Sequence
<220>
   <223> SLC9A2
<400> 1398
   Thr Ile Pro Leu Thr Phe Lys
   1 5
<210> 1399
   <211> 6
   <212> PRT
   <213> Artificial Sequence
<220>
   <223> SLC9A2
<400> 1399
   Leu Phe Asp His Val Lys
   1 5
<210> 1400
   <211> 6
   <212> PRT
   <213> Artificial Sequence
<220>
   <223> SLC9A2
<400> 1400
   Asn Leu Tyr Gln Ile Arg
   1 5
<210> 1401
   <211> 6
   <212> PRT
   <213> Artificial Sequence
<220>
   <223> SLC9A2
<400> 1401
   Thr Leu Ser Tyr Asn Arg
   1 5
<210> 1402
   <211> 7
   <212> PRT
   <213> Artificial Sequence
<220>
   <223> SLC9A2
<400> 1402
   Ala Ser Thr Ser Thr Ser Arg
   1 5
<210> 1403
   <211> 6
   <212> PRT
   <213> Artificial Sequence
<220>
   <223> SLC9A2
<400> 1403
   Tyr Leu Ser Leu Pro Lys
   1               5
<210> 1404
   <211> 15
   <212> PRT
   <213> Artificial Sequence
<220>
   <223> SLC9A2
<400> 1404
   Gly Thr Gln Thr Ser Gly Leu Leu Gln Gln Pro Leu Leu Ser Lys
   1               5                   10                  15
<210> 1405
   <211> 8
   <212> PRT
   <213> Artificial Sequence
<220>
   <223> SLC9A3
<400> 1405
   Ile Gly Phe His Leu Ser His Lys
   1               5
<210> 1406
   <211> 15
   <212> PRT
   <213> Artificial Sequence
<220>
   <223> SLC9A3
<400> 1406
   Ser Pro Ser Thr Asp Asn Val Val Asn Val Asp Phe Thr Pro Arg
   1               5                   10                  15
<210> 1407
   <211> 28
   <212> PRT
   <213> Artificial Sequence
<220>
   <223> SLC9A3
<400> 1407
   Gly Ile Val Ser Phe Phe Val Val Ser Leu Gly Gly Thr Leu Val Gly
   1               5                   10                  15
   Val Val Phe Ala Phe Leu Leu Ser Leu Val Thr Arg
               20                  25
<210> 1408
   <211> 8
   <212> PRT
   <213> Artificial Sequence
<220>
   <223> SLC9A4
<400> 1408
   Ile Gly Phe His Leu Tyr His Arg
   1               5
<210> 1409
   <211> 15
   <212> PRT
   <213> Artificial Sequence
<220>
   <223> SLC9A4
<400> 1409
   Ala Ile Ser Val Phe Ala Leu Phe Tyr Ile Ser Asn Gln Phe Arg
   1               5                   10                  15
<210> 1410
   <211> 7
   <212> PRT
   <213> Artificial Sequence
<220>
   <223> SLC9A4
<400> 1410
   Thr Phe Pro Phe Ser Ile Lys
   1               5
<210> 1411
   <211> 14
   <212> PRT
   <213> Artificial Sequence
<220>
   <223> SLC9A4
<400> 1411
   Tyr Leu Ser Tyr Pro Tyr Gly Asn Pro Gln Ser Ala Gly Arg
   1 5 10
<210> 1412
   <211> 11
   <212> PRT
   <213> Artificial Sequence
<220>
   <223> SLC9A5
<400> 1412
   Ser Ser Phe Ala Phe Pro Pro Ser Leu Ala Lys
   1 5 10
<210> 1413
   <211> 6
   <212> PRT
   <213> Artificial Sequence
<220>
   <223> SLC9A5
<400> 1413
   Leu Val Pro Leu Asp Lys
   1 5
<210> 1414
   <211> 7
   <212> PRT
   <213> Artificial Sequence
<220>
   <223> SLC9A6
<400> 1414
   Asp Thr Ala Thr Tyr Ala Arg
   1 5
<210> 1415
   <211> 8
   <212> PRT
   <213> Artificial Sequence
<220>
   <223> SLC9A7
<400> 1415
   Ala Thr Gly Ala Pro Pro Pro Arg
   1 5
<210> 1416
   <211> 7
   <212> PRT
   <213> Artificial Sequence
<220>
   <223> SLC9A7
<400> 1416
   Asp Thr Ala Ser Tyr Ala Arg
   1 5
<210> 1417
   <211> 19
   <212> PRT
   <213> Artificial Sequence
<220>
   <223> SLC9A7
<400> 1417
   His Val Phe Ser Pro Ile Phe Ile Ile Gly Ala Phe Val Ala Ile Phe
   1 5 10 15
   Leu Gly Arg
<210> 1418
   <211> 15
   <212> PRT
   <213> Artificial Sequence
<220>
   <223> SLC9A8
<400> 1418
   Ala Val Asn Ile Phe Pro Leu Ser Tyr Leu Leu Asn Phe Phe Arg
   1 5 10 15
<210> 1419
   <211> 6
   <212> PRT
   <213> Artificial Sequence
<220>
   <223> SLC9A9
<400> 1419
   Leu Gly Leu Asp Gln Lys
   1 5
<210> 1420
   <211> 8
   <212> PRT
   <213> Artificial Sequence
<220>
   <223> SLC9A9
<400> 1420
   Ala Ser Pro Gln Thr Pro Gly Lys
   1               5

## Description

### Technical Field

The present invention relates to a method for measuring quantitatively a plasma membrane protein, specifically to a method for measuring quantitatively a membrane protein present in a plasma membrane, by using a stable-isotope labeled peptide as a probe, by a mass spectrometry using liquid chromatography-tandem mass spectrometry (LC-MSMS).

### Background Art

Plasma membrane protein (sometimes referred to as membrane protein) is present as a protein constituting a biological membrane. A plasma membrane protein has a function as a receptor of an enzyme, peptide hormone, growth factor, autacoid, etc.; transporter of sugar, etc.; ion channel; or plasma membrane antigen, and is associated with dynamic functions of a cell such as penetration/transportation of a substance, by receiving a signal from the surface of a cell. A plasma membrane protein is a protein or a glycoprotein incorporated in a plasma membrane lipid bilayer, which is present in various forms, for example, penetrating all layers of a plasma membrane (transmembrane protein), located on the surface layer (cell surface protein), or undercoating a plasma membrane. In either case, it is a high molecular protein.

As one of plasma membrane protein, ABCG2 (ATP binding cassette transporter G2) is known. ABCG2 is known as a member of human ATP binding cassette (ABC) transporter, and it is reported that ABC transporter is associated with causes of diseases or drug transport. ABC transporter is one of the biggest superfamily of protein which is found in any cell of from bacterium to higher animal such as human, and about 250 members (about 50 in human) are known. Almost all ABC proteins function as a transporter, while some function as an ion channel. ABCG2 gene has been first isolated by Allikmets et al (Humann Mol. Genet. 5(10), 1649-1655, 1996). This protein is a transporter being the cause of drug resistance, and has been revealed to have a function to discharge anticancer agents to the outside of the cell (Proc. Natl. Acad. Sci., 95, 15665-15670, 1998; Clin. Cancer. Res. 7, 935-941, 2001).

Further, also as one of plasma membrane proteins, P-glycoprotein (PGP; also known as multidrug transporter MDR1) is known (Medicine,Stanford University School of Medicine, Stanford, CA 94306, USA ; Nature 323(6090), 728-731, 1986 ; Biochem. Biophys. Res. Commun. 162(1), 224-231, 1989). P-glycoprotein is a member of the ABC transporter superfamily, and is expressed in human intestines, liver or other tissues. The enzyme is incorporated in the plasma membrane and acts as an efflux pump transporting small molecules. It has been known these last several years that expression of a high-level P-glycoprotein is a mechanism of tumor resistance against chemotherapy of cancer. Further, expression of P-glycoprotein in intestines, affects oral bioavailability of an agent molecule which is a substrate of this transporter, enables effective discharge of drug in intestine lumen, thus reducing the drug amount to circulate.

Further, ABCC4 is known as one of plasma membrane proteins (Genetics, 123(1), 45-54, 1989). ABCC4 is known as a member of ATP binding domain (ABC) transporter. ABC transporter superfamily is one of the biggest gene families, which encodes membrane protein groups having various functions related to energy-dependent transportation of a wide variety of substances passing through the membrane (Curr. Opin. Genet. Dev., 5, 779-85, 1995). ABC proteins are associated with extracellular and intracellular membrane transport of various substances, including ion, amino acid, peptide, sugar, vitamin, and steroid hormone. Human ABCC subfamily has at present 10 identified members (ABCC 1-10), among which 7 are derived from multidrug resistance-like (MRP) subgroup, 2 are derived from sulfonyaurea receptor (SUR) subgroup, and 1 is CFTR gene. MRP-like protein is an organic anion transporter. ABCC4 and ABCC5 proteins are known to impart resistance against nucleotide analog including PMEA and purine base analog.

As for biogenic proteins such as the above-mentioned plasma membrane protein, it is necessary to detect proteins contained in the cells or the like, and measure them quantitatively in order to elucidate the function of the protein, or when testing or screening for the protein, or to use the protein. Particularly, when developing a novel drug, it is significant to quantify absolutely proteins such as membrane proteins to test a novel drug candidate, with a simple and accurate method.

For example, more than 90% of substances as novel drug candidates, which pharmaceuticals companies have developed by spending much time and cost, are dropped out (halt development). Most of the reasons are drug kinetics (absorption, distribution, metabolism, excretion) such as low-absorption to digestive tracts, or drug transfer into inappropriate organs, and side effects. As drug kinetics are considered by using animals conventionally, it cannot be considered at an early phase of the development as high throughput screening. Further, as there are species differences of drug kinetics, some data obtained from animal experiments are not reproduced in human. In other words, the problem of drug kinetics is often revealed in clinical experiments at a late stage of the development, which cause significant losses for pharmaceutical companies. Due to such problems, it is awaited that human drug kinetics can be estimated at an early phase of development by using a system that enables high throughput screening in vitro.

Performance of the above system is actualized for metabolisms of drug kinetics. In other words, the absolute amount of each metabolic enzyme in human liver is revealed. Further, the metabolic velocity per molecule by each metabolic enzyme of the subject drug in vitro is calculated by using a recombinant protein. From the information from each metabolic enzyme functioning mainly in liver, the metabolic velocity of the subject drug can be estimated by calculation. In other words, a means for obtaining the absolute amount of enzyme protein in organs and in vitro is indispensable.

On the other hand, a membrane protein associated with drug kinetics (drug transporter, receptor), which transports drugs is playing a crucial role for absorption, distribution and excretion among the drug kinetics. Therefore, if the absolute amount of the membrane protein associated with drug kinetics in organs, and the transportation ability or the binding ability of the drug per molecule can be measured, it would be possible to estimate the metabolic velocity from the obtained information by calculation, similarly with metabolism. Presently, there is a system to estimate in vitro the transportation or binding by using a membrane protein forcibly expressing cell or an expressed membrane vesicle, while it is very difficult to quantify the absolute amount of membrane protein. Therefore, if a more general method for quantifying the absolute amount of membrane protein with a high sensitivity is established, it becomes possible to estimate drug kinetics from an experiment system in vitro. Thus, it would be possible to estimate drug kinetics in human at an early stage of drug development, and reduce significantly time and cost necessary for drug development. The development of a method for absolutely quantifying membrane protein is awaited also even in the field of drug development.

Conventionally, a method using electrophoresis such as two-dimensional electrophoresis was conducted for quantifying biogenic proteins. With this method, detection and quantification were performed by staining the protein to be quantified, or by autoradiography, or by using an antibody specific to a particular protein (western blot). Particularly, a method using an antibody was conducted for quantifying a plasma membrane protein. For a method for quantifying a plasma membrane protein using an antibody, it is necessary to prepare an antibody against the protein, and it cannot be applied to a membrane protein against which an antibody cannot be prepared. Further, the method for quantifying comprises solubilizing the membrane protein when preparing a sample, and then electrophoresing to stain the antibody. However, as solubilizing conditions vary upon membrane proteins, conditions must be considered individually, which takes time. Further, as it is not possible to electrophorese insoluble proteins or high-molecular proteins, it was difficult to apply these methods for quantifying a plasma membrane protein.

On the other hand, mass spectrometry is making a significant progress recently, and the method is considered and used for detecting or measuring various biological materials. Mass spectrometers having various function have been developed including : a mass spectrometer having an electrospray ionization (ESI) source, a mass spectrometer having a liquid chromatography spectromotry (LC-MS), a MS/MS spectrum or tandem mass spectrum (tandemu MS) mass spectrometer wherein two mass spectrometers are bound. They are used for detecting or measuring biological materials (Japanese Laid-Open Patent Application No. 2004-28993; Japanese Laid-Open Patent Application No. 2004-77276; Published Japanese translation of PCT international publication No. 2004-533610).

Recently, a mass spectrometry using a stable-isotope label has been developed, and is used for detecting or measuring biological materials. This method quantifies proteins or peptides in a sample by mass spectrometry, by using proteins or peptides labeled with a stable-isotope. Examples of using the method for quantifying C-reactive protein (CRP) which is a diagnostic marker in the serum of a patient with rheumatism, and β-amyloid in a sample from mammalian tissues or in body fluid have been reported. However, no example of using such mass spectrometry for quantifying a plasma membrane protein which is insoluble and high-molecular is known.

Conventionally, LC-MSMS is used for detecting or measuring biological materials by using mass spectrometry. Quantification by using LC-MSMS is performed by using channel in which MS spectrum of the intended compound, and a particular MSMS spectrum peak are combined. For a low-molecular compound, usually the method is performed by using a single channel. However, in case of a peptide, which is high molecular, it was estimated that quantification using a single channel would cause some problems, and indeed did.

In other words, when there is a peptide with a different sequence from that of the selected peptide detected in the same channel, plural peaks are detected in the selected channel. It is possible to synthesize an internal standard peptide, and to identify the peak of the elution time identical with the internal standard peptide, as a peak of the intended peptide. However, when plural channels are prepared for various membrane proteins, it is very difficult to synthesize internal standard peptides for all of them, from the view points of time and cost. Therefore, a means for identifying the peak of the intended peptide without an internal standard peptide is necessary. Further, it is possible that a peptide with a different sequence detected in the same channel is detected within the same elution time as the intended peptide, which lowers quantification accuracy.

Patent reference 1: Japanese Laid-Open Patent Application No. 2004-28993
Patent reference 2: Japanese Laid-Open Patent Application No. 2004-77276
Patent reference 3: Japanese Laid-Open Patent Application No. 2004-157124
Patent reference 4: Published Japanese translation of PCT International Publication NO. 2004-533610
Non-patent reference 1: Humann Mol. Genet. 5(10), 1649-1655, 1996
Non-patent reference 2: Proc. Natl. Acad. Sci., 95, 15665-15670,1998
Non-patent reference 3: Clin. Cancer. Res. 7, 935-941, 2001
Non-patent reference 4: Medicine, Stanford University School of Medicine, Stanford, CA 94306,USA
Non-patent reference 5: Nature 323(6090), 728-731, 1986
Non-patent reference 6: Biochem. Biophys. Res. Commun. 162(1), 224-231, 1989
Non-patent reference 7: Genetics, 123(1), 45-54, 1989
Non-patent reference 8: Curr. Opin. Genet. Dev., 5, 779-85, 1995
Non-patent reference 9: Proteomics 4, 1175-1186,2004

### Disclosure of the Invention

### Object to be solved by the Invention

A plasma membrane protein constituting a biological membrane has important functions including receptor against factors acting on organisms, transporter of biological substances, ion channel, and plasma membrane antigen. Elucidation of functions of the protein, screening of active substances using the protein, and test or diagnosis using expression of the protein have been performed. When elucidating functions of the protein, screening of active substances, or testing/diagnosis utilizing expression of the protein, it is necessary to detect the protein contained in cells and to measure it quantitatively. Particularly, when developing a novel drug, it is significantly important to quantify absolutely the plasma protein with a simple and accurate method for testing a material being a novel drug candidate.

Conventionally, when detecting and quantifying biological proteins, methods such as staining method, autoradiography, or electrophoresis using an antibody were used. However, as a plasma membrane protein is high molecular and insoluble, it is difficult to conduct electrophoresis, and thus difficult to quantify with these conventional methods. For a method for quantifying a plasma membrane protein using an antibody, it is necessary to prepare an antibody against the protein, and it cannot be applied to a membrane protein against which an antibody cannot be prepared. Further, the method for quantifying comprises solubilizing the plasma membrane protein when preparing a sample, and then electrophoresing to stain the antibody. However, as solubilizing conditions vary upon plasma membrane proteins, conditions must be considered individually, which takes time. Further, a lot of time, technique, and cost are necessary to prepare an antibody.

Thus, the object of the present disclosure is to provide a method for measuring accurately and quantitatively a high molecular and insoluble plasma membrane protein with a simple method, more specifically a method for measuring quantitatively and accurately a membrane protein which is present in a cell membrane by using a stable-isotope labeled peptide as a probe, by mass spectrometry in a simple and accurate manner, with a simple means. Further, the present disclosure is to provide a method for measuring a plasma membrane protein accurately and easily by improving selection of measuring samples or measuring means, when quantifying a plasma membrane protein by mass spectrometry.
The scope of protection is defined by the claims.

### Means to solve the object

The present inventors made a keen study to resolve the above object, and found out that a high molecular and insoluble plasma membrane protein can be quantified simply and quickly, and accurately by the following steps:
fragmentating a separated plasma membrane protein to be quantified with a protease to prepare an oligopeptide fragment, identifying it by LC-MSMS; setting as essential criteria that it is a peptide obtained by fragmentating with a protease, and that it is a peptide sequence specific to the target molecule, and by establishing selective criteria with scores for content of hydrophobic amino acid, sequence conditions, amino acid residue numbers, specific amino acid sequence conditions, etc.; selecting the peptide fragment that can be ionized by ESI, according to a selective criterion by which a peptide with a high score is preferentially selected; selecting the peptide fragment as a subject peptide for quantification; on the other hand, by preparing a stable-isotope labeled peptide having the same sequence as the subject peptide for quantification, and labeled with a stable-isotope element ; preparing in advance a calibration curve with the subject peptide for quantification, and a stable-isotope labeled peptide from a mass spectrometry by LC-MSMS by using a sample of a certain concentration; and calculating from the calibration curve from the results of LC-MSMS mass spectrometry of an oligopeptide fragment of a sample of a plasma membrane protein to be quantified, and the stable-isotope labeled peptide.

One of the features of the method for quantifying a plasma membrane protein by using LC-MSMS mass spectrometry of the present invention, is to select the oligopeptide fragment enabling ionization by ESI method, and to select a subject peptide for quantification, using the selective criteria for a subject peptide for quantification, established for at least, a protease to use, hydrophobic amino acid content and sequence conditions, amino acid residue numbers, and specific amino acid sequence conditions. In other words, when quantifying a plasma membrane protein by mass spectrometry, it is effective to quantify by using LC-MSMS method. As a problem of a conventional method, for quantifying a protein by using LC-MSMS method, it is necessary to select a peptide that can be separated by liquid chromatography, and has a superior ionization efficiency. For this, it is necessary to measure a sample containing a large amount of the protein by LC-MSMS, and to confirm peptide that ionizes. When there is a little amount of the plasma membrane protein, a very long time and burden are required for pre-treatment of separation/concentration. The determination of a subject peptide for quantification is a rate-controlling step of the conventional LC-MSMS method, and it was difficult to apply the method for quantifying of a large amount of membrane proteins (example: 48 ABC transporters, and 319 SCL transporters in human). To resolve this problem, in the present invention, the above criteria for selecting peptide (selective standard) were established to select a subject peptide for quantification, and by selecting a peptide having an excellent ionization efficiency, it became possible to quantify a plasma membrane protein by LC-MSMS mass spectromety accurately and effectively. The selection of a peptide having an excellent ionization efficiency by using the criteria for selecting peptide can be performed at a high speed with a computer, and it is possible to select an appropriate peptide.

In other words, the present disclosure relates to: ("1") a method for quantifying a plasma membrane protein by liquid chromatograph-tandem mass spectrometer (LC-MSMS) using a stable-isotope labeled peptide, comprising the following steps (a) to (e):
(a)a step of preparing and identifying an oligopeptide fragment by fragmentating a separated plasma membrane protein to be quantified; selecting a subject peptide for quantification that can be ionized by ESI method, according to a criteria for selecting a subject peptide for quantification including essential criteria consisting of at least as: 1) that it is a peptide obtained from a protease comprising trypsin, endoproteinase, and pepsin; and 2) that it is a peptide sequence specific to the target molecule;
b) a step of preparing a stable-isotope labeled peptide having the same sequence as the subject peptide for quantification, and labeled with a stable-isotope element by a peptide synthesis-method;
c) a step of preparing a calibration curve by using the subject peptide for quantification and the stable-isotope labeled peptide, and performing mass spectrometry using LC-MSMS for each predetermined concentration level;
(d) a step of performing mass spectrometry by using LC-MSMS by adding a stable-isotope labeled peptide to the peptide fragment obtained by fragmentating the plasma membrane protein to be quantified in a sample with the protease, and calculating the mass spectrum area ratio of a plasma membrane protein peptide to be quantified/stable-isotope labeled peptide;
(e) a step of calculating the quantitative level from the area ratio by using the calibration curve.

Further, the present disclosure relates to ("2"), the method for quantifying a plasma membrane protein according to ("1"), wherein the criteria for selecting the subject peptide for quantification are criteria for selecting preferentially a peptide with a high score by setting as selective criteria with score, further to the above essential criteria (1) and (2), the followings:
3) that it is a peptide wherein the content of hydrophobic amino acids is 80% or less, and that not more than 10 hydrophobic amino acids are consecutive, as for the content of hydrophobic amino acids consisting of tryptophan, tyrosine, valine, leucine, isoleucine, phenylalanine, and for sequence conditions [score 2];
4) that it is a peptide wherein the number of amino acid residues is 4 - 30 [score 3];
5) that it is a peptide that does not contain the sequence of asparagine-X (wherein X represents amino acids other than proline)-serine or -threonine, -cysteine, as specific amino acid sequence conditions [score 2];
6) that it is a peptide that does not contain a post-translation modified site (it is not limited to this when quantifying a post-translation modified protein) [score 3];
7) that it is a peptide that does not contain a single nucleotide polymorphism (SNP) site [score 4];
8) that it is a peptide wherein the protease cleavage site is not arginine-arginine, arginine-lysine, lysine-arginine, lysine-lysine [score 5];
9) that it is a peptide that does not contain a transmembrane domain when the protein structure is determined or estimated [score 2];
10) that it is a peptide that does not contain methionine and cysteine [score 3];
11) that it is a peptide that does not contain tryptophan and glutamic acid [score 1].

Further, the present disclosure relates to: ("3") the method for quantifying a plasma membrane protein according to ("2"), wherein the hydrophobic amino acid content is 50% or less, and the number of amino acid residues is 8 - 12; ("4") the method for quantifying a plasma membrane protein according to any one of ("1") to ("3"), wherein an additional criterion that it is the same amino acid sequence in plural animal species is added to the criteria for selecting a subject peptide for quantification; ("5") the method for quantifying a plasma membrane protein according to any one of ("1") to ("4"), wherein plural specific measurement channels are prepared by combining parent ion (m/z) and peptide fragment ion (m/z) of candidate peptides selected according to the criteria for selecting subject peptide for quantification, and measured; ("6") the method for quantifying a plasma membrane protein according to ("5"), wherein the plural specific channels are prepared for plural candidate peptides of the same protein and for a candidate peptide for plural proteins, and measured at the same time; ("7") the method for quantifying a plasma membrane protein according to any one of ("1") to ("6"), wherein the stable-isotope labeled peptide is labeled with an amino acid containing any one of ¹⁵N, ¹³C, ¹⁸O, or ²H; ("8") the method for quantifying a plasma membrane protein according to any one of ("1") to ("7") by a mass spectrometry using a stable-isotope labeled peptide, wherein a source of plasma membrane protein is a plasma membrane protein obtained from a tissue sample.

Further, the present disclosure relates to ("9") the method for quantifying a plasma membrane protein according to any one of ("1") to ("8"), wherein the plasma membrane protein is 1 or more proteins selected from the group consisting of human ABCA1, human ABCA2, human ABCA3, human ABCA4, human ABCA5, human ABCA6, human ABCA7 , human ABCA8, human ABCA9, human ABCA10, human ABCA12, human ABCA13, human ABCB1, human ABCB4,human ABCB5, human ABCB11, human ABCC1, human ABCC2, human ABCC3, human ABCC4, human ABCC5, human ABCC6, human ABCC7, human ABCC8, human ABCC9, human ABCC10, human ABCC11, human ABCC12, human ABCC13, human ABCG1, human ABCG2, human ABCG4, human ABCG5, human ABCG8, and human P-glycoprotein [hereinafter referred to as "the present ABC transporter"].

Further, the present disclosure relates to ("10") the method for quantifying a plasma membrane protein according to any one of ("1") to ("8"), wherein the plasma protein is 1 or more proteins selected from the group consisting of human SLC10A1, human SLC10A2, human SLC15A1, human SLC15A2, human SLC16A1, human SLC16A7, human SLC19A1, human SLC19A2, human SLC19A3, human SLC21A1, human SLC21A10, human SLC21A11, human SLC21A12, human SLC21A13, human SLC21A14, human SLC21A15, human SLC21A19 , human SLC21A2, human SLC21A20, human SLC21A3, human SLC21A4, human SLC21A5, human SLC21A6, human SLC21A7, human SLC21A8, human SLC21A9, human SLC22A1, human SLC22A10, human SLC22A11, human SLC22A12, human SLC22A13, human SLC22A14, human SLC22A15, human SLC22A16, human SLC22A17, human SLC22A18, human SLC22A2, human SLC22A3, human SLC22A4, human SLC22A5, human SLC22A6, human SLC22A7, human SLC22A8, human SLC22A9, human SLC23A1, human SLC23A2, human SLC28A1, human SLC28A2, human SLC28A3, human SLC29A1, human SLC29A2, human SLC29A3, human SLC29A4, human SLC31A1, human SLC3A2, human SLC43A1, human SLC43A2, human SLC43A3, human SLC7A5, human SLC7A6, and human SLC7A8 [hereinafter referred to as "the present SLC transporter (group A)"].

Further, the present disclosure relates to ("11") the method for quantifying a plasma membrane protein according to any one of ("1") to ("8"), wherein the plasma protein is 1 or more proteins selected from the group consisting of human SLC10A3, human SLC10A4, human SLC10A5, human SLC10A6, human SLC11A1, human SLC11A2, human SLC12A1, human SLC12A2, human SLC12A3, human SLC12A4, human SLC12A5, human SLC12A6, human SLC12A7, human SLC12A8, human SLC12A9, human SLC13A1, human SLC13A2, human SLC13A3, human SLC13A4, human SLC13A5, human SLC14A1, human SLC14A2, human SLC15A3, human SLC15A4, human SLC16A10, human SLC16A11, human SLC16A12, human SLC16A13, human SLC16A14, human SLC16A2, human SLC16A3, human SLC16A4, human SLC16A5, human SLC16A6, human SLC16A8, human SLC16A9, human SLC17A1, human SLC17A2, human SLC17A3, human SLC17A4, human SLC17A5, human SLC17A6, human SLC17A7, human SLC17A8, human SLC18A1, human SLC18A2, human SLC18A3, human SLC1A1, human SLC1A2 , human SLC1A3, human SLC1A4, human SLC1A5, human SLC1A6, human SLC1A7, human SLC20A1, human SLC20A2, human SLC23A3, human SLC24A1, human SLC24A2, human SLC24A3, human SLC24A4, human SLC24A5, human SLC24A6, human SLC25A1, human SLC25A10, human SLC25A11, human SLC25A12, human SLC25A13, human SLC25A14, human SLC25A15, human SLC25A16, human SLC25A17, human SLC25A18, human SLC25A19, human SLC25A2, human SLC25A20, human SLC25A21, human SLC25A22, human SLC25A23, human SLC25A24, human SLC25A25, human SLC25A26, human SLC25A27, human SLC25A28, human SLC25A29, human SLC25A3, human SLC25A30, human SLC25A31, human SLC25A32, human SLC25A33, human SLC25A34, human SLC25A35, human SLC25A36, human SLC25A37, human SLC25A4, human SLC25A5, human SLC25A6, human SLC25A7, human SLC25A8, human SLC25A9, human SLC26A1, human SLC26A10, human SLC26A11, human SLC26A2, human SLC26A3, human SLC26A4, human SLC26A5, human SLC26A6, human SLC26A7, human SLC26A8, human SLC26A9, human SLC27A1, human SLC27A2, human SLC27A3, human SLC27A4, human SLC27A5, human SLC27A6, human SLC2A1, human SLC2A10, human SLC2A11, human SLC2A12, human SLC2A13, human SLC2A14, human SLC2A2, human SLC2A3, human SLC2A4, human SLC2A5, human SLC2A6, human SLC2A7, human SLC2A8, human SLC2A9, human SLC30A1, human SLC30A10, human SLC30A11, human SLC30A2, human SLC30A3, human SLC30A4, human SLC30A5, human SLC30A6, human SLC30A7, human SLC30A8, human SLC30A9, human SLC31A2, human SLC32A1, human SLC33A1, human SLC34A1, human SLC34A2, human SLC34A3, human SLC35B1, human SLC35B2, human SLC35B3, human SLC35B4, human SLC35C1, human SLC35C2, human SLC35D1, human SLC35D2, human SLC35D3, human SLC36A1, human SLC36A2, human SLC36A3, human SLC36A4, human SLC37A1, human SLC37A2, human SLC37A3, human SLC37A4, human SLC38A1, human SLC38A2, human SLC38A3, human SLC38A4, human SLC38A5, human SLC38A6, human SLC3A1, human SLC40A1, human SLC41A1, human SLC41A2, human SLC41A3, human SLC44A1, human SLC44A2, human SLC44A3, human SLC44A4, human SLC44A5, human SLC45A1, human SLC45A2, human SLC45A3, human SLC45A4, human SLC4A1, human SLC4A10, human SLC4A11, human SLC4A2, human SLC4A3, human SLC4A4, human SLC4A5, human SLC4A7, human SLC4A8, human SLC4A9, human SLC5A1, human SLC5A11, human SLC5A12, human SLC5A2, human SLC5A3, human SLC5A4, human SLC5A5, human SLC5A9, human SLC6A1, human SLC6A10, human SLC6A11, human SLC6A12, human SLC6A13, human SLC6A14, human SLC6A15, human SLC6A16, human SLC6A17, human SLC6A18, human SLC6A19, human SLC6A2, human SLC6A20, human SLC6A3, human SLC6A4, human SLC6A5, human SLC6A6, human SLC6A7, human SLC6A8, human SLC6A9, human SLC7A1, human SLC7A10, human SLC7A11, human SLC7A13, human SLC7A14, human SLC7A2, human SLC7A3, human SLC7A4, human SLC7A7, human SLC7A9, human SLC8A1, human SLC8A2, human SLC8A3, human SLC9A1, human SLC9A2, human SLC9A3, human SLC9A4, human SLC9A5, human SLC9A6, human SLC9A7, human SLC9A8, and human SLC9A9 [hereinafter referred to as "the present SLC transporter (group B)"].

Further, the present disclosure relates to ("12") the method for quantifying a plasma membrane protein according to any one of ("1") to ("8"), wherein the plasma protein is 1 or more proteins selected from the group consisting of human SLC35A1, human SLC35A2, human SLC35A3, human SLC35A4, human SLC35A5, human SLC35E1, human SLC35E2, human SLC35E3, human SLC35E4, human SLC35F1, human SLC35F2, human SLC35F3, human SLC35F5, human SLC39A1, human SLC39A10, human SLC39A11, human SLC39A12, human SLC39A13, human SLC39A14, human SLC39A2, human SLC39A3, human SLC39A4, human SLC39A5, human SLC39A6, human SLC39A7, human SLC39A8, human SLC39A9, human SLC42A1, human SLC42A2, and human SLC42A3 [hereinafter referred to as "the present SLC transporter (group C)"].

Further, the present disclosure relates to ("13") the method for quantifying a plasma membrane protein according to any one of ("1") to ("8"), wherein the plasma membrane protein is human MATE1 and/or human MATE2 [hereinafter referred to as "the present MATE transporter"].

Further, the present disclosure relates to ("14") the method for quantifying a plasma membrane protein according to any one of ("1") to ("8"), wherein the plasma membrane protein is 1 or more proteins selected from the group consisting of human ABCA1, human ABCA2, human ABCA3, human ABC4, human ABCA5, human ABCA6, human ABCA7, human ABCA8, human ABCA9, human ABCA10, human ABCA12, human ABCA13, human ABCB1, human ABCB4, human ABCB5, human ABCB11, human ABCC1, human ABCC2, human ABCC3, human ABCC4, human ABCC5, human ABCC6, human ABCC10, human ABCC11, human ABCC12, human ABCC13, human ABCG1, human ABCG2, human ABCG4, human ABCG5, human ABCG8, human MATE1, human MATE2, human SLC3A2, human SLC7A5, human SLC7A6, human SLC10A1, human SLC10A2, human SLC15A1, human SLC15A2, human SLC16A1, human SLC16A7, human SLC19A1, human SLC21A2, human SLC21A3, human SLC21A4, human SLC21A5, human SLC21A6, human SLC21A7, human SLC21A8, human SLC21A9, human SLC21A11, human SLC21A12, human SLC21A13, human SLC21A14, human SLC21A15, human SLC21A19, human SLC21A20, human SLC22A1, human SLC22A2, human SLC22A3, human SLC22A4, human SLC22A5, human SLC22A6, human SLC22A7, human SLC22A8, human SLC22A9, human SLC22A10, human SLC22A11, human SLC22A12, human SLC22A13, human SLC22A14, human SLC22A15, human SLC22A16, human SLC22A17, human SLC23A1, human SLC23A2, human SLC28A1, human SLC28A2, human SLC28A3, human SLC29A1, human SLC29A2, and human SLC31A1 [hereinafter referred to as "the suitable transporter of the present invention"].

### Brief Description of Drawings

[Fig. 1]
   It is a figure showing the flow of the method for quantifying a plasma membrane protein by mass spectrometry of the present invention.
[Fig. 2-1]
   It is a figure showing the results of LC-MSMS measurement of a peptide library of a sample containing the subject membrane protein using the prepared 5 channels, when quantifying by peptide multichannel using LC-MSMS in the present invention.
[Fig. 2-2]
   It is a figure showing the results of LC-MSMS measurement of a peptide library of a sample containing the subject membrane protein using the prepared 5 channels, when quantifying by peptide multichannel using LC-MSMS in the present invention.
[Fig. 3-1]
   It is a figure showing the results of mass spectrometry by human ABCG2 peptide in an Example of the present invention. A) shows the MS spectrum of 5 fmol standard peptide and 50 fmol ¹³C₆-, ¹⁵N-labeled peptide.
[Fig. 3-2]
   It is a figure showing the results of mass spectrometry by human ABCG2 peptide in an Example of the present invention. B) shows the MS spectrum of 50 fmol standard peptide and 50 fmol ¹³C₆-, ¹⁵N-labeled peptide.
[Fig. 4]
   It is a figure showing a calibration curve prepared by adding 10 fmol of ¹³C₆-, ¹⁵N-labeled peptide, to 0.5 fmol, 1 fmol, 5 fmol, 10 fmol, and 50 fmol of standard peptide, respectively in an Example of the present invention. The calibration curve was prepared by calculating MS spectrum area ratio (standard peptide/¹³C₆-, ¹⁵N-labeled peptide).
[Fig. 5]
   It is a figure showing the results of mass spectrometry in an experiment for quantifying ABCG2 content in 1 µg of a plasma membrane peptide sample of a conditionally immortalized brain capillary endothelial cell strain, in an Example of the present invention. 10 fmol of ¹³C₆-, ¹⁵N-labeled peptide was added to a sample and measured. The ABCG2 content in the sample calculated from the calibration curve was 4.7 fmol/µg.
[Fig. 6]
   It is a figure showing the results of mass spectrometry, in an experiment measuring content of ABCB1, ABCB4, ABCB5, ABCB11, ABCC1, ABCC2, ABCC3, ABCC4, ABC C5, ABCC6, ABCC7, ABCC8, ABCC9, ABCC10, ABCC11, ABCC12, ABCC 13, ABCG1, ABCG4, ABCG5, and ABCG8 at the same time, in 50 µg of a plasma membrane peptide sample of leukemia cells, in Example 2 of the present invention.

### Best Mode of Carrying Out the Invention

A method for quantifying a plasma membrane protein by LC-MSMS using a stable-isotope labeled peptide is not particularly limited as long as it is a method comprising the following steps:
(a) a step of fragmentating a separated plasma membrane protein for quantification and preparing and identifying an oligopeptide fragment, and selecting a subject peptide for quantification that can be ionized by ESI, according to criteria for selecting a subject peptide for quantification including essential criteria set from at least: (1) that it is a peptide obtained by fragmentating with a protease consisting of trypsin, endoproteinase, or pepsine, (2) that it is a peptide sequence specific to a target molecule;
(b) a step of preparing a stable-isotope labeled peptide having the same sequence as the subject peptide for quantification, and labeled with a stable-isotope element by a peptide synthesis method;
(c) a step of preparing a calibration curve by using a subject peptide for quantification and the stable-isotope labeled peptide, and performing mass spectrometry using LC-MSMS for each predetermined concentration level;
(d) a step of calculating the mass spectrum area ratio of subject plasma membrane protein peptide for quantification/stable-isotope labeled peptide, by adding a stable-isotope labeled peptide to the peptide fragment obtained by fragmentating a plasma membrane protein for quantification of a sample with the protease and performing mass spectrometry using LC-MSMS;
(e) a step of calculating the quantitative level from the area ratio by using the calibration curve. A plasma membrane protein obtained from a tissue sample or cultured cell can be suitably exemplified as a source
for the above plasma membrane protein.

It is preferable to add to the above essential criteria set as (1) that it is a peptide obtained from a protease consisting of trypsin, endoproteinase, or pepsine, (2) that it is a peptide sequence specific to a target molecule, the following selective criteria with score, in order to apply criteria for preferentially selecting a peptide with high total score. 3) that it is a peptide wherein the content of hydrophobic amino acids is 80% or less, preferably 50% or less and that not more than 10 hydrophobic amino acids are consecutive, as for the content of hydrophobic amino acids consisting of tryoptophan, tyrosine, valine, leucine, isoleucine, phenylalanine, and sequence conditions [score 2];
4) that it is a peptide wherein the number of amino acid residues is 4 - 30, preferably 8 - 12 [score 3]
5) that it is a peptide that does not contain the sequence of asparagine-X (wherein X represents amino acids other than proline)-serine or -threonine, -cysteine, as a specific amino acid sequence condition [score 2];
6) that it is a peptide that does not contain a post-translation modified site (it is not limited to this when quantifying a post-translation modified protein) [score 3];
7) that it is a peptide that does not contain a single nucleotide polymorphism (SNP) site [score 4];
8) that it is a peptide wherein the protease cleavage site is not arginine-arginine, arginine-lysine, lysine-arginine, lysine-lysine [score 5];
9) that it is a peptide that does not contain a transmembrane domain when the protein structure is determined or estimated [score 2];
10) that it is a peptide that does not contain methionine and cysteine [score 3];
11) that it is a peptide that does not contain tryptophan and glutamic acid [score 1]. Further, an additional criterion that it is the same amino acid sequence in plural animal species (for example, human, mouse, rat) can be added, and by adding this criterion, it would be possible to quantify plasma membrane proteins of plural animal species with a single peptide.

When the membrane protein level contained in the source (sample) of a protein to be a measuring subject is less than measuring threshold limit of mass spectrometry using LC-MSMS, it is preferred to use a plasma membrane separated by high pressure nitrogen gas filling method, etc. An oligopeptide fragment is prepared by enzymatically digesting a plasma membrane protein with a protease consisting of trypsin, endoproteinase, and/or pepsine. For example, a peptide sample can be prepared by isolating a plasma membrane by high pressure nitrogen gas filling method, etc. from a cell in which the plasma membrane protein is expressed, followed by an enzymatic digestion. As for the fragmentating level, it is preferred to clarify the separation by increasing the mass difference between a stable-isotope labeled peptide and a non-labeled peptide that can be separated by mass spectrometry, as well as to lower molecules so that the number of amino acid residues becomes 4 - 30, in order to retain the specificity of the sequence.

It is preferred to identify the fragmentated oligopeptide fragment sample with LC-MSMS and to select a subject peptide for quantification. Thus, it is preferred that a peptide being the subject for quantification can be separated by liquid chromatography, ionized by ESI method, and detected by LC-MSMS. Then, by using the above criteria for selecting the subject peptide for quantification, a subject peptide for quantification that can be separated by liquid chromatography, and ionized by ESI method can be selected.

Based on the selected subject peptide for quantification, a stable-isotope labeled peptide which is labeled with a stable-isotope is prepared. The peptide labeled with a stable-isotope and added as an internal standard has the same amino acid sequence as the subject peptide for quantification, and is labeled with at least one stable-isotope selected from ¹⁵N, ¹³C, ¹⁸O, and ²H. As the internal standard is separated from the subject peptide according to the mass, a mass difference that can be separated with LC-MSMS is necessary. For example, it is preferred to use a peptide containing leucine wherein 6 sites are labeled with ¹³C.

As for a stable-isotope labeled peptide, it is necessary that at least one amino acid is labeled with a stable-isotope element. The peptide can be prepared by any method known to a person skilled in the art. For example, the intended stable-isotope labeled peptide can be chemically synthesized with a suitable means such as F-moc method (Amblard M, Fehrentz JA, Martinez J, Subra G. Methods Mol Biol. 298:3-24(2005)). A stable-isotope labeled peptide thus obtained is chemically identical with a subject peptide for quantification except for that the mass of the labeled amino acid is different, exert the same behavior in the measurement with LC-MSMS, and the loss levels of the analyte and standard are equal.

The selected subject peptides for quantification and prepared stable-isotope labeled peptides are used and subjected to mass spectrometry using LC-MSMS for each predetermined concentration level, to prepare a calibration curve. The calibration curve is prepared by adding a determined amount of stable-isotope labeled peptide to non-labeled peptides of several concentration levels, and measuring by LC-MSMS. Area ratio of MS spectrum or peak height ratio of non-labled peptide and stable-isotope labeled peptide at each concentration is calculated to prepare a calibration curve. The amount of non-labeled peptide is preferred to be within the linear measurement range of the mass spectrometry.

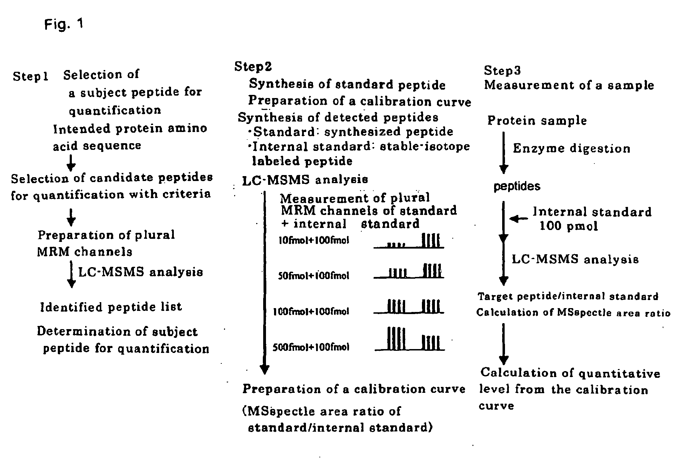

The prepared calibration curve is used to quantify a plasma membrane protein sample. For measurement, a stable-isotope labeled peptide is added to the peptide fragment obtained by fragmentating a plasma membrane protein to be quantified of a sample with the same protease used for selecting the subject peptide for quantification, to perform mass spectrometry by using LC-MSMS, the mass spectrum area ratio of the plasma membrane protein peptide to be quantified/stable-isotope labeled peptide is calculated, and the quantitative level is calculated from the area ratio by using the calibration curve. The flow of the method for quantifying a plasma membrane protein of the present invention is shown in Fig. 1.

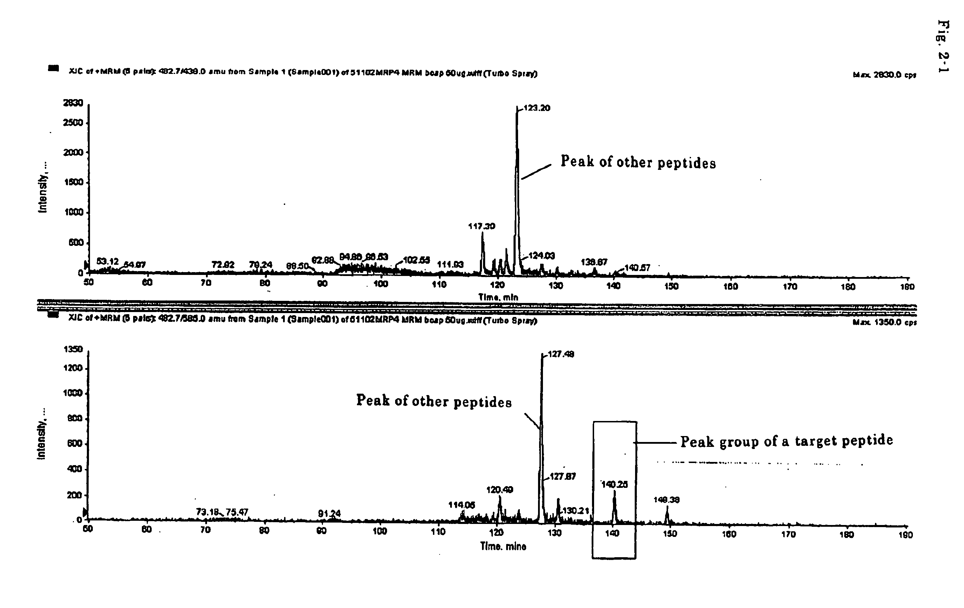

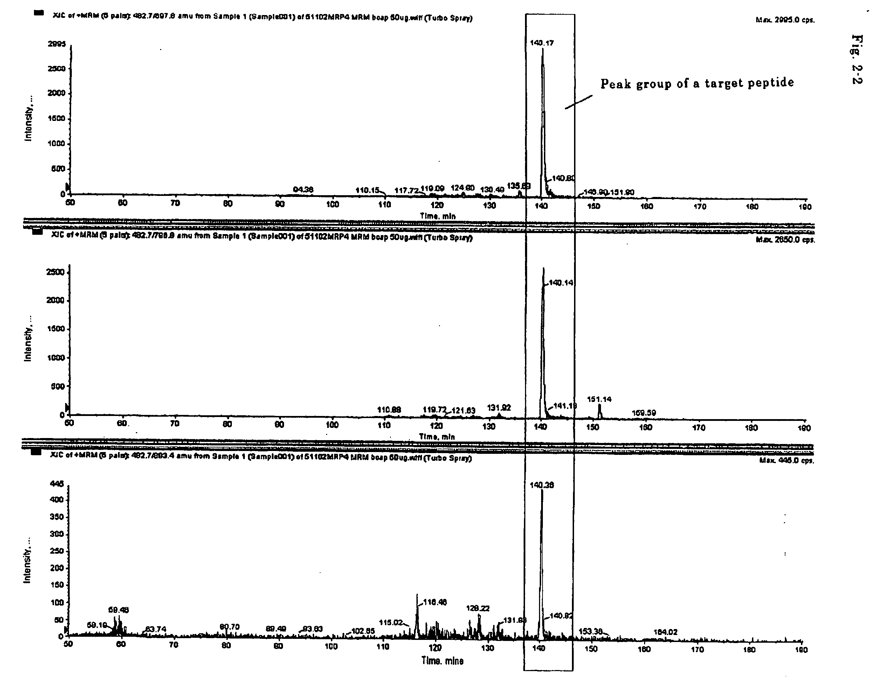

It is preferred to improve the quantitative accuracy by peptide multichannel by using LC-MSMS. By using LC-MSMS analysis, plural specific measurement channels are prepared by combining parent ion (m/z) and peptide fragment ion (m/z) for peptide sequence candidates selected according to the criteria and measured. It is preferred to prepare 5 or more specific measurement channels. It is preferred to select a divalent ion of the candidate peptide as MS spectrum, and to select 5 monovalent ions in descending order in terms of the mass for MSMS spectrum. By using the prepared 5 channels, a peptide library of a sample containing the subject membrane protein is measured with LC-MSMS, and the ion peak of the candidate peptide is confirmed by the prepared channel. The ion peaks in which the detecting time conforms within plus minus 1.0 sec for 3 or more out of 5 channels are identified as the intended peptide peak. The measurement results of mass spectrometry of the 5 prepared channels are shown in Fig. 2.

The present disclosure encompasses a method for quantifying the above-mentioned present ABC transporters, present SLC transporters (group A: those transporting drugs, and those which drug transporting function is estimated from amino acid sequence identity), present SLC transporters (group B: those transporting endogenous substances, and those which endogenous substance function is estimated from amino acid sequence identity), present SLC transporters (group C: those which transporting function is not estimated), present MATE transporter, and "the suitable transporters of the present invention", by using the method for quantifying a plasma membrane protein .

In the quantification by mass spectrometry of human ABCG2 of the present invention (ATP binding cassette transporter G2; Humann Mol. Genet. 5(10), 1649-1655, 1996); its sequence is registered at the data base of the Gene Bank with the accession no.: NM_004827), a quantification with high accuracy can be performed by using a peptide which amino acid sequence is ENLQFSAALR(SEQ ID No: 1), LAEIYVNSSFYK(SEQ ID No: 2), LFDSLTLLASGR(SEQ ID No: 3), SSLLDVLAAR(SEQ ID No: 4), and VIQELGLDK(SEQ ID No : 5), which has been selected in the present disclosure, as a subject peptide for quantification and a stable-isotope labeled peptide.

In the quantification by mass spectrometry of P-glycoprotein (P-glycoprotein ; Medicine, Stanford University School of Medicine, Stanford, CA 94306, USA ; Nature 323(6090), 728-731, 1986;Biochem. Biophys. Res. Commun. 162(1), 224-231, 1989; its sequence is registered at the data base of the Gene Bank with the accession no.: AF016535, NM_000927, M14758), a quantification with high accuracy can be performed by using a peptide which amino acid sequence is SEIDALEMSSNDSR(SEQ ID No: 6), EALDESIPPVSFWR(SEQ ID No: 7), IATEAIENFR(SEQ ID No: 8), NADVIAGFDDGVIVEK(SEQ ID No: 9), LYDPTEGMVSVDGQDIR(SEQ ID No: 10), ILLLDEATSALDTESEAVVQVALDK(SEQ ID No: 11), TVVSLTQEQK(SEQ ID No: 12), STVVQLLER(SEQ ID No: 13), ENVTMDEIEK(SEQ ID No: 14), NTTGALTTR(SEQ ID No: 15), VVQEALDK(SEQ ID No: 16), FYDPLAGK(SEQ ID No: 17), and STTVQLMQR(SEQ ID No: 18), which has been selected in the present disclosure, as a subject peptide for quantification and a stable-isotope labeled peptide.

In the quantification by mass spectrometry of human ABCC4 (Curr. Opin. Genet. Dev., 5, 779-85, 1995; its sequence is registered in the data base of the Gene Bank with the accession no.: NM_005845), a quantification with high accuracy can be performed by using a peptide which amino acid sequence is SQHLGEELQGFWDK(SEQ ID No: 19), MDTELAESGSNFSVGQR(SEQ ID No: 20), DGALESQDTENVPVTLSEENR(SEQ ID No: 21), ITILVTHQLQYLK (SEQ ID No: 22), IAYVSQQPWVFSGTLR(SEQ ID No: 23), IQTFLLLDEISQR (SEQ ID No: 24), DLQLLEDGDLTVIGDR(SEQ ID No: 25), MVHVQDFTAFWDK(SEQ ID No: 26), VFFWWLNPLFK(SEQ ID No: 27), DNEESEQPPVPGTPTLR(SEQ ID No: 28), APVLFFDR(SEQ ID No: 29), VAMZHMIYR(SEQ ID No: 30), SSLLSAVLGELAPSHGLVSVHGR(SEQ ID No: 31), TFSESSVWSQQSSRPSLK(SEQ ID No: 32), VSEAIVSIR(SEQ ID No: 33), SGIDFGSLLK(SEQ ID No: 34), DLQLLEDGDLTVIGDR(SEQ ID No: 35), and MSIIPQEPVLFTGTMR(SEQ ID No: 36), which has been selected in the present disclosure, as a subject peptide for quantification and a stable-isotope labeled peptide.

Similarly, as the above plasma membrane protein to be quantified, human ABCA1 can be exemplified. As a subject peptide for quantification and a stable-isotope labeled peptide used for measuring human ABCA1, one or more peptides selected from a peptide having an amino acid sequence shown by SEQ ID No : 37, SEQ ID No: 38, SEQ ID No: 39, SEQ ID No: 40, SEQ ID No: 41, SEQ ID No: 42, SEQ ID No: 43, SEQ ID No: 44, SEQ ID No: 45, SEQ ID No: 46, SEQ ID No: 47, SEQ ID No: 48, SEQ ID No: 49, SEQ ID No: 50, SEQ ID No: 51, SEQ ID No: 52, and SEQ ID No: 53 of the sequence listing can be specifically exemplified.

Similarly, as the above plasma membrane protein to be quantified, human ABCA2 can be exemplified. As a subject peptide for quantification and a stable-isotope labeled peptide used for measuring human ABCA2, one or more peptides selected from a peptide having an amino acid sequence shown by SEQ ID No: 54, SEQ ID No: 55, SEQ ID No: 56, SEQ ID No: 57, SEQ ID No: 58, SEQ ID No: 59, SEQ ID No: 60, SEQ ID No: 61, SEQ ID No: 62, SEQ ID No: 63, SEQ ID No: 64, SEQ ID No: 65, SEQ ID No: 66, SEQ ID No: 67, SEQ ID No: 68, SEQ ID No: 69, SEQ ID No: 70, SEQ ID No: 71, SEQ ID No: 72, SEQ ID No: 73, SEQ ID No: 74, and SEQ ID No: 75 of the sequence listing can be specifically exemplified.

Similarly, as the above plasma membrane protein to be quantified, human ABCA3 can be exemplified. As a subject peptide for quantification and a stable-isotope labeled peptide used for measuring human ABCA3, one or more peptides selected from a peptide having an amino acid sequence shown by SEQ ID No: 76, SEQ ID No : 77, SEQ ID No: 78, SEQ ID No: 79, SEQ ID No: 80, SEQ ID No: 81, SEQ ID No: 82, SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, and SEQ ID No: 92 of the sequence listing can be specifically exemplified.

Similarly, as the above plasma membrane protein to be quantified, human ABCA4 can be exemplified. As a subject peptide for quantification and a stable-isotope labeled peptide used for measuring human ABCA4, one or more peptides selected from a peptide having an amino acid sequence shown by SEQ ID No: 93, SEQ ID No: 94, SEQ ID No: 95, SEQ ID No: 96, SEQ ID No: 97, SEQ ID No: 98, SEQ ID No: 99, SEQ ID No: 100, SEQ ID No: 101, SEQ ID No: 102, SEQ ID No: 103, SEQ ID No: 104, and SEQ ID No: 105 of the sequence listing can be specifically exemplified.

Similarly, as the above plasma membrane protein to be quantified, human ABCAS can be exemplified. As a subject peptide for quantification and a stable-isotope labeled peptide used for measuring human ABCA5, one or more peptides selected from a peptide having an amino acid sequence shown by SEQ ID No: 106, SEQ ID No : 107, SEQ ID No: 108, SEQ ID No: 109, SEQ ID No: 110, SEQ ID No: 111, SEQ ID No: 112, SEQ ID No: 113, SEQ ID No: 114, and SEQ ID No: 115 of the sequence listing can be specifically exemplified.

Similarly, as the above plasma membrane protein to be quantified, human ABCA6 can be exemplified. As a subject peptide for quantification and a stable-isotope labeled peptide used for measuring human ABCA6, one or more peptides selected from a peptide having an amino acid sequence shown by SEQ ID No: 116, SEQ ID No: 117, SEQ ID No: 118, SEQ ID No: 119, SEQ ID No: 120, SEQ ID No: 121, SEQ ID No: 122, SEQ ID No: 123, SEQ ID No: 124, SEQ ID No: 125, SEQ ID No: 125, SEQ ID No: 126, SEQ ID No: 127, SEQ ID No: 128, SEQ ID No: 129, SEQ ID No: 130, SEQ ID No: 131, SEQ ID No: 132, and SEQ ID No: 133 of the sequence listing can be specifically exemplified.

Similarly, as the above plasma membrane protein to be quantified, human ABCA7 can be exemplified. As a subject peptide for quantification and a stable-isotope labeled peptide used for measuring human ABCA7, one or more peptides selected from a peptide having an amino acid sequence shown by SEQ ID No: 134 of the sequence listing can be specifically exemplified.

Similarly, as the above plasma membrane protein to be quantified, human ABCA8 can be exemplified. As a subject peptide for quantification and a stable-isotope labeled peptide used for measuring human ABCA8, one or more peptides selected from a peptide having an amino acid sequence shown by SEQ ID No: 135 of the sequence listing can be specifically exemplified.

Similarly, as the above plasma membrane protein to be quantified, human ABCA9 can be exemplified. As a subject peptide for quantification and a stable-isotope labeled peptide used for measuring human ABCA9, one or more peptides selected from a peptide having an amino acid sequence shown by SEQ ID No: 136 of the sequence listing can be specifically exemplified.

Similarly, as the above plasma membrane protein to be quantified, human ABCA10 can be exemplified. As a subject peptide for quantification and a stable-isotope labeled peptide used for measuring human ABCA10, one or more peptides selected from a peptide having an amino acid sequence shown by SEQ ID No: 137 of the sequence listing can be specifically exemplified.

Similarly, as the above plasma membrane protein to be quantified, human ABCA12 can be exemplified. As a subject peptide for quantification and a stable-isotope labeled peptide used for measuring human ABCA12, one or more peptides selected from a peptide having an amino acid sequence shown by SEQ ID No: 138 of the sequence listing can be specifically exemplified.

Similarly, as the above plasma membrane protein to be quantified, human ABCA13 can be exemplified. As a subject peptide for quantification and a stable-isotope labeled peptide used for measuring human ABCA13, one or more peptides selected from a peptide having an amino acid sequence shown by SEQ ID No: 139 of the sequence listing can be specifically exemplified.

Similarly, as the above plasma membrane protein to be quantified, human ABCB1 can be exemplified. As a subject peptide for quantification and a stable-isotope labeled peptide used for measuring human ABCB1, one or more peptides selected from a peptide having an amino acid sequence shown by SEQ ID No: 140 of the sequence listing can be specifically exemplified.

Similarly, as the above plasma membrane protein to be quantified, human ABCB4 can be exemplified. As a subject peptide for quantification and a stable-isotope labeled peptide used for measuring human ABCB4, one or more peptides selected from a peptide having an amino acid sequence shown by SEQ ID No: 141 of the sequence listing can be specifically exemplified.

Similarly, as the above plasma membrane protein to be quantified, human ABCB5 can be exemplified. As a subject peptide for quantification and a stable-isotope labeled peptide used for measuring human ABCB5, one or more peptides selected from a peptide having an amino acid sequence shown by SEQ ID No: 142 of the sequence listing can be specifically exemplified.

Similarly, as the above plasma membrane protein to be quantified, human ABCB11 can be exemplified. As a subject peptide for quantification and a stable-isotope labeled peptide used for measuring human ABCB11, one or more peptides selected from a peptide having an amino acid sequence shown by SEQ ID No: 143 of the sequence listing can be specifically exemplified.

Similarly, as the above plasma membrane protein to be quantified, human ABCC1 can be exemplified. As a subject peptide for quantification and a stable-isotope labeled peptide used for measuring human ABCC1, one or more peptides selected from a peptide having an amino acid sequence shown by SEQ ID No: 144, SEQ ID No: 145, SEQ ID No: 146, SEQ ID No: 147, SEQ ID No: 148, SEQ ID No: 149, SEQ ID No: 150, SEQ ID No: 151, SEQ ID No: 152, SEQ ID No: 153, SEQ ID No: 154, SEQ ID No: 155, SEQ ID No: 156, SEQ ID No: 157, and SEQ ID No: 158 of the sequence listing can be specifically exemplified.

Similarly, as the above plasma membrane protein to be quantified, human ABCC2 can be exemplified. As a subject peptide for quantification and a stable-isotope labeled peptide used for measuring human ABCC2, one or more peptides selected from a peptide having an amino acid sequence shown by SEQ ID No: 159, SEQ ID No: 160, SEQ ID No: 161, SEQ ID No: 162, SEQ ID No: 163, SEQ ID No: 164, SEQ ID No: 165, SEQ ID No: 166, SEQ ID No: 167, SEQ ID No: 168, SEQ ID No: 169, SEQ ID No: 170, SEQ ID No: 171, and SEQ ID No: 172 of the sequence listing can be specifically exemplified.

Similarly, as the above plasma membrane protein to be quantified, human ABCC3 can be exemplified. As a subject peptide for quantification and a stable-isotope labeled peptide used for measuring human ABCC3, one or more peptides selected from a peptide having an amino acid sequence shown by SEQ ID No: 173, SEQ ID No: 174, SEQ ID No: 175, SEQ ID No: 176, SEQ ID No: 177, SEQ ID No: 178, SEQ ID No: 179, SEQ ID No: 180, SEQ ID No: 181, SEQ ID No: 182, SEQ ID No: 183, SEQ ID No: 184, SEQ ID No: 185, and SEQ ID No: 186 of the sequence listing can be specifically exemplified.

Similarly, as the above plasma membrane protein to be quantified, human ABCC5 can be exemplified. As a subject peptide for quantification and a stable-isotope labeled peptide used for measuring human ABCC5, one or more peptides selected from a peptide having an amino acid sequence shown by SEQ ID No: 187 of the sequence listing can be specifically exemplified.

Similarly, as the above plasma membrane protein to be quantified, human ABCC6 can be exemplified. As a subject peptide for quantification and a stable-isotope labeled peptide used for measuring human ABCC6, one or more peptides selected from a peptide having an amino acid sequence shown by SEQ ID No: 188, SEQ ID No: 189, SEQ ID No: 190, SEQ ID No: 191, SEQ ID No: 192, SEQ ID No: 193, SEQ ID No: 194, SEQ ID No: 195, SEQ ID No: 196, SEQ ID No: 197, SEQ ID No: 198, SEQ ID No: 199, SEQ ID No: 200, SEQ ID No: 201, SEQ ID No: 202, SEQ ID No: 203, and SEQ ID No: 204 of the sequence listing can be specifically exemplified.

Similarly, as the above plasma membrane protein to be quantified, human ABCC7 can be exemplified. As a subject peptide for quantification and a stable-isotope labeled peptide used for measuring human ABCC7, one or more peptides selected from a peptide having an amino acid sequence shown by SEQ ID No: 205, SEQ ID No: 206, SEQ ID No: 207, SEQ ID No: 208, SEQ ID No: 209, SEQ ID No: 210, SEQ ID No: 211, SEQ ID No: 212, SEQ ID No: 212, SEQ ID No: 213, and SEQ ID No: 214 of the sequence listing can be specifically exemplified.

Similarly, as the above plasma membrane protein to be quantified, human ABCC8 can be exemplified. As a subject peptide for quantification and a stable-isotope labeled peptide used for measuring human ABCC8, one or more peptides selected from a peptide having an amino acid sequence shown by SEQ ID No: 215, SEQ ID No: 216, SEQ ID No: 217, SEQ ID No: 218, SEQ ID No: 219, SEQ ID No: 220, SEQ ID No: 221, SEQ ID No: 222, SEQ ID No: 223, and SEQ ID No: 224 of the sequence listing can be specifically exemplified.

Similarly, as the above plasma membrane protein to be quantified, human ABCC9 can be exemplified. As a subject peptide for quantification and a stable-isotope labeled peptide used for measuring human ABCC9, one or more peptides selected from a peptide having an amino acid sequence shown by SEQ ID No: 225, SEQ ID No: 226, SEQ ID No: 227, SEQ ID No: 228, SEQ ID No: 229, SEQ ID No: 230, SEQ ID No: 231, and SEQ ID No: 232 of the sequence listing can be specifically exemplified.

Similarly, as the above plasma membrane protein to be quantified, human ABCC10 can be exemplified. As a subject peptide for quantification and a stable-isotope labeled peptide used for measuring human ABCC10, one or more peptides selected from a peptide having an amino acid sequence shown by SEQ ID No: 233, SEQ ID No: 234, SEQ ID No: 235, SEQ ID No: 236, SEQ ID No: 237, SEQ ID No: 238, SEQ ID No: 239, SEQ ID No: 240, SEQ ID No: 241, SEQ ID No: 242, SEQ ID No: 243, SEQ ID No: 244, SEQ ID No: 245, and SEQ ID No: 246, SEQ ID No: 247, SEQ ID No: 248, SEQ ID No: 249, SEQ ID No : 250, SEQ ID No: 251, SEQ ID No: 252, SEQ ID No: 253, SEQ ID No: 254, and SEQ ID No: 255 of the sequence listing can be specifically exemplified.

Similarly, as the above plasma membrane protein to be quantified, human ABCC11 can be exemplified. As a subject peptide for quantification and a stable-isotope labeled peptide used for measuring human ABCC11, one or more peptides selected from a peptide having an amino acid sequence shown by SEQ ID No: 256, SEQ ID No: 257, SEQ ID No: 258, SEQ ID No: 259, SEQ ID No: 260, SEQ ID No: 261, SEQ ID No: 262, SEQ ID No: 263, SEQ ID No: 264, SEQ ID No: 265, SEQ ID No: 266, and SEQ ID No: 267 of the sequence listing can be specifically exemplified,

Similarly, as the above plasma membrane protein to be quantified, human ABCC12 can be exemplified. As a subject peptide for quantification and a stable-isotope labeled peptide used for measuring human ABCC12, one or more peptides selected from a peptide having an amino acid sequence shown by SEQ ID No: 268, and SEQ ID No: 269 of the sequence listing can be specifically exemplified.

Similarly, as the above plasma membrane protein to be quantified, human ABCC13 can be exemplified. As a subject peptide for quantification and a stable-isotope labeled peptide used for measuring human ABCC13, one or more peptides selected from a peptide having an amino acid sequence shown by SEQ ID No: 270 of the sequence listing can be specifically exemplified.

Similarly, as the above plasma membrane protein to be quantified, human ABCG1 can be exemplified. As a subject peptide for quantification and a stable-isotope labeled peptide used for measuring human ABCG1, one or more peptides selected from a peptide having an amino acid sequence shown by SEQ ID No: 271, SEQ ID No: 272, SEQ ID No: 273, SEQ ID No: 274, and SEQ ID No: 275 can be specifically exemplified.

Similarly, as the above plasma membrane protein to be quantified, human ABCG4 can be exemplified. As a subject peptide for quantification and a stable-isotope labeled peptide used for measuring human ABCG4, one or more peptides selected from a peptide having an amino acid sequence shown by SEQ ID No: 276, SEQ ID No: 277, and SEQ ID No: 278 of the sequence listing can be specifically exemplified.

Similarly, as the above plasma membrane protein to be quantified, human ABCG5 can be exemplified. As a subject peptide for quantification and a stable-isotope labeled peptide used for measuring human ABCG5, one or more peptides selected from a peptide having an amino acid sequence shown by SEQ ID No: 279, SEQ ID No: 280, SEQ ID No: 281, SEQ ID No: 282, and SEQ ID No: 283 of the sequence listing can be specifically exemplified.

Similarly, as the above plasma membrane protein to be quantified, human ABCG8 can be exemplified. As a subject peptide for quantification and a stable-isotope labeled peptide used for measuring human ABCG8, one or more peptides selected from a peptide having an amino acid sequence shown by SEQ ID No: 284 of the sequence listing can be specifically exemplified.

Similarly, as the above plasma membrane protein to be quantified, human SLC10A1 can be exemplified. As a subject peptide for quantification and a stable-isotope labeled peptide used for measuring human SLC10A1, one or more peptides selected from a peptide having an amino acid sequence shown by SEQ ID No: 285 of the sequence listing can be specifically exemplified.

Similarly, as the above plasma membrane protein to be quantified, human SLC15A1 can be exemplified. As a subject peptide for quantification and a stable-isotope labeled peptide used for measuring human SLC15A1, one or more peptides selected from a peptide having an amino acid sequence shown by SEQ ID No: 286 of the sequence listing can be specifically exemplified.

Similarly, as the above plasma membrane protein to be quantified, human SLC15A2 can be exemplified. As a subject peptide for quantification and a stable-isotope labeled peptide used for measuring human SLC15A2, one or more peptides selected from a peptide having an amino acid sequence shown by SEQ ID No: 287 of the sequence listing can be specifically exemplified.

Similarly, as the above plasma membrane protein to be quantified, human SLC16A1 can be exemplified. As a subject peptide for quantification and a stable-isotope labeled peptide used for measuring human SLC16A1, one or more peptides selected from a peptide having an amino acid sequence shown by SEQ ID No: 288 of the sequence listing can be specifically exemplified.

Similarly, as the above plasma membrane protein to be quantified, human SLC16A7 can be exemplified. As a subject peptide for quantification and a stable-isotope labeled peptide used for measuring human SLC16A7, one or more peptides selected from a peptide having an amino acid sequence shown by SEQ ID No: 289 of the sequence listing can be specifically exemplified.

Similarly, as the above plasma membrane protein to be quantified, human SLC21A2 can be exemplified. As a subject peptide for quantification and a stable-isotope labeled peptide used for measuring human SLC21A2, one or more peptides selected from a peptide having an amino acid sequence shown by SEQ ID No: 290 of the sequence listing can be specifically exemplified.

Similarly, as the above plasma membrane protein to be quantified, human SLC21A3 can be exemplified. As a subject peptide for quantification and a stable-isotope labeled peptide used for measuring human SLC21A3, one or more peptides selected from a peptide having an amino acid sequence shown by SEQ ID No: 291 of the sequence listing can be specifically exemplified.

Similarly, as the above plasma membrane protein to be quantified, human SLC21A6 can be exemplified. As a subject peptide for quantification and a stable-isotope labeled peptide used for measuring human SLC21A6, one or more peptides selected from a peptide having an amino acid sequence shown by SEQ ID No: 292 of the sequence listing can be specifically exemplified.

Similarly, as the above plasma membrane protein to be quantified, human SLC21A8 can be exemplified. As a subject peptide for quantification and a stable-isotope labeled peptide used for measuring human SLC21A8, one or more peptides selected from a peptide having an amino acid sequence shown by SEQ ID No: 293 of the sequence listing can be specifically exemplified.

Similarly, as the above plasma membrane protein to be quantified, human SLC21A9 can be exemplified. As a subject peptide for quantification and a stable-isotope labeled peptide used for measuring human SLC21A9, one or more peptides selected from a peptide having an amino acid sequence shown by SEQ ID No: 294 of the sequence listing can be specifically exemplified.

Similarly, as the above plasma membrane protein to be quantified, human SLC21A11 can be exemplified. As a subject peptide for quantification and a stable-isotope labeled peptide used for measuring human SLC21A11, one or more peptides selected from a peptide having an amino acid sequence shown by SEQ ID No: 295 of the sequence listing can be specifically exemplified.

Similarly, as the above plasma membrane protein to be quantified, human SLC21A12 can be exemplified. As a subject peptide for quantification and a stable-isotope labeled peptide used for measuring human SLC21A12, one or more peptides selected from a peptide having an amino acid sequence shown by SEQ ID No: 296 of the sequence listing can be specifically exemplified.

Similarly, as the above plasma membrane protein to be quantified, human SLC21A14 can be exemplified. As a subject peptide for quantification and a stable-isotope labeled peptide used for measuring human SLC21A14, one or more peptides selected from a peptide having an amino acid sequence shown by SEQ ID No: 297 of the sequence listing can be specifically exemplified.

Similarly, as the above plasma membrane protein to be quantified, human SLC21A15 can be exemplified. As a subject peptide for quantification and a stable-isotope labeled peptide used for measuring human SLC21A15, one or more peptides selected from a peptide having an amino acid sequence shown by SEQ ID No: 298 of the sequence listing can be specifically exemplified.

Similarly, as the above plasma membrane protein to be quantified, human SLC21A19 can be exemplified. As a subject peptide for quantification and a stable-isotope labeled peptide used for measuring human SLC21A19, one or more peptides selected from a peptide having an amino acid sequence shown by SEQ ID No: 299 of the sequence listing can be specifically exemplified.

Similarly, as the above plasma membrane protein to be quantified, human SLC21A20 can be exemplified. As a subject peptide for quantification and a stable-isotope labeled peptide used for measuring human SLC21A20, one or more peptides selected from a peptide having an amino acid sequence shown by SEQ ID No: 300 of the sequence listing can be specifically exemplified.

Similarly, as the above plasma membrane protein to be quantified, human SLC22A1 can be exemplified. As a subject peptide for quantification and a stable-isotope labeled peptide used for measuring human SLC22A1, one or more peptides selected from a peptide having an amino acid sequence shown by SEQ ID No: 301 of the sequence listing can be specifically exemplified.

Similarly, as the above plasma membrane protein to be quantified, human SLC22A2 can be exemplified. As a subject peptide for quantification and a stable-isotope labeled peptide used for measuring human SLC22A2, one or more peptides selected from a peptide having an amino acid sequence shown by SEQ ID No: 302 of the sequence listing can be specifically exemplified.

Similarly, as the above plasma membrane protein to be quantified, human SLC22A3 can be exemplified. As a subject peptide for quantification and a stable-isotope labeled peptide used for measuring human SLC22A3, one or more peptides selected from a peptide having an amino acid sequence shown by SEQ ID No: 303 of the sequence listing can be specifically exemplified.

Similarly, as the above plasma membrane protein to be quantified, human SLC22A4 can be exemplified. As a subject peptide for quantification and a stable-isotope labeled peptide used for measuring human SLC22A4, one or more peptides selected from a peptide having an amino acid sequence shown by SEQ ID No: 304 of the sequence listing can be specifically exemplified.

Similarly, as the above plasma membrane protein to be quantified, human SLC22A5 can be exemplified. As a subject peptide for quantification and a stable-isotope labeled peptide used for measuring human SLC22A5, one or more peptides selected from a peptide having an amino acid sequence shown by SEQ ID No: 305 of the sequence listing can be specifically exemplified.

Similarly, as the above plasma membrane protein to be quantified, human SLC22A6 can be exemplified. As a subject peptide for quantification and a stable-isotope labeled peptide used for measuring human SLC22A6, one or more peptides selected from a peptide having an amino acid sequence shown by SEQ ID No: 306 of the sequence listing can be specifically exemplified.

Similarly, as the above plasma membrane protein to be quantified, human SLC22A7 can be exemplified. As a subject peptide for quantification and a stable-isotope labeled peptide used for measuring human SLC22A7, one or more peptides selected from a peptide having an amino acid sequence shown by SEQ ID No: 307 of the sequence listing can be specifically exemplified.

Similarly, as the above plasma membrane protein to be quantified, human SLC22A8 can be exemplified. As a subject peptide for quantification and a stable-isotope labeled peptide used for measuring human SLC22A8, one or more peptides selected from a peptide having an amino acid sequence shown by SEQ ID No: 308 of the sequence listing can be specifically exemplified.

Similarly, as the above plasma membrane protein to be quantified, human SLC22A9 can be exemplified. As a subject peptide for quantification and a stable-isotope labeled peptide used for measuring human SLC22A9, one or more peptides selected from a peptide having an amino acid sequence shown by SEQ ID No: 309 of the sequence listing can be specifically exemplified.

Similarly, as the above plasma membrane protein to be quantified, human SLC22A10 can be exemplified. As a subject peptide for quantification and a stable-isotope labeled peptide used for measuring human SLC22A10, one or more peptides selected from a peptide having an amino acid sequence shown by SEQ ID No: 310 of the sequence listing can be specifically exemplified.

Similarly, as the above plasma membrane protein to be quantified, human SLC22A11 can be exemplified. As a subject peptide for quantification and a stable-isotope labeled peptide used for measuring human SLC22A11, one or more peptides selected from a peptide having an amino acid sequence shown by SEQ ID No: 311 of the sequence listing can be specifically exemplified.

Similarly, as the above plasma membrane protein to be quantified, human SLC22A12 can be exemplified. As a subject peptide for quantification and a stable-isotope labeled peptide used for measuring human SLC22A12, one or more peptides selected from a peptide having an amino acid sequence shown by SEQ ID No: 312 of the sequence listing can be specifically exemplified.

Similarly, as the above plasma membrane protein to be quantified, human SLC22A13 can be exemplified. As a subject peptide for quantification and a stable-isotope labeled peptide used for measuring human SLC22A13, one or more peptides selected from a peptide having an amino acid sequence shown by SEQ ID No: 313 of the sequence listing can be specifically exemplified.

Similarly, as the above plasma membrane protein to be quantified, human SLC22A14 can be exemplified. As a subject peptide for quantification and a stable-isotope labeled peptide used for measuring human SLC22A14, one or more peptides selected from a peptide having an amino acid sequence shown by SEQ ID No: 314 of the sequence listing can be specifically exemplified.

Similarly, as the above plasma membrane protein to be quantified, human SLC22A15 can be exemplified. As a subject peptide for quantification and a stable-isotope labeled peptide used for measuring human SLC22A15, one or more peptides selected from a peptide having an amino acid sequence shown by SEQ ID No: 315 of the sequence listing can be specifically exemplified.

Similarly, as the above plasma membrane protein to be quantified, human SLC22A16 can be exemplified. As a subject peptide for quantification and a stable-isotope labeled peptide used for measuring human SLC22A16, one or more peptides selected from a peptide having an amino acid sequence shown by SEQ ID No: 316 of the sequence listing can be specifically exemplified.

Similarly, as the above plasma membrane protein to be quantified, human SLC22A17 can be exemplified. As a subject peptide for quantification and a stable-isotope labeled peptide used for measuring human SLC22A17, one or more peptides selected from a peptide having an amino acid sequence shown by SEQ ID No: 317 of the sequence listing can be specifically exemplified.

Similarly, as the above plasma membrane protein to be quantified, human SLC22A18 can be exemplified. As a subject peptide for quantification and a stable-isotope labeled peptide used for measuring human SLC22A18, one or more peptides selected from a peptide having an amino acid sequence shown by SEQ ID No: 318 of the sequence listing can be specifically exemplified.

Similarly, as the above plasma membrane protein to be quantified, human SLC29A1 can be exemplified. As a subject peptide for quantification and a stable-isotope labeled peptide used for measuring human SLC29A1, one or more peptides selected from a peptide having an amino acid sequence shown by SEQ ID No: 319 of the sequence listing can be specifically exemplified.

Similarly, as the above plasma membrane protein to be quantified, human SLC29A2 can be exemplified. As a subject peptide for quantification and a stable-isotope labeled peptide used for measuring human SLC29A2, one or more peptides selected from a peptide having an amino acid sequence shown by SEQ ID No: 320 of the sequence listing can be specifically exemplified.

Similarly, as the above plasma membrane protein to be quantified, human SLC28A1 can be exemplified. As a subject peptide for quantification and a stable-isotope labeled peptide used for measuring human SLC28A1, one or more peptides selected from a peptide having an amino acid sequence shown by SEQ ID No: 321 of the sequence listing can be specifically exemplified.

Similarly, as the above plasma membrane protein to be quantified, human SLC28A3 can be exemplified. As a subject peptide for quantification and a stable-isotope labeled peptide used for measuring human SLC28A3, one or more peptides selected from a peptide having an amino acid sequence shown by SEQ ID No: 322 of the sequence listing can be specifically exemplified.

Similarly, as the above plasma membrane protein to be quantified, human SLC19A1 can be exemplified. As a subject peptide for quantification and a stable-isotope labeled peptide used for measuring human SLC19A1, one or more peptides selected from a peptide having an amino acid sequence shown by SEQ ID No: 323 of the sequence listing can be specifically exemplified.

Similarly, as the above plasma membrane protein to be quantified, human SLC1A1 can be exemplified. As a subject peptide for quantification and a stable-isotope labeled peptide used for measuring human SLC1A1, one or more peptides selected from a peptide having an amino acid sequence shown by SEQ ID No: 324 of the sequence listing can be specifically exemplified.

Similarly, as the above plasma membrane protein to be quantified, human SLC1A2 can be exemplified. As a subject peptide for quantification and a stable-isotope labeled peptide used for measuring human SLC1A2, one or more peptides selected from a peptide having an amino acid sequence shown by SEQ ID No: 325 of the sequence listing can be specifically exemplified.

Similarly, as the above plasma membrane protein to be quantified, human SLC1A3 can be exemplified. As a subject peptide for quantification and a stable-isotope labeled peptide used for measuring human SLC1A3, one or more peptides selected from a peptide having an amino acid sequence shown by SEQ ID No: 326 of the sequence listing can be specifically exemplified.

Similarly, as the above plasma membrane protein to be quantified, human SLC1A4 can be exemplified. As a subject peptide for quantification and a stable-isotope labeled peptide used for measuring human SLC1A4, one or more peptides selected from a peptide having an amino acid sequence shown by SEQ ID No: 327 of the sequence listing can be specifically exemplified.

Similarly, as the above plasma membrane protein to be quantified, human SLC1A5 can be exemplified. As a subject peptide for quantification and a stable-isotope labeled peptide used for measuring human SLC1A5, one or more peptides selected from a peptide having an amino acid sequence shown by SEQ ID No: 328 of the sequence listing can be specifically exemplified.

Similarly, as the above plasma membrane protein to be quantified, human SLC1A6 can be exemplified. As a subject peptide for quantification and a stable-isotope labeled peptide used for measuring human SLC1A6, one or more peptides selected from a peptide having an amino acid sequence shown by SEQ ID No: 329 of the sequence listing can be specifically exemplified.

Similarly, as the above plasma membrane protein to be quantified, human SLC1A7 can be exemplified. As a subject peptide for quantification and a stable-isotope labeled peptide used for measuring human SLC1A7 , one or more peptides selected from a peptide having an amino acid sequence shown by SEQ ID No: 330 of the sequence listing can be specifically exemplified.

Similarly, as the above plasma membrane protein to be quantified, human SLC2A1 can be exemplified. As a subject peptide for quantification and a stable-isotope labeled peptide used for measuring human SLC2A1, one or more peptides selected from a peptide having an amino acid sequence shown by SEQ ID No: 331 of the sequence listing can be specifically exemplified.

Similarly, as the above plasma membrane protein to be quantified, human SLC6A12 can be exemplified. As a subject peptide for quantification and a stable-isotope labeled peptide used for measuring human SLC6A12, one or more peptides selected from a peptide having an amino acid sequence shown by SEQ ID No: 332 of the sequence listing can be specifically exemplified.

Similarly, as the above plasma membrane protein to be quantified, human SLC7A5 can be exemplified. As a subject peptide for quantification and a stable-isotope labeled peptide used for measuring human SLC7A5, one or more peptides selected from a peptide having an amino acid sequence shown by SEQ ID No: 333 of the sequence listing can be specifically exemplified.

Similarly, as the above plasma membrane protein to be quantified, human SLC17A1 can be exemplified. As a subject peptide for quantification and a stable-isotope labeled peptide used for measuring human SLC17A1, one or more peptides selected from a peptide having an amino acid sequence shown by SEQ ID No: 334 of the sequence listing can be specifically exemplified.

Similarly, as the above plasma membrane protein to be quantified, human SLC17A2 can be exemplified. As a subject peptide for quantification and a stable-isotope labeled peptide used for measuring human SLC17A2, one or more peptides selected from a peptide having an amino acid sequence shown by SEQ ID No: 335 of the sequence listing can be specifically exemplified.

Similarly, as the above plasma membrane protein to be quantified, human SLC17A3 can be exemplified. As a subject peptide for quantification and a stable-isotope labeled peptide used for measuring human SLC17A3, one or more peptides selected from a peptide having an amino acid sequence shown by SEQ ID No: 336 of the sequence listing can be specifically exemplified.

Similarly, as the above plasma membrane protein to be quantified, human SLC17A4 can be exemplified. As a subject peptide for quantification and a stable-isotope labeled peptide used for measuring human SLC17A4, one or more peptides selected from a peptide having an amino acid sequence shown by SEQ ID No: 337 of the sequence listing can be specifically exemplified.

Similarly, as the above plasma membrane protein to be quantified, human SLC17A5 can be exemplified. As a subject peptide for quantification and a stable-isotope labeled peptide used for measuring human SLC17A5, one or more peptides selected from a peptide having an amino acid sequence shown by SEQ ID No: 338 of the sequence listing can be specifically exemplified.

Similarly, as the above plasma membrane protein to be quantified, human SLC17A6 can be exemplified. As a subject peptide for quantification and a stably-isotope labeled peptide used for measuring human SLC17A6, one or more peptides selected from a peptide having an amino acid sequence shown by SEQ ID No: 339 of the sequence listing can be specifically exemplified.

Similarly, as the above plasma membrane protein to be quantified, human SLC17A7 can be exemplified. As a subject peptide for quantification and a stable-isotope labeled peptide used for measuring human SLC17A7, one or more peptides selected from a peptide having an amino acid sequence shown by SEQ ID No: 340 of the sequence listing can be specifically exemplified.

Similarly, as the above plasma membrane protein to be quantified, human SLC17A8 can be exemplified. As a subject peptide for quantification and a stable-isotope labeled peptide used for measuring human SLC17A8, one or more peptides selected from a peptide having an amino acid sequence shown by SEQ ID No: 341 of the sequence listing can be specifically exemplified.

Similarly, as the above plasma membrane protein to be quantified, human SLC29A4 can be exemplified. As a subject peptide for quantification and a stable-isotope labeled peptide used for measuring human SLC29A4, one or more peptides selected from a peptide having an amino acid sequence shown by SEQ ID No: 342 of the sequence listing can be specifically exemplified.

Similarly, as the above plasma membrane protein to be quantified, human SLC44A1 can be exemplified. As a subject peptide for quantification and a stable-isotope labeled peptide used for measuring human SLC44A1, one or more peptides selected from a peptide having an amino acid sequence shown by SEQ ID No: 343 of the sequence listing can be specifically exemplified.

Similarly, as the above plasma membrane protein to be quantified, human SLC44A2 can be exemplified. As a subject peptide for quantification and a stable-isotope labeled peptide used for measuring human SLC44A2, one or more peptides selected from a peptide having an amino acid sequence shown by SEQ ID No: 344 of the sequence listing can be specifically exemplified.

Similarly, as the above plasma membrane protein to be quantified, human SLC44A3 can be exemplified. As a subject peptide for quantification and a stable-isotope labeled peptide used for measuring human SLC44A3, one or more peptides selected from a peptide having an amino acid sequence shown by SEQ ID No: 345 of the sequence listing can be specifically exemplified.

Similarly, as the above plasma membrane protein to be quantified, human SLC44A4 can be exemplified. As a subject peptide for quantification and a stable-isotope labeled peptide used for measuring human SLC44A4, one or more peptides selected from a peptide having an amino acid sequence shown by SEQ ID No: 346 of the sequence listing can be specifically exemplified.

Similarly, as the above plasma membrane protein to be quantified, human SLC44A5 can be exemplified. As a subject peptide for quantification and a stable-isotope labeled peptide used for measuring human SLC44A5, one or more peptides selected from a peptide having an amino acid sequence shown by SEQ ID No: 347 of the sequence listing can be specifically exemplified.

Similarly, as the above plasma membrane protein to be quantified, human MATE1 can be exemplified. As a subject peptide for quantification and a stable-isotope labeled peptide used for measuring human MATE1, one or more peptides selected from a peptide having an amino acid sequence shown by SEQ ID No: 348 of the sequence listing can be specifically exemplified.

Similarly, as the above plasma membrane protein to be quantified, human MATE2 can be exemplified. As a subject peptide for quantification and a stable-isotope labeled peptide used for measuring human MATE2, one or more peptides selected from a peptide having an amino acid sequence shown by SEQ ID No: 349 of the sequence listing can be specifically exemplified.

Similarly, as the above plasma membrane protein to be quantified, human SLC2A2 can be exemplified. As a subject peptide for quantification and a stable-isotope labeled peptide used for measuring human SLC2A2, one or more peptides selected from a peptide having an amino acid sequence shown by SEQ ID No: 350 and SEQ ID NO: 351 of the sequence listing can be specifically exemplified.

Similarly, as the above plasma membrane protein to be quantified, human SLC2A8 can be exemplified. As a subject peptide for quantification and a stable-isotope labeled peptide used for measuring human SLC2A8, one or more peptides selected from a peptide having an amino acid sequence shown by SEQ ID No: 352 and SEQ ID NO: 353 of the sequence listing can be specifically exemplified.

Similarly, as the above plasma membrane protein to be quantified, human SLC2A9 can be exemplified. As a subject peptide for quantification and a stable-isotope labeled peptide used for measuring human SLC2A9, one or more peptides selected from a peptide having an amino acid sequence shown by SEQ ID No: 354, SEQ ID NO: 355 and SEQ ID NO: 356 of the sequence listing can be specifically exemplified.

Similarly, as the above plasma membrane protein to be quantified, human SLC2A10 can be exemplified. As a subject peptide for quantification and a stable-isotope labeled peptide used for measuring human SLC2A10, one or more peptides selected from a peptide having an amino acid sequence shown by SEQ ID No: 357, SEQ ID No: 358, SEQ ID No: 359, and SEQ ID No: 360 of the sequence listing can be specifically exemplified.

Similarly, as the above plasma membrane protein to be quantified, human SLC4A5 can be exemplified. As a subject peptide for quantification and a stable-isotope labeled peptide used for measuring human SLC4A5, one or more peptides selected from a peptide having an amino acid sequence shown by SEQ ID No: 361, SEQ ID No: 362, SEQ ID No: 363, SEQ ID No: 364, SEQ ID No: 365, SEQ ID No: 366, SEQ ID No: 367, SEQ ID No: 368, SEQ ID No: 369, SEQ ID No: 370, SEQ ID No: 371, SEQ ID No: 372, and SEQ ID No: 373 of the sequence listing can be specifically exemplified.

Similarly, as the above plasma membrane protein to be quantified, human SLC4A7 can be exemplified. As a subject peptide for quantification and a stable-isotope labeled peptide used for measuring human SLC4A7, one or more peptides selected from a peptide having an amino acid sequence shown by SEQ ID No: 374, SEQ ID No: 375, SEQ ID No: 376, SEQ ID No: 377, SEQ ID No: 378, and SEQ ID No: 379 of the sequence listing can be specifically exemplified.

Similarly, as the above plasma membrane protein to be quantified, human SLC5A9 can be exemplified. As a subject peptide for quantification and a stable-isotope labeled peptide used for measuring human SLC5A9, one or more peptides selected from a peptide having an amino acid sequence shown by SEQ ID No: 380 and SEQ ID NO: 381 of the sequence listing can be specifically exemplified.

Similarly, as the above plasma membrane protein to be quantified, human SLC5A11 can be exemplified. As a subject peptide for quantification and a stable-isotope labeled peptide used for measuring human SLC5A11, one or more peptides selected from a peptide having an amino acid sequence shown by SEQ ID No: 382, SEQ ID No: 383, SEQ ID No: 384, and SEQ ID No: 385 of the sequence listing can be specifically exemplified.

Similarly, as the above plasma membrane protein to be quantified, human SLC6A9 can be exemplified. As a subject peptide for quantification and a stable-isotope labeled peptide used for measuring human SLC6A9, one or more peptides selected from a peptide having an amino acid sequence shown by SEQ ID No: 386 and SEQ ID NO: 387 of the sequence listing can be specifically exemplified.

Similarly, as the above plasma membrane protein to be quantified, human SLC6A13 can be exemplified. As a subject peptide for quantification and a stable-isotope labeled peptide used for measuring human SLC6A13, one or more peptides selected from a peptide having an amino acid sequence shown by SEQ ID No: 388 of the sequence listing can be specifically exemplified.

Similarly, as the above plasma membrane protein to be quantified, human SLC7A2 can be exemplified. As a subject peptide for quantification and a stable-isotope labeled peptide used for measuring human SLC7A2, one or more peptides selected from a peptide having an amino acid sequence shown by SEQ ID No: 389, SEQ ID No: 390, SEQ ID No: 391, SEQ ID No: 392, SEQ ID No: 393, SEQ ID No: 394, SEQ ID No: 395, SEQ ID No: 396, SEQ ID No: 397 of the sequence listing can be specifically exemplified.

Similarly, as the above plasma membrane protein to be quantified, human SLC7A6 can be exemplified. As a subject peptide for quantification and a stable-isotope labeled peptide used for measuring human SLC7A6, one or more peptides selected from a peptide having an amino acid sequence shown by SEQ ID No: 398, SEQ ID No: 399, and SEQ ID No: 400 of the sequence listing can be specifically exemplified.

Similarly, as the above plasma membrane protein to be quantified, human SLC7A9 can be exemplified. As a subject peptide for quantification and a stable-isotope labeled peptide used for measuring human SLC7A9, one or more peptides selected from a peptide having an amino acid sequence shown by SEQ ID No: 401 of the sequence listing can be specifically exemplified.

Similarly, as the above plasma membrane protein to be quantified, human SLC10A6 can be exemplified. As a subject peptide for quantification and a stable-isotope labeled peptide used for measuring human SLC10A6, one or more peptides selected from a peptide having an amino acid sequence shown by SEQ ID No: 402 and SEQ ID No: 403 of the sequence listing can be specifically exemplified.

Similarly, as the above plasma membrane protein to be quantified, human SLC13A3 can be exemplified. As a subject peptide for quantification and a stable-isotope labeled peptide used for measuring human SLC13A3, one or more peptides selected from a peptide having an amino acid sequence shown by SEQ ID No: 404, SEQ ID No: 405, SEQ ID No: 406, and SEQ ID No: 407 of the sequence listing can be specifically exemplified.

Similarly, as the above plasma membrane protein to be quantified, human SLC13A5 can be exemplified. As a subject peptide for quantification and a stable-isotope labeled peptide used for measuring human SLC13A5, one or more peptides selected from a peptide having an amino acid sequence shown by SEQ ID No: 408 of the sequence listing can be specifically exemplified.

Similarly, as the above plasma membrane protein to be quantified, human SLC16A2 can be exemplified. As a subject peptide for quantification and a stable-isotope labeled peptide used for measuring human SLC16A2, one or more peptides selected from a peptide having an amino acid sequence shown by SEQ ID No: 409, SEQ ID No: 410, and SEQ ID No: 411 of the sequence listing can be specifically exemplified.

Similarly, as the above plasma membrane protein to be quantified, human SLC16A4 can be exemplified. As a subject peptide for quantification and a stable-isotope labeled peptide used for measuring human SLC16A4, one or more peptides selected from a peptide having an amino acid sequence shown by SEQ ID No: 412, SEQ ID No: 413, and SEQ ID No: 414 of the sequence listing can be specifically exemplified.

Similarly, as the above plasma membrane protein to be quantified, human SLC10A2 can be exemplified. As a subject peptide for quantification and a stable-isotope labeled peptide used for measuring human SLC10A2, one or more peptides selected from a peptide having an amino acid sequence shown by SEQ ID No: 415 of the sequence listing can be specifically exemplified.

Similarly, as the above plasma membrane protein to be quantified, human SLC10A3 can be exemplified. As a subject peptide for quantification and a stable-isotope labeled peptide used for measuring human SLC10A3, one or more peptides selected from a peptide having an amino acid sequence shown by SEQ ID No: 416, and SEQ ID No: 417 of the sequence listing can be specifically exemplified.

Similarly, as the above plasma membrane protein to be quantified, human SLC10A4 can be exemplified. As a subject peptide for quantification and a stable-isotope labeled peptide used for measuring human SLC10A4, one or more peptides selected from a peptide having an amino acid sequence shown by SEQ ID No: 418 of the sequence listing can be specifically exemplified.

Similarly, as the above plasma membrane protein to be quantified, human SLC10A5 can be exemplified. As a subject peptide for quantification and a stable-isotope labeled peptide used for measuring human SLC10A5, one or more peptides selected from a peptide having an amino acid sequence shown by SEQ ID No: 419, SEQ ID No: 420, and SEQ ID No: 421 of the sequence listing can be specifically exemplified.

Similarly, as the above plasma membrane protein to be quantified, human SLC11A1 can be exemplified. As a subject peptide for quantification and a stable-isotope labeled peptide used for measuring human SLC11A1, one or more peptides selected from a peptide having an amino acid sequence shown by SEQ ID No: 422 of the sequence listing can be specifically exemplified.

Similarly, as the above plasma membrane protein to be quantified, human SLC11A2 can be exemplified. As a subject peptide for quantification and a stable-isotope labeled peptide used for measuring human SLC11A2, one or more peptides selected from a peptide having an amino acid sequence shown by SEQ ID No: 423 of the sequence listing can be specifically exemplified.

Similarly, as the above plasma membrane protein to be quantified, human SLC12A1 can be exemplified. As a subject peptide for quantification and a stable-isotope labeled peptide used for measuring human SLC12A1, one or more peptides selected from a peptide having an amino acid sequence shown by SEQ ID No: 424, SEQ ID No: 425, SEQ ID No: 426, and SEQ ID No: 427 of the sequence listing can be specifically exemplified.

Similarly, as the above plasma membrane protein to be quantified, human SLC12A2 can be exemplified. As a subject peptide for quantification and a stable-isotope labeled peptide used for measuring human SLC12A2, one or more peptides selected from a peptide having an amino acid sequence shown by SEQ ID No: 428, SEQ ID No: 429, SEQ ID No: 430, SEQ ID No: 431, and SEQ ID No: 432 of the sequence listing can be specifically exemplified.

Similarly, as the above plasma membrane protein to be quantified, human SLC12A3 can be exemplified. As a subject peptide for quantification and a stable-isotope labeled peptide used for measuring human SLC12A3, one or more peptides selected from a peptide having an amino acid sequence shown by SEQ ID No: 433, SEQ ID No: 434, and SEQ ID No: 435 of the sequence listing can be specifically exemplified.

Similarly, as the above plasma membrane protein to be quantified, human SLC12A4 can be exemplified. As a subject peptide for quantification and a stable-isotope labeled peptide used for measuring human SLC12A4, one or more peptides selected from a peptide having an amino acid sequence shown by SEQ ID No: 436, SEQ ID No: 437, SEQ ID No: 438, SEQ ID No: 439, SEQ ID No: 440, and SEQ ID No: 441 of the sequence listing can be specifically exemplified.

Similarly, as the above plasma membrane protein to be quantified, human SLC12A5 can be exemplified. As a subject peptide for quantification and a stable-isotope labeled peptide used for measuring human SLC12A5, one or more peptides selected from a peptide having an amino acid sequence shown by SEQ ID No: 442, SEQ ID No: 443, SEQ ID No: 444, and SEQ ID No: 445 of the sequence listing can be specifically exemplified.

Similarly, as the above plasma membrane protein to be quantified, human SLC12A6 can be exemplified. As a subject peptide for quantification and a stable-isotope labeled peptide used for measuring human SLC12A6, one or more peptides selected from a peptide having an amino acid sequence shown by SEQ ID No: 446, SEQ ID No: 447, SEQ ID No: 448, SEQ ID No: 449, SEQ ID No: 450, SEQ ID No: 451, SEQ ID No: 452, SEQ ID No: 453, and SEQ ID No: 454 of the sequence listing can be specifically exemplified.

Similarly, as the above plasma membrane protein to be quantified, human SLC12A7 can be exemplified. As a subject peptide for quantification and a stable-isotope labeled peptide used for measuring human SLC12A7, one or more peptides selected from a peptide having an amino acid sequence shown by SEQ ID No: 455, SEQ ID No: 456, SEQ ID No: 457, SEQ ID No: 458, SEQ ID No: 459, SEQ ID No: 460, SEQ ID No: 461, SEQ ID No: 462, SEQ ID No: 463, and SEQ ID No: 464 of the sequence listing can be specifically exemplified.

Similarly, as the above plasma membrane protein to be quantified, human SLC12A8 can be exemplified. As a subject peptide for quantification and a stable-isotope labeled peptide used for measuring human SLC12A8, one or more peptides selected from a peptide having an amino acid sequence shown by SEQ ID No: 465, and SEQ ID No: 466 of the sequence listing can be specifically exemplified.

Similarly, as the above plasma membrane protein to be quantified, human SLC12A9 can be exemplified. As a subject peptide for quantification and a stable-isotope labeled peptide used for measuring human SLC12A9, one or more peptides selected from a peptide having an amino acid sequence shown by SEQ ID No: 467, SEQ ID No: 468, SEQ ID No: 469, SEQ ID No: 470, SEQ ID No: 471, SEQ ID No: 472, SEQ ID No: 473, SEQ ID No: 474, SEQ ID No: 475, and SEQ ID No: 476 of the sequence listing can be specifically exemplified.

Similarly, as the above plasma membrane protein to be quantified, human SLC13A1 can be exemplified. As a subject peptide for quantification and a stable-isotope labeled peptide used for measuring human SLC13A1, one or more peptides selected from a peptide having an amino acid sequence shown by SEQ ID No: 477 of the sequence listing can be specifically exemplified.

Similarly, as the above plasma membrane protein to be quantified, human SLC13A2 can be exemplified. As a subject peptide for quantification and a stable-isotope labeled peptide used for measuring human SLC13A2, one or more peptides selected from a peptide having an amino acid sequence shown by SEQ ID No: 478, and SEQ ID No: 479 of the sequence listing can be specifically exemplified.

Similarly, as the above plasma membrane protein to be quantified, human SLC13A4 can be exemplified. As a subject peptide for quantification and a stable-isotope labeled peptide used for measuring human SLC13A4, one or more peptides selected from a peptide having an amino acid sequence shown by SEQ ID No: 480, and SEQ ID No: 481 of the sequence listing can be specifically exemplified.

Similarly, as the above plasma membrane protein to be quantified, human SLC14A1 can be exemplified. As a subject peptide for quantification and a stable-isotope labeled peptide used for measuring human SLC14A1, one or more peptides selected from a peptide having an amino acid sequence shown by SEQ ID No: 482 of the sequence listing can be specifically exemplified.

Similarly, as the above plasma membrane protein to be quantified, human SLC14A2 can be exemplified. As a subject peptide for quantification and a stable-isotope labeled peptide used for measuring human SLC14A2, one or more peptides selected from a peptide having an amino acid sequence shown by SEQ ID No: 483 of the sequence listing can be specifically exemplified.

Similarly, as the above plasma membrane protein to be quantified, human SLC15A3 can be exemplified. As a subject peptide for quantification and a stable-isotope labeled peptide used for measuring human SLC15A3, one or more peptides selected from a peptide having an amino acid sequence shown by SEQ ID No: 484, SEQ ID No: 485, SEQ ID No: 486, SEQ ID No: 487, and SEQ ID No: 488 of the sequence listing can be specifically exemplified.

Similarly, as the above plasma membrane protein to be quantified, human SLC15A4 can be exemplified. As a subject peptide for quantification and a stable-isotope labeled peptide used for measuring human SLC15A4, one or more peptides selected from a peptide having an amino acid sequence shown by SEQ ID No: 489, and SEQ ID No: 490 of the sequence listing can be specifically exemplified.

Similarly, as the above plasma membrane protein to be quantified, human SLC16A10 can be exemplified. As a subject peptide for quantification and a stable-isotope labeled peptide used for measuring human SLC16A10, one or more peptides selected from a peptide having an amino acid sequence shown by SEQ ID No: 491 of the sequence listing can be specifically exemplified.

Similarly, as the above plasma membrane protein to be quantified, human SLC16A11 can be exemplified. As a subject peptide for quantification and a stable-isotope labeled peptide used for measuring human SLCA11, one or more peptides selected from a peptide having an amino acid sequence shown by SEQ ID No: 492, and SEQ ID No: 493 of the sequence listing can be specifically exemplified.

Similarly, as the above plasma membrane protein to be quantified, human SLC16A12 can be exemplified. As a subject peptide for quantification and a stable-isotope labeled peptide used for measuring human SLC16A12, one or more peptides selected from a peptide having an amino acid sequence shown by SEQ ID No: 494 of the sequence listing can be specifically exemplified.

Similarly, as the above plasma membrane protein to be quantified, human SLC16A13 can be exemplified. As a subject peptide for quantification and a stable-isotope labeled peptide used for measuring human SLC16A13, one or more peptides selected from a peptide having an amino acid sequence shown by SEQ ID No: 495 of the sequence listing can be specifically exemplified.

Similarly, as the above plasma membrane protein to be quantified, human SLC16A14 can be exemplified. As a subject peptide for quantification and a stable-isotope labeled peptide used for measuring human SLC16A14, one or more peptides selected from a peptide having an amino acid sequence shown by SEQ ID No: 496 of the sequence listing can be specifically exemplified.

Similarly, as the above plasma membrane protein to be quantified, human SLC16A3 can be exemplified. As a subject peptide for quantification and a stable-isotope labeled peptide used for measuring human SLC16A3, one or more peptides selected from a peptide having an amino acid sequence shown by SEQ ID No: 497, and SEQ ID No: 498 of the sequence listing can be specifically exemplified.

Similarly, as the above plasma membrane protein to be quantified, human SLC16A5 can be exemplified. As a subject peptide for quantification and a stable-isotope labeled peptide used for measuring human SLC16A5, one or more peptides selected from a peptide having an amino acid sequence shown by SEQ ID No: 499 of the sequence listing can be specifically exemplified.

Similarly, as the above plasma membrane protein to be quantified, human SLC16A6 can be exemplified. As a subject peptide for quantification and a stable-isotope labeled peptide used for measuring human SLC16A6, one or more peptides selected from a peptide having an amino acid sequence shown by SEQ ID No: 500 of the sequence listing can be specifically exemplified.

Similarly, as the above plasma membrane protein to be quantified, human SLC16A8 can be exemplified. As a subject peptide for quantification and a stable-isotope labeled peptide used for measuring human SLC16A8, one or more peptides selected from a peptide having an amino acid sequence shown by SEQ ID No: 501, SEQ ID No: 502, and SEQ ID No: 503 of the sequence listing can be specifically exemplified.

Similarly, as the above plasma membrane protein to be quantified, human SLC16A9 can be exemplified. As a subject peptide for quantification and a stable-isotope labeled peptide used for measuring human SLC16A9, one or more peptides selected from a peptide having an amino acid sequence shown by SEQ ID No: 504, SEQ ID No: 505, and SEQ ID No: 506 of the sequence listing can be specifically exemplified.

Similarly, as the above plasma membrane protein to be quantified, human SLC18A1 can be exemplified. As a subject peptide for quantification and a stable-isotope labeled peptide used for measuring human SLC18A1, one or more peptides selected from a peptide having an amino acid sequence shown by SEQ ID No: 507, and SEQ ID No: 508 of the sequence listing can be specifically exemplified.

Similarly, as the above plasma membrane protein to be quantified, human SLC18A2 can be exemplified. As a subject peptide for quantification and a stable-isotope labeled peptide used for measuring human SLC18A2, one or more peptides selected from a peptide having an amino acid sequence shown by SEQ ID No: 509, and SEQ ID No: 510 of the sequence listing can be specifically exemplified.

Similarly, as the above plasma membrane protein to be quantified, human SLC18A3 can be exemplified. As a subject peptide for quantification and a stable-isotope labeled peptide used for measuring human SLC18A3, one or more peptides selected from a peptide having an amino acid sequence shown by SEQ ID No: 511, SEQ ID No: 512, SEQ ID No: 513, SEQ ID No: 514, and SEQ ID No: 515 of the sequence listing can be specifically exemplified.

Similarly, as the above plasma membrane protein to be quantified, human SLC19A2 can be exemplified. As a subject peptide for quantification and a stable-isotope labeled peptide used for measuring human SLC19A2, one or more peptides selected from a peptide having an amino acid sequence shown by SEQ ID No: 516, and SEQ ID No: 517 of the sequence listing can be specifically exemplified.

Similarly, as the above plasma membrane protein to be quantified, human SLC19A3 can be exemplified. As a subject peptide for quantification and a stable-isotope labeled peptide used for measuring human SLC19A3, one or more peptides selected from a peptide having an amino acid sequence shown by SEQ ID No: 518, and SEQ ID No: 519 of the sequence listing can be specifically exemplified.

Similarly, as the above plasma membrane protein to be quantified, human SLC20A1 can be exemplified. As a subject peptide for quantification and a stable-isotope labeled peptide used for measuring human SLC20A1, one or more peptides selected from a peptide having an amino acid sequence shown by SEQ ID No: 520, SEQ ID No: 521, SEQ ID No: 522, SEQ ID No: 523, SEQ ID No: 524, SEQ ID No: 525, SEQ ID No: 526, SEQ ID No: 527, SEQ ID No: 528, SEQ ID No: 529, SEQ ID No: 530, SEQ ID No: 531, and SEQ ID No: 532 of the sequence listing can be specifically exemplified.

Similarly, as the above plasma membrane protein to be quantified, human SLC20A2 can be exemplified. As a subject peptide for quantification and a stable-isotope labeled peptide used for measuring human SLC20A2, one or more peptides selected from a peptide having an amino acid sequence shown by SEQ ID No: 533, SEQ ID No: 534, SEQ ID No: 535, SEQ ID No: 536, SEQ ID No: 537, SEQ ID No: 538, SEQ ID No: 539, SEQ ID No: 540, SEQ ID No: 541, SEQ ID No: 542, SEQ ID No: 543, SEQ ID No: 544, SEQ ID No: 545, and SEQ ID No: 546 of the sequence listing can be specifically exemplified.

Similarly, as the above plasma membrane protein to be quantified, human SLC23A1 can be exemplified. As a subject peptide for quantification and a stable-isotope labeled peptide used for measuring human SLC23A1, one or more peptides selected from a peptide having an amino acid sequence shown by SEQ ID No: 547, SEQ ID No: 548, SEQ ID No: 549, and SEQ ID No: 550 of the sequence listing can be specifically exemplified.

Similarly, as the above plasma membrane protein to be quantified, human SLC23A2 can be exemplified. As a subject peptide for quantification and a stable-isotope labeled peptide used for measuring human SLC23A2, one or more peptides selected from a peptide having an amino acid sequence shown by SEQ ID No: 551, SEQ ID No: 552, SEQ ID No: 553, SEQ ID No: 554, and SEQ ID No: 555 of the sequence listing can be specifically exemplified.

Similarly, as the above plasma membrane protein to be quantified, human SLC23A3 can be exemplified. As a subject peptide for quantification and a stable-isotope labeled peptide used for measuring human SLC23A3, one or more peptides selected from a peptide having an amino acid sequence shown by SEQ ID No: 556, SEQ ID No: 557, SEQ ID No: 558, SEQ ID No: 559, and SEQ ID No: 560 of the sequence listing can be specifically exemplified.

Similarly, as the above plasma membrane protein to be quantified, human SLC24A1 can be exemplified. As a subject peptide for quantification and a stable-isotope labeled peptide used for measuring human SLC24A1, one or more peptides selected from a peptide having an amino acid sequence shown by SEQ ID No: 561, SEQ ID No: 562, SEQ ID No: 563, SEQ ID No: 564, SEQ ID No: 565, SEQ ID No: 566, SEQ ID No: 567, SEQ ID No: 568, SEQ ID No: 569, SEQ ID No: 570, SEQ ID No: 571, SEQ ID No: 572, SEQ ID No: 573, and SEQ ID No: 574 of the sequence listing can be specifically exemplified.

Similarly, as the above plasma membrane protein to be quantified, human SLC24A2 can be exemplified. As a subject peptide for quantification and a stable-isotope labeled peptide used for measuring human SLC24A2, one or more peptides selected from a peptide having an amino acid sequence shown by SEQ ID No: 575, SEQ ID No: 576, SEQ ID No: 577, SEQ ID No: 578, SEQ ID No: 579, and SEQ ID No: 580 of the sequence listing can be specifically exemplified.

Similarly, as the above plasma membrane protein to be quantified, human SLC24A3 can be exemplified. As a subject peptide for quantification and a stable-isotope labeled peptide used for measuring human SLC24A3, one or more peptides selected from a peptide having an amino acid sequence shown by SEQ ID No: 581, SEQ ID No: 582, SEQ ID No: 583, and SEQ ID No: 584 of the sequence listing can be specifically exemplified.

Similarly, as the above plasma membrane protein to be quantified, human SLC24A4 can be exemplified. As a subject peptide for quantification and a stable-isotope labeled peptide used for measuring human SLC24A4, one or more peptides selected from a peptide having an amino acid sequence shown by SEQ ID No: 585, and SEQ ID No: 586 of the sequence listing can be specifically exemplified.

Similarly, as the above plasma membrane protein to be quantified, human SLC24A5 can be exemplified. As a subject peptide for quantification and a stable-isotope labeled peptide used for measuring human SLC24A5, one or more peptides selected from a peptide having an amino acid sequence shown by SEQ ID No: 587 of the sequence listing can be specifically exemplified.

Similarly, as the above plasma membrane protein to be quantified, human SLC24A6 can be exemplified. As a subject peptide for quantification and a stable-isotope labeled peptide used for measuring human SLC24A6, one or more peptides selected from a peptide having an amino acid sequence shown by SEQ ID No: 588, SEQ ID No: 589, and SEQ ID No: 590 of the sequence listing can be specifically exemplified.

Similarly, as the above plasma membrane protein to be quantified, human SLC25A1 can be exemplified. As a subject peptide for quantification and a stable-isotope labeled peptide used for measuring human SLC25A1, one or more peptides selected from a peptide having an amino acid sequence shown by SEQ ID No: 591, SEQ ID No: 592, SEQ ID No: 593, SEQ ID No: 594, SEQ ID No: 595, SEQ ID No: 596, and SEQ ID No: 597 of the sequence listing can be specifically exemplified.

Similarly, as the above plasma membrane protein to be quantified, human SLC25A10 can be exemplified. As a subject peptide for quantification and a stable-isotope labeled peptide used for measuring human SLC25A10, one or more peptides selected from a peptide having an amino acid sequence shown by SEQ ID No: 598, SEQ ID No: 599, SEQ ID No: 600, and SEQ ID No: 601 of the sequence listing can be specifically exemplified.

Similarly, as the above plasma membrane protein to be quantified, human SLC25A11 can be exemplified. As a subject peptide for quantification and a stable-isotope labeled peptide used for measuring human SLC25A11, one or more peptides selected from a peptide having an amino acid sequence shown by SEQ ID No: 602, SEQ ID No: 603, SEQ ID No: 604, SEQ ID No: 605, SEQ ID No: 606, and SEQ ID No: 607 of the sequence listing can be specifically exemplified.

Similarly, as the above plasma membrane protein to be quantified, human SLC25A12 can be exemplified. As a subject peptide for quantification and a stable-isotope labeled peptide used for measuring human SLC25A12, one or more peptides selected from a peptide having an amino acid sequence shown by SEQ ID No: 608, SEQ ID No: 607, SEQ ID No: 608, SEQ ID No: 609, SEQ ID No: 610, SEQ ID No: 611, SEQ ID No: 612, SEQ ID No: 613, SEQ ID No: 614, SEQ ID No: 615, SEQ ID No: 616, SEQ ID No: 617, SEQ ID No: 618, SEQ ID No: 619, SEQ ID No: 620, SEQ ID No: 621, SEQ ID No: 622, SEQ ID No: 623, SEQ ID No: 624, SEQ ID No: 625, SEQ ID No: 626, and SEQ ID No: 627 of the sequence listing can be specifically exemplified.

Similarly, as the above plasma membrane protein to be quantified, human SLC25A13 can be exemplified. As a subject peptide for quantification and a stable-isotope labeled peptide used for measuring human SLC25A13, one or more peptides selected from a peptide having an amino acid sequence shown by SEQ ID No: 628, SEQ ID No: 629, SEQ ID No: 630, SEQ ID No: 631, SEQ ID No: 632, SEQ ID No: 633, SEQ ID No: 634, SEQ ID No: 635, SEQ ID No: 636, SEQ ID No: 637, SEQ ID No: 638, SEQ ID No: 639, SEQ ID No: 640, SEQ ID No: 641, SEQ ID No: 642, SEQ ID No: 643, SEQ ID No: 644, and SEQ ID No: 645 of the sequence listing can be specifically exemplified.

Similarly, as the above plasma membrane protein to be quantified, human SLC25A14 can be exemplified. As a subject peptide for quantification and a stable-isotope labeled peptide used for measuring human SLC25A14, one or more peptides selected from a peptide having an amino acid sequence shown by SEQ ID No: 646, SEQ ID No: 647, SEQ ID No: 648, SEQ ID No: 649and SEQ ID No: 650 of the sequence listing can be specifically exemplified.

Similarly, as the above plasma membrane protein to be quantified, human SLC25A15 can be exemplified. As a subject peptide for quantification and a stable-isotope labeled peptide used for measuring human SLC25A15, one or more peptides selected from a peptide having an amino acid sequence shown by SEQ ID No: 651, SEQ ID No: 652, and SEQ ID No: 653 of the sequence listing can be specifically exemplified.

Similarly, as the above plasma membrane protein to be quantified, human SLC25A16 can be exemplified. As a subject peptide for quantification and a stable-isotope labeled peptide used for measuring human SLC25A16, one or more peptides selected from a peptide having an amino acid sequence shown by SEQ ID No: 654, SEQ ID No: 655, SEQ ID No: 656, SEQ ID No: 657, SEQ ID No: 658, SEQ ID No: 659, SEQ ID No: 660, SEQ ID No: 661, SEQ ID No: 662, SEQ ID No: 663, SEQ ID No: 664, SEQ ID No: 665, and SEQ ID No: 666 of the sequence listing can be specifically exemplified.

Similarly, as the above plasma membrane protein to be quantified, human SLC25A17 can be exemplified. As a subject peptide for quantification and a stable-isotope labeled peptide used for measuring human SLC25A17, one or more peptides selected from a peptide having an amino acid sequence shown by SEQ ID No: 667, SEQ ID No: 668, SEQ ID No: 669, SEQ ID No: 670, SEQ ID No: 671, SEQ ID No: 672, SEQ ID No: 673, SEQ ID No: 674, and SEQ ID No: 675 of the sequence listing can be specifically exemplified.

Similarly, as the above plasma membrane protein to be quantified, human SLC25A18 can be exemplified. As a subject peptide for quantification and a stable-isotope labeled peptide used for measuring human SLC25A18, one or more peptides selected from a peptide having an amino acid sequence shown by SEQ ID No: 676, SEQ ID No: 677, SEQ ID No: 678, SEQ ID No: 679, and SEQ ID No: 680 of the sequence listing can be specifically exemplified.

Similarly, as the above plasma membrane protein to be quantified, human SLC25A19 can be exemplified. As a subject peptide for quantification and a stable-isotope labeled peptide used for measuring human SLC25A19, one or more peptides selected from a peptide having an amino acid sequence shown by SEQ ID No: 681, SEQ ID No: 682, SEQ ID No: 683, SEQ ID No: 684, SEQ ID No: 685, SEQ ID No: 686, and SEQ ID No: 687 of the sequence listing can be specifically exemplified.

Similarly, as the above plasma membrane protein to be quantified, human SLC25A2 can be exemplified. As a subject peptide for quantification and a stable-isotope labeled peptide used for measuring human SLC25A2, one or more peptides selected from a peptide having an amino acid sequence shown by SEQ ID No: 688, SEQ ID No: 689, SEQ ID No: 690, SEQ ID No: 691, and SEQ ID No: 692 of the sequence listing can be specifically exemplified.

Similarly, as the above plasma membrane protein to be quantified, human SLC25A20 can be exemplified. As a subject peptide for quantification and a stable-isotope labeled peptide used for measuring human SLC25A20, one or more peptides selected from a peptide having an amino acid sequence shown by SEQ ID No: 693, SEQ ID No: 694, SEQ ID No: 695, and SEQ ID No: 696 of the sequence listing can be specifically exemplified.

Similarly, as the above plasma membrane protein to be quantified, human SLC25A21 can be exemplified. As a subject peptide for quantification and a stable-isotope labeled peptide used for measuring human SLC25A21, one or more peptides selected from a peptide having an amino acid sequence shown by SEQ ID No: 697, SEQ ID No: 698, SEQ ID No: 699, SEQ ID No: 700, SEQ ID No: 701, SEQ ID No: 702, and SEQ ID No: 703 of the sequence listing can be specifically exemplified.

Similarly, as the above plasma membrane protein to be quantified, human SLC25A22 can be exemplified. As a subject peptide for quantification and a stable-isotope labeled peptide used for measuring human SLC25A22, one or more peptides selected from a peptide having an amino acid sequence shown by SEQ ID No: 704, SEQ ID No: 705, SEQ ID No: 706, SEQ ID No: 707, SEQ ID No: 708, SEQ ID No: 709, SEQ ID No: 710, SEQ ID No: 711, SEQ ID No: 712, and SEQ ID No: 713 of the sequence listing can be specifically exemplified.

Similarly, as the above plasma membrane protein to be quantified, human SLC25A23 can be exemplified. As a subject peptide for quantification and a stable-isotope labeled peptide used for measuring human SLC25A23, one or more peptides selected from a peptide having an amino acid sequence shown by SEQ ID No: 714, SEQ ID No: 715, SEQ ID No: 716, SEQ ID No: 717, SEQ ID No: 718, SEQ ID No: 719, SEQ ID No: 720, SEQ ID No: 721, SEQ ID No: 722, SEQ ID No: 723, SEQ ID No: 724, SEQ ID No: 725, and SEQ ID No: 726 of the sequence listing can be specifically exemplified.

Similarly, as the above plasma membrane protein to be quantified, human SLC25A24 can be exemplified. As a subject peptide for quantification and a stable-isotope labeled peptide used for measuring human SLC25A24, one or more peptides selected from a peptide having an amino acid sequence shown by SEQ ID No: 727, SEQ ID No: 728, SEQ ID No: 729, SEQ ID No: 730, SEQ ID No: 731. SEQ ID No: 732, SEQ ID No: 733, SEQ ID No: 734, SEQ ID No: 735, SEQ ID No: 736, SEQ ID No: 737, SEQ ID No: 738, SEQ ID No: 739, and SEQ ID No: 740 of the sequence listing can be specifically exemplified.

Similarly, as the above plasma membrane protein to be quantified, human SLC25A25 can be exemplified. As a subject peptide for quantification, and a stable-isotope labeled peptide used for measuring human SLC25A25, one or more peptides selected from a peptide having an amino acid sequence shown by SEQ ID No: 741, SEQ ID No: 742, SEQ ID No: 743, SEQ ID No: 744, SEQ ID No: 745, SEQ ID No: 746, SEQ ID No: 747, SEQ ID No: 748, SEQ ID No: 749, SEQ ID No: 750, SEQ ID No: 751, and SEQ ID No: 752 of the sequence listing can be specifically exemplified.

Similarly, as the above plasma membrane protein to be quantified, human SLC25A26 can be exemplified. As a subject peptide for quantification and a stable-isotope labeled peptide used for measuring human SLC25A26, one or more peptides selected from a peptide having an amino acid sequence shown by SEQ ID No: 753, SEQ ID No: 754, SEQ ID No: 755, and SEQ ID No: 756 of the sequence listing can be specifically exemplified.

Similarly, as the above plasma membrane protein to be quantified, human SLC25A27 can be exemplified. As a subject peptide for quantification and a stable-isotope labeled peptide used for measuring human SLC25A27, one or more peptides selected from a peptide having an amino acid sequence shown by SEQ ID No: 757, SEQ ID No: 758, SEQ ID No: 759, SEQ ID No: 760, SEQ ID No: 761, SEQ ID No: 762, SEQ ID No: 763, and SEQ ID No: 764 of the sequence listing can be specifically exemplified.

Similarly, as the above plasma membrane protein to be quantified, human SLC25A28 can be exemplified. As a subject peptide for quantification and a stable-isotope labeled peptide used for measuring human SLC25A28, one or more peptides selected from a peptide having an amino acid sequence shown by SEQ ID No: 765, and SEQ ID No: 766 of the sequence listing can be specifically exemplified.

Similarly, as the above plasma membrane protein to be quantified, human SLC25A29 can be exemplified. As a subject peptide for quantification and a stable-isotope labeled peptide used for measuring human SLC25A29, one or more peptides selected from a peptide having an amino acid sequence shown by SEQ ID No: 767, SEQ ID No: 768, SEQ ID No: 769, SEQ ID No: 770, SEQ ID No: 771, and SEQ ID No: 772 of the sequence listing can be specifically exemplified.

Similarly, as the above plasma membrane protein to be quantified, human SLC25A3 can be exemplified. As a subject peptide for quantification and a stable-isotope labeled peptide used for measuring human SLC25A3, one or more peptides selected from a peptide having an amino acid sequence shown by SEQ ID No: 773, SEQ ID No: 774, SEQ ID No: 775, SEQ ID No: 776, and SEQ ID No: 777 of the sequence listing can be specifically exemplified.

Similarly, as the above plasma membrane protein to be quantified, human SLC25A30 can be exemplified. As a subject peptide for quantification and a stable-isotope labeled peptide used for measuring human SLC25A30, one or more peptides selected from a peptide having an amino acid sequence shown by SEQ ID No: 778, SEQ ID No: 779, SEQ ID No: 780, SEQ ID No: 781, SEQ ID No: 782, and SEQ ID No: 783 of the sequence listing can be specifically exemplified.

Similarly, as the above plasma membrane protein to be quantified, human SLC25A31 can be exemplified. As a subject peptide for quantification and a stable-isotope labeled peptide used for measuring human SLC25A31, one or more peptides selected from a peptide having an amino acid sequence shown by SEQ ID No: 784, SEQ ID No: 785, SEQ ID No: 786, SEQ ID No: 787, SEQ ID No: 798, SEQ ID No: 789, SEQ ID No: 790, SEQ ID No: 791, SEQ ID No: 792, SEQ ID No: 793, SEQ ID No: 794, SEQ ID No: 795, SEQ ID No: 796, and SEQ ID No: 797 of the sequence listing can be specifically exemplified.

Similarly, as the above plasma membrane protein to be quantified, human SLC25A32 can be exemplified. As a subject peptide for quantification and a stable-isotope labeled peptide used for measuring human SLC25A32, one or more peptides selected from a peptide having an amino acid sequence shown by SEQ ID No: 798, SEQ ID No: 799, SEQ ID No: 800, and SEQ ID No: 801 of the sequence listing can be specifically exemplified.

Similarly, as the above plasma membrane protein to be quantified, human SLC25A34 can be exemplified. As a subject peptide for quantification and a stable-isotope labeled peptide used for measuring human SLC25A34, one or more peptides selected from a peptide having an amino acid sequence shown by SEQ ID No: 802, SEQ ID No: 803, SEQ ID No: 804, SEQ ID No: 805, SEQ ID No: 806, SEQ ID No: 807 , and SEQ ID No: 808 of the sequence listing can be specifically exemplified.

Similarly, as the above plasma membrane protein to be quantified, human SLC25A35 can be exemplified. As a subject peptide for quantification and a stable-isotope labeled peptide used for measuring human SLC25A35, one or more peptides selected from a peptide having an amino acid sequence shown by SEQ ID No: 809, SEQ ID No: 810, SEQ ID No: 811, SEQ ID No: 812, SEQ ID No: 813, SEQ ID No: 814, SEQ ID No: 815, and SEQ ID No: 816 of the sequence listing can be specifically exemplified.

Similarly, as the above plasma membrane protein to be quantified, human SLC25A36 can be exemplified. As a subject peptide for quantification and a stable-isotope labeled peptide used for measuring human SLC25A36, one or more peptides selected from a peptide having an amino acid sequence shown by SEQ ID No: 817, SEQ ID No: 818, SEQ ID No: 819, SEQ ID No: 820, SEQ ID No: 821, and SEQ ID No: 822 of the sequence listing can be specifically exemplified.

Similarly, as the above plasma membrane protein to be quantified, human SLC25A37 can be exemplified. As a subject peptide for quantification and a stable-isotope labeled peptide used for measuring human SLC35A37, one or more peptides selected from a peptide having an amino acid sequence shown by SEQ ID No: 823, SEQ ID No: 824, SEQ ID No: 825, and SEQ ID No: 826 of the sequence listing can be specifically exemplified.

Similarly, as the above plasma membrane protein to be quantified, human SLC25A4 can be exemplified. As a subject peptide for quantification and a stable-isotope labeled peptide used for measuring human SLC25A4, one or more peptides selected from a peptide having an amino acid sequence shown by SEQ ID No: 827, SEQ ID No: 828, and SEQ ID No: 829 of the sequence listing can be specifically exemplified.

Similarly, as the above plasma membrane protein to be quantified, human SLC25A5 can be exemplified. As a subject peptide for quantification and a stable-isotope labeled peptide used for measuring human SLC25A5, one or more peptides selected from a peptide having an amino acid sequence shown by SEQ ID No: 830, and SEQ ID No: 831 of the sequence listing can be specifically exemplified.

Similarly, as the above plasma membrane protein to be quantified, human SLC25A6 can be exemplified. As a subject peptide for quantification and a stable-isotope labeled peptide used for measuring human SLC25A6, one or more peptides selected from a peptide having an amino acid sequence shown by SEQ ID No: 832, SEQ ID No: 833, and SEQ ID No: 834 of the sequence listing can be specifically exemplified.

Similarly, as the above plasma membrane protein to be quantified, human SLC25A7 can be exemplified. As a subject peptide for quantification and a stable-isotope labeled peptide used for measuring human SLC25A7, one or more peptides selected from a peptide having an amino acid sequence shown by SEQ ID No: 835, SEQ ID No: 836, SEQ ID No: 837, SEQ ID No: 838, and SEQ ID No: 839 of the sequence listing can be specifically exemplified.

Similarly, as the above plasma membrane protein to be quantified, human SLC25A8 can be exemplified. As a subject peptide for quantification and a stable-isotope labeled peptide used for measuring human SLC25A8, one or more peptides selected from a peptide having an amino acid sequence shown by SEQ ID No: 840, SEQ ID No: 841, SEQ ID No: 842, SEQ ID No: 843, SEQ ID No: 844, and SEQ ID No: 845 of the sequence listing can be specifically exemplified.

Similarly, as the above plasma membrane protein to be quantified, human SLC25A9 can be exemplified. As a subject peptide for quantification and a stable-isotope labeled peptide used for measuring human SLC25A9, one or more peptides selected from a peptide having an amino acid sequence shown by SEQ ID No: 846, SEQ ID No: 847, and SEQ ID No: 848 of the sequence listing can be specifically exemplified.

Similarly, as the above plasma membrane protein to be quantified, human SLC26A1 can be exemplified. As a subject peptide for quantification and a stable-isotope labeled peptide used for measuring human SLC26A1, one or more peptides selected from a peptide having an amino acid sequence shown by SEQ ID No: 849, SEQ ID No: 850, SEQ ID No: 851, SEQ ID No: 852, SEQ ID No: 853, SEQ ID No: 854, SEQ ID No: 855, SEQ ID No: 856, SEQ ID No: 857, and SEQ ID No: 858 of the sequence listing can be specifically exemplified.

Similarly, as the above plasma membrane protein to be quantified, human SLC26A10 can be exemplified. As a subject peptide for quantification and a stable-isotope labeled peptide used for measuring human SLC26A10, one or more peptides selected from a peptide having an amino acid sequence shown by SEQ ID No: 859, SEQ ID No: 860, SEQ ID No: 861, SEQ ID No: 862, SEQ ID No: 863, SEQ ID No: 864, SEQ ID No: 865, SEQ ID No: 866, SEQ ID No: 867, SEQ ID No: 868, SEQ ID No: 869, SEQ ID No: 870, and SEQ ID No: 871 of the sequence listing can be specifically exemplified.

Similarly, as the above plasma membrane protein to be quantified, human SLC26A11 can be exemplified. As a subject peptide for quantification and a stable-isotope labeled peptide used for measuring human SLC26A11, one or more peptides selected from a peptide having an amino acid sequence shown by SEQ ID No: 872, SEQ ID No: 873, SEQ ID No: 874, SEQ ID No: 875, SEQ ID No: 876, SEQ ID No: 877, SEQ ID No: 878, SEQ ID No: 879, SEQ ID No: 880, and SEQ ID No: 881 of the sequence listing can be specifically exemplified.

Similarly, as the above plasma membrane protein to be quantified, human SLC26A2 can be exemplified. As a subject peptide for quantification and a stable-isotope labeled peptide used for measuring human SLC26A2, one or more peptides selected from a peptide having an amino acid sequence shown by SEQ ID No: 882, SEQ ID No: 883, SEQ ID No: 884, SEQ ID No: 885, SEQ ID No: 886, SEQ ID No: 887, SEQ ID No: 888, SEQ ID No: 889, SEQ ID No: 890, SEQ ID No: 891, and SEQ ID No: 892 of the sequence listing can be specifically exemplified.

Similarly, as the above plasma membrane protein to be quantified, human SLC26A3 can be exemplified. As a subject peptide for quantification and a stable-isotope labeled peptide used for measuring human SLC26A3, one or more peptides selected from a peptide having an amino acid sequence shown by SEQ ID No: 893, SEQ ID No: 894, SEQ ID No: 895, SEQ ID No: 896, SEQ ID No: 897, SEQ ID No: 898, SEQ ID No: 899, SEQ ID No: 900, SEQ ID No: 901, SEQ ID No: 902, SEQ ID No: 903, SEQ ID No: 904, SEQ ID No: 905, and SEQ ID No: 906 of the sequence listing can be specifically exemplified.

Similarly, as the above plasma membrane protein to be quantified, human SLC26A4 can be exemplified. As a subject peptide for quantification and a stable-isotope labeled peptide used for measuring human SLC26A4, one or more peptides selected from a peptide having an amino acid sequence shown by SEQ ID No: 907, SEQ ID No: 908, SEQ ID No: 909, SEQ ID No: 910, SEQ ID No: 911, SEQ ID No: 912, SEQ ID No : 913, and SEQ ID No: 914 of the sequence listing can be specifically exemplified.

Similarly, as the above plasma membrane protein to be quantified, human SLC26A5 can be exemplified. As a subject peptide for quantification and a stable-isotope labeled peptide used for measuring human SLC26A5, one or more peptides selected from a peptide having an amino acid sequence shown by SEQ ID No: 915, SEQ ID No: 916, and SEQ ID No: 917 of the sequence listing can be specifically exemplified.

Similarly, as the above plasma membrane protein to be quantified, human SLC26A6 can be exemplified. As a subject peptide for quantification and a stable-isotope labeled peptide used for measuring human SLC26A6, one or more peptides selected from a peptide having an amino acid sequence shown by SEQ ID No: 918, SEQ ID No: 919, SEQ ID No: 920, SEQ ID No: 921, SEQ ID No: 922, SEQ ID No: 923, SEQ ID No: 924, SEQ ID No: 925, SEQ ID No: 926, and SEQ ID No: 927 of the sequence listing can be specifically exemplified.

Similarly, as the above plasma membrane protein to be quantified, human SLC26A7 can be exemplified. As a subject peptide for quantification and a stable-isotope labeled peptide used for measuring human SLC26A7, one or more peptides selected from a peptide having an amino acid sequence shown by SEQ ID No: 928, and SEQ ID No: 929 of the sequence listing can be specifically exemplified.

Similarly, as the above plasma membrane protein to be quantified, human SLC26A8 can be exemplified. As a subject peptide for quantification and a stable-isotope labeled peptide used for measuring human SLC26A8, one or more peptides selected from a peptide having an amino acid sequence shown by SEQ ID No: 930, SEQ ID No: 931, and SEQ ID No: 932 of the sequence listing can be specifically exemplified.

Similarly, as the above plasma membrane protein to be quantified, human SLC26A9 can be exemplified. As a subject peptide for quantification and a stable-isotope labeled peptide used for measuring human SLC26A9, one or more peptides selected from a peptide having an amino acid sequence shown by SEQ ID No: 933, SEQ ID No: 934, SEQ ID No: 935, SEQ ID No: 936, SEQ ID No: 937, SEQ ID No: 938, SEQ ID No: 939, SEQ ID No: 940, SEQ ID No: 941, SEQ ID No: 942, SEQ ID No: 943, SEQ ID No: 944, and SEQ ID No: 945 of the sequence listing can be specifically exemplified.

Similarly, as the above plasma membrane protein to be quantified, human SLC27A1 can be exemplified. As a subject peptide for quantification and a stable-isotope labeled peptide used for measuring human SLC27A1, one or more peptides selected from a peptide having an amino acid sequence shown by SEQ ID No: 946, SEQ ID No: 947, and SEQ ID No: 948 of the sequence listing can be specifically exemplified.

Similarly, as the above plasma membrane protein to be quantified, human SLC27A2 can be exemplified. As a subject peptide for quantification and a stable-isotope labeled peptide used for measuring human SLC27A2, one or more peptides selected from a peptide having an amino acid sequence shown by SEQ ID No: 949, SEQ ID No: 950, SEQ ID No: 951, SEQ ID No: 952, and SEQ ID No: 953 of the sequence listing can be specifically exemplified.

Similarly, as the above plasma membrane protein to be quantified, human SLC27A3 can be exemplified. As a subject peptide for quantification and a stable-isotope labeled peptide used for measuring human SLC27A3, one or more peptides selected from a peptide having an amino acid sequence shown by SEQ ID No: 954, SEQ ID No: 955, SEQ ID No: 956, and SEQ ID No: 957 of the sequence listing can be specifically exemplified.

Similarly, as the above plasma membrane protein to be quantified, human SLC27A4 can be exemplified. As a subject peptide for quantification and a stable-isotope labeled peptide used for measuring human SLC27A4, one or more peptides selected from a peptide having an amino acid sequence shown by SEQ ID No: 958, SEQ ID No: 959, SEQ ID No: 960, SEQ ID No: 961, and SEQ ID No: 962 of the sequence listing can be specifically exemplified.

Similarly, as the above plasma membrane protein to be quantified, human SLC27A5 can be exemplified. As a subject peptide for quantification and a stable-isotope labeled peptide used for measuring human SLC27A5, one or more peptides selected from a peptide having an amino acid sequence shown by SEQ ID No: 963, SEQ ID No: 964, and SEQ ID No: 965 of the sequence listing can be specifically exemplified.

Similarly, as the above plasma membrane protein to be quantified, human SLC27A6 can be exemplified. As a subject peptide for quantification and a stable-isotope labeled peptide used for measuring human SLC27A6, one or more peptides selected from a peptide having an amino acid sequence shown by SEQ ID No: 966, SEQ ID No: 967, SEQ ID No: 968, and SEQ ID No: 969 of the sequence listing can be specifically exemplified.

Similarly, as the above plasma membrane protein to be quantified, human SLC28A2 can be exemplified. As a subject peptide for quantification and a stable-isotope labeled peptide used for measuring human SLC28A2, one or more peptides selected from a peptide having an amino acid sequence shown by SEQ ID No: 970, SEQ ID No: 971, and SEQ ID No: 972 of the sequence listing can be specifically exemplified.

Similarly, as the above plasma membrane protein to be quantified, human SLC29A3 can be exemplified. As a subject peptide for quantification and a stable-isotope labeled peptide used for measuring human SLC29A3, one or more peptides selected from a peptide having an amino acid sequence shown by SEQ ID No: 973, SEQ ID No: 974, SEQ ID No: 975, and SEQ ID No: 976 of the sequence listing can be specifically exemplified.

Similarly, as the above plasma membrane protein to be quantified, human SLC2A11 can be exemplified. As a subject peptide for quantification and a stable-isotope labeled peptide used for measuring human SLC2A11, one or more peptides selected from a peptide having an amino acid sequence shown by SEQ ID No: 977, SEQ ID No: 978, SEQ ID No: 979, and SEQ ID No: 980 of the sequence listing can be specifically exemplified.

Similarly, as the above plasma membrane protein to be quantified, human SLC2A12 can be exemplified. As a subject peptide for quantification and a stable-isotope labeled peptide used for measuring human SLC2A12, one or more peptides selected from a peptide having an amino acid sequence shown by SEQ ID No: 981, and SEQ ID No: 982 of the sequence listing can be specifically exemplified.

Similarly, as the above plasma membrane protein to be quantified, human SLC2A13 can be exemplified. As a subject peptide for quantification and a stable-isotope labeled peptide used for measuring human SLC2A13, one or more peptides selected from a peptide having an amino acid sequence shown by SEQ ID No: 983, SEQ ID No: 984, SEQ ID No: 985, SEQ ID No: 986, SEQ ID No: 987, and SEQ ID No: 988 of the sequence listing can be specifically exemplified.

Similarly, as the above plasma membrane protein to be quantified, human SLC2A14 can be exemplified. As a subject peptide for quantification and a stable-isotope labeled peptide used for measuring human SLC2A14, one or more peptides selected from a peptide having an amino acid sequence shown by SEQ ID No: 989, SEQ ID No: 990 and SEQ ID No: 991 of the sequence listing can be specifically exemplified.

Similarly, as the above plasma membrane protein to be quantified, human SLC2A3 can be exemplified. As a subject peptide for quantification and a stable-isotope labeled peptide used for measuring human SLC2A3, one or more peptides selected from a peptide having an amino acid sequence shown by SEQ ID No: 992, SEQ ID No: 993, SEQ ID No: 994 and SEQ ID No: 995 of the sequence listing can be specifically exemplified.

Similarly, as the above plasma membrane protein to be quantified, human SLC2A4 can be exemplified. As a subject peptide for quantification and a stable-isotope labeled peptide used for measuring human SLC2A4, one or more peptides selected from a peptide having an amino acid sequence shown by SEQ ID No: 996, and SEQ ID No: 997 of the sequence listing can be specifically exemplified.

Similarly, as the above plasma membrane protein to be quantified, human SLC2A5 can be exemplified. As a subject peptide for quantification and a stable-isotope labeled peptide used for measuring human SLC2A5, one or more peptides selected from a peptide having an amino acid sequence shown by SEQ ID No: 998, and SEQ ID No: 999 of the sequence listing can be specifically exemplified.

Similarly, as the above plasma membrane protein to be quantified, human SLC2A6 can be exemplified. As a subject peptide for quantification and a stable-isotope labeled peptide used for measuring human SLC2A6, one or more peptides selected from a peptide having an amino acid sequence shown by SEQ ID No: 1000, and SEQ ID No: 1001 of the sequence listing can be specifically exemplified.

Similarly, as the above plasma membrane protein to be quantified, human SLC2A7 can be exemplified. As a subject peptide for quantification and a stable-isotope labeled peptide used for measuring human SLC2A7, one or more peptides selected from a peptide having an amino acid sequence shown by SEQ ID NO: 1002 of the sequence listing can be specifically exemplified.

Similarly, as the above plasma membrane protein to be quantified, human SLC30A1 can be exemplified. As a subject peptide for quantification and a stable-isotope labeled peptide used for measuring human SLC30A1, one or more peptides selected from a peptide having an amino acid sequence shown by SEQ ID No: 1003, SEQ ID No: 1004, and SEQ ID No: 1005 of the sequence listing can be specifically exemplified.

Similarly, as the above plasma membrane protein to be quantified, human SLC30A10 can be exemplified. As a subject peptide for quantification and a stable-isotope labeled peptide used for measuring human SLC30A10, one or more peptides selected from a peptide having an amino acid sequence shown by SEQ ID No: 1006, and SEQ ID No: 1007 of the sequence listing can be specifically exemplified.

Similarly, as the above plasma membrane protein to be quantified, human SLC30A11 can be exemplified. As a subject peptide for quantification and a stable-isotope labeled peptide used for measuring human SLC30A11, one or more peptides selected from a peptide having an amino acid sequence shown by SEQ ID No: 1008, SEQ ID No: 1009, and SEQ ID No: 1010 of the sequence listing can be specifically exemplified.

Similarly, as the above plasma membrane protein to be quantified, human SLC30A2 can be exemplified. As a subject peptide for quantification and a stable-isotope labeled peptide used for measuring human SLC30A2, one or more peptides selected from a peptide having an amino acid sequence shown by SEQ ID No: 1011, and SEQ ID No: 1012 of the sequence listing can be specifically exemplified.

Similarly, as the above plasma membrane protein to be quantified, human SLC30A3 can be exemplified. As a subject peptide for quantification and a stable-isotope labeled peptide used for measuring human SLC30A3, one or more peptides selected from a peptide having an amino acid sequence shown by SEQ ID No: 1013, SEQ ID No: 1014, SEQ ID No: 1015, and SEQ ID No: 1016 of the sequence listing can be specifically exemplified.

Similarly, as the above plasma membrane protein to be quantified, human SLC30A4 can be exemplified. As a subject peptide for quantification and a stable-isotope labeled peptide used for measuring human SLC30A4, one or more peptides selected from a peptide having an amino acid sequence shown by SEQ ID No: 1017, SEQ ID No: 1018, SEQ ID No: 1019, SEQ ID No: 1020, and SEQ ID No: 1021 of the sequence listing can be specifically exemplified.

Similarly, as the above plasma membrane protein to be quantified, human SLC30A5 can be exemplified. As a subject peptide for quantification and a stable-isotope labeled peptide used for measuring human SLC30A5, one or more peptides selected from a peptide having an amino acid sequence shown by SEQ ID No: 1022, and SEQ ID No: 1023 of the sequence listing can be specifically exemplified.

Similarly, as the above plasma membrane protein to be quantified, human SLC30A6 can be exemplified. As a subject peptide for quantification and a stable-isotope labeled peptide used for measuring human SLC30A6, one or more peptides selected from a peptide having an amino acid sequence shown by SEQ ID No: 1024, SEQ ID No: 1025, and SEQ ID No: 1026 of the sequence listing can be specifically exemplified.

Similarly, as the above plasma membrane protein to be quantified, human SLC30A7 can be exemplified. As a subject peptide for quantification and a stable-isotope labeled peptide used for measuring human SLC30A7, one or more peptides selected from a peptide having an amino acid sequence shown by SEQ ID No: 1027, SEQ ID No: 1028, and SEQ ID No: 1029 of the sequence listing can be specifically exemplified.

Similarly, as the above plasma membrane protein to be quantified, human SLC30A8 can be exemplified. As a subject peptide for quantification and a stable-isotope labeled peptide used for measuring human SLC30A8, one or more peptides selected from a peptide having an amino acid sequence shown by SEQ ID No: 1030, SEQ ID No: 1031, SEQ ID No: 1032, SEQ ID No: 1033, and SEQ ID No: 1034 of the sequence listing can be specifically exemplified.

Similarly, as the above plasma membrane protein to be quantified, human SLC30A9 can be exemplified. As a subject peptide for quantification and a stable-isotope labeled peptide used for measuring human SLC30A9, one or more peptides selected from a peptide having an amino acid sequence shown by SEQ ID No: 1035, SEQ ID No: 1036, SEQ ID No: 1037, SEQ ID No: 1038, SEQ ID No: 1039, and SEQ ID No: 1040 of the Sequence listing can be specifically exemplified.

Similarly, as the above plasma membrane protein to be quantified, human SLC31A1 can be exemplified. As a subject peptide for quantification and a stable-isotope labeled peptide used for measuring human SLC31A1, one or more peptides selected from a peptide having an amino acid sequence shown by SEQ ID No: 1041, and SEQ ID No: 1042 of the sequence listing can be specifically exemplified.

Similarly, as the above plasma membrane protein to be quantified, human SLC31A2 can be exemplified. As a subject peptide for quantification and a stable-isotope labeled peptide used for measuring human SLC31A2, one or more peptides selected from a peptide having an amino acid sequence shown by SEQ ID No: 1043 of the sequence listing can be specifically exemplified.

Similarly, as the above plasma membrane protein to be quantified, human SLC32A1 can be exemplified. As a subject peptide for quantification, and a stable-isotope labeled peptide used for measuring human SLC32A1, one or more peptides selected from a peptide having an amino acid sequence shown by SEQ ID No: 1044, SEQ ID No: 1045, and SEQ ID No: 1046 of the sequence listing can be specifically exemplified.

Similarly, as the above plasma membrane protein to be quantified, human SLC33A1 can be exemplified. As a subject peptide for quantification and a stable-isotope labeled peptide used for measuring human SLC33A1, one or more peptides selected from a peptide having an amino acid sequence shown by SEQ ID No: 1047, and SEQ ID No: 1048 of the sequence listing can be specifically exemplified.

Similarly, as the above plasma membrane protein to be quantified, human SLC34A1 can be exemplified. As a subject peptide for quantification and a stable-isotope labeled peptide used for measuring human SLC34A1, one or more peptides selected from a peptide having an amino acid sequence shown by SEQ ID No: 1049, SEQ ID No: 1050, SEQ ID No: 1051, SEQ ID No: 1052, and SEQ ID No: 1053 of the sequence listing can be specifically exemplified.

Similarly, as the above plasma membrane protein to be quantified, human SLC34A2 can be exemplified. As a subject peptide for quantification and a stable-isotope labeled peptide used for measuring human SLC34A2, one or more peptides selected from a peptide having an amino acid sequence shown by SEQ ID No: 1054, SEQ ID No: 1055, and SEQ ID No: 1056 of the sequence listing can be specifically exemplified.

Similarly, as the above plasma membrane protein to be quantified, human SLC34A3 can be exemplified. As a subject peptide for quantification and a stable-isotope labeled peptide used for measuring human SLC34A3, one or more peptides selected from a peptide having an amino acid sequence shown by SEQ ID No: 1057, SEQ ID No: 1058, SEQ ID No: 1059, SEQ ID No: 1060, and SEQ ID No: 1061 of the sequence listing can be specifically exemplified.

Similarly, as the above plasma membrane protein to be quantified, human SLC35A1 can be exemplified. As a subject peptide for quantification and a stable-isotope labeled peptide used for measuring human SLC35A1. one or more peptides selected from a peptide having an amino acid sequence shown by SEQ ID No: 1062 of the sequence listing can be specifically exemplified.

Similarly, as the above plasma membrane protein to be quantified, human SLC35A2 can be exemplified. As a subject peptide for quantification and a stable-isotope labeled peptide used for measuring human SLC35A2, one or more peptides selected from a peptide having an amino acid sequence shown by SEQ ID No: 1063, SEQ ID No: 1064, and SEQ ID No: 1065 of the sequence listing can be specifically exemplified.

Similarly, as the above plasma membrane protein to be quantified, human SLC35A3 can be exemplified. As a subject peptide for quantification and a stable-isotope labeled peptide used for measuring human SLC35A3, one or more peptides selected from a peptide having an amino acid sequence shown by SEQ ID No: 1066, and SEQ ID No: 1067 of the sequence listing can be specifically exemplified.

Similarly, as the above plasma membrane protein to be quantified, human SLC35A4 can be exemplified. As a subject peptide for quantification and a stable-isotope labeled peptide used for measuring human SLC35A4, one or more peptides selected from a peptide having an amino acid sequence shown by SEQ ID No: 1068, and SEQ ID No: 1069 of the sequence listing can be specifically exemplified.

Similarly, as the above plasma membrane protein to be quantified, human SLC35A5 can be exemplified. As a subject peptide for quantification and a stable-isotope labeled peptide used for measuring human SLC35A5, one or more peptides selected from a peptide having an amino acid sequence shown by SEQ ID No: 1070, SEQ ID No: 1071, and SEQ ID No: 1072 of the sequence listing can be specifically exemplified.

Similarly, as the above plasma membrane protein to be quantified, human SLC35B1 can be exemplified. As a subject peptide for quantification and a stable-isotope labeled peptide used for measuring human SLC35B1, one or more peptides selected from a peptide having an amino acid sequence shown by SEQ ID No: 1073 of the sequence listing can be specifically exemplified.

Similarly, as the above plasma membrane protein to be quantified, human SLC35B2 can be exemplified. As a subject peptide for quantification and a stable-isotope labeled peptide used for measuring human SLC35B2, one or more peptides selected from a peptide having an amino acid sequence shown by SEQ ID No: 1074, SEQ ID No: 1075, SEQ ID No: 1076, and SEQ ID No: 1077 of the sequence listing can be specifically exemplified.

Similarly, as the above plasma membrane protein to be quantified, human SLC35B3 can be exemplified. As a subject peptide for quantification and a stable-isotope labeled peptide used for measuring human SLC35B3, one or more peptides selected from a peptide having an amino acid sequence shown by SEQ ID No: 1078, SEQ ID No: 1079, and SEQ ID No: 1080 of the sequence listing can be specifically exemplified.

Similarly, as the above plasma membrane protein to be quantified, human SLC35B4 can be exemplified. As a subject peptide for quantification and a stable-isotope labeled peptide used for measuring human SLC35B4, one or more peptides selected from a peptide having an amino acid sequence shown by SEQ ID No: 1081, and SEQ ID No: 1082 of the sequence listing can be specifically exemplified.

Similarly, as the above plasma membrane protein to be quantified, human SLC35C1 can be exemplified. As a subject peptide for quantification and a stable-isotope labeled peptide used for measuring human SLC35C1, one or more peptides selected from a peptide having an amino acid sequence shown by SEQ ID No: 1083, and SEQ ID No: 1084 of the sequence listing can be specifically exemplified.

Similarly, as the above plasma membrane protein to be quantified, human SLC35C2 can be exemplified. As a subject peptide for quantification and a stable-isotope labeled peptide used for measuring human SLC35C2, one or more peptides selected from a peptide having an amino acid sequence shown by SEQ ID No: 1085, SEQ ID No: 1086 and SEQ ID No: 1087 of the sequence listing can be specifically exemplified.

Similarly, as the above plasma membrane protein to be quantified, human SLC35D1 can be exemplified. As a subject peptide for quantification and a stable-isotope labeled peptide used for measuring human SLC35D1, one or more peptides selected from a peptide having an amino acid sequence shown by SEQ ID No: 1088, and SEQ ID No: 1089 of the sequence listing can be specifically exemplified.

Similarly, as the above plasma membrane protein to be quantified, human SLC35D2 can be exemplified. As a subject peptide for quantification and a stable-isotope labeled peptide used for measuring human SLC35D2, one or more peptides selected from a peptide having an amino acid sequence shown by SEQ ID No: 1090, SEQ ID No: 1091, and SEQ ID No: 1092 of the sequence listing can be specifically exemplified.

Similarly, as the above plasma membrane protein to be quantified, human SLC35D3 can be exemplified. As a subject peptide for quantification and a stable-isotope labeled peptide used for measuring human SLC35D3, one or more peptides selected from a peptide having an amino acid sequence shown by SEQ ID No: 1093, SEQ ID No: 1094, and SEQ ID No: 1095 of the sequence listing can be specifically exemplified.

Similarly, as the above plasma membrane protein to be quantified, human SLC35E1 can be exemplified. As a subject peptide for quantification and a stable-isotope labeled peptide used for measuring human SLC35E1, one or more peptides selected from a peptide having an amino acid sequence shown by SEQ ID No: 1096, SEQ ID No: 1097, and SEQ ID No: 1098 of the sequence listing can be specifically exemplified.

Similarly, as the above plasma membrane protein to be quantified, human SLC35E2 can be exemplified. As a subject peptide for quantification and a stable-isotope labeled peptide used for measuring human SLC35E2, one or more peptides selected from a peptide having an amino acid sequence shown by SEQ ID No: 1099, SEQ ID No: 1100, SEQ ID No: 1101, SEQ ID No: 1102, SEQ ID No: 1103, SEQ ID No: 1104, SEQ ID No: 1105, SEQ ID No: 1106, and SEQ ID No: 1107 of the sequence listing can be specifically exemplified.

Similarly, as the above plasma membrane protein to be quantified, human SLC35E3 can be exemplified. As a subject peptide for quantification and a stable-isotope labeled peptide used for measuring human SLC35E3, one or more peptides selected from a peptide having an amino acid sequence shown by SEQ ID No: 1108, SEQ ID No: 1109, and SEQ ID No: 1110 of the sequence listing can be specifically exemplified.

Similarly, as the above plasma membrane protein to be quantified, human SLC35E4 can be exemplified. As a subject peptide for quantification and a stable-isotope labeled peptide used for measuring human SLC35E4, one or more peptides selected from a peptide having an amino acid sequence shown by SEQ ID No: 1111, and SEQ ID No: 1112 of the sequence listing can be specifically exemplified.

Similarly, as the above plasma membrane protein to be quantified, human SLC35F1 can be exemplified. As a subject peptide for quantification and a stable-isotope labeled peptide used for measuring human SLC35F1, one or more peptides selected from a peptide having an amino acid sequence shown by SEQ ID No: 1113 of the sequence listing can be specifically exemplified.

Similarly, as the above plasma membrane protein to be quantified, human SLC35F2 can be exemplified. As a subject peptide for quantification and a stable-isotope labeled peptide used for measuring human SLC35F2, one or more peptides selected from a peptide having an amino acid sequence shown by SEQ ID No: 1114, and SEQ ID No: 1115 of the sequence listing can be specifically exemplified.

Similarly, as the above plasma membrane protein to be quantified, human SLC35F3 can be exemplified. As a subject peptide for quantification and a stable-isotope labeled peptide used for measuring human SLC35F3, one or more peptides selected from a peptide having an amino acid sequence shown by SEQ ID No: 1116, and SEQ ID No: 1117 of the sequence listing can be specifically exemplified.

Similarly, as the above plasma membrane protein to be quantified, human SLC35F5 can be exemplified. As a subject peptide for quantification and a stable-isotope labeled peptide used for measuring human SLC35F5, one or more peptides selected from a peptide having an amino acid sequence shown by SEQ ID No: 1118, and SEQ ID No: 1119 of the sequence listing can be specifically exemplified.

Similarly, as the above plasma membrane protein to be quantified, human SLC36A1 can be exemplified. As a subject peptide for quantification and a stable-isotope labeled peptide used for measuring human SLC36A1, one or more peptides selected from a peptide having an amino acid sequence shown by SEQ ID No: 1120 of the sequence listing can be specifically exemplified.

Similarly, as the above plasma membrane protein to be quantified, human SLC36A2 can be exemplified. As a subject peptide for quantification and a stable-isotope labeled peptide used for measuring human SLC36A2, one or more peptides selected from a peptide having an amino acid sequence shown by SEQ ID No: 1121, SEQ ID No: 1122, and SEQ ID No: 1123 of the sequence listing can be specifically exemplified.

Similarly, as the above plasma membrane protein to be quantified, human SLC36A3 can be exemplified. As a subject peptide for quantification and a stable-isotope labeled peptide used for measuring human SLC36A3, one or more peptides selected from a peptide having an amino acid sequence shown by SEQ ID No: 1124, and SEQ ID No: 1125 of the sequence listing can be specifically exemplified.

Similarly, as the above plasma membrane protein to be quantified, human SLC36A4 can be exemplified. As a subject peptide for quantification and a stable-isotope labeled peptide used for measuring human SLC36A4, one or more peptides selected from a peptide having an amino acid sequence shown by SEQ ID No: 1126, SEQ ID No: 1127, SEQ ID No: 1128, and SEQ ID No: 1129 of the sequence listing can be specifically exemplified.

Similarly, as the above plasma membrane protein to be quantified, human SLC37A1 can be exemplified. As a subject peptide for quantification and a stable-isotope labeled peptide used for measuring human SLC37A1, one or more peptides selected from a peptide having an amino acid sequence shown by SEQ ID No: 1130, SEQ ID No: 1131, and SEQ ID No: 1132 of the sequence listing can be specifically exemplified.

Similarly, as the above plasma membrane protein to be quantified, human SLC37A2 can be exemplified. As a subject peptide for quantification and a stable-isotope labeled peptide used for measuring human SLC37A2, one or more peptides selected from a peptide having an amino acid sequence shown by SEQ ID No: 1133, and SEQ ID No: 1134 of the sequence listing can be specifically exemplified.

Similarly, as the above plasma membrane protein to be quantified, human SLC37A3 can be exemplified. As a subject peptide for quantification and a stable-isotope labeled peptide used for measuring human SLC37A3, one or more peptides selected from a peptide having an amino acid sequence shown by SEQ ID No: 1135, SEQ ID No: 1136, and SEQ ID No: 1137 of the sequence listing can be specifically exemplified.

Similarly, as the above plasma membrane protein to be quantified, human SLC37A4 can be exemplified. As a subject peptide for quantification and a stable-isotope labeled peptide used for measuring human SLC37A4, one or more peptides selected from a peptide having an amino acid sequence shown by SEQ ID No: 1138, and SEQ ID No: 1139 of the sequence listing can be specifically exemplified.

Similarly, as the above plasma membrane protein to be quantified, human SLC38A1 can be exemplified. As a subject peptide for quantification and a stable-isotope labeled peptide used for measuring human SLC38A1, one or more peptides selected from a peptide having an amino acid sequence shown by SEQ ID No: 1140, and SEQ ID No: 1141 of the sequence listing can be specifically exemplified.

Similarly, as the above plasma membrane protein to be quantified, human SLC38A2 can be exemplified. As a subject peptide for quantification and a stable-isotope labeled peptide used for measuring human SLC38A2, one or more peptides selected from a peptide having an amino acid sequence shown by SEQ ID No: 1142, SEQ ID No: 1143, and SEQ ID No: 1144 of the sequence listing can be specifically exemplified.

Similarly, as the above plasma membrane protein to be quantified, human SLC38A3 can be exemplified. As a subject peptide for quantification and a stable-isotope labeled peptide used for measuring human SLC38A3, one or more peptides selected from a peptide having an amino acid sequence shown by SEQ ID No: 1145, SEQ ID No: 1146, and SEQ ID No: 1147 of the sequence listing can be specifically exemplified.

Similarly, as the above plasma membrane protein to be quantified, human SLC38A4 can be exemplified. As a subject peptide for quantification and a stable-isotope labeled peptide used for measuring human SLC38A4, one or more peptides selected from a peptide having an amino acid sequence shown by SEQ ID No: 1148, and SEQ ID No: 1149 of the sequence listing can be specifically exemplified.

Similarly, as the above plasma membrane protein to be quantified, human SLC38A5 can be exemplified. As a subject peptide for quantification and a stable-isotope labeled peptide used for measuring human SLC38A5, one or more peptides selected from a peptide having an amino acid sequence shown by SEQ ID No: 1150, SEQ ID No: 1151, SEQ ID No: 1152, SEQ ID No: 1153, and SEQ ID No: 1154 of the sequence listing can be specifically exemplified.

Similarly, as the above plasma membrane protein to be quantified, human SLC38A6 can be exemplified. As a subject peptide for quantification and a stable-isotope labeled peptide used for measuring human SLC38A6, one or more peptides selected from a peptide having an amino acid sequence shown by SEQ ID No: 1155, SEQ ID No: 1156, and SEQ ID No: 1157 of the sequence listing can be specifically exemplified.

Similarly, as the above plasma membrane protein to be quantified, human SLC39A1 can be exemplified. As a subject peptide for quantification and a stable-isotope labeled peptide used for measuring human SLC39A1, one or more peptides selected from a peptide having an amino acid sequence shown by SEQ ID No: 1158, and SEQ ID No: 1159 of the sequence listing can be specifically exemplified.

Similarly, as the above plasma membrane protein to be quantified, human SLC39A10 can be exemplified. As a subject peptide for quantification and a stable-isotope labeled peptide used for measuring human SLC39A10, one or more peptides selected from a peptide having an amino acid sequence shown by SEQ ID No: 1160, SEQ ID No: 1161, and SEQ ID No: 1162 of the sequence listing can be specifically exemplified.

Similarly, as the above plasma membrane protein to be quantified, human SLC39A11 can be exemplified. As a subject peptide for quantification and a stable-isotope labeled peptide used for measuring human SLC39A11, one or more peptides selected from a peptide having an amino acid sequence shown by SEQ ID No: 1163, and SEQ ID No: 1164 of the sequence listing can be specifically exemplified.

Similarly, as the above plasma membrane protein to be quantified, human SLC39A12 can be exemplified. As a subject peptide for quantification and a stable-isotope labeled peptide used for measuring human SLC39A12, one or more peptides selected from a peptide having an amino acid sequence shown by SEQ ID No: 1165, and SEQ ID No: 1166 of the sequence listing can be specifically exemplified.

Similarly, as the above plasma membrane protein to be quantified, human SLC39A13 can be exemplified. As a subject peptide for quantification and a stable-isotope labeled peptide used for measuring human SLC39A13, one or more peptides selected from a peptide having an amino acid sequence shown by SEQ ID No: 1167, SEQ ID No: 1168, and SEQ ID No: 1169 of the sequence listing can be specifically exemplified.

Similarly, as the above plasma membrane protein to be quantified, human SLC39A14 can be exemplified. As a subject peptide for quantification and a stable-isotope labeled peptide used for measuring human SLC39A14, one or more peptides selected from a peptide having an amino acid sequence shown by SEQ ID No: 1170, and SEQ ID No: 1171 of the sequence listing can be specifically exemplified.

Similarly, as the above plasma membrane protein to be quantified, human SLC39A2 can be exemplified. As a subject peptide for quantification and a stable-isotope labeled peptide used for measuring human SLC39A2, one or more peptides selected from a peptide having an amino acid Sequence shown by SEQ ID No: 1172, SEQ ID No: 1173, SEQ ID No: 1174, and SEQ ID No: 1175 of the sequence listing can be specifically exemplified.

Similarly, as the above plasma membrane protein to be quantified, human SLC39A3 can be exemplified. As a subject peptide for quantification and a stable-isotope labeled peptide used for measuring human SLC39A3, one or more peptides selected from a peptide having an amino acid sequence shown by SEQ ID No: 1176, SEQ ID No: 1177, SEQ ID No: 1178, SEQ ID No: 1179 of the sequence listing can be specifically exemplified.

Similarly, as the above plasma membrane protein to be quantified, human SLC39A4 can be exemplified. As a subject peptide for quantification and a stable-isotope labeled peptide used for measuring human SLC39A4, one or more peptides selected from a peptide having an amino acid sequence shown by SEQ ID No: 1180, SEQ ID No: 1181, SEQ ID No: 1182, and SEQ ID No: 1183 of the sequence listing can be specifically exemplified.

Similarly, as the above plasma membrane protein to be quantified, human SLC39A5 can be exemplified. As a subject peptide for quantification and a stable-isotope labeled peptide used for measuring human SLC39A5, one or more peptides selected from a peptide having an amino acid sequence shown by SEQ ID No: 1184, SEQ ID No: 1185, and SEQ ID No: 1186 of the sequence listing can be specifically exemplified.

Similarly, as the above plasma membrane protein to be quantified, human SLC39A6 can be exemplified. As a subject peptide for quantification and a stable-isotope labeled peptide used for measuring human SLC39A6, one or more peptides selected from a peptide having an amino acid sequence shown by SEQ ID No: 1187, SEQ ID No: 1188, and SEQ ID No: 1189 of the sequence listing can be specifically exemplified.

Similarly, as the above plasma membrane protein to be quantified, human SLC39A7 can be exemplified. As a subject peptide for quantification and a stable-isotope labeled peptide used for measuring human SLC39A7, one or more peptides selected from a peptide having an amino acid sequence shown by SEQ ID No: 1190, SEQ ID No: 1191, and SEQ ID No: 1192 of the sequence listing can be specifically exemplified.

Similarly, as the above plasma membrane protein to be quantified, human SLC39A8 can be exemplified. As a subject peptide for quantification and a stable-isotope labeled peptide used for measuring human SLC39A8, one or more peptides selected from a peptide having an amino acid sequence shown by SEQ ID No: 1193, SEQ ID No: 1194, and SEQ ID No: 1195 of the sequence listing can be specifically exemplified.

Similarly, as the above plasma membrane protein to be quantified, human SLC39A9 can be exemplified. As a subject peptide for quantification and a stable-isotope labeled peptide used for measuring human SLC39A9, one or more peptides selected from a peptide having an amino acid sequence shown by SEQ ID No: 1196, SEQ ID No : 1197, and SEQ ID No: 1198 of the sequence listing can be specifically exemplified.

Similarly, as the above plasma membrane protein to be quantified, human SLC3A1 can be exemplified. As a subject peptide for quantification and a stable-isotope labeled peptide used for measuring human SLC3A1, one or more peptides selected from a peptide having an amino acid sequence shown by SEQ ID No: 1199, SEQ ID No: 1200, SEQ ID No: 1201, SEQ ID No: 1202, and SEQ ID No: 1203 of the sequence listing can be specifically exemplified.

Similarly, as the above plasma membrane protein to be quantified, human SLC3A2 can be exemplified. As a subject peptide for quantification and a stable-isotope labeled peptide used for measuring human SLC3A2, one or more peptides selected from a peptide having an amino acid sequence shown by SEQ ID No: 1204, SEQ ID No: 1205, SEQ ID No: 1206, SEQ ID No: 1207, and SEQ ID No: 1208 of the sequence listing can be specifically exemplified.

Similarly, as the above plasma membrane protein to be quantified, human SLC40A1 can be exemplified. As a subject peptide for quantification and a stable-isotope labeled peptide used for measuring human SLC40A1, one or more peptides selected from a peptide having an amino acid sequence shown by SEQ ID No: 1209, SEQ ID No: 1210, and SEQ ID No: 1211 of the sequence listing can be specifically exemplified.

Similarly, as the above plasma membrane protein to be quantified, human SLC41A1 can be exemplified. As a subject peptide for quantification and a stable-isotope labeled peptide used for measuring human SLC41A1, one or more peptides selected from a peptide having an amino acid sequence shown by SEQ ID No: 1212, SEQ ID No: 1213, and SEQ ID No: 1214 of the sequence listing can be specifically exemplified.

Similarly, as the above plasma membrane protein to be quantified, human SLC41A2 can be exemplified. As a subject peptide for quantification and a stable-isotope labeled peptide used for measuring human SLC41A2, one or more peptides selected from a peptide having an amino acid sequence shown by SEQ ID No: 1215, SEQ ID No: 1216, and SEQ ID No: 1217 of the sequence listing can be specifically exemplified.

Similarly, as the above plasma membrane protein to be quantified, human SLC41A3 can be exemplified. As a subject peptide for quantification and a stable-isotope labeled peptide used for measuring human SLC41A3, one or more peptides selected from a peptide having an amino acid sequence shown by SEQ ID No: 1218, and SEQ ID No: 1219 of the sequence listing can be specifically exemplified.

Similarly, as the above plasma membrane protein to be quantified, human SLC42A1 can be exemplified. As a subject peptide for quantification and a stable-isotope labeled peptide used for measuring human SLC42A1, one or more peptides selected from a peptide having an amino acid sequence shown by SEQ ID No: 1220, and SEQ ID No: 1221 of the sequence listing can be specifically exemplified.

Similarly, as the above plasma membrane protein to be quantified, human SLC42A2 can be exemplified. As a subject peptide for quantification and a stable-isotope labeled peptide used for measuring human SLC42A2, one or more peptides selected from a peptide having an amino acid sequence shown by SEQ ID No: 1222, and SEQ ID No: 1223 of the sequence listing can be specifically exemplified.

Similarly, as the above plasma membrane protein to be quantified, human SLC42A3 can be exemplified. As a subject peptide for quantification and a stable-isotope labeled peptide used for measuring human SLC42A3, one or more peptides selected from a peptide having an amino acid sequence shown by SEQ ID No: 1224, SEQ ID No: 1225, SEQ ID No: 1226, and SEQ ID No: 1227 of the sequence listing can be specifically exemplified.

Similarly, as the above plasma membrane protein to be quantified, human SLC43A1 can be exemplified. As a subject peptide for quantification and a stable-isotope labeled peptide used for measuring human SLC43A1, one or more peptides selected from a peptide having an amino acid sequence shown by SEQ ID No: 1228, SEQ ID No: 1229, and SEQ ID No: 1230 of the sequence listing can be specifically exemplified.

Similarly, as the above plasma membrane protein to be quantified, human SLC43A2 can be exemplified. As a subject peptide for quantification and a stable-isotope labeled peptide used for measuring human SLC43A2, one or more peptides selected from a peptide having an amino acid sequence shown by SEQ ID No: 1231, SEQ ID No: 1232, and SEQ ID No: 1233 of the sequence listing can be specifically exemplified.

Similarly, as the above plasma membrane protein to be quantified, human SLC43A3 can be exemplified. As a subject peptide for quantification and a stable-isotope labeled peptide used for measuring human SLC43A3, one or more peptides selected from a peptide having an amino acid sequence shown by SEQ ID No: 1234, SEQ ID No: 1235, and SEQ ID No: 1236 of the sequence listing can be specifically exemplified.

Similarly, as the above plasma membrane protein to be quantified, human SLC45A1 can be exemplified. As a subject peptide for quantification and a stable-isotope labeled peptide used for measuring human SLC45A1, one or more peptides selected from a peptide having an amino acid sequence shown by SEQ ID No: 1237, SEQ ID No: 1238, SEQ ID No: 1239, SEQ ID No: 1240 of the sequence listing can be specifically exemplified.

Similarly, as the above plasma membrane protein to be quantified, human SLC45A2 can be exemplified. As a subject peptide for quantification and a stable-isotope labeled peptide used for measuring human SLC45A2, one or more peptides selected from a peptide having an amino acid sequence shown by SEQ ID No: 1241, SEQ ID No: 1242, and SEQ ID No: 1243 of the sequence listing can be specifically exemplified.

Similarly, as the above plasma membrane protein to be quantified, human SLC45A3 can be exemplified. As a subject peptide for quantification and a stable-isotope labeled peptide used for measuring human SLC45A3, one or more peptides selected from a peptide having an amino acid sequence shown by SEQ ID No: 1244, SEQ ID No: 1245, SEQ ID No: 1246, SEQ ID No: 1247, SEQ ID No: 1248 of the sequence listing can be specifically exemplified.

Similarly, as the above plasma membrane protein to be quantified, human SLC45A4 can be exemplified. As a subject peptide for quantification and a stable-isotope labeled peptide used for measuring human SLC45A4, one or more peptides selected from a peptide having an amino acid sequence shown by SEQ ID No: 1249, SEQ ID No: 1250, SEQ ID No: 1251 of the sequence listing can be specifically exemplified.

Similarly, as the above plasma membrane protein to be quantified, human SLC4A1 can be exemplified. As a subject peptide for quantification and a stable-isotope labeled peptide used for measuring human SLC4A1, one or more peptides selected from a peptide having an amino acid sequence shown by SEQ ID No: 1252, SEQ ID No: 1253, SEQ ID No: 1254, SEQ ID No: 1255, SEQ ID No: 1256, SEQ ID No: 1257, and SEQ ID No: 1258 of the sequence listing can be specifically exemplified.

Similarly, as the above plasma membrane protein to be quantified, human SLC4A10 can be exemplified. As a subject peptide for quantification and a stable-isotope labeled peptide used for measuring human SLC4A10, one or more peptides selected from a peptide having an amino acid sequence shown by SEQ ID No: 1259, SEQ ID No: 1260, SEQ ID No: 1261, SEQ ID No: 1262, SEQ ID No: 1263, SEQ ID No: 1264, and SEQ ID No: 1265 of the sequence listing can be specifically exemplified.

Similarly, as the above plasma membrane protein to be quantified, human SLC4A11 can be exemplified. As a subject peptide for quantification and a stable-isotope labeled peptide used for measuring human SLC4A11, one or more peptides selected from a peptide having an amino acid sequence shown by SEQ ID No: 1266, SEQ ID No: 1267, SEQ ID No: 1268, SEQ ID No: 1269, SEQ ID No: 1270, SEQ ID No: 1271, SEQ ID No: 1272, and SEQ ID No: 1273 of the sequence listing can be specifically exemplified.

Similarly, as the above plasma membrane protein to be quantified, human SLC4A2 can be exemplified. As a subject peptide for quantification and a stable-isotope labeled peptide used for measuring human SLC4A2, one or more peptides selected from a peptide having an amino acid sequence shown by SEQ ID No: 1274, SEQ ID No: 1275, SEQ ID No: 1276, SEQ ID No: 1277, SEQ ID No: 1278, SEQ ID No: 1279, SEQ ID No: 1280, SEQ ID No: 1281, and SEQ ID No: 1282 of the sequence listing can be specifically exemplified.

Similarly, as the above plasma membrane protein to be quantified, human SLC4A3 can be exemplified. As a subject peptide for quantification and a stable-isotope labeled peptide used for measuring human SLC4A3, one or more peptides selected from a peptide having an amino acid sequence shown by SEQ ID No: 1283, SEQ ID No: 1284, SEQ ID No: 1285, SEQ ID No: 1286, SEQ ID No: 1287, SEQ ID No: 1288, SEQ ID No: 1289, SEQ ID No: 1290, SEQ ID No: 1291, SEQ ID No: 1292, SEQ ID No: 1293 of the sequence listing can be specifically exemplified.

Similarly, as the above plasma membrane protein to be quantified, human SLC4A4 can be exemplified. As a subject peptide for quantification and a stable-isotope labeled peptide used for measuring human SLC4A4 , one or more peptides selected from a peptide having an amino acid sequence shown by SEQ ID No: 1294, SEQ ID No: 1295, SEQ ID No: 1296, SEQ ID No: 1297, SEQ ID No: 1298, SEQ ID No: 1299, and SEQ ID No: 1300 of the sequence listing can be specifically exemplified.

Similarly, as the above plasma membrane protein to be quantified, human SLC4A8 can be exemplified. As a subject peptide for quantification and a stable-isotope labeled peptide used for measuring human SLC4A8, one or more peptides selected from a peptide having an amino acid sequence shown by SEQ ID No: 1301, SEQ ID No: 1302, SEQ ID No: 1303, SEQ ID No: 1304, SEQ ID No: 1305, SEQ ID No: 1306, SEQ ID No: 1307 of the sequence listing can be specifically exemplified.

Similarly, as the above plasma membrane protein to be quantified, human SLC4A9 can be exemplified. As a subject peptide for quantification and a stable-isotope labeled peptide used for measuring human SLC4A9, one or more peptides selected from a peptide having an amino acid sequence shown by SEQ ID No: 1308, SEQ ID No: 1309, SEQ ID No: 1310, and SEQ ID No: 1311 of the sequence listing can be specifically exemplified.

Similarly, as the above plasma membrane protein to be quantified, human SLC5A1 can be exemplified. As a subject peptide for quantification and a stable-isotope labeled peptide used for measuring human SLC5A1, one or more peptides selected from a peptide having an amino acid sequence shown by SEQ ID No: 1312 of the sequence listing can be specifically exemplified.

Similarly, as the above plasma membrane protein to be quantified, human SLC5A12 can be exemplified. As a subject peptide for quantification and a stable-isotope labeled peptide used for measuring human SLC5A12, one or more peptides selected from a peptide having an amino acid sequence shown by SEQ ID No: 1313 of the sequence listing can be specifically exemplified.

Similarly, as the above plasma membrane protein to be quantified, human SLC5A2 can be exemplified. As a subject peptide for quantification and a stable-isotope labeled peptide used for measuring human SLC5A2, one or more peptides selected from a peptide having an amino acid sequence shown by SEQ ID No : 1314, SEQ ID No: 1315, SEQ ID No: 1316, and SEQ ID No: 1317 of the sequence listing can be specifically exemplified.

Similarly, as the above plasma membrane protein to be quantified, human SLC5A3 can be exemplified. As a subject peptide for quantification and a stable-isotope labeled peptide used for measuring human SLC5A3, one or more peptides selected from a peptide having an amino acid sequence shown by SEQ ID No: 1318, and SEQ ID No: 1319 of the sequence listing can be specifically exemplified.

Similarly, as the above plasma membrane protein to be quantified, human SLC5A4 can be exemplified. As a subject peptide for quantification and a stable-isotope labeled peptide used for measuring human SLC5A4, one or more peptides selected from a peptide having an amino acid sequence shown by SEQ ID No: 1320, and SEQ ID No: 1321 of the sequence listing can be specifically exemplified.

Similarly, as the above plasma membrane protein to be quantified, human SLC5A5 can be exemplified. As a subject peptide for quantification and a stable-isotope labeled peptide used for measuring human SLC5A5, one or more peptides selected from a peptide having an amino acid sequence shown by SEQ ID No: 1322, SEQ ID No: 1323, and SEQ ID No: 1324 of the sequence listing can be specifically exemplified.

Similarly, as the above plasma membrane protein to be quantified, human SLC6A1 can be exemplified. As a subject peptide for quantification and a stable-isotope labeled peptide used for measuring human SLC6A1, one or more peptides selected from a peptide having an amino acid sequence shown by SEQ ID No: 1325, and SEQ ID No: 1326 of the sequence listing can be specifically exemplified.

Similarly, as the above plasma membrane protein to be quantified, human SLC6A11 can be exemplified. As a subject peptide for quantification and a stable-isotope labeled peptide used for measuring human SLC6A11, one or more peptides selected from a peptide having an amino acid sequence shown by SEQ ID No: 1327, SEQ ID No: 1328, and SEQ ID No: 1329 of the sequence listing can be specifically exemplified.

Similarly, as the above plasma membrane protein to be quantified, human SLC6A14 can be exemplified. As a subject peptide for quantification and a stable-isotope labeled peptide used for measuring human SLC6A14, one or more peptides selected from a peptide having an amino acid sequence shown by SEQ ID No: 1330 of the sequence listing can be specifically exemplified.

Similarly, as the above plasma membrane protein to be quantified, human SLC6A15 can be exemplified. As a subject peptide for quantification and a stable-isotope labeled peptide used for measuring human SLC6A15, one or more peptides selected from a peptide having an amino acid sequence shown by SEQ ID No: 1331, and SEQ ID No: 1332 of the sequence listing can be specifically exemplified.

Similarly, as the above plasma membrane protein to be quantified, human SLC6A16 can be exemplified. As a subject peptide for quantification and a stable-isotope labeled peptide used for measuring human SLC6A16, one or more peptides selected from a peptide having an amino acid sequence shown by SEQ ID No: 1333 of the sequence listing can be specifically exemplified.

Similarly, as the above plasma membrane protein to be quantified, human SLC6A17 can be exemplified. As a subject peptide for quantification and a stable-isotope labeled peptide used for measuring human SLC6A17, one or more peptides selected from a peptide having an amino acid sequence shown by SEQ ID No: 1334, and SEQ ID No: 1335 of the sequence listing can be specifically exemplified.

Similarly, as the above plasma membrane protein to be quantified, human SLC6A18 can be exemplified. As a subject peptide for quantification and a stable-isotope labeled peptide used for measuring human SLC6A18, one or more peptides selected from a peptide having an amino acid sequence shown by SEQ ID No: 1336 of the sequence listing can be specifically exemplified.

Similarly, as the above plasma membrane protein to be quantified, human SLC6A19 can be exemplified. As a subject peptide for quantification and a stable-isotope labeled peptide used for measuring human SLC6A19, one or more peptides selected from a peptide having an amino acid sequence shown by SEQ ID No: 1337 of the sequence listing can be specifically exemplified.

Similarly, as the above plasma membrane protein to be quantified, human SLC6A2 can be exemplified. As a subject peptide for quantification and a stable-isotope labeled peptide used for measuring human SLC6A2, one or more peptides selected from a peptide having an amino acid sequence shown by SEQ ID No: 1338 of the sequence listing can be specifically exemplified.

Similarly, as the above plasma membrane protein to be quantified, human SLC6A2 can be exemplified. As a subject peptide for quantification and a stable-isotope labeled peptide used for measuring human SLC6A2, one or more peptides selected from a peptide having an amino acid sequence shown by SEQ ID No: 1339, SEQ ID No: 1340, and SEQ ID No: 1341 of the sequence listing can be specifically exemplified.

Similarly, as the above plasma membrane protein to be quantified, human SLC6A3 can be exemplified. As a subject peptide for quantification and a stable-isotope labeled peptide used for measuring human SLC6A3, one or more peptides selected from a peptide having an amino acid sequence shown by SEQ ID No: 1342, SEQ ID No: 1343, and SEQ ID No: 1344 of the sequence listing can be specifically exemplified.

Similarly, as the above plasma membrane protein to be quantified, human SLC6A4 can be exemplified. As a subject peptide for quantification and a stable-isotope labeled peptide used for measuring human SLC6A4, one or more peptides selected from a peptide having an amino acid sequence shown by SEQ ID No: 1345, SEQ ID No: 1346, and SEQ ID No: 1347 of the sequence listing can be specifically exemplified.

Similarly, as the above plasma membrane protein to be quantified, human SLC6A5 can be exemplified. As a subject peptide for quantification and a stable-isotope labeled peptide used for measuring human SLC6A5, one or more peptides selected from a peptide having an amino acid sequence shown by SEQ ID No: 1348, and SEQ ID No: 1349 of the sequence listing can be specifically exemplified.

Similarly, as the above plasma membrane protein to be quantified, human SLC6A6 can be exemplified. As a subject peptide for quantification and a stable-isotope labeled peptide used for measuring human SLC6A6, one or more peptides selected from a peptide having an amino acid sequence shown by SEQ ID No: 1350, SEQ ID No: 1351, SEQ ID No: 1352, SEQ ID No: 1353, and SEQ ID No: 1354 of the sequence listing can be specifically exemplified.

Similarly, as the above plasma membrane protein to be quantified, human SLC6A7 can be exemplified. As a subject peptide for quantification and a stable-isotope labeled peptide used for measuring human SLC6A7, one or more peptides selected from a peptide having an amino acid sequence shown by SEQ ID No: 1355, SEQ ID No: 1356, and SEQ ID No: 1357 of the sequence listing can be specifically exemplified.

Similarly, as the above plasma membrane protein to be quantified, human SLC6A8 can be exemplified. As a subject peptide for quantification and a stable-isotope labeled peptide used for measuring human SLC6A8, one or more peptides selected from a peptide having an amino acid sequence shown by SEQ ID No: 1358, SEQ ID No: 1359, and SEQ ID No: 1360 of the sequence listing can be specifically exemplified.

Similarly, as the above plasma membrane protein to be quantified, human SLC7A1 can be exemplified. As a subject peptide for quantification and a stable-isotope labeled peptide used for measuring human SLC7A1, one or more peptides selected from a peptide having an amino acid sequence shown by SEQ ID No: 1361 of the sequence listing can be specifically exemplified.

Similarly, as the above plasma membrane protein to be quantified, human SLC7A10 can be exemplified. As a subject peptide for quantification and a stable-isotope labeled peptide used for measuring human SLC7A10, one or more peptides selected from a peptide having an amino acid sequence shown by SEQ ID No: 1362 of the sequence listing can be specifically exemplified.

Similarly, as the above plasma membrane protein to be quantified, human SLC7A11 can be exemplified. As a subject peptide for quantification and a stable-isotope labeled peptide used for measuring human SLC7A11, one or more peptides selected from a peptide having an amino acid sequence shown by SEQ ID No: 1363, SEQ ID No: 1364, SEQ ID No: 1365, SEQ ID No: 1366, SEQ ID No: 1367, and SEQ ID No: 1368 of the sequence listing can be specifically exemplified.

Similarly, as the above plasma membrane protein to be quantified, human SLC7A13 can be exemplified. As a subject peptide for quantification and a stable-isotope labeled peptide used for measuring human SLC7A13, one or more peptides selected from a peptide having an amino acid sequence shown by SEQ ID No: 1369, and SEQ ID No: 1370 of the sequence listing can be specifically exemplified.

Similarly, as the above plasma membrane protein to be quantified, human SLC7A14 can be exemplified. As a subject peptide for quantification and a stable-isotope labeled peptide used for measuring human SLC7A14, one or more peptides selected from a peptide having an amino acid sequence shown by SEQ ID No: 1371 of the sequence listing can be specifically exemplified.

Similarly, as the above plasma membrane protein to be quantified, human SLC7A3 can be exemplified. As a subject peptide for quantification and a stable-isotope labeled peptide used for measuring human SLC7A3, one or more peptides selected from a peptide having an amino acid sequence shown by SEQ ID No: 1372, and SEQ ID No: 1373 of the sequence listing can be specifically exemplified.

Similarly, as the above plasma membrane protein to be quantified, human SLC7A4 can be exemplified. As a subject peptide for quantification and a stable-isotope labeled peptide used for measuring human SLC7A4, one or more peptides selected from a peptide having an amino acid sequence shown by SEQ ID No: 1374, and SEQ ID No: 1375 of the sequence listing can be specifically exemplified.

Similarly, as the above plasma membrane protein to be quantified, human SLC7A7 can be exemplified. As a subject peptide for quantification and a stable-isotope labeled peptide used for measuring human SLC7A7, one or more peptides selected from a peptide having an amino acid sequence shown by SEQ ID No: 1376 of the sequence listing can be specifically exemplified.

Similarly, as the above plasma membrane protein to be quantified, human SLC7A8 can be exemplified. As a subject peptide for quantification and a stable-isotope labeled peptide used for measuring human SLC7A8, one or more peptides selected from a peptide having an amino acid sequence shown by SEQ ID No: 1377, SEQ ID No: 1378, and SEQ ID No: 1379 of the sequence listing can be specifically exemplified.

Similarly, as the above plasma membrane protein to be quantified, human SLC8A1 can be exemplified. As a subject peptide for quantification and a stable-isotope labeled peptide used for measuring human SLC8A1, one or more peptides selected from a peptide having an amino acid sequence shown by SEQ ID No: 1380, SEQ ID No: 1381, SEQ ID No: 1382 of the sequence listing can be specifically exemplified.

Similarly, as the above plasma membrane protein to be quantified, human SLC8A2 can be exemplified. As a subject peptide for quantification and a stable-isotope labeled peptide used for measuring human SLC8A2, one or more peptides selected from a peptide having an amino acid sequence shown by SEQ ID No: 1383, and SEQ ID No: 1384 of the sequence listing can be specifically exemplified.

Similarly, as the above plasma membrane protein to be quantified, human SLC8A3 can be exemplified. As a subject peptide for quantification and a stable-isotope labeled peptide used for measuring human SLC8A3, one or more peptides selected from a peptide having an amino acid sequence shown by SEQ ID No: 1385, and SEQ ID No: 1386 of the sequence listing can be specifically exemplified.

Similarly, as the above plasma membrane protein to be quantified, human SLC9A1 can be exemplified. As a subject peptide for quantification and a stable-isotope labeled peptide used for measuring human SLC9A1, one or more peptides selected from a peptide having an amino acid sequence shown by SEQ ID No: 1387, SEQ ID No: 1388, SEQ ID No: 1389, SEQ ID No: 1390, SEQ ID No: 1391, SEQ ID No: 1392, and SEQ ID No: 1393 of the sequence listing can be specifically exemplified.

Similarly, as the above plasma membrane protein to be quantified, human SLC9A2 can be exemplified. As a subject peptide for quantification and a stable-isotope labeled peptide used for measuring human SLC9A2, one or more peptides selected from a peptide having an amino acid sequence shown by SEQ ID No: 1394, SEQ ID No: 1395, SEQ ID No: 1396, SEQ ID No: 1397, SEQ ID No: 1398, SEQ ID No: 1399, SEQ ID No: 1400, SEQ ID No: 1401, SEQ ID No: 1402, SEQ ID No: 1403, and SEQ ID No: 1404 of the sequence listing can be specifically exemplified.

Similarly, as the above plasma membrane protein to be quantified, human SLC9A3 can be exemplified. As a subject peptide for quantification and a stable-isotope labeled peptide used for measuring human SLC9A3, one or more peptides selected from a peptide having an amino acid sequence shown by SEQ ID No: 1405, SEQ ID No: 1406, and SEQ ID No: 1407 of the sequence listing can be specifically exemplified.

Similarly, as the above plasma membrane protein to be quantified, human SLC9A4 can be exemplified. As a subject peptide for quantification and a stable-isotope labeled peptide used for measuring human SLC9A4, one or more peptides selected from a peptide having an amino acid sequence shown by SEQ ID No: 1408, SEQ ID No : 1409, SEQ ID No: 1410, and SEQ ID No: 1411 of the sequence listing can be specifically exemplified.

Similarly, as the above plasma membrane protein to be quantified, human SLC9A5 can be exemplified. As a subject peptide for quantification and a stable-isotope labeled peptide used for measuring human SLC9A5, one or more peptides selected from a peptide having an amino acid sequence shown by SEQ ID No: 1412, and SEQ ID No: 1413 of the sequence listing can be specifically exemplified.

Similarly, as the above plasma membrane protein to be quantified, human SLC9A6 can be exemplified. As a subject peptide for quantification and a stable-isotope labeled peptide used for measuring human SLC9A6, one or more peptides selected from a peptide having an amino acid sequence shown by SEQ ID No: 1414 of the sequence listing can be specifically exemplified.

Similarly, as the above plasma membrane protein to be quantified, human SLC9A7 can be exemplified. As a subject peptide for quantification and a stable-isotope labeled peptide used for measuring human SLC9A7, one or more peptides selected from a peptide having an amino acid sequence shown by SEQ ID No: 1415, SEQ ID No: 1416, and SEQ ID No: 1417 of the sequence listing can be specifically exemplified.

Similarly, as the above plasma membrane protein to be quantified, human SLC9A8 can be exemplified. As a subject peptide for quantification and a stable-isotope labeled peptide used for measuring human SLC9A8, one or more peptides selected from a peptide having an amino acid sequence shown by SEQ ID No: 1418 of the sequence listing can be specifically exemplified.

Similarly, as the above plasma membrane protein to be quantified, human SLC9A9 can be exemplified. As a subject peptide for quantification and a stable-isotope labeled peptide used for measuring human SLC9A9, one or more peptides selected from a peptide having an amino acid sequence shown by SEQ ID No: 1419, and SEQ ID No: 1420 of the sequence listing can be specifically exemplified.

### [Example 1]

### (Preparation of a peptide sample)

Human ABCG2 (human ATB binding cassette transporter G2) was selected as a plasma membrane protein, and a peptide sample was prepared to select a peptide that can be selected by LC-MSMS. First, a plasma membrane of human ABCG2-expressing cell was used as a peptide source, and the peptide sample was prepared as follows. That is, ABCC2-expresssing plasma membrane protein was denatured in buffer solution of 7 M guanidine hydrochloride, 0.1 M Tris-HCl, 10 mM EDTA ph 8.5, and a reduction treatment by DTT and a carbamidemethylation treatmemt by Iodo acetamide were performed in order to protect SH group of cysteine residue. After a dialysis against 50 mM hydrogen carbonate ammonium buffer solution, trypsin was added in an amount of 1/100 of protein mass, the mixture was enzymatically digested at 37°C for 16 hours, to obtain a peptide sample.

### (Preparation of a subject peptide for quantification and a stable-isotope labeled peptide)

Human ABCG2-expressing plasma membrane peptide sample was measured by LC-MSMS. From the measurement results, according to the criteria for selecting a subject peptide for quantification, 5 peptides derived from human ABCG2 protein were selected: ENLQFSAALR (SEQ ID No: 1), LAEIYVNSSFYK (SEQ ID No:2), LFDSLTLLASGR (SEQ ID No: 3), SSLLDVLAAR (SEQ ID No: 4), and VIQELGLDK (SEQ ID No: 5). The applied criteria for ENLQFSAALR (SEQ ID No: 1) were (1), (2), (3), (4), (5), (8), (9), and (10). The applied criteria for LAEIYVNSSFYK (SEQ ID No: 2) were (1), (2), (3), (4), (5), (6), (7), (8), (9), and (10). The applied criteria for LFDSLTLLASGR (SEQ ID No: 3) were (1), (2), (3), (4), (5), (6), (7), (8), (9), (10) and (11). The applied criteria for SSLLDVLAAR(SEQ ID No:4) were (1), (2), (3), (4), (5), (6), (7), (8), (9), (10) and (11). The applied criteria for VIQELGLDK(SEQ ID No:5) were (1), (2), (3), (4), (5), (6), (7), (8), (9), and (10). Among these, the peptide SSLLDVLAAR (SEQ ID No:4), which is the same amino acid sequence as those of rats and mice, was set as a measurement subject. By selecting a peptide having homology among species, the quantification of not only human-derived sample, but rat and mouse-derived sample can be performed by using the same standard peptide. For the subject peptide, a non-labeled peptide, and a stable-isotope peptide in which the 7 th leucine is labeled with ¹³C₆, and ¹⁵N, were synthesized by F-moc method: SSLLDVL (¹³C₆,¹⁵N)AAR. It was confirmed that the intended peptide was synthesized, by mass spectrometry by using LC-MSMS.

### (Preparation of a calibration curve)

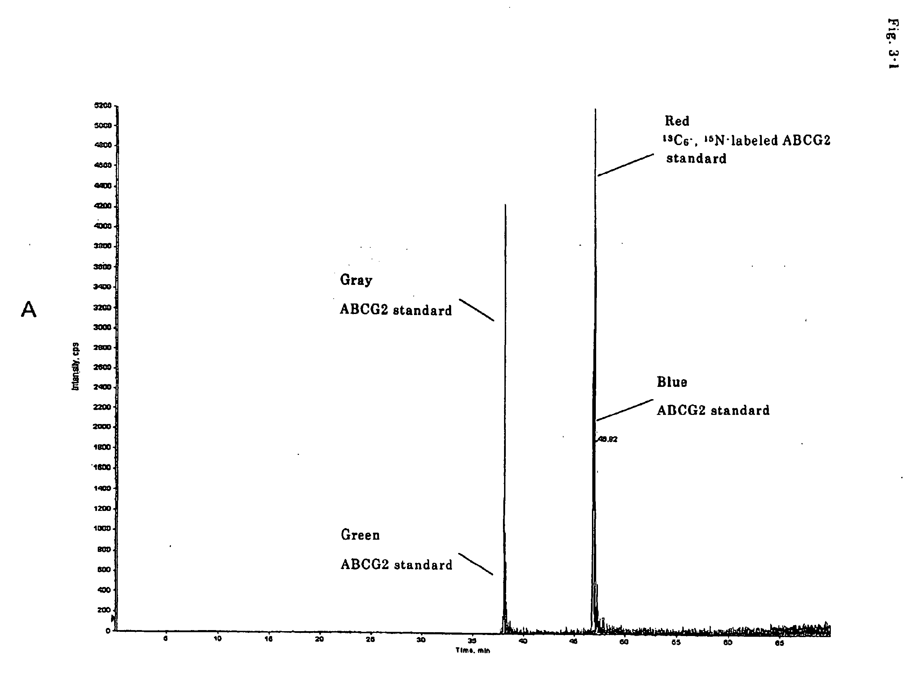

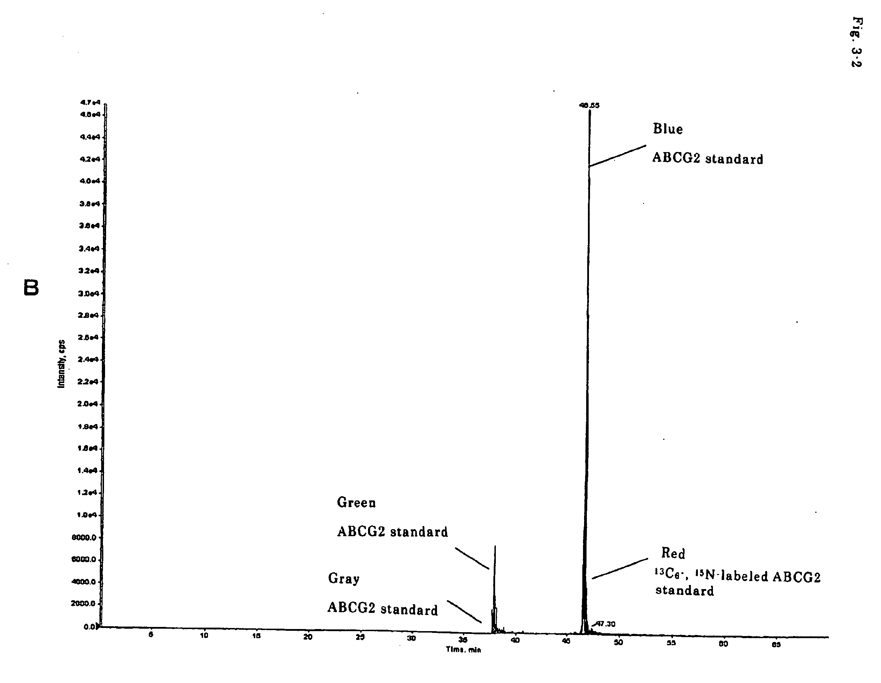

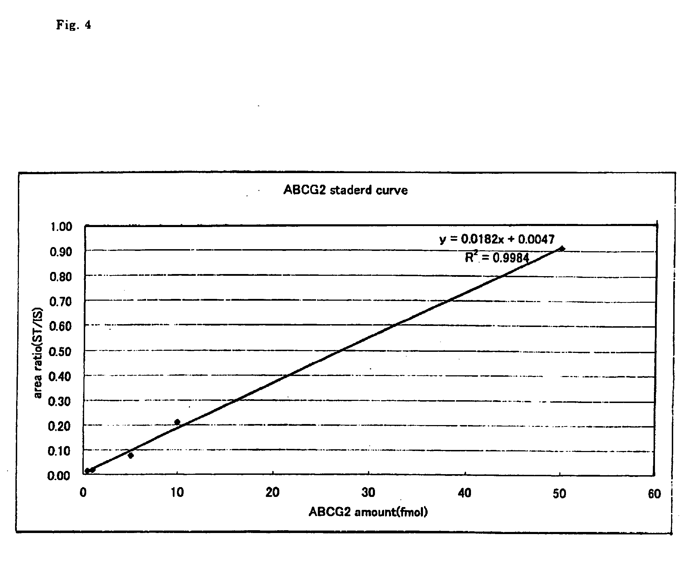

By using the selected subject peptide for quantification (non-labeled peptide: SSLLDVLAAR) and the synthesized stable-isotope labeled peptide (isotope labeled peptide: SSLLDVL(¹³C₆, ¹⁵N)AAR), a calibration curve was prepared (consideration of linearity). 10 f mol of ¹³C₆-, ¹⁵N-labeled peptide was added to each of 0.5 fmol, 1 fmol, 5 fmol, 10 fmol, 50 fmol of non-labeled peptide, respectively, and measured by LC-MSMS (Fig. 3). The MS spectrum area ratio (non-labeled peptide /³C₆-, ¹⁵N-labeled peptide) was calculated to prepare a calibration curve (Fig. 4). The linearity of the calibration curve within the measured range, 0.5 fmol - 50 fmol, was shown, and it was confirmed that quantification was possible within this range.

### (Quantification of ABCG2 protein sample)

As ABCG2 protein source, a plasma membrane protein of mouse conditionally immortalized brain capillary endothelial cell strain was selected and quantified. First, the cultured cells were suspended in 10 mM Tris-HCl, 250 mM Sucrose, 1 mM EGTA pH 7.4. High nitrogen gas filling method was conducted for 15 min, and the solution was centrifuged at 10,000 × g, for 15 min. Further, the supernatant was added on 38% sucrose solution, which mixture was centrifuged at 10,000 × g for 40 min to obtain a plasma membrane sample.

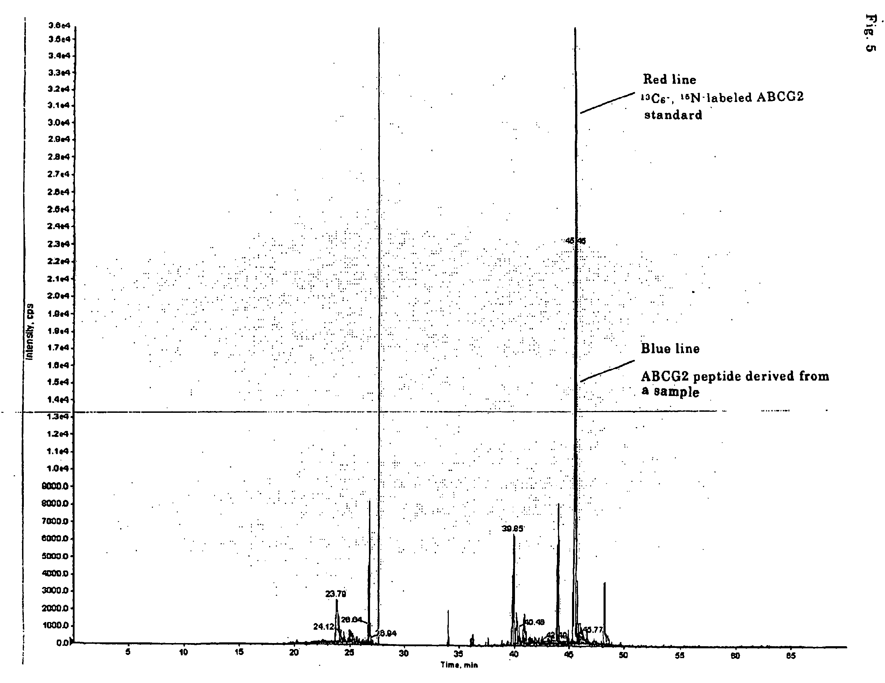

With the method described in the above section "(Preparation of a peptide sample)", a peptide sample was prepared from a plasma membrane sample. In 1 µg of the plasma membrane peptide sample thus obtained from mouse conditionally immortalized brain capillary endothelial cell strain, 10 f mol of ¹³C₆-, ¹⁵N-labeled peptide was added and the resultant was measured by LC-MSMS (Fig. 5). After measurement, MS spectrum area ratio (endogenous ABCG2 peptide ¹³C₆-, ¹⁵N-labeled peptide) was calculated, and the quantitative level was calculated by using the above calibration curve. As a result, the ABCG2 protein mass of a plasma membrane of mouse conditionally immortalized brain capillary endothelial cell strain was calculated to be 4.7 fmol/µg.

### Example 2

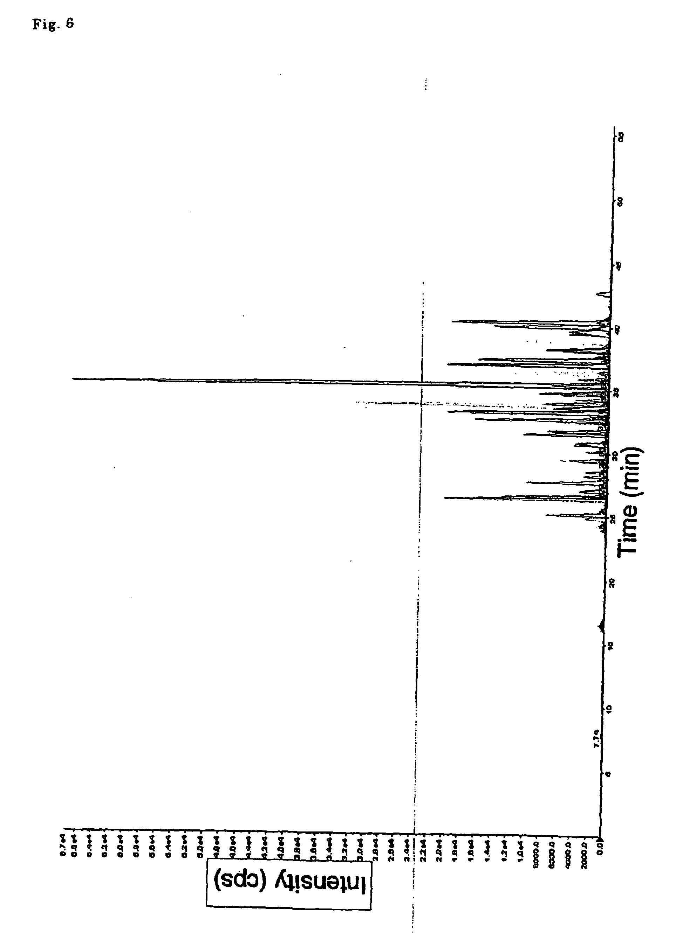

As criteria for selecting a subject peptide for quantification, criteria (1), (2), (3), (4), (5), (6), (7), (8), (9), and (10) were applied as well as (11) according to need. Subject peptides for quantification for the proteins listed on the following Table 1 were synthesized in the same manner as Example 1, and their linearity was considered by preparing a calibration curve. 500 f mol of a stable-isotope labeled peptide was added to each of 10 fmol, 50 fmol, 100 fmol, 500 fmol, 1000 fmol of non-labeled peptide, respectively, and measured by LC-MSMS. In each of the peptides, the linearity of the calibration curve was shown within 10 f mol - 1000 fmol, and it was confirmed that quantification of the target protein is possible within this range (Fig. 6). Moreover, a plasma membrane of rotein of human leukemia cell strain was selected as a transporter protein source, and quantified. The quantification threshold value and the quantitative level of plasmid membrane of leukemia cells of the obtained calibration curve are shown in Table 1.

**[Table 1]**

| protein | sequence | Isotope sequence | Quantitative threshold (fmol) | Quantitative level of leukemia cells (fmol/µg) |
|---|---|---|---|---|
| ABCB1 | EALDESIPPVSFWR | EALDESIPPVSF (13C9,15N)WR | 10 | < 0.12 |
| ABCB4 | IATEAIENIR | IATEA(13C3,15N)IENIR | 1 | 0 |
| ABCB5 | SADLIVTLK | SADLIVTL(13C6,15N)K | 5 | 0.15 |
| ABCB11 | STALQLIQR | STALQL(13C6,15N)IQR | 1 | 0.45 |
| ABCC1 | TPSGNLVNR | TPSGNL(13C6,15N)VNR | 5 | 0.67 |
| ABCC2 | QLLNNILR | QLLNNIL(13C6,15N) R | 10 | < 0.01 |
| ABCC3 | AEGEISDPFR | AEGEISDPF(13C9,15N)R | 5 | < 0.06 |
| ABCC4 | APVLFFDR | APVL(13C6,15N) FFDR | 5 | 0.65 |
| ABCC5 | SLSEASVAVDR | SL(13C6,15N)SEASVAVDR | 10 | 0.28 |
| ABCC6 | APETEPFLR | APETEPFL(13C6,15N)R | 1 | 0 |
| ABCC7 | GLPLVHTLITVSK | GLPLVHTLITV(13C5,15N)SK | 10 | < 0.22 |
| ABCC8 | TVVTIAHR | TVVTIA(13C3,15N)HR | 5 | < 0.06 |
| ABCC9 | NLHHNLLNK | NLHHNLL(13C8,15N)NK | 50 | < 0.29 |
| ABCC10 | VFTALALVR | VFTALAL(13C6,15N)VR | 5 | < 0.01 |
| ABCC11 | FNLDPFDR | FNLDPF(13C9,15N)DR | 5 | < 0.11 |
| ABCC12 | FSIAILPFSIK | FSIAILPF(13C9,15N)SIK | 50 | < 0.43 |
| ABCC13 | EDLFELK | EDLFEL(13C6,15N)K | 5 | 0 |
| ABCG1 | GAVLINGLPR | GAVLINGL(13C6,15N)PR | 5 | 0 |
| ABCG4 | GVVTNLIPYLK | GVVTNLIPYL(13C6,15N)K | 5 | < 0.21 |
| ABCG5 | DSPGVFSK | DSPGVF(13C9,15N)SK | 50 | < 0.09 |
| ABCG8 | ASLLDVITGR | ASLL(13C6,15N)DVITGR | 5 | 0 |

### [Example 3]

As criteria for selecting a subject peptide for quantification, criteria (1), (2), (3), (4), (5), (6), (7), (8), (9), and (10) were applied as well as (11) according to need, subject peptides for quantification for the proteins listed on the following Table 2 were synthesized in the same manner as Example 1, and their linearity was considered by preparing a calibration curve. 100 f mol of a stable-isotope labeled peptide was added to each of 1 fmol, 5 fmol, 10 fmol, 50 fmol, 100 fmol, 500 fmol, and 1000 fmol of non-labeled peptide, respectively, and measured by LC-MSMS. In each of the peptides, the linearity of the calibration curve was shown, and it was confirmed that quantification of the intended peptide is possible. The quantification threshold value and the quantitative level of plasma membrane of leukemia cells of the obtained calibration curve are shown in Table 2.

**[Table 2]**

| protein | sequence | Isotope sequence | Quantitative threshold(fmol) |
|---|---|---|---|
| SLC10A1 | GIYDGDLK | GIYDGDL(13C6,15N)K | 5 |
| SLC15A1 | TLPVFPK | TLPVFP(13C5,15N)K | 5 |
| SLC15A2 | SQDFHFHLK | SODFHFHL(13C6,15N)K | 10 |
| SLC21A2 | VNTAAVNLVPGDPR | VNTAAVNLVPGDP(13C5,15N)R | 5 |
| SLC21A6 | LNTVGIAK | LNTVGI(13C6,15N)AK | 1 |
| SLC21A8 | IYNSVFFGR | IYNSVFF(13C9,15N)GR | 5 |
| SLC21A9 | VLLQTLR | VLLQTL(13C6,15N)R | 5 |
| SLC22A1 | LSPSFADLFR | LSPSFADL(13C6,15N)FR | 5 |
| SLC22A3 | FLQGVFGK | FLQGVF(13C9,15N)GK | 5 |
| SLC22A7 | NVALLALPR | NVALLAL(13C6,15N)PR | 10 |
| SLC22A9 | DTLTLEILK | DTLTLEIL(13C6,15N)K | 50 |
| SLC22A10 | NLPLPDTIK | NLPLPDTI(13C6,15N)K | 5 |
| SLC22A15 | VGGIIAPFIPSLK | VGGIIAPFIPSL(13C6,15N)K | 5 |

### Industrial Applicability

With the method for quantifying a plasma membrane protein by mass spectrometry by using a stable-isotope labeled peptide, it is possible to quantify insoluble and high molecular membrane proteins in a simple, rapid and accurate manner, which was difficult with the conventional methods. Further, with the method for quantifying a plasma membrane protein, it is possible to quantify the membrane protein without using an antibody. Therefore, the step of preparing an antibody which took much time in the conventional methods can be omitted, and the quantification of a membrane protein to which an antibody cannot be prepared became also possible. Therefore, a method for quantifying accurately a plasma membrane protein widely applicable can be provided. Therefore, a method for quantifying a plasma membrane protein can be expected to contribute significantly for elucidating functions of a plasma membrane protein having important functions as receptor against factors acting on organisms, transporter of biological substances, ion channel, and plasma membrane antigen; screening of active substances, test, diagnosis, etc. using the expression of the protein. Particularly, when developing a novel drug, the absolute quantification of a protein such as membrane proteins by a simple and accurate method, is very critical for testing a novel drug candidate. The method for quantifying a plasma membrane protein is expected to contribute greatly for promoting development in this field.

## Claims

1. A method for quantifying a plasma membrane protein by liquid chromatography-tandem mass spectrometry (LC/MS/MS) using a stable-isotope labeled peptide, comprising the following steps (a) to (f):
(a) preparing and identifying an oligopeptide fragment by fragmenting a separated plasma membrane protein target molecule to be quantified;
(b) selecting a subject peptide for quantification that can be ionized by ESI method comprising:
selecting an oligopeptide fragment that meets an essential criteria consisting of (1) and (2) set forth below, and
further calculating a total score value for each of the oligopeptide fragment that meets the essential criteria, based on selective criteria consisting of (3) to (11) set forth below, each selective criterion having a score value,
wherein an oligopeptide fragment having a high total score relative to the other fragments of said target molecule is preferentially selected as the subject peptide for quantification:
(1) the peptide is obtained by fragmenting with a protease selected from trypsin, endoproteinase, and pepsin;
(2) the peptide sequence is specific to the target molecule;
(3) where it is a peptide wherein the content of hydrophobic amino acids is 80% or less, and wherein not more than 10 hydrophobic amino acids are consecutive, where hydrophobic amino acids are selected from tryptophan, tyrosine, valine, leucine, isoleucine and phenylalanine, score 2 is given;
(4) where it is a peptide wherein the number of amino acid residues is 4 to 30, score 3 is given;
(5) where it is a peptide that does not contain the sequence of asparagine-X-serine, asparagine-X-threonine, or asparagine-X-cysteine as specific amino acid sequence conditions, wherein X represents an amino acid other than proline, score 2 is given;
(6) where, except when a post-translational modified protein is being quantified, it is a peptide that does not contain a post-translational modification site, score 3 is given;
(7) where it is a peptide that does not contain a single nucleotide polymorphism (SNP) site, score 4 is given;
(8) where it is a peptide wherein a cleavage site of the protease is not arginine-arginine, arginine-lysine, lysine-arginine, or lysine-lysine, score 5 is given;
(9) where it is a peptide that does not contain a transmembrane domain when the protein structure is determined or estimated, score 2 is given;
(10) where it is a peptide that does not contain methionine or cysteine, score 3 is given; and
(11) where it is a peptide that does not contain tryptophan or glutamic acid, score 1 is given;
(c) preparing a stable-isotope labeled peptide having the same sequence as the subject peptide for quantification and labeled with a stable isotope by a peptide synthesis method;
(d) preparing a calibration curve by using the subject peptide for quantification and the stable-isotope labeled peptide and performing mass spectrometry using LC/MS/MS for each predetermined concentration level;
(e) performing mass spectrometry using LC/MS/MS by adding the stable-isotope labeled peptide to the peptide fragment obtained by fragmenting the plasma membrane protein to be quantified, and calculating the mass spectrum area ratio of the peptide fragment to the stable-isotope labeled peptide; and
(f) calculating a quantitative level of the plasma membrane protein from the mass spectrum area ratio by using the calibration curve.

2. The method for quantifying a plasma membrane protein according to claim 1, wherein the hydrophobic amino acid content in selective criterion (3) is 50% or less, and the number of amino acid residues in selective criterion (4) is 8 to 12.

3. The method for quantifying a plasma membrane protein according to claim 1 or 2, further comprising an additional criterion (12):
(12) the same amino acid sequence of the peptide is found in plural animal species;
wherein the peptide is preferentially selected as the subject peptide for quantification if it meets at least criteria (1), (2) and (12) and if it has a high total score value based on said score values of selective criteria (3) to (11).

4. The method for quantifying a plasma membrane protein according to any one of claims 1 to 3, wherein plural specific measurement channels are prepared by combining parent ion (m/z) and peptide fragment ion (m/z) of candidate peptides selected according to the criteria for selecting subject peptide for quantification, and measured.

5. The method for quantifying a plasma membrane protein according to claim 4, wherein the plural specific channels are prepared for plural candidate peptides of the same protein and for a candidate peptide for plural proteins, and measured at the same time.

6. The method for quantifying a plasma membrane protein according to any one of claims 1 to 5, wherein the stable-isotope labeled peptide is labeled with an amino acid containing any one of ¹⁵N, ¹³C, ¹⁸O, or ²H.

7. The method for quantifying a plasma membrane protein according to any one of claims 1 to 6, wherein a source of plasma membrane protein is a tissue sample.

8. The method for quantifying a plasma membrane protein according to any one of claims 1 to 7, wherein the plasma membrane protein is 1 or more proteins selected from the group consisting of human ABCA1, human ABCA2, human ABCA3, human ABCA4, human ABCA5, human ABCA6, human ABCA7, human ABCA8, human ABCA9, human ABCA10, human ABCA12, human ABCA13, human ABCB1, human ABCB4,human ABCB5, human ABCB11, human ABCC1, human ABCC2, human ABCC3, human ABCC4, human ABCC5, human ABCC6, human ABCC7, human ABCC8, human ABCC9, human ABCC10, human ABCC11, human ABCC12, human ABCC13, human ABCG1, human ABCG2, human ABCG4, human ABCG5, human ABCG8, and human P-glycoprotein.

9. The method for quantifying a plasma membrane protein according to any one of claims 1 to 7, wherein the plasma membrane protein is one or more proteins selected from the group consisting of human SLC10A1, human SLC10A2, human SLC15A1. human SLC15A2, human SLC16A1, human SLC16A7, human SLC19A1, human SLC19A2, human SLC19A3, human SLC21A1. human SLC21A10, human SLC21A11, human SLC21A12, human SLC21A13, human SLC21A14, human SLC21A15, human SLC21A19, human SLC21A2, human SLC21A20, human SLC21A3, human SLC21A4, human SLC21A5, human SLC21A6, human SLC21A7, human SLC21A8, human SLC21A9, human SLC22A1, human SLC22A10, human SLC22A11. human SLC22A12, human SLC22A13, human SLC22A14, human SLC22A15, human SLC22A16. human SLC22A17, human SLC22A18, human SLC22A2, human SLC22A3, human SLC22A4, human SLC22A5, human SLC22A6, human SLC22A7, human SLC22A8, human SLC22A9, human SLC23A1, human SLC23A2, human SLC28A1, human SLC28A2, human SLC28A3, human SLC29A1, human SLC29A2, human SLC29A3, human SLC29A4, human SLC31A1, human SLC3A2, human SLC43A1. human SLC43A2, human SLC43A3, human SLC7A5, human SLC7A6, and human SLC7A8.

10. The method for quantifying a plasma membrane protein according to any one of claims 1 to 7, wherein the plasma membrane protein is one or more proteins selected from the group consisting of human SLC10A3, human SLC10A4. human SLC10A5. human SLC10A6, human SLC11A1, human SLC11A2, human SLC12A1, human SLC12A2, human SLC12A3, human SLC12A4, human SLC12A5, human SLC12A6, human SLC12A7, human SLC12A8, human SLC12A9, human SLC13A1, human SLC13A2, human SLC13A3, human SLC13A4, human SLC13A5, human SLC14A1, human SLC14A2, human SLC15A3, human SLC15A4, human SLC16A10, human SLC16A11, human SLC16A12, human SLC16A13, human SLC16A14, human SLC16A2, human SLC16A3, human SLC16A4, human SLC16A5, human SLC16A6, human SLC16A8, human SLC16A9, human SLC17A1, human SLC17A2, human SLC17A3, human SLC17A4, human SLC17A5, human SLC17A6, human SLC17A7, human SLC17A8, human SLC18A1, human SLC18A2, human SLC18A3, human SLC1A1, human SLC1A2, human SLC1A3, human SLC1A4, human SLC1A5, human SLC1A6, human SLC1A7, human SLC20A1, human SLC20A2, human SLC23A3, human SLC24A1, human SLC24A2, human SLC24A3, human SLC24A4, human SLC24A5, human SLC24A6, human SLC25A1, human SLC25A10, human SLC25A11, human SLC25A12, human SLC25A13, human SLC25A14, human SLC25A15, human SLC25A16, human SLC25A17, human SLC25A18, human SLC25A19, human SLC25A2, human SLC25A20, human SLC25A21, human SLC25A22, human SLC25A23, human SLC25A24, human SLC25A25, human SLC25A26, human SLC25A27, human SLC25A28, human SLC25A29, human SLC25A3, human SLC25A30, human SLC25A31, human SLC25A32, human SLC25A33, human SLC25A34, human SLC25A35, human SLC25A36, human SLC25A37, human SLC25A4, human SLC25A5, human SLC25A6, human SLC25A7, human SLC25A8, human SLC25A9, human SLC26A1, human SLC26A10, human SLC26A11, human SLC26A2, human SLC26A3, human SLC26A4, human SLC26A5, human SLC26A6, human SLC26A7, human SLC26A8, human SLC26A9, human SLC27A1, human SLC27A2, human SLC27A3, human SLC27A4, human SLC27A5, human SLC27A6, human SLC2A1, human SLC2A10, human SLC2A11. human SLC2A12, human SLC2A13, human SLC2A14, human SLC2A2, human SLC2A3, human SLC2A4, human SLC2A5, human SLC2A6, human SLC2A7, human SLC2A8. human SLC2A9, human SLC30A1, human SLC30A10, human SLC30A11, human SLC30A2, human SLC30A3, human SLC30A4, human SLC30A5, human SLC30A6, human SLC30A7, human SLC30A8, human SLC30A9, human SLC31A2, human SLC32A1, human SLC33A1, human SLC34A1, human SLC34A2, human SLC34A3, human SLC35B1, human SLC35B2, human SLC35B3, human SLC35B4, human SLC35C1, human SLC35C2, human SLC35D1, human SLC35D2, human SLC35D3, human SLC36A1, human SLC36A2, human SLC36A3, human SLC36A4, human SLC37A1, human SLC37A2, human SLC37A3, human SLC37A4, human SLC38A1, human SLC38A2, human SLC38A3, human SLC38A4, human SLC38A5, human SLC38A6, human SLC3A1. human SLC40A1, human SLC41A1, human SLC41A2, human SLC41A3, human SLC44A1, human SLC44A2, human SLC44A3, human SLC44A4, human SLC44A5, human SLC45A1, human SLC45A2, human SLC45A3, human SLC45A4, human SLC4A1, human SLC4A10, human SLC4A11, human SLC4A2, human SLC4A3, human SLC4A4, human SLC4A5, human SLC4A7, human SLC4A8, human SLC4A9, human SLC5A1, human SLC5A11, human SLC5A12, human SLC5A2, human SLC5A3, human SLC5A4, human SLC5A5, human SLC5A9, human SLC6A1, human SLC6A10, human SLC6A11, human SLC6A12, human SLC6A13. human SLC6A14, human SLC6A15, human SLC6A16, human SLC6A17, human SLC6A18, human SLC6A19, human SLC6A2, human SLC6A20, human SLC6A3, human SLC6A4, human SLC6A5, human SLC6A6, human SLC6A7, human SLC6A8, human SLC6A9, human SLC7A1, human SLC7A10, human SLC7A11, human SLC7A13, human SLC7A14, human SLC7A2, human SLC7A3, human SLC7A4, human SLC7A7, human SLC7A9, human SLC8A1, human SLC8A2, human SLC8A3, human SLC9A1, human SLC9A2, human SLC9A3, human SLC9A4, human SLC9A5, human SLC9A6, human SLC9A7, human SLC9A8, and human SLC9A9.

11. The method for quantifying a plasma membrane protein according to any one of claims 1 to 7, wherein the plasma membrane protein is one or more proteins selected from the group consisting of human SLC35A1, human SLC35A2, human SLC35A3, human SLC35A4, human SLC35A5, human SLC35E1, human SLC35E2, human SLC35E3, human SLC35E4, human SLC35F1, human SLC35F2, human SLC35F3, human SLC35F5, human SLC39A1, human SLC39A10, human SLC39A11, human SLC39A12, human SLC39A13, human SLC39A14, human SLC39A2, human SLC39A3, human SLC39A4, human SLC39A5, human SLC39A6, human SLC39A7, human SLC39A8, human SLC39A9, human SLC42A1, human SLC42A2, and human SLC42A3.

12. The method for quantifying a plasma membrane protein according to any one of claims 1 to 7, wherein the plasma membrane protein is human MATE1 or human MATE2.

13. The method for quantifying a plasma membrane protein according to any one of claims 1 to 7, wherein the plasma membrane protein is one or more proteins selected from the group consisting of human ABCA1, human ABCA2, human ABCA3, human ABCA4, human ABCA5, human ABCA6, human ABCA7, human ABCA8, human ABCA9, human ABCA10, human ABCA12, human ABCA13, human ABCB1, human ABCB4, human ABCB5, human ABCB11, human ABCC1, human ABCC2, human ABCC3, human ABCC4, human ABCC5, human ABCC6, human ABCC10, human ABCC11, human ABCC12, human ABCC13, human ABCG1, human ABCG2, human ABCG4, human ABCG5, human ABCG8, human MATE1, human MATE2, human SLC3A2, human SLC7A5, human SLC7A6, human SLC10A1, human SLC10A2, human SLC15A1, human SLC15A2, human SLC16A1, human SLC16A7, human SLC19A1, human SLC21A2, human SLC21A3, human SLC21A4, human SLC21A5, human SLC21A6, human SLC21A7, human SLC21A8, human SLC21A9, human SLC21A11, human SLC21A12, human SLC21A13, human SLC21A14, human SLC21A15, human SLC21A19, human SLC21A20, human SLC22A1, human SLC22A2, human SLC22A3, human SLC22A4, humain SLC22A5, human SLC22A6, human SLC22A7, human SLC22A8, human SLC22A9, human SLC22A10, human SLC22A11. human SLC22A12, human SLC22A13, human SLC22A14, human SLC22A15, human SLC22A16, human SLC22A17, human SLC23A1, human SLC23A2, human SLC28A1, human SLC28A2, human SLC28A3, human SLC29A1, human SLC29A2, and human SLC31A1.

## Patentansprüche

1. Verfahren zum Quantifizieren eines Plasmamembranproteins durch Flüssigkeitschromatographie-Tandem-Massenspektrometrie (LC/MS/MS) unter Verwendung eines mit einem stabilen Isotop markierten Peptids, umfassend die folgenden Schritte (a) bis (f):
(a) Herstellen und Identifizieren eines Oligopeptidfragments durch Fragmentieren eines zu quantifizierenden, abgetrennten Plasmamembranprotein-Zielmoleküls;
(b) Auswählen eines Subjektpeptids, das durch ESI-Verfahren ionisiert werden kann, zum Quantifizieren, umfassend:
Auswählen eines Oligopeptidfragments, das einem notwendigen Kriterium, das aus den nachstehend dargelegten (1) und (2) besteht, entspricht und
ferner Berechnen eines Gesamtpunktewerts für jedes der Oligopeptidfragmente, die dem notwendigen Kriterium entsprechen, auf der Grundlage selektiver Kriterien, die aus den nachstehend dargelegten (3) bis (11) bestehen, wobei jedes selektive Kriterium einen Punktewert aufweist,
wobei ein Oligopeptidfragment mit einem hohen Gesamtpunktewert im Vergleich zu anderen Fragmenten des Zielmoleküls vorzugsweise als Subjektpeptid für die Quantifizierung ausgewählt wird:
(1) das Peptid ist durch Fragmentieren mit einer Protease, ausgewählt aus Trypsin, Endoproteinase und Pepsin, erhalten;
(2) die Peptidsequenz ist für das Zielmolekül spezifisch;
(3) wenn es ein Peptid ist, bei dem der Gehalt an hydrophoben Aminosäuren 80 % oder weniger beträgt und bei dem nicht mehr als 10 hydrophobe Aminosäuren aufeinander folgen, wobei hydrophobe Aminosäuren ausgewählt sind aus Tryptophan, Tyrosin, Valin, Leucin, Isoleucin und Phenylalanin, wird ein Punktewert von 2 gegeben;
(4) wenn es ein Peptid ist, bei dem die Anzahl der Aminosäurereste 4 bis 30 ist, wird ein Punktewert von 3 gegeben;
(5) wenn es ein Peptid ist, das nicht die Sequenz Asparagin-X-Serin, Asparagin-X-Threonin oder Asparagin-X-Cystein als spezifische Aminosäuresequenzbedingungen enthält, wobei X eine von Prolin verschiedene Aminosäure darstellt, wird ein Punktewert von 2 gegeben;
(6) wenn es, mit der Ausnahme der Quantifizierung eines posttranslational modifizierten Proteins, ein Peptid ist, das keine posttranslationale Modifizierungsstelle enthält, wird ein Punktewert von 3 gegeben;
(7) wenn es ein Peptid ist, das keine Einzelnucleotidpolymorphismus(SNP)-Stelle enthält, wird ein Punktewert von 4 gegeben;
(8) wenn es ein Peptid ist, bei dem eine Spaltungsstelle der Protease nicht Arginin-Arginin, Arginin-Lysin, Lysin-Arginin oder Lysin-Lysin ist, wird ein Punktewert von 5 gegeben;
(9) wenn es ein Peptid ist, das, falls die Proteinstruktur bestimmt oder geschätzt ist, keine Transmembrandomäne enthält, wird ein Punktewert von 2 gegeben;
(10) wenn es ein Peptid ist, das nicht Methionin oder Cystein enthält, wird ein Punktewert von 3 gegeben; und
(11) wenn es ein Peptid ist, das nicht Tryptophan oder Glutaminsäure enthält, wird ein Punktewert von 1 gegeben;
(c) Herstellen eines mit einem stabilen Isotop markierten Peptids mit der gleichen Sequenz wie das Subjektpeptid für die Quantifizierung, markiert mit einem stabilen Isotop, durch ein Peptidsynthese-Verfahren;
(d) Erstellen einer Kalibrierkurve unter Verwendung des Subjektpeptids für die Quantifizierung und des mit einem stabilen Isotop markierten Peptids und Durchführen von Massenspektrometrie unter Verwendung von LC/MS/MS für jedes vorbestimmte Konzentrationsniveau;
(e) Durchführen von Massenspektrometrie unter Verwendung von LC/MS/MS durch Zugeben des mit einem stabilen Isotop markierten Peptids zu dem durch Fragmentieren des zu quantifizierenden Plasmamembranproteins erhaltenen Peptidfragment und Berechnen des Massenspektrum-Flächenverhältnisses des Peptidfragments zu dem mit einem stabilen Isotop markierten Peptid; und
(f) Berechnen eines quantitativen Niveaus des Plasmamembranproteins aus dem Massenspektrum-Flächenverhältnis durch Verwendung der Kalibrierkurve.

2. Verfahren zum Quantifizieren eines Plasmamembranproteins gemäß Anspruch 1, wobei der Gehalt an hydrophoben Aminosäuren bei dem selektiven Kriterium. (3) 50 % oder weniger beträgt und die Anzahl der Aminosäurereste bei dem selektiven Kriterium (4) 8 bis 12 beträgt.

3. Verfahren zum Quantifizieren eines Plasmamembranproteins gemäß Anspruch 1 oder 2, ferner umfassend ein zusätzliches Kriterium (12):
(12) die gleiche Aminosäuresequenz des Peptids wird in mehreren Tierspezies gefunden;
wobei das Peptid vorzugsweise als Subjektpeptid für die Quantifizierung ausgewählt wird, wenn es wenigstens den Kriterien (1), (2) und (12) entspricht und wenn es einen hohen Gesamtpunktewert auf der Grundlage der Punktewerte der selektiven Kriterien (3) bis (11) aufweist.

4. Verfahren zum Quantifizieren eines Plasmamembranproteins gemäß einem der Ansprüche 1 bis 3, wobei mehrere spezifische Messkanäle durch Kombinieren des Ausgangsionen-(m/z) und des Peptidfragmentionen-(m/z) von Kandidatenpeptiden, ausgewählt gemäß den Kriterien zum Auswählen von Subjektpeptiden für die Quantifizierung, erstellt und gemessen werden.

5. Verfahren zum Quantifizieren eines Plasmamembranproteins gemäß Anspruch 4, wobei die mehreren spezifischen Kanäle für mehrere Kandidatenpeptide des gleichen Proteins und für ein Kandidatenpeptid für mehrere Proteine erstellt und gleichzeitig gemessen werden.

6. Verfahren zum Quantifizieren eines Plasmamembranproteins gemäß einem der Ansprüche 1 bis 5, wobei das mit einem stabilen Isotop markierte Peptid mit einer Aminosäure markiert ist, die eines von ¹⁵N, ¹³C, ¹⁸O oder ²H enthält.

7. Verfahren zum Quantifizieren eines Plasmamembranproteins gemäß einem der Ansprüche 1 bis 6, wobei eine Quelle von Plasmamembranprotein eine Gewebeprobe ist.

8. Verfahren zum Quantifizieren eines Plasmamembranproteins gemäß einem der Ansprüche 1 bis 7, wobei das Plasmamembranprotein 1 oder mehrere Proteine ist, ausgewählt aus der Gruppe bestehend aus humanem ABCA1, humanem ABCA2, humanem ABCA3, humanem ABCA4, humanem ABCA5, humanem ABCA6, humanem ABCA7, humanem ABCA8, humanem ABCA9, humanem ABCA10, humanem ABCA12, humanem ABCA13, humanem ABCB1, humanem ABCB4,humanem ABCB5, humanem ABCB11, humanem ABCC1, humanem ABCC2, humanem ABCC3, humanem ABCC4, humanem ABCC5, humanem ABCC6, humanem ABCC7, humanem ABCC8, humanem ABCC9, humanem ABCC10, humanem ABCC11, humanem ABCC12, humanem ABCC13, humanem ABCG1, humanem ABCG2, humanem ABCG4, humanem ABCG5, humanem ABCG8 und humanem P-Glycoprotein.

9. Verfahren zum Quantifizieren eines Plasmamembranproteins gemäß einem der Ansprüche 1 bis 7, wobei das Plasmamembranprotein ein oder mehrere Proteine ist, ausgewählt aus der Gruppe bestehend aus humanem SLC10A1, humanem SLC10A2, humanem SLC15A1, humanem SLC15A2, humanem SLC16A1, humanem SLC16A7, humanem SLC19A1, humanem SLC19A2, humanem SLC19A3, humanem SLC21A1, humanem SLC21A10, humanem SLC21A11, humanem SLC21A12, humanem SLC21A13, humanem SLC21A14, humanem SLC21A15, humanem SLC21A19, humanem SLC21A2, humanem SLC21A20, humanem SLC21A3, humanem SLC21A4, humanem SLC21A5, humanem SLC21A6, humanem SLC21A7, humanem SLC21A8, humanem SLC21A9, humanem SLC22A1, humanem SLC22A10, humanem SLC22A11, humanem SLC22A12, humanem SLC22A13, humanem SLC22A14, humanem SLC22A15, humanem SLC22A16, humanem SLC22A17, humanem SLC22A18, humanem SLC22A2, humanem SLC22A3, humanem SLC22A4, humanem SLC22A5, humanem SLC22A6, humanem SLC22A7, humanem SLC22A8, humanem SLC22A9, humanem SLC23A1, humanem SLC23A2, humanem SLC28A1, humanem SLC28A2, humanem SLC28A3, humanem SLC29A1, humanem SLC29A2, humanem SLC29A3, humanem SLC29A4, humanem SLC31A1, humanem SLC3A2, humanem SLC43A1, humanem SLC43A2, humanem SLC43A3, humanem SLC7A5, humanem SLC7A6 und humanem SLC7A8.

10. Verfahren zum Quantifizieren eines Plasmamembranproteins gemäß einem der Ansprüche 1 bis 7, wobei das Plasmamembranprotein ein oder mehrere Proteine ist, ausgewählt aus der Gruppe bestehend aus humanem SLC10A3, humanem SLC10A4, humanem SLC10A5, humanem SLC10A6, humanem SLC11A1, humanem SLC11A2, humanem SLC12A1, humanem SLC12A2, humanem SLC12A3, humanem SLC12A4, humanem SLC12A5, humanem SLC12A6, humanem SLC12A7, humanem SLC12A8, humanem SLC12A9, humanem SLC13A1, humanem SLC13A2, humanem SLC13A3, humanem SLC13A4, humanem SLC13A5, humanem SLC14A1, humanem SLC14A2, humanem SLC15A3, humanem SLC15A4, humanem SLC16A10, humanem SLC16A11, humanem SLC16A12, humanem SLC16A13, humanem SLC16A14, humanem SLC16A2, humanem SLC16A3, humanem SLC16A4, humanem SLC16A5, humanem SLC16A6, humanem SLC16A8, humanem SLC16A9, humanem SLC17A1, humanem SLC17A2, humanem SLC17A3, humanem SLC17A4, humanem SLC17A5, humanem SLC17A6, humanem SLC17A7, humanem SLC17A8, humanem SLC18A1, humanem SLC18A2, humanem SLC18A3, humanem SLC1A1, humanem SLC1A2, humanem SLC1A3, humanem SLC1A4, humanem SLC1A5, humanem SLC1A6, humanem SLC1A7, humanem SLC20A1, humanem SLC20A2, humanem SLC23A3, humanem SLC24A1, humanem SLC24A2, humanem SLC24A3, humanem SLC24A4, humanem SLC24A5, humanem SLC24A6, humanem SLC25A1, humanem SLC25A10, humanem SLC25A11, humanem SLC25A12, humanem SLC25A13, humanem SLC25A14, humanem SLC25A15, humanem SLC25A16, humanem SLC25A17, humanem SLC25A18, humanem SLC25A19, humanem SLC25A2, humanem SLC25A20, humanem SLC25A21, humanem SLC25A22, humanem SLC25A23, humanem SLC25A24, humanem SLC25A25, humanem SLC25A26, humanem SLC25A27, humanem SLC25A28, humanem SLC25A29, humanem SLC25A3, humanem SLC25A30, humanem SLC25A31, humanem SLC25A32, humanem SLC25A33, humanem SLC25A34, humanem SLC25A35, humanem SLC25A36, humanem SLC25A37, humanem SLC25A4, humanem SLC25A5, humanem SLC25A6, humanem SLC25A7, humanem SLC25A8, humanem SLC25A9, humanem SLC26A1, humanem SLC26A10, humanem SLC26A11, humanem SLC26A2, humanem SLC26A3, humanem SLC26A4, humanem SLC26A5, humanem SLC26A6, humanem SLC26A7, humanem SLC26A8, humanem SLC26A9, humanem SLC27A1, humanem SLC27A2, humanem SLC27A3, humanem SLC27A4, humanem SLC27A5, humanem SLC27A6, humanem SLC2A1, humanem SLC2A10, humanem SLC2A11, humanem SLC2A12, humanem SLC2A13, humanem SLC2A14, humanem SLC2A2, humanem SLC2A3, humanem SLC2A4, humanem SLC2A5, humanem SLC2A6, humanem SLC2A7, humanem SLC2A8, humanem SLC2A9, humanem SLC30A1, humanem SLC30A10, humanem SLC30A11, humanem SLC30A2, humanem SLC30A3, humanem SLC30A4, humanem SLC30A5, humanem SLC30A6, humanem SLC30A7, humanem SLC30A8, humanem SLC30A9, humanem SLC31A2, humanem SLC32A1, humanem SLC33A1, humanem SLC34A1, humanem SLC34A2, humanem SLC34A3, humanem SLC35B1, humanem SLC35B2, humanem SLC35B3, humanem SLC35B4, humanem SLC35C1, humanem SLC35C2, humanem SLC35D1, humanem SLC35D2, humanem SLC35D3, humanem SLC36A1, humanem SLC36A2, humanem SLC36A3, humanem SLC36A4, humanem SLC37A1, humanem SLC37A2, humanem SLC37A3, humanem SLC37A4, humanem SLC38A1, humanem SLC38A2, humanem SLC38A3, humanem SLC38A4, humanem SLC38A5, humanem SLC38A6, humanem SLC3A1, humanem SLC40A1, humanem SLC41A1, humanem SLC41A2, humanem SLC41A3, humanem SLC44A1, humanem SLC44A2, humanem SLC44A3, humanem SLC44A4, humanem SLC44A5, humanem SLC45A1, humanem SLC45A2, humanem SLC45A3, humanem SLC45A4, humanem SLC4A1, humanem SLC4A10, humanem SLC4A11, humanem SLC4A2, humanem SLC4A3, humanem SLC4A4, humanem SLC4A5, humanem SLC4A7, humanem SLC4A8, humanem SLC4A9, humanem SLC5A1, humanem SLC5A11, humanem SLC5A12, humanem SLC5A2, humanem SLC5A3, humanem SLC5A4, humanem SLC5A5, humanem SLC5A9, humanem SLC6A1, humanem SLC6A10, humanem SLC6A11, humanem SLC6A12, humanem SLC6A13, humanem SLC6A14, humanem SLC6A15, humanem SLC6A16, humanem SLC6A17, humanem SLC6A18, humanem SLC6A19, humanem SLC6A2, humanem SLC6A20, humanem SLC6A3, humanem SLC6A4, humanem SLC6A5, humanem SLC6A6, humanem SLC6A7, humanem SLC6A8, humanem SLC6A9, humanem SLC7A1, humanem SLC7A10, humanem SLC7A11, humanem SLC7A13, humanem SLC7A14, humanem SLC7A2, humanem SLC7A3, humanem SLC7A4, humanem SLC7A7, humanem SLC7A9, humanem SLC8A1, humanem SLC8A2, humanem SLC8A3, humanem SLC9A1, humanem SLC9A2, humanem SLC9A3, humanem SLC9A4, humanem SLC9A5, humanem SLC9A6, humanem SLC9A7, humanem SLC9A8 und humanem SLC9A9.

11. Verfahren zum Quantifizieren eines Plasmamembranproteins gemäß einem der Ansprüche 1 bis 7, wobei das Plasmamembranprotein ein oder mehrere Proteine ist, ausgewählt aus der Gruppe bestehend aus humanem SLC35A1, humanem SLC35A2, humanem SLC35A3, humanem SLC35A4, humanem SLC35A5, humanem SLC35E1, humanem SLC35E2, humanem SLC35E3, humanem SLC35E4, humanem SLC35F1, humanem SLC35F2, humanem SLC35F3, humanem SLC35F5, humanem SLC39A1, humanem SLC39A10, humanem SLC39A11, humanem SLC39A12, humanem SLC39A13, humanem SLC39A14, humanem SLC39A2, humanem SLC39A3, humanem SLC39A4, humanem SLC39A5, humanem SLC39A6, humanem SLC39A7, humanem SLC39A8, humanem SLC39A9, humanem SLC42A1, humanem SLC42A2 und humanem SLC42A3.

12. Verfahren zum Quantifizieren eines Plasmamembranproteins gemäß einem der Ansprüche 1 bis 7, wobei das Plasmamembranprotein humanes MATE1 und/oder humanes MATE2 ist.

13. Verfahren zum Quantifizieren eines Plasmamembranproteins gemäß einem der Ansprüche 1 bis 7, wobei das Plasmamembranprotein ein oder mehrere Proteine ist, ausgewählt aus der Gruppe bestehend aus humanem ABCA1, humanem ABCA2, humanem ABCA3, humanem ABCA4, humanem ABCA5, humanem ABCA6, humanem ABCA7, humanem ABCA8, humanem ABCA9, humanem ABCA10, humanem ABCA12, humanem ABCA13, humanem ABCB1, humanem ABCB4, humanem ABCB5, humanem ABCB11, humanem ABCC1, humanem ABCC2, humanem ABCC3, humanem ABCC4, humanem ABCC5, humanem ABCC6, humanem ABCC10, humanem ABCC11, humanem ABCC12, humanem ABCC13, humanem ABCG1, humanem ABCG2, humanem ABCG4, humanem ABCG5, humanem ABCG8, humanem MATE1, humanem MATTE2, humanem SLC3A2, humanem SLC7A5, humanem SLC7A6, humanem SLC10A1, humanem SLC10A2, humanem SLC15A1, humanem SLC15A2, humanem SLC16A1, humanem SLC16A7, humanem SLC19A1, humanem SLC21A2, humanem SLC21A3, humanem SLC21A4, humanem SLC21A5, humanem SLC21A6, humanem SLC21A7, humanem SLC21A8, humanem SLC21A9, humanem SLC21A11, humanem SLC21A12, humanem SLC21A13, humanem SLC21A14, humanem SLC21A15, humanem SLC21A19, humanem SLC21A20, humanem SLC22A1, humanem SLC22A2, humanem SLC22A3, humanem SLC22A4, humanem SLC22A5, humanem SLC22A6, humanem SLC22A7, humanem SLC22A8, humanem SLC22A9, humanem SLC22A10, humanem SLC22A11, humanem SLC22A12, humanem SLC22A13, humanem SLC22A14, humanem SLC22A15, humanem SLC22A16, humanem SLC22A17, humanem SLC23A1, humanem SLC23A2, humanem SLC28A1, humanem SLC28A2, humanem SLC28A3, humanem SLC29A1, humanem SLC29A2, und humanem SLC31A1.

## Revendications

1. Procédé pour la quantification d'une protéine de la membrane plasmique par chromatographie en phase liquide - spectrométrie de masse en tandem (LC/MS/MS) par utilisation d'un peptide marqué par un isotope stable, comprenant les étapes (a) à (f) ci-après :
(a) préparation et identification d'un fragment oligopeptidique par fragmentation d'une molécule cible protéine de la membrane plasmique séparée, à quantifier ;
(b) sélection d'un peptide sujet pour la quantification, qui peut être ionisé par la méthode ESI, comprenant :
la sélection d'un fragment oligopeptidique qui satisfait à un critère essentiel consistant en les points (1) et (2) ci-dessous, et
puis le calcul d'une note totale pour chacun des fragments oligopeptidiques qui satisfait au critère essentiel, sur la base de critères sélectifs consistant en les points (3) à (11) présentés ci-dessous, chaque critère sélectif ayant une note,
un fragment oligopeptidique ayant une note totale élevée, par comparaison avec les autres fragments de ladite molécule cible, étant préférentiellement choisi en tant que peptide sujet pour la quantification :
(1) le peptide est obtenu par fragmentation avec une protéase choisie parmi la trypsine, l'endoprotéinase et la pepsine ;
(2) la séquence peptidique est spécifique à la molécule cible ;
(3) quand il s'agit d'un peptide dont la teneur en acides aminés hydrophobes est de 80 % ou moins, et que pas plus de 10 acides aminés hydrophobes sont consécutifs, les acides aminés hydrophobes étant choisis parmi le tryptophane, la tyrosine, la valine, la leucine, l'isoleucine et la phénylalanine, on donne la note 2 ;
(4) quand il s'agit d'un peptide dans lequel le nombre de résidus d'acides aminés est de 4 à 30, on donne la note 3 ;
(5) quand il s'agit d'un peptide qui ne contient pas la séquence asparagine-X-sérine, asparagine-X-thréonine ou asparagine-X-cystéine en tant que conditions spécifiques de séquences d'acides aminés, X représentant un acide aminé autre que la proline, on donne la note 2;
(6) quand, sauf quand une protéine ayant subi une modification post-traductionnelle est en cours de quantification, il s'agit d'un peptide qui ne contient pas de site de modification post-traductionnelle, on donne la note 3 ;
(7) quand il s'agit d'un peptide qui ne contient pas de site d'un polymorphisme d'un seul nucléotide (SNP), on donne la note 4 ;
(8) quand il s'agit d'un peptide dans lequel un site de clivage de la protéase n'est pas l'arginine-arginine, l'arginine-lysine, la lysine-arginine ou la lysine-lysine, on donne la note 5 ;
(9) quand il s'agit d'un peptide qui ne contient pas de domaine transmembranaire lors de la détermination ou de l'estimation de la structure de la protéine, on donne la note 2 ;
(10) quand il s'agit d'un peptide qui ne contient pas de méthionine ou de cystéine, on donne la note 3 ; et
(11) quand il s'agit d'un peptide qui ne contient pas de tryptophane ou d'acide glutamique, on donne la note 1 ;
(c) préparation d'un peptide marqué par un isotope stable, ayant la même séquence que le peptide sujet pour la quantification, et marqué par un isotope stable, par une méthode de synthèse des peptides ;
(d) préparation d'une courbe d'étalonnage par utilisation du peptide sujet pour la quantification et du peptide marqué par un isotope stable, et mise en oeuvre d'une spectrométrie de masse par utilisation d'une LC/MS/MS pour chaque niveau de concentration prédéterminé ;
(e) mise en oeuvre d'une spectrométrie de masse par LC/MS/MS, par addition du peptide marqué par un isotope stable au fragment peptidique obtenu par fragmentation de la protéine de la membrane plasmique à quantifier, et calcul du rapport des aires du spectre de masse, entre le fragment peptidique et le peptide marqué par un isotope stable ; et
(f) calcul d'un niveau quantitatif de la protéine de la membrane plasmique à partir du rapport des aires du spectre de masse, par utilisation d'une courbe d'étalonnage.

2. Procédé pour la quantification d'une protéine de la membrane plasmique selon la revendication 1, dans lequel la teneur en acides aminés hydrophobes, dans le critère sélectif (3), est de 50 % ou moins, et le nombre de résidus d'acides aminés dans le critère sélectif (4) est de 8 à 12.

3. Procédé pour la quantification d'une protéine de la membrane plasmique selon la revendication 1 ou 2, comprenant en outre un critère additionnel (12) :
(12) la même séquence d'acides aminés du peptide est trouvée dans plusieurs espèces animales ;
le peptide étant préférentiellement choisi en tant que peptide sujet pour la quantification s'il satisfait au moins aux critères (1), (2) et (12), et s'il a une note totale élevée, sur la base desdites notes des critères sélectifs (3) à (11).

4. Procédé pour la quantification d'une protéine de la membrane plasmique selon l'une quelconque des revendications 1 à 3, dans lequel plusieurs canaux de mesures spécifiques sont préparés par combinaison d'un ion parent (m/z) et d'un ion d'un fragment peptidique (m/z) de peptides candidats choisis conformément aux critères de sélection du peptide sujet pour la quantification, et mesurés.

5. Procédé pour la quantification d'une protéine de la membrane plasmique selon la revendication 4, dans lequel les plusieurs canaux spécifiques sont préparés pour plusieurs peptides candidats de la même protéine et pour un peptide candidat pour plusieurs protéines, et sont mesurés en même temps.

6. Procédé pour la quantification d'une protéine de la membrane plasmique selon l'une quelconque des revendications 1 à 5, dans lequel le peptide marqué par un isotope stable est marqué par un acide aminé contenant l' un quelconque de ¹⁵N, ¹³C, ¹⁸O ou ²H.

7. Procédé pour la quantification d'une protéine de la membrane plasmique selon l'une quelconque des revendications 1 à 6, dans lequel une source de la protéine de la membrane plasmique est un échantillon de tissu.

8. Procédé pour la quantification d'une protéine de la membrane plasmique selon l'une quelconque des revendications 1 à 7, dans lequel la protéine de la membrane plasmique est constituée d'une ou plusieurs protéines choisies dans le groupe consistant en l'ABCA1 humain, l'ABCA2 humain, l'ABCA3 humain, l'ABCA4 humain, l'ABCA5 humain, l'ABCA6 humain, l'ABCA7 humain, l'ABCA8 humain, l'ABCA9 humain, l'ABCA10 humain, l'ABCA12 humain, l'ABCA13 humain, l'ABCB1 humain, l'ABCB4 humain, l'ABCB5 humain, l'ABCB11 humain, l'ABCC1 humain, l'ABCC2 humain, l'ABCC3 humain, l'ABCC4 humain, l'ABCC5 humain, l'ABCC6 humain, l'ABCC7 humain, l'ABCC8 humain, l'ABCC9 humain, l'ABCC10 humain, l'ABCC11 humain, l'ABCC12 humain, l'ABCC13 humain, l'ABCG1 humain, l'ABCG2 humain, l'ABCG4 humain, l'ABCG5 humain, l'ABCG8 humain et la P-glycoprotéine humaine.

9. Procédé pour la quantification d'une protéine de la membrane plasmique selon l'une quelconque des revendications 1 à 7, dans lequel la protéine de la membrane plasmique est constituée d'une ou plusieurs protéines choisies dans le groupe consistant en le SLC10A1 humain, le SLC10A2 humain, le SLC15A1 humain, le SLC15A2 humain, le SLC16A1 humain, le SLC16A7 humain, le SLC19A1 humain, le SLC19A2 humain, le SLC19A3 humain, le SLC21A1 humain, le SLC21A10 humain, le SLC21A11 humain, le SLC21A12 humain, le SLC21A13 humain, le SLC21A14 humain, le SLC21A15 humain, le SLC21A19 humain, le SLC21A2 humain, le SLC21A20 humain, le SLC21A3 humain, le SLC21A4 humain, le SLC21A5 humain, le SLC21A6 humain, le SLC21A7 humain, le SLC21A8 humain, le SLC21A9 humain, le SLC22A1 humain, le SLC2A10 humain, le SLC22A11 humain, le SLC22A12 humain, le SLC22A13 humain, le SLC22A14 humain, le SLC22A15 humain, le SLC22A16 humain, le SLC22A17 humain, le SLC22A18 humain, le SLC22A2 humain, le SLC22A3 humain, le SLC22A4 humain, le SLC22A5 humain, le SLC22A6 humain, le SLC22A7 humain, le SLC22A8 humain, le SLC22A9 humain, le SLC23A1 humain, le SLC23A2 humain, le SLC28A1 humain, le SLC28A2 humain, le SLC28A3 humain, le SLC29A1 humain, le SLC29A2 humain, le SLC29A3 humain, le SLC29A4 humain, le SLC31A1 humain, le SLC3A2 humain, le SLC43A1 humain, le SLC43A2 humain, le SLC43A3 humain, le SLC7A5 humain, le SLC7A6 humain et le SLC7A8 humain.

10. Procédé pour la quantification d'une protéine de la membrane plasmique selon l'une quelconque des revendications 1 à 7, dans lequel la protéine de la membrane plasmique est constituée d'une ou plusieurs protéines choisies dans le groupe consistant en le SLC10A3 humain, le SLC10A4 humain, le SLC10A5 humain, le SLC10A6 humain, le SLC11A1 humain, le SLC11A2 humain, le SLC12A1 humain, le SLC12A2 humain, le SLC12A3 humain, le SLC12A4 humain, le SLC12A5 humain, le SLC12A6 humain, le SLC12A7 humain, le SLC12A8 humain, le SLC12A9 humain, le SLC13A1 humain, le SLC13A2 humain, le SLC13A3 humain, le SLC13A4 humain, le SLC13A5 humain, le SLC14A1 humain, le SLC14A2 humain, le SLC15A3 humain, le SLC15A4 humain, le SLC16A10 humain, le SLC16A11 humain, le SLC16A12 humain, le SLC16A13 humain, le SLC16A14 humain, le SLC16A2 humain, le SLC16A3 humain, le SLC16A4 humain, le SLC16A5 humain, le SLC16A6 humain, le SLC16A8 humain, le SLC16A9 humain, le SLC17A1 humain, le SLC17A2 humain, le SLC17A3 humain, le SLC17A4 humain, le SLC17A5 humain, le SLC17A6 humain, le SLC17A7 humain, le SLC17A8 humain, le SLC18A1 humain, le SLC18A2 humain, le SLC18A3 humain, le SLC1A1 humain, le SLC1A2 humain, le SLC1A3 humain, le SLC1A4 humain, le SLC1A5 humain, le SLC1A6 humain, le SLC1A7 humain, le SLC20A1 humain, le SLC20A2 humain, le SLC23A3 humain, le SLC24A1 humain, le SLC24A2 humain, le SLC24A3 humain, le SLC24A4 humain, le SLC24A5 humain, le SLC24A6 humain, le SLC25A1 humain, le SLC25A10 humain, le SLC25A11 humain, le SLC25A12 humain, le SLC25A13 humain, le SLC25A14 humain, le SLC25A15 humain, le SLC25A16 humain, le SLC25A17 humain, le SLC25A18 humain, le SLC25A19 humain, le SLC25A2 humain, le SLC25A20 humain, le SLC25A21 humain, le SLC25A22 humain, le SLC25A23 humain, le SLC25A24 humain, le SLC25A25 humain, le SLC25A26 humain, le SLC25A27 humain, le SLC25A28 humain, le SLC25A29 humain, le SLC25A3 humain, le SLC25A30 humain, le SLC25A31 humain, le SLC25A32 humain, le SLC25A33 humain, le SLC25A34 humain, le SLC25A35 humain, le SLC25A36 humain, le SLC25A37 humain, le SLC25A4 humain, le SLC25A5 humain, le SLC25A6 humain, le SLC25A7 humain, le SLC25A8 humain, le SLC25A9 humain, le SLC26A1 humain, le SLC26A10 humain, le SLC26A11 humain, le SLC26A2 humain, le SLC26A3 humain, le SLC26A4 humain, le SLC26A5 humain, le SLC26A6 humain, le SLC26A7 humain, le SLC26A8 humain, le SLC26A9 humain, le SLC27A1 humain, le SLC27A2 humain, le SLC27A3 humain, le SLC27A4 humain, le SLC27A5 humain, le SLC27A6 humain, le SLC2A1 humain, le SLC2A10 humain, le SLC2A11 humain, le SLC2A12 humain, le SLC2A13 humain, le SLC2A14 humain, le SLC2A2 humain, le SLC2A3 humain, le SLC2A4 humain, le SLC2A5 humain, le SLC2A6 humain, le SLC2A7 humain, le SLC2A8 humain, le SLC2A9 humain, le SLC30A1 humain, le SLC30A10 humain, le SLC30A11 humain, le SLC30A2 humain, le SLC30A3 humain, le SLC30A4 humain, le SLC30A5 humain, le SLC30A6 humain, le SLC30A7 humain, le SLC30A8 humain, le SLC30A9 humain, le SLC31A2 humain, le SLC32A1 humain, le SLC33A1 humain, le SLC34A1 humain, le SLC34A2 humain, le SLC34A3 humain, le SLC35B1 humain, le SLC35B2 humain, le SLC35B3 humain, le SLC35B4 humain, le SLC35C1 humain, le SLC35C2 humain, le SLC35D1 humain, le SLC35D2 humain, le SLC35D3 humain, le SLC36A1 humain, le SLC36A2 humain, le SLC36A3 humain, le SLC36A4 humain, le SLC37A1 humain, le SLC37A2 humain, le SLC37A3 humain, le SLC37A4 humain, le SLC38A1 humain, le SLC38A2 humain, le SLC38A3 humain, le SLC38A4 humain, le SLC38A5 humain, le SLC38A6 humain, le SLC3A1 humain, le SLC40A1 humain, le SLC41A1 humain, le SLC41A2 humain, le SLC41A3 humain, le SLC44A1 humain, le SLC44A2 humain, le SLC44A3 humain, le SLC44A4 humain, le SLC44A5 humain, le SLC45A1 humain, le SLC45A2 humain, le SLC45A3 humain, le SLC45A4 humain, le SLC4A1 humain, le SLC4A10 humain, le SLC4A11 humain, le SLC4A2 humain, le SLC4A3 humain, le SLC4A4 humain, le SLC4A5 humain, le SLC4A7 humain, le SLC4A8 humain, le SLC4A9 humain, le SLC5A1 humain, le SLC5A11 humain, le SLC5A12 humain, le SLC5A2 humain, le SLC5A3 humain, le SLC5A4 humain, le SLC5A5 humain, le SLC5A9 humain, le SLC6A1 humain, le SLC6A10 humain, le SLC6A11 humain, le SLC6A12 humain, le SLC6A13 humain, le SLC6A14 humain, le SLC6A15 humain, le SLC6A16 humain, le SLC6A17 humain, le SLC6A18 humain, le SLC6A19 humain, le SLC6A2 humain, le SLC6A20 humain, le SLC6A3 humain, le SLC6A4 humain, le SLC6A5 humain, le SLC6A6 humain, le SLC6A7 humain, le SLC6A8 humain, le SLC6A9 humain, le SLC7A1 humain, le SLC7A10 humain, le SLC7A11 humain, le SLC7A13 humain, le SLC7A14 humain, le SLC7A2 humain, le SLC7A3 humain, le SLC7A4 humain, le SLC7A7 humain, le SLC7A9 humain, le SLC8A1 humain, le SLC8A2 humain, le SLC8A3 humain, le SLC9A1 humain, le SLC9A2 humain, le SLC9A3 humain, le SLC9A4 humain, le SLC9A5 humain, le SLC9A6 humain, le SLC9A7 humain, le SLC9A8 humain et le SLC9A9 humain.

11. Procédé pour la quantification d'une protéine de la membrane plasmique selon l'une quelconque des revendications 1 à 7, dans lequel la protéine de la membrane plasmique est constituée d'une ou plusieurs protéines choisies dans le groupe consistant en le SLC35A1 humain, le SLC35A2 humain, le SLC35A3 humain, le SLC35A4 humain, le SLC35A5 humain, le SLC35E1 humain, le SLC35E2 humain, le SLC35E3 humain, le SLC35E4 humain, le SLC35F1 humain, le SLC35F2 humain, le SLC35F3 humain, le SLC35F5 humain, le SLC39A1 humain, le SLC39A10 humain, le SLC39A11 humain, le SLC39A12 humain, le SLC39A13 humain, le SLC39A14 humain, le SLC39A2 humain, le SLC39A3 humain, le SLC39A4 humain, le SLC39A5 humain, le SLC39A6 humain, le SLC39A7 humain, le SLC39A8 humain, le SLC39A9 humain, le SLC42A1 humain, le SLC42A2 humain et le SLC42A3 humain.

12. Procédé pour la quantification d'une protéine de la membrane plasmique selon l'une quelconque des revendications 1 à 7, dans lequel la protéine de la membrane plasmique est le MATE1 humain ou le MATE2 humain.

13. Procédé pour la quantification d'une protéine de la membrane plasmique selon l'une quelconque des revendications 1 à 7, dans lequel la protéine de la membrane plasmique est constituée d'une ou plusieurs protéines choisies dans le groupe consistant en l'ABCA1 humain, l'ABCA2 humain, l'ABCA3 humain, l'ABCA4 humain, l'ABCA5 humain, l'ABCA6 humain, l'ABCA7 humain, l'ABCA8 humain, l'ABCA9 humain, l'ABCA10 humain, l'ABCA12 humain, l'ABCA13 humain, l'ABCB1 humain, l'ABCB4 humain, l'ABCB5 humain, l'ABCB11 humain, l'ABCC1 humain, l'ABCC2 humain, l'ABCC3 humain, l'ABCC4 humain, l'ABCC5 humain, l'ABCC6 humain, l'ABCC10 humain, l'ABCC11 humain, l'ABCC12 humain, l'ABCC13 humain, l'ABCG1 humain, l'ABCG2 humain, l'ABCG4 humain, l'ABCG5 humain, l'ABCG8 humain, le MATE1 humain, le MATE2 humain, le SLC3A2 humain, le SLC7A5 humain, le SLC7A6 humain, le SLC10A1 humain, le SLC10A2 humain, le SLC15A1 humain, le SLC15A2 humain, le SLC16A1 humain, le SLC16A7 humain, le SLC19A1 humain, le SLC21A2 humain, le SLC21A3 humain, le SLC21A4 humain, le SLC21A5 humain, le SLC21A6 humain, le SLC21A7 humain, le SLC21A8 humain, le SLC21A9 humain, le SLC21A11 humain, le SLC21A12 humain, le SLC21A13 humain, le SLC21A14 humain, le SLC21A15 humain, le SLC21A19 humain, le SLC21A20 humain, le SLC22A1 humain, le SLC22A2 humain, le SLC22A3 humain, le SLC22A4 humain, le SLC22A5 humain, le SLC22A6 humain, le SLC22A7 humain, le SLC22A8 humain, le SLC22A9 humain, le SLC22A10 humain, le SLC22A11 humain, le SLC22A12 humain, le SLC22A13 humain, le SLC22A14 humain, le SLC22A15 humain, le SLC22A16 humain, le SLC22A17 humain, le SLC23A1 humain, le SLC23A2 humain, le SLC28A1 humain, le SLC28A2 humain, le SLC28A3 humain, le SLC29A1 humain, le SLC29A2 humain et le SLC31A1 humain.
